# EUROPEAN PATENT APPLICATION

(11) **EP 4 640 684 A1**
(43) Date of publication of application: **29.10.2025**
(21) Application number: 23905989.2
(22) Date of filing: 20.12.2023
(51) Int. Cl.: C07D 471/00, A61K 31/435, A61P 35/00

(54) **KRAS G12D INHIBITORS AND USES THEREOF**

(30) Priority: 23.12.2022 CN 202211663805; 18.05.2023 CN 202310564827
(71) Applicant: Abbisko Therapeutics Co., Ltd., Shanghai 201203 (CN)
(72) Inventor: YANG, Fei, Shanghai 201203 (CN); DENG, Haibing, Shanghai 201203 (CN); MA, Zhixiong, Shanghai 201203 (CN); XUN, Guoliang, Shanghai 201203 (CN); YU, Hongping, Shanghai 201203 (CN); CHEN, Zhui, Shanghai 201203 (CN); XU, Yaochang, Shanghai 201203 (CN)
(74) Representative: Potter Clarkson
(86) International application number: PCT/CN2023/140181
(87) International publication number: WO 2024/131827

(57) **Abstract**

The present invention relates to KRAS G12D inhibitors and the uses thereof. In particular, the present invention relates to KRAS G12D inhibitors having structures shown as formula (I), a preparation method therefor, a pharmaceutical composition containing same, the use thereof as KRAS G12D inhibitors, and the use thereof in the treatment and/or prevention of cancers or tumors related to KRAS G12D, each substituent of formula (I) being as defined in the description.

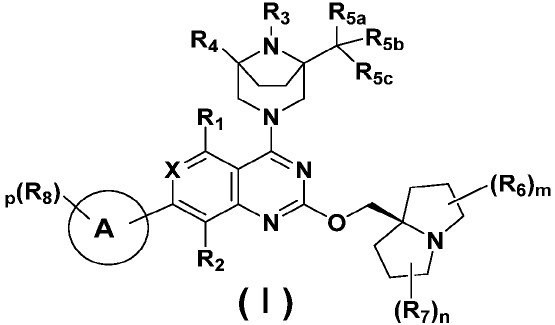

## Description

### TECHNICAL FIELD

The present invention belongs to the field of pharmaceutical synthesis, and particularly relates to KRAS inhibitors, preparation method therefor, and pharmaceutical use thereof.

### BACKGROUND

The RAS gene family includes HRAS, KRAS and NRAS, which act as oncogenes and are frequently mutated in cancer. Mutated RAS proteins are found in 20-30% of human tumors. Activated RAS proteins contribute to the malignant phenotype of cancer cells, including dysregulation of cell growth and programmed cell death, increased invasiveness and neovascularization. The development of drugs targeting RAS proteins has been slow due to their high affinity for GTP/GDP and lack of a clear binding pocket.

Under normal conditions, the RAS protein acts as a molecular switch, alternating between a GDP-bound inactive state and a GTP-bound active state. After stimulation by exogenous growth factors, promoted by guanine nucleotide exchange factors (GEFs), the RAS protein changes from an inactive GDP-bound form, to an activated GTP-bound form, which can bind and activate downstream signaling pathways. Subsequently, the RAS protein reverts to the inactive GDP-bound form, with the help of its intrinsic GTPase activity and GTPase activating/accelerating protein (GAP).

Missense mutations in codons 12, 13, or 61 lead to abnormal activation of RAS. These mutations prolong the time the RAS protein remains bound to GTP, resulting in sustained activation of downstream signaling pathways. K-RAS is the most common mutation subtype of the RAS family in human cancers, including pancreatic cancer (71%), small intestine cancer (35%), colon cancer (35%), biliary tract cancer (26%), endometrial cancer (17%) and lung cancer (19%). In terms of mutation sites, G12D/G12V/G12C/G13D are the most common mutation types of K-RAS in pancreatic cancer, lung cancer, and colon cancer.

The development of KRAS inhibitors is challenging because the protein lacks a clear pocket. Recent research has uncovered a previously undiscovered pocket in the GDP-bound form of KRAS. Based on these new discoveries, covalently bound inhibitors targeting the mutated cysteine at codon 12 have become a hot spot in the research and development of KRAS inhibitors, which have made certain progress. However, in addition to the G12C mutation, other activating mutations targeting KRAS still need to be solved, especially the KRAS G12D mutation. Therefore, it is necessary to develop safe and effective KRAS G12D inhibitors to treat KRAS-G12D-mediated cancers.

### SUMMARY

The objective of the present invention is to provide KRAS G12D inhibitors and the uses thereof. The series of compounds of the present invention have a strong inhibitory effect on KRAS G12D and can be widely used in the preparation of a medicament for the treatment and/or prevention of cancers or tumors related to KRAS G12D, thereby promising the development of a new generation of KRAS G12D inhibitors.

The first aspect of the present invention provides a compound of formula (I), a stereoisomer or pharmaceutically acceptable salt thereof: 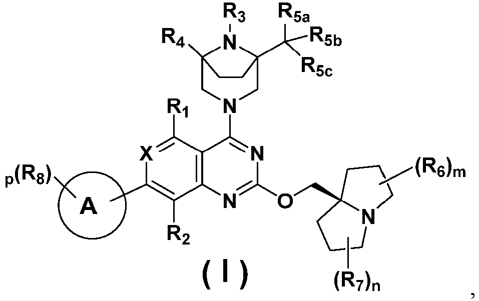
wherein, X is CR₉ or N;
ring A is C₆₋₁₀ aryl or 5-10 membered heteroaryl, preferably, the C₆₋₁₀ aryl or 5-10 membered heteroaryl is selected from the group consisting of phenyl, naphthyl, pyridyl, pyrimidinyl, pyridazinyl, quinolinyl, isoquinolinyl, indolyl, benzothiophenyl, benzimidazolyl, benzopyrazolyl, benzoxazolyl and benzothiazolyl;
R₁ is selected from the group consisting of hydrogen, deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂-₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R15, -C₀₋₈ alkyl-N(R₁₆)-C(=NR₁₇)Ris15, -C₀₋₈ alkyl-C(O)NR₁₆R₁₇ and -C₀₋₈ alkyl-N(R₁₆)-C(O)R₁₅;
R₂ is selected from the group consisting of hydrogen, deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₃ is selected from the group consisting of hydrogen, C₁₋₁₀ alkyl, C₂₋₁₀ alkenyl, C₃₋₁₂ cycloalkyl, 3-12 membered heterocyclyl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-C(O)R₁₅, -C₀₋₈ alkyl-O-C(O)R₁₅, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅, and the above groups are each independently optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R10)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₄ is selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, and the above groups are each independently optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₅ₐ is selected from the group consisting of hydrogen, deuterium, halogen, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R10)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-NH-S(O)₂R₁₃, -C₀₋₈ alkyl-NH-S(O)₂NR₁₆R₁₇, -C₀₋₈ alkyl-S(O)ᵣR₁₃, -C₀₋₈ alkyl-S(O)ᵣNR₁₆R₁₇, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, and the above groups are each independently optionally further substituted by one or more substituents selected from the group consisting of deuterium, halogen, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R_{5b} and R_{5c} are each independently selected from the group consisting of hydrogen, deuterium, halogen, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-NH-S(O)₂R₁₃, -C₀₋₈ alkyl-NH-S(O)₂NR₁₆R₁₇, -C₀₋₈ alkyl-S(O)ᵣR₁₃, -C₀₋₈ alkyl-S(O)ᵣNR₁₆R₁₇, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, and the above groups are each independently optionally further substituted by one or more substituents selected from the group consisting of deuterium, halogen, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, provided that R₅ₐ, R_{5b} and R_{5c} are not hydrogen at the same time;
or, R_{5b} and R_{5c}, together with the carbon atom to which they are directly attached, form a C(O), C₃₋₁₀ cycloalkyl or 3-10 membered heterocyclyl, and the above groups are each independently optionally further substituted by one or more substituents selected from the group consisting of deuterium, halogen, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, wherein R₅ₐ is defined as above;
or, R_{5b} and R_{5c}, together with the carbon atom to which they are directly attached, form 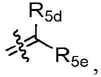 wherein R₅ₐ is defined as above;
or, R₅ₐ, R_{5b} and R_{5c}, together with the carbon atom to which they are directly attached, form 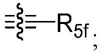
R_{5d}, R₅ₑ and R_{5f} are each independently selected from the group consisting of halogen, cyano, C₁₋₄ alkyl, C₃₋₆ cycloalkyl, 3-6 membered heterocyclyl and -C₀₋₈ alkyl-NR₁₆R₁₇, and the above groups are each independently optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl and 5-10 membered heteroaryl;
each R₆ and each R₇ are independently selected from the group consisting of hydrogen, deuterium, halogen, cyano, nitro, azido, C₁₋₁₀ alkyl, C₁₋₁₀ haloalkyl, C₁₋₁₀ deuterioalkyl, C₁₋₁₀ alkylene, C₁₋₁₀ haloalkylene, C₁₋₁₀ deuterioalkylene, C₂₋₁₀ alkenyl, C₂₋₁₀ alkynyl, C₃₋₁₂ cycloalkyl, C₃₋₁₂ cycloalkylene, C₃₋₁₂ halocycloalkylene, C₃₋₁₂ deuteriocycloalkylene, 3-12 membered heterocyclyl, C₃₋₁₂ heterocycloalkylene, C₃₋₁₂ haloheterocycloalkylene, C₃₋₁₂ deuterioheterocycloalkylene, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, provided that at least one of R₆ or R₇ is C₁₋₁₀ alkylene, C₁₋₁₀ haloalkylene, C₁₋₁₀ deuterioalkylene, C₁₋₁₀ cycloalkylene, C₁₋₁₀ halocycloalkylene, C₁₋₁₀ deuteriocycloalkylene, C₁₋₁₀ heterocycloalkylene, C₁₋₁₀ haloheterocycloalkylene or C₁₋₁₀ deuterioheterocycloalkylene;
each R₈ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, or, when p≥2, two adjacent R₈, together with the moiety to which they are directly attached, form a C₅₋₁₂ cycloalkyl or 5-12 membered heterocyclyl, and the above groups are each independently optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₉ is selected from the group consisting of hydrogen, deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
or, R₉ and R₁, together with the moiety to which they are directly attached, form a C₃₋₁₀ cycloalkyl or 3-10 membered heterocyclyl, and the C₃₋₁₀ cycloalkyl or 3-10 membered heterocyclyl is independently optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
each R₁₀ is independently selected from the group consisting of hydrogen, deuterium, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-C(O)OR₁₄, -C₀₋₈ alkyl-C(O)R₁₅ and -C₀₋₈ alkyl-C(O)NR₁₆R₁₇, and the above groups are each independently optionally further substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)R₁₅, -C₀₋₈ alkyl-O-C(O)R₁₅, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
each R₁₁ and each R₁₂ are independently selected from the group consisting of hydrogen, deuterium, hydroxy, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl and 5-10 membered heteroaryl, or, R₁₁ and R₁₂, together with the sulfur atom to which they are directly attached, form a 3-10 membered heterocyclyl, and the above groups are each independently optionally further substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)R₁₅, -C₀₋₈ alkyl-O-C(O)R₁₅, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
each R₁₃ is independently selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl and -NR₁₆R₁₇, and the above groups are each independently optionally further substituted by one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₄ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl and 5-10 membered heteroaryl, and the above groups are each independently optionally further substituted by one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₅ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇, and the above groups are each independently optionally further substituted by one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₆ and each R₁₇ are independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and C₁₋₁₀ alkanoyl, and the above groups are each independently optionally further substituted by one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, monoC₁₋₁₀ alkylamino, diC₁₋₁₀ alkylamino and C₁₋₁₀ alkanoyl;
or, R₁₆ and R₁₇, together with the nitrogen atom to which they are directly attached, form a 4-10 membered heterocyclyl or 5-10 membered heteroaryl, and the 4-10 membered heterocyclyl or 5-10 membered heteroaryl is optionally further substituted by one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, monoC₁₋₁₀ alkylamino, diC₁₋₁₀ alkylamino and C₁₋₁₀ alkanoyl;
m is 0, 1 or 2; n is 0, 1 or 2;
p is 0, 1, 2, 3, 4, 5 or 6; and
each r is independently 0, 1 or 2.

As a preferred embodiment, in the compound of formula (I), the stereoisomer or pharmaceutically acceptable salt thereof, X is CR₉ or N;
R₁ is selected from the group consisting of hydrogen, deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₂ is selected from the group consisting of hydrogen, deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₃ is selected from the group consisting of hydrogen, C₁₋₄ alkyl, C₂₋₄ alkenyl, C₃₋₆ cycloalkyl, 3-6 membered heterocyclyl, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-C(O)R₁₅, -C₀₋₄ alkyl-O-C(O)R₁₅, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₄ is selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₅ₐ is selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-NH-S(O)₂R₁₃, -C₀₋₄ alkyl-NH-S(O)₂NR₁₆R₁₇, -C₀₋₄ alkyl-S(O)ᵣR₁₃, -C₀₋₄ alkyl-S(O)ᵣNR₁₆R₁₇, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R_{5b} and R_{5c} are each independently selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-NH-S(O)₂R₁₃, -C₀₋₄ alkyl-NH-S(O)₂NR₁₆R₁₇, -C₀₋₄ alkyl-S(O)ᵣR₁₃, -C₀₋₄ alkyl-S(O)ᵣNR₁₆R₁₇, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, provided that R₅ₐ, R_{5b} and R_{5c} are not hydrogen at the same time;
or, R_{5b} and R_{5c}, together with the carbon atom to which they are directly attached, form a C(O), C₃₋₆ cycloalkyl or 3-6 membered heterocyclyl, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, wherein R₅ₐ is defined as above;
or, R_{5b} and R_{5c}, together with the carbon atom to which they are directly attached, form 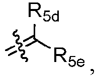 wherein R₅ₐ is defined as above;
or, R₅ₐ, R_{5b} and R_{5c}, together with the carbon atom to which they are directly attached, form 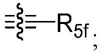
R_{5d}, R₅ₑ and R_{5f} are each independently selected from the group consisting of halogen, cyano, C₁₋₄ alkyl, C₃₋₆ cycloalkyl, 3-6 membered heterocyclyl and -C₀₋₄ alkyl-NR₁₆R₁₇, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl and 5-8 membered heteroaryl;
each R₆ and each R₇ are independently selected from the group consisting of hydrogen, deuterium, halogen, cyano, nitro, azido, C₁₋₄ alkyl, C₁₋₄ haloalkyl, C₁₋₄ deuterioalkyl, C₁₋₄ alkylene, C₁₋₄ haloalkylene, C₁₋₄ deuterioalkylene, C₂₋₄ alkenyl, C₂₋₄ alkynyl, C₃₋₆ cycloalkyl, C₃₋₆ cycloalkylene, C₃₋₆ halocycloalkylene, C₃₋₆ deuteriocycloalkylene, 3-6 membered heterocyclyl, C₃₋₆ heterocycloalkylene, C₃₋₆ haloheterocycloalkylene, C₃₋₆ deuterioheterocycloalkylene, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, provided that at least one of R₆ or R₇ is C₁₋₄ alkylene, C₁₋₄ haloalkylene, C₁₋₄ deuterioalkylene, C₃₋₆ cycloalkylene, C₃₋₆ halocycloalkylene, C₃₋₆ deuteriocycloalkylene, C₃₋₆ heterocycloalkylene, C₃₋₆ haloheterocycloalkylene or C₃₋₆ deuterioheterocycloalkylene;
each R₈ is independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, or, when p≥2, two adjacent R₈, together with the moiety to which they are directly attached, form a C₅₋₁₀ cycloalkyl or 5-10 membered heterocyclyl, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₉ is selected from the group consisting of hydrogen, deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
wherein, R₁₀, R₁₁, R₁₂, R₁₃, R₁₄, R₁₅, R₁₆, R₁₇ and r are defined as in the compound of formula (I).

As a preferred embodiment, in the compound of formula (I), the stereoisomer or pharmaceutically acceptable salt thereof, each R₁₀ is independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-C(O)OR₁₄, -C₀₋₄ alkyl-C(O)R₁₅ and -C₀₋₄ alkyl-C(O)NR₁₆R₁₇, and the above groups are each independently optionally further substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
each R₁₁ and each R₁₂ are independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, or, R₁₁ and R₁₂, together with the sulfur atom to which they are directly attached, form a 3-6 membered heterocyclyl, and the above groups are each independently optionally further substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
each R₁₃ is independently selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, 5-8 membered heteroaryl and -NR₁₆R₁₇, and the above groups are each independently optionally further substituted by one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₄ is independently selected from the group consisting of hydrogen, deuterium, C₁₋₄ alkyl, C₂₋₄ alkenyl, C₃₋₆ cycloalkyl, 3-6 membered heterocyclyl, C₆₋₈ aryl and 5-8 membered heteroaryl, and the above groups are each independently optionally further substituted by one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₅ is independently selected from the group consisting of hydrogen, deuterium, hydroxy, C₁₋₄ alkyl, C₁₋₄ alkoxy, C₂₋₄ alkenyl, C₂₋₄ alkynyl, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇, and the above groups are each independently optionally further substituted by one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇,
each R₁₆ and each R₁₇ are independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and C₁₋₄ alkanoyl, and the above groups are independently optionally further substituted by one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, monoC₁₋₄ alkylamino, diC₁₋₄ alkylamino and C₁₋₄ alkanoyl;
or, R₁₆ and R₁₇, together with the nitrogen atom to which they are directly attached, form a 4-8 membered heterocyclyl or 5-8 membered heteroaryl, and the 4-8 membered heterocyclyl or 5-8 membered heteroaryl is optionally further substituted by one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, monoC₁₋₄ alkylamino, diC₁₋₄ alkylamino and C₁₋₄ alkanoyl,
each r is independently 0, 1 or 2.

As a preferred embodiment, in the compound of formula (I), the stereoisomer or pharmaceutically acceptable salt thereof, R₂ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R₃ is selected from the group consisting of hydrogen, C₁₋₄ alkyl, C₂₋₄ alkenyl, C₃₋₆ cycloalkyl, 3-6 membered heterocyclyl, -S(O)ᵣR₁₃, -O-R₁₄, -C(O)OR₁₄, -C(O)SR₁₄, -C(O)R₁₅, -O-C(O)R₁₅, -NR₁₆R₁₇, -C(O)NR₁₆R₁₇ and -N(R₁₆)-C(O)R₁₅, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R₄ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇ and r are defined as in the compound of formula (I).

As a further preferred embodiment, in the compound of formula (I), the stereoisomer or pharmaceutically acceptable salt thereof, R₂ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
R₃ is selected from the group consisting of hydrogen, C₁₋₄ alkyl, C₂₋₄ alkenyl, C₃₋₆ cycloalkyl, 3-6 membered heterocyclyl, -S(O)ᵣR₁₃, -O-R₁₄, -C(O)OR₁₄, -C(O)SR₁₄, -C(O)R₁₅, -O-C(O)R₁₅, -NR₁₆R₁₇, -C(O)NR₁₆R₁₇ and -N(R₁₆)-C(O)R₁₅, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
R₄ is selected from the group consisting of hydrogen, deuterium, halogen, cyano, C₁₋₄ alkyl, C₂₋₄ alkenyl, C₂₋₄ alkynyl, C₃₋₆ cycloalkyl and 3-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O and =S;
wherein, R₁₀, R₁₁, R₁₂, R₁₃, R₁₄, R₁₅, R₁₆, R₁₇ and r are defined as in the compound of formula (I).

As a preferred embodiment, in the compound of formula (I), the stereoisomer or pharmaceutically acceptable salt thereof, the compound of formula (I) is a compound of formula (IIa) or a compound of formula (IIb) as shown below: 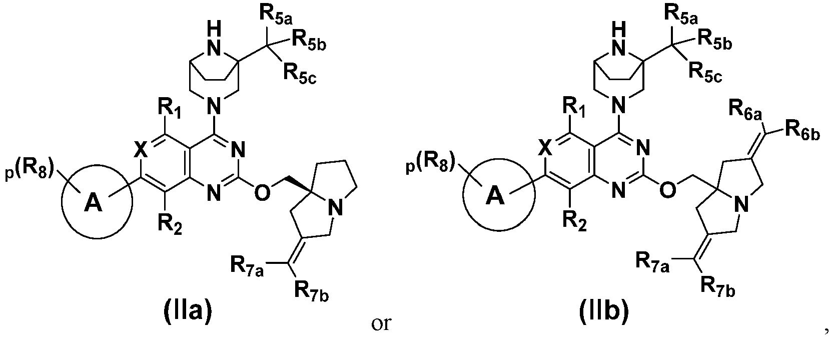
wherein, each X is independently CR₉ or N;
each ring A is independently phenyl, naphthyl, pyridyl, pyrimidinyl, pyridazinyl, quinolinyl, isoquinolinyl, benzothiophenyl or benzothiazolyl;
each R₁ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
each R₂ is independently selected from the group consisting of hydrogen, deuterium, fluorine, chlorine, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
each R₅ₐ is independently selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
each R_{5b} and each R_{5c} are independently selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, provided that R₅ₐ, R_{5b} and R_{5c} are not hydrogen at the same time;
or, R_{5b} and R_{5c}, together with the carbon atom to which they are directly attached, form a C(O), C₃₋₆ cycloalkyl or 3-6 membered heterocyclyl, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, wherein R₅ₐ is defined as above;
or, R_{5b} and R_{5c}, together with the carbon atom to which they are directly attached, form 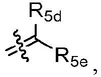 wherein R₅ₐ is defined as above;
or, R₅ₐ, R_{5b} and R_{5c}, together with the carbon atom to which they are directly attached, form 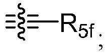
each R_{5d} and each R₅ₑ are independently selected from the group consisting of hydrogen, halogen, cyano, C₁₋₄ alkyl, C₃₋₆ cycloalkyl, 3-6 membered heterocyclyl and -C₀₋₄ alkyl-NR₁₆R₁₇, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl and 5-8 membered heteroaryl, provided that R_{5d} and R₅ₑ are not hydrogen at the same time;
each R_{5f} is independently selected from the group consisting of halogen, cyano, C₁₋₄ alkyl, C₃₋₆ cycloalkyl, 3-6 membered heterocyclyl and -C₀₋₄ alkyl-NR₁₆R₁₇, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl and 5-8 membered heteroaryl;
in the compound of formula (IIb), R₆ₐ and R_{6b} are each independently selected from the group consisting of hydrogen, deuterium, halogen, C₁₋₃ alkyl, C₁₋₃ haloalkyl and C₁₋₃ deuterioalkyl, or, R₆ₐ and R_{6b}, together with the carbon atom to which they are directly attached, form a C₃₋₆ cycloalkyl or 3-6 membered heterocyclyl, and the C₃₋₆ cycloalkyl or 3-6 membered heterocyclyl is independently optionally further substituted by one or more substituents selected from the group consisting of deuterium or halogen;
each R₇ₐ and each R_{7b} are independently selected from the group consisting of hydrogen, deuterium, halogen, C₁₋₃ alkyl, C₁₋₃ haloalkyl and C₁₋₃ deuterioalkyl;
or, R₇ₐ and R_{7b}, together with the carbon atom to which they are directly attached, form a C₃₋₆ cycloalkyl or 3-6 membered heterocyclyl, and the C₃₋₆ cycloalkyl or 3-6 membered heterocyclyl is independently optionally further substituted by one or more substituents which are deuterium or halogen;
each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, or, when p≥2, two adjacent R₈, together with the moiety to which they are directly attached, form a C₅₋₆ cycloalkyl or 5-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
each R₉ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇, p and r are defined as in the compound of formula (I).

As a further preferred embodiment, in the compound of formula (I), the stereoisomer or pharmaceutically acceptable salt thereof, R₇ₐ and R_{7b} are each independently selected from the group consisting of hydrogen, deuterium, fluorine, chlorine, bromine, methyl, ethyl, propyl, isopropyl, monofluoromethyl, difluoromethyl, trifluoromethyl, monodeuteriomethyl, dideuteriomethyl and trideuteriomethyl;
or, R₇ₐ and R_{7b}, together with the carbon atom to which they are directly attached, form a C₃₋₆ cycloalkyl or 3-6 membered heterocyclyl, and the C₃₋₆ cycloalkyl or 3-6 membered heterocyclyl is independently optionally further substituted by one or more substituents selected from deuterium, fluorine, chlorine and bromine.

As a further preferred embodiment, in the compound of formula (I), the stereoisomer or pharmaceutically acceptable salt thereof, in the compound of formula (IIb), R₆ₐ and R_{6b} are each independently selected from the group consisting of hydrogen, deuterium, fluorine, chlorine, bromine, methyl, ethyl, propyl, isopropyl, monofluoromethyl, difluoromethyl, trifluoromethyl, monodeuteriomethyl, dideuteriomethyl and trideuteriomethyl, or, R₆ₐ and R_{6b}, together with the carbon atom to which they are directly attached, form a C₃₋₆ cycloalkyl or 3-6 membered heterocyclyl, and the C₃₋₆ cycloalkyl or 3-6 membered heterocyclyl is independently optionally further substituted by one or more substituents selected from the group consisting of deuterium, fluorine, chlorine and bromine.

As a further preferred embodiment, in the compound of formula (I), the stereoisomer or pharmaceutically acceptable salt thereof, X is CR₉ or N;
R₁ is selected from the group consisting of hydrogen, deuterium, halogen, cyano, C₁₋₄ alkyl, C₁₋₄ haloalkyl, C₁₋₄ deuterioalkyl, C₃₋₆ cycloalkyl, 3-6 membered heterocyclyl, -O-R₁₄ and -NR₁₆R₁₇;
R₉ is selected from the group consisting of hydrogen, deuterium, halogen, cyano, C₁₋₄ alkyl, C₁₋₄ haloalkyl, C₁₋₄ deuterioalkyl, C₃₋₆ cycloalkyl, 3-6 membered heterocyclyl, -O-R₁₄ and -NR₁₆R₁₇;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, C₃₋₆ cycloalkyl, 3-6 membered heterocyclyl, =O, =S, -O-R₁₄ and -NR₁₆R₁₇;
wherein, R₁₄, R₁₆ and R₁₇ are defined as in the compound of formula (I).

As a more further preferred embodiment, in the compound of formula (I), the stereoisomer or pharmaceutically acceptable salt thereof, X is CR₉ or N;
R₁ is selected from the group consisting of hydrogen, deuterium, fluorine, chlorine, bromine, cyano, methyl, ethyl, propyl, isopropyl, butyl, isobutyl, monofluoromethyl, difluoromethyl, trifluoromethyl, monodeuteriomethyl, dideuteriomethyl, trideuteriomethyl, cyclopropyl, cyclobutyl, cyclopentyl, cyclohexyl, oxacyclopropyl, azacyclopropyl, oxacyclobutyl, azacyclobutyl, methoxy, ethoxy, propoxy, trideuteriomethoxy, isopropoxy, butoxy, cyclopropyloxy, cyclobutyloxy, monomethylamino, dimethylamino and amino;
R₉ is selected from the group consisting of hydrogen, deuterium, fluorine, chlorine, bromine, cyano, methyl, ethyl, propyl, isopropyl, butyl, isobutyl, monofluoromethyl, difluoromethyl, trifluoromethyl, monodeuteriomethyl, dideuteriomethyl, trideuteriomethyl, cyclopropyl, cyclobutyl, cyclopentyl, cyclohexyl, oxacyclopropyl, azacyclopropyl, oxacyclobutyl, azacyclobutyl, methoxy, ethoxy, propoxy, isopropoxy, butoxy, cyclopropyloxy, cyclobutyloxy, monomethylamino, dimethylamino and amino;
or, R₉ and R₁, together with the moiety to which they are directly attached, form an oxacyclohexyl, and the oxacyclohexyl is optionally further substituted by one or more substituents selected from the group consisting of deuterium, fluorine, chlorine, bromine, cyano, methyl, ethyl, propyl, isopropyl, butyl, isobutyl, monofluoromethyl, difluoromethyl, trifluoromethyl, monodeuteriomethyl, dideuteriomethyl, trideuteriomethyl, cyclopropyl, cyclobutyl, cyclopentyl, cyclohexyl, oxacyclopropyl, azacyclopropyl, oxacyclobutyl, azacyclobutyl, =O, =S, methoxy, ethoxy, propoxy, isopropoxy, butoxy, cyclopropyloxy, cyclobutyloxy, monomethylamino, dimethylamino and amino.

As a further preferred embodiment, in the compound of formula (I), the stereoisomer or pharmaceutically acceptable salt thereof, each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇ and r are defined as in the compound of formula (I).

As a more further preferred embodiment, in the compound of formula (I), the stereoisomer or pharmaceutically acceptable salt thereof, each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -O-R₁₄ and -NR₁₆R₁₇,
wherein, R₁₄, R₁₆ and R₁₇ are defined as in the compound of formula (I).

As a still more further preferred embodiment, in the compound of formula (I), the stereoisomer or pharmaceutically acceptable salt thereof, each R₈ is independently selected from the group consisting of hydrogen, deuterium, fluorine, chlorine, bromine, cyano, hydroxy, methyl, ethyl, propyl, isopropyl, butyl, isobutyl, vinyl, ethynyl, cyclopropyl, cyclobutyl, cyclopentyl, cyclohexyl, oxacyclopropyl, azacyclopropyl, oxacyclobutyl, azacyclobutyl, methoxy, ethoxy, propoxy, isopropoxy, butoxy, cyclopropyloxy, cyclobutyloxy, monomethylamino, dimethylamino and amino, and the above groups are each independently optionally further substituted by one or more substituents selected from the group consisting of deuterium, fluorine, chlorine, bromine, cyano, hydroxy, methyl, ethyl, propyl, isopropyl, butyl, isobutyl, vinyl, ethynyl, monofluoromethyl, difluoromethyl, trifluoromethyl, monodeuteriomethyl, dideuteriomethyl, trideuteriomethyl, cyclopropyl, cyclobutyl, cyclopentyl, cyclohexyl, oxacyclopropyl, azacyclopropyl, oxacyclobutyl, azacyclobutyl, =O, =S, methoxy, ethoxy, propoxy, isopropoxy, butoxy, cyclopropyloxy, cyclobutyloxy, monomethylamino, dimethylamino and amino.

As a further preferred embodiment, in the compound of formula (I), the stereoisomer or pharmaceutically acceptable salt thereof, R₅ₐ is selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R_{5b} and R_{5c} are each independently selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇ and r are defined as in the compound of formula (I).

As a more further preferred embodiment, in the compound of formula (I), the stereoisomer or pharmaceutically acceptable salt thereof, R₅ₐ is selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, -O-R₁₄ and -C(O)NR₁₆R₁₇, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O and =S;
R_{5b} and R_{5c} are each independently hydrogen, deuterium, halogen or C₁₋₄ alkyl, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -O-R₁₄;
wherein, R₁₄, R₁₆ and R₁₇ are defined as in the compound of formula (I).

As a further preferred embodiment, in the compound of formula (I), the stereoisomer or pharmaceutically acceptable salt thereof, R₅ₐ is selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R_{5b} and R_{5c}, together with the carbon atom to which they are directly attached, form a C(O), C₃₋₆ cycloalkyl or 3-6 membered heterocyclyl, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇ and r are defined as in the compound of formula (I).

As a more further preferred embodiment, in the compound of formula (I), the stereoisomer or pharmaceutically acceptable salt thereof, R₅ₐ is selected from the group consisting of hydrogen, deuterium, halogen, C₁₋₄ alkyl and -O-R₁₄, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -O-R₁₄;
R_{5b} and R_{5c}, together with the carbon atom to which they are directly attached, form a C(O), C₃₋₆ cycloalkyl or 3-6 membered heterocyclyl, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -O-R₁₄ and -NR₁₆R₁₇,
wherein, R₁₄, R₁₆ and R₁₇ are defined as in the compound of formula (I).

As a further preferred embodiment, in the compound of formula (I), the stereoisomer or pharmaceutically acceptable salt thereof, R₅ₐ is selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R_{5b} and R_{5c}, together with the carbon atom to which they are directly attached, form 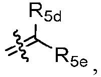
R_{5d} and R₅ₑ are each independently selected from the group consisting of hydrogen, halogen, cyano, C₁₋₄ alkyl, C₃₋₆ cycloalkyl, 3-6 membered heterocyclyl and -C₀₋₄ alkyl-NR₁₆R₁₇, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl and 5-8 membered heteroaryl, provided that R_{5d} and R₅ₑ are not hydrogen at the same time;
wherein, R₁₀, R₁₁, R₁₂, R₁₃, R₁₄, R₁₅, R₁₆, R₁₇ and r are defined as in the compound of formula (I).

As a more further preferred embodiment, in the compound of formula (I), the stereoisomer or pharmaceutically acceptable salt thereof, R₅ₐ is selected from the group consisting of hydrogen, deuterium, halogen and C₁₋₄ alkyl, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -O-R₁₄;
R_{5b} and R_{5c}, together with the carbon atom to which they are directly attached, form 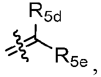
R_{5d} and R₅ₑ are each independently selected from the group consisting of hydrogen, halogen, cyano, C₁₋₄ alkyl, C₃₋₆ cycloalkyl, 3-6 membered heterocyclyl and -C₀₋₄ alkyl-NR₁₆R₁₇, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl and 5-8 membered heteroaryl, provided that R_{5d} and R₅ₑ are not hydrogen at the same time;
wherein, R₁₄, R₁₆ and R₁₇ are defined as in the compound of formula (I).

As a further preferred embodiment, in the compound of formula (I), the stereoisomer or pharmaceutically acceptable salt thereof, R₅ₐ, R_{5b} and R_{5c}, together with the carbon atom to which they are directly attached, form 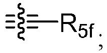
R_{5f} is selected from the group consisting of halogen, cyano, C₁₋₄ alkyl, C₃₋₆ cycloalkyl, 3-6 membered heterocyclyl and -C₀₋₄ alkyl-NR₁₆R₁₇, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl and 5-8 membered heteroaryl;
wherein, R₁₆ and R₁₇ are defined as in the compound of formula (I).

As a preferred embodiment, in the compound of formula (I), the stereoisomer or pharmaceutically acceptable salt thereof, the compound of formula (I) is a compound of formula (IIIa1) or a compound of formula (IIIb1) as shown below: 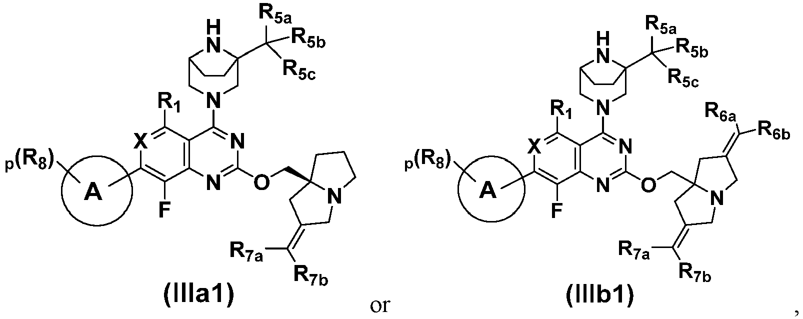
wherein, each X is independently CR₉ or N;
each ring A is independently phenyl, naphthyl, pyridyl, pyrimidinyl, pyridazinyl, quinolinyl, isoquinolinyl, benzothiophenyl or benzothiazolyl;
each R₁ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
each 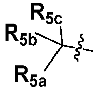 is independently selected from the group consisting of deuteriomethyl, ethyl, isopropyl, methoxymethyl, cyclopropyloxymethyl and 1-hydroxy-1-methyl-ethyl, and the ethyl, isopropyl, methoxymethyl, cyclopropyloxymethyl or 1-hydroxy-1-methyl-ethyl is each independently optionally further substituted by one or more deuterium(s);
in the compound of formula (IIIb 1), R₆ₐ and R_{6b} are each independently selected from the group consisting of hydrogen, deuterium, fluorine, C₁₋₃ alkyl, C₁₋₃ haloalkyl and C₁₋₃ deuterioalkyl;
each R₇ₐ and each R_{7b} are independently selected from the group consisting of hydrogen, deuterium, fluorine, C₁₋₃ alkyl, C₁₋₃ haloalkyl and C₁₋₃ deuterioalkyl;
each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
each R₉ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
wherein, R₁₄, R₁₆, R₁₇ and p are defined as in the compound of formula (I).

As a further preferred embodiment, in the compound of formula (I), the stereoisomer or pharmaceutically acceptable salt thereof, each 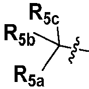 is independently selected from the group consisting of deuteriomethyl, ethyl, isopropyl, cyclopropyloxymethyl and 1-hydroxy-1-methyl-ethyl, and the ethyl, isopropyl, cyclopropyloxymethyl or 1-hydroxy-1-methyl-ethyl is each independently further substituted by at least 1, 2, 3, 4, 5, 6 or 7 deuterium(s).

As a further preferred embodiment, in the compound of formula (I), the stereoisomer or pharmaceutically acceptable salt thereof, each 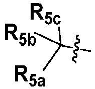 is independently selected from the group consisting of -CH₂D, -CHD₂, -CD₃,

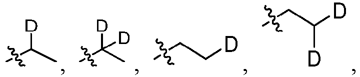

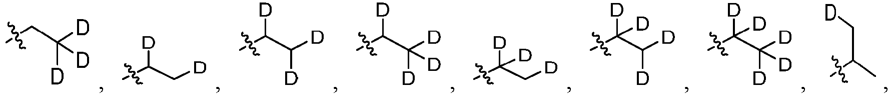

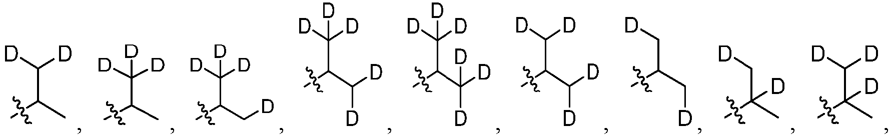

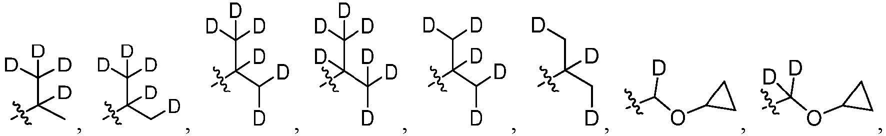

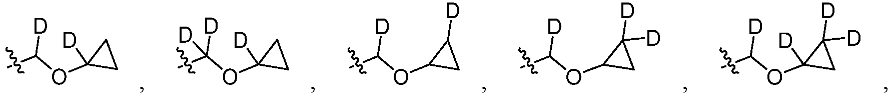

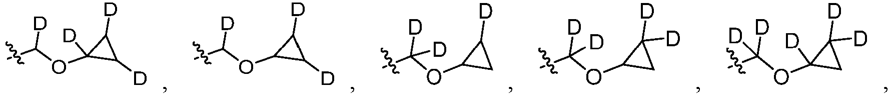

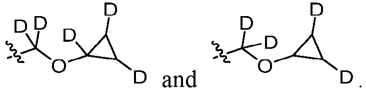

As a further preferred embodiment, in the compound of formula (I), the stereoisomer or pharmaceutically acceptable salt thereof, each 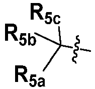 is independently selected from the group consisting of -CH₂D, -CHD₂, -CD₃, 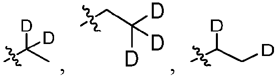 and

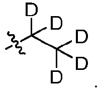

As a further preferred embodiment, in the compound of formula (I), the stereoisomer or pharmaceutically acceptable salt thereof, each 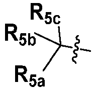 is independently ethyl or isopropyl.

As a further preferred embodiment, in the compound of formula (I), the stereoisomer or pharmaceutically acceptable salt thereof, each 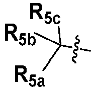 is independently methoxymethyl, and the methoxymethyl is optionally further substituted by one or more deuterium(s); provided that the compound of formula (I), the stereoisomer or pharmaceutically acceptable salt thereof is not the following compound:

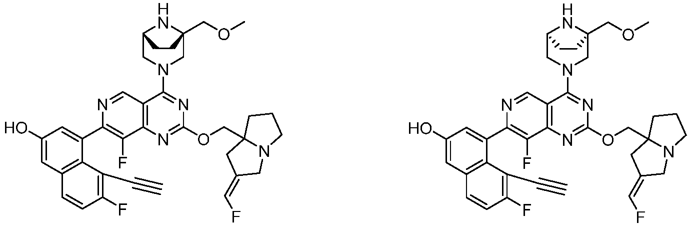

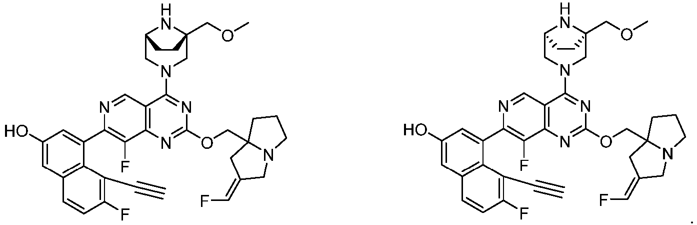

As a more further preferred embodiment, in the compound of formula (I), the stereoisomer or pharmaceutically acceptable salt thereof, each 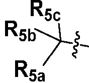 is independently methoxymethyl, and the methoxymethyl is optionally further substituted by 1, 2, 3, 4 or 5 deuterium(s).

As a more further preferred embodiment, in the compound of formula (I), the stereoisomer or pharmaceutically acceptable salt thereof, each 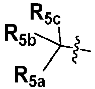 is independently selected from the group consisting of

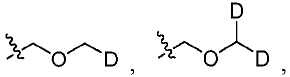

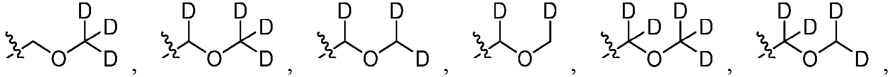

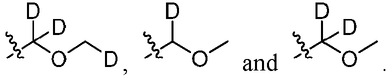

As a more further preferred embodiment, in the compound of formula (I), the stereoisomer or pharmaceutically acceptable salt thereof, each 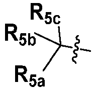 is independently

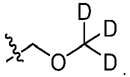

As a further preferred embodiment, in the compound of formula (I), the stereoisomer or pharmaceutically acceptable salt thereof, each X is independently CR₉ or N;
each R₁ is independently selected from the group consisting of methoxy, ethoxy, isopropoxy, cyclopropyloxy, trideuteriomethoxy, monomethylamino, dimethylamino and amino;
each R₉ is independently selected from the group consisting of hydrogen, fluorine, chlorine, methyl, monofluoromethyl, difluoromethyl, trifluoromethyl, monodeuteriomethyl, dideuteriomethyl and trideuteriomethyl.

As a further preferred embodiment, in the compound of formula (I), the stereoisomer or pharmaceutically acceptable salt thereof, each X is independently CR₉, and R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
wherein, R₁₄, R₁₆ and R₁₇ are defined as in the compound of formula (I).

As a preferred embodiment, in the compound of formula (I), the stereoisomer or pharmaceutically acceptable salt thereof, the compound of formula (I) is a compound of formula (IIIa2) or a compound of formula (IIIb2) as shown below: 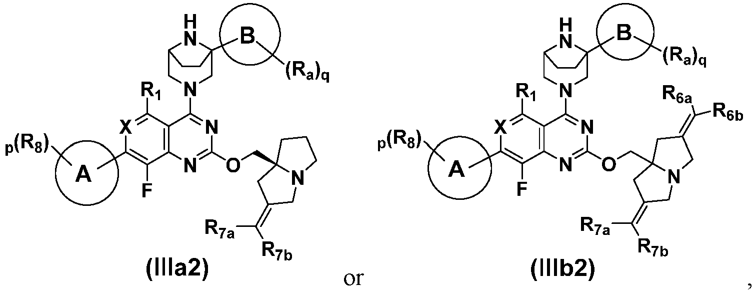
wherein, each X is independently CR₉ or N;
each ring A is independently phenyl, naphthyl, pyridyl, pyrimidinyl, pyridazinyl, quinolinyl, isoquinolinyl, benzothiophenyl or benzothiazolyl;
each ring B is independently selected from the group consisting of oxacyclobutyl, oxacyclopentyl and oxacyclohexyl;
each Rₐ is independently selected from the group consisting of hydrogen, deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -O-R₁₄ and -NR₁₆R₁₇;
each R₁ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
in the compound of formula (IIIb2), R₆ₐ and R_{6b} are each independently selected from the group consisting of hydrogen, deuterium, fluorine, C₁₋₃ alkyl, C₁₋₃ haloalkyl and C₁₋₃ deuterioalkyl;
each R₇ₐ and each R_{7b} are independently selected from the group consisting of hydrogen, deuterium, fluorine, C₁₋₃ alkyl, C₁₋₃ haloalkyl and C₁₋₃ deuterioalkyl;
each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
each R₉ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
q is 0, 1, 2 or 3;
wherein, R₁₄, R₁₆, R₁₇ and p are defined as in the compound of formula (I).

As a further preferred embodiment, in the compound of formula (I), the stereoisomer or pharmaceutically acceptable salt thereof, each ring B is independently oxacyclobutyl or oxacyclohexyl;
each Rₐ is independently selected from the group consisting of hydrogen, deuterium, fluorine, chlorine, methyl, ethyl, propyl, isopropyl, monofluoromethyl, difluoromethyl, trifluoromethyl, monodeuteriomethyl, dideuteriomethyl and trideuteriomethyl.

As a preferred embodiment, in the compound of formula (I), the stereoisomer or pharmaceutically acceptable salt thereof, the compound of formula (I) is a compound of formula (IIIa3) or a compound of formula (IIIb3) as shown below: 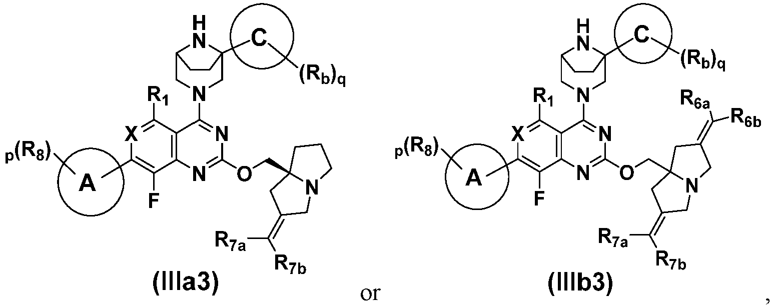
wherein, each X is independently CR₉ or N;
each ring A is independently phenyl, naphthyl, pyridyl, pyrimidinyl, pyridazinyl, quinolinyl, isoquinolinyl, benzothiophenyl or benzothiazolyl;
each ring C is independently selected from the group consisting of cyclopropyl, cyclobutyl, cyclopentyl and cyclohexyl;
each R_{b} is independently selected from the group consisting of hydrogen, deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -O-R₁₄ and -NR₁₆R₁₇;
each R₁ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
in the compound of formula (IIIb3), R₆ₐ and R_{6b} are each independently selected from the group consisting of hydrogen, deuterium, fluorine, C₁₋₃ alkyl, C₁₋₃ haloalkyl and C₁₋₃ deuterioalkyl;
each R₇ₐ and each R_{7b} are independently selected from the group consisting of hydrogen, deuterium, fluorine, C₁₋₃ alkyl, C₁₋₃ haloalkyl and C₁₋₃ deuterioalkyl;
each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
each R₉ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
q is 0, 1, 2 or 3;
wherein, R₁₄, R₁₆, R₁₇ and p are defined as in the compound of formula (I).

As a further preferred embodiment, in the compound of formula (I), the stereoisomer or pharmaceutically acceptable salt thereof, each ring C is independently cyclopropyl;
each R_{b} is independently selected from the group consisting of hydrogen, deuterium, fluorine, chlorine, methyl, ethyl, propyl, isopropyl, monofluoromethyl, difluoromethyl, trifluoromethyl, monodeuteriomethyl, dideuteriomethyl and trideuteriomethyl.

As a preferred embodiment, in the compound of formula (I), the stereoisomer or pharmaceutically acceptable salt thereof, the compound of formula (I) is a compound of formula (IIIa4) or a compound of formula (IIIb4) as shown below: 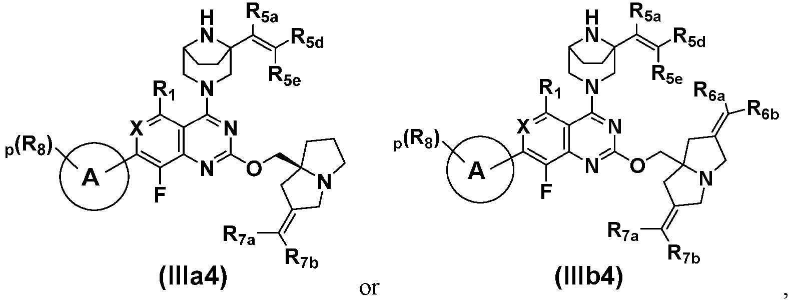
wherein, each X is independently CR₉ or N;
each ring A is independently phenyl, naphthyl, pyridyl, pyrimidinyl, pyridazinyl, quinolinyl, isoquinolinyl, benzothiophenyl or benzothiazolyl;
each R₁ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
R₅ₐ is selected from the group consisting of hydrogen, fluorine and methyl;
R_{5d} is selected from the group consisting of hydrogen, fluorine and methyl;
R₅ₑ is selected from the group consisting of hydrogen, fluorine and methyl;
in the compound of formula (IIIb4), R₆ₐ and R_{6b} are each independently selected from the group consisting of hydrogen, deuterium, fluorine, C₁₋₃ alkyl, C₁₋₃ haloalkyl and C₁₋₃ deuterioalkyl;
each R₇ₐ and each R_{7b} are independently selected from the group consisting of hydrogen, deuterium, fluorine, C₁₋₃ alkyl, C₁₋₃ haloalkyl and C₁₋₃ deuterioalkyl;
each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
each R₉ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
wherein, R₁₄, R₁₆, R₁₇ and p are defined as in the compound of formula (I).

As a further preferred embodiment, in the compound of formula (I), the stereoisomer or pharmaceutically acceptable salt thereof, R₅ₐ is hydrogen; R_{5d} is fluorine; R₅ₑ is hydrogen or fluorine.

As a preferred embodiment, in the compound of formula (I), the stereoisomer or pharmaceutically acceptable salt thereof, the compound of formula (I) is a compound of formula (IIIa5) or a compound of formula (IIIb5) as shown below: 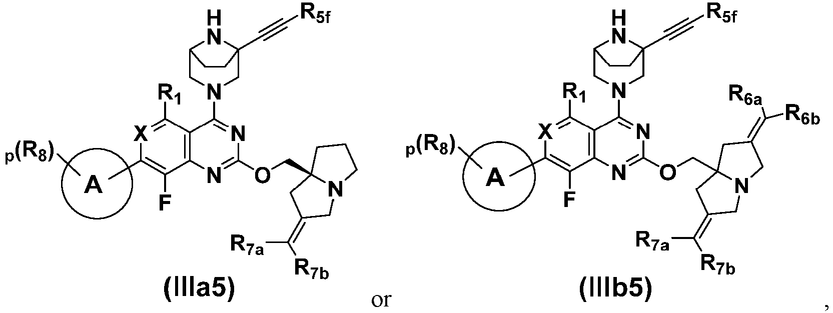
wherein, each X is independently CR₉ or N;
each ring A is independently phenyl, naphthyl, pyridyl, pyrimidinyl, pyridazinyl, quinolinyl, isoquinolinyl, benzothiophenyl or benzothiazolyl;
each R₁ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
R_{5f} is selected from the group consisting of hydrogen, fluorine and methyl;
in the compound of formula (IIIb5), R₆ₐ and R_{6b} are each independently selected from the group consisting of hydrogen, deuterium, fluorine, C₁₋₃ alkyl, C₁₋₃ haloalkyl and C₁₋₃ deuterioalkyl;
each R₇ₐ and each R_{7b} are independently selected from the group consisting of hydrogen, deuterium, fluorine, C₁₋₃ alkyl, C₁₋₃ haloalkyl and C₁₋₃ deuterioalkyl;
each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
each R₉ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
wherein, R₁₄, R₁₆, R₁₇ and p are defined as in the compound of formula (I).

As a further preferred embodiment, in the compound of formula (I), the stereoisomer or pharmaceutically acceptable salt thereof, R_{5f} is methyl.

As a further preferred embodiment, in the compound of formula (I), the stereoisomer or pharmaceutically acceptable salt thereof, each X is independently CR₉ or N;
each R₁ is independently selected from the group consisting of hydrogen, halogen, -O-R₁₄ and -NR₁₆R₁₇;
each R₉ is independently selected from the group consisting of hydrogen, halogen, C₁₋₄ alkyl, C₁₋₄ haloalkyl and C₁₋₄ deuterioalkyl;
or, R₉ and R₁, together with the moiety to which they are directly attached, form a N-containing or O-containing 3-8 membered heterocyclyl;
wherein, R₁₄, R₁₆ and R₁₇ are defined as in the compound of formula (I).

As a more further preferred embodiment, in the compound of formula (I), the stereoisomer or pharmaceutically acceptable salt thereof, each X is independently CR₉ or N;
each R₁ is independently selected from the group consisting of hydrogen, fluorine, chlorine, hydroxy, methoxy, ethoxy, isopropoxy, cyclopropyloxy, trideuteriomethoxy, monomethylamino, dimethylamino and amino;
each R₉ is independently selected from the group consisting of hydrogen, fluorine, chlorine, methyl, monofluoromethyl, difluoromethyl, trifluoromethyl, monodeuteriomethyl, dideuteriomethyl and trideuteriomethyl;
or, R₉ and R₁, together with the moiety to which they are directly attached, form an oxacyclohexyl.

As a further preferred embodiment, in the compound of formula (I), the stereoisomer or pharmaceutically acceptable salt thereof, wherein each ring A is independently selected from the group consisting of phenyl, naphthyl, pyridyl, pyrimidinyl, benzothiophenyl and benzothiazolyl;
each R₈ is independently selected from the group consisting of hydrogen, deuterium, halogen, cyano, C₁₋₄ alkyl, C₂₋₄ alkenyl, C₂₋₄ alkynyl, -O-R₁₄ and -NH₂, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NH₂;
wherein, R₁₄ is defined as in the compound of formula (I).

As a further preferred embodiment, in the compound of formula (I), the stereoisomer or pharmaceutically acceptable salt thereof, each 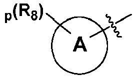 is independently selected from the group consisting of the following structures: 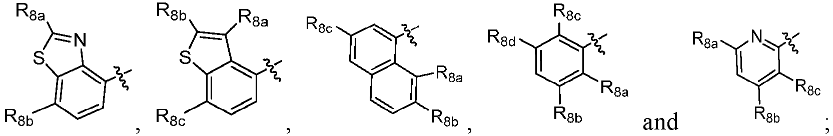 each R₈ₐ, each R_{8b}, each R_{8c} and each R_{8d} are independently selected from the group consisting of hydrogen, deuterium, fluorine, chlorine, bromine, cyano, hydroxy, methyl, ethyl, propyl, isopropyl, butyl, isobutyl, vinyl, ethynyl, cyclopropyl, cyclobutyl, cyclopentyl, cyclohexyl, oxacyclopropyl, azacyclopropyl, oxacyclobutyl, azacyclobutyl, methoxy, ethoxy, propoxy, isopropoxy, butoxy, cyclopropyloxy, cyclobutyloxy, monomethylamino, dimethylamino and amino, and the above groups are each independently optionally further substituted by one or more substituents selected from the group consisting of deuterium, fluorine, chlorine, bromine, cyano, hydroxy, methyl, ethyl, propyl, isopropyl, butyl, isobutyl, vinyl, ethynyl, monofluoromethyl, difluoromethyl, trifluoromethyl, monodeuteriomethyl, dideuteriomethyl, trideuteriomethyl, cyclopropyl, cyclobutyl, cyclopentyl, cyclohexyl, oxacyclopropyl, azacyclopropyl, oxacyclobutyl, azacyclobutyl, =O, methoxy, ethoxy, propoxy, isopropoxy, butoxy, cyclopropyloxy, cyclobutyloxy, monomethylamino, dimethylamino and amino.

As a further preferred embodiment, in the compound of formula (I), the stereoisomer or pharmaceutically acceptable salt thereof, wherein R₆ₐ and R_{6b} are each independently selected from the group consisting of hydrogen, deuterium, fluorine, methyl, ethyl, monofluoromethyl, difluoromethyl, trifluoromethyl, monodeuteriomethyl, dideuteriomethyl and trideuteriomethyl.

As a more further preferred embodiment, in the compound of formula (I), the stereoisomer or pharmaceutically acceptable salt thereof, R₆ₐ and R_{6b} are each independently hydrogen, deuterium or fluorine.

As a further preferred embodiment, in the compound of formula (I), the stereoisomer or pharmaceutically acceptable salt thereof, each R₇ₐ and each R_{7b} are independently selected from the group consisting of hydrogen, deuterium, fluorine, methyl, ethyl, monofluoromethyl, difluoromethyl, trifluoromethyl, monodeuteriomethyl, dideuteriomethyl and trideuteriomethyl.

As a more further preferred embodiment, in the compound of formula (I), the stereoisomer or pharmaceutically acceptable salt thereof, each R₇ₐ and each R_{7b} are independently hydrogen, deuterium or fluorine.

As the most preferred embodiment, the compound of formula (I), the stereoisomer or pharmaceutically acceptable salt thereof includes, but is not limited to, the following compounds:

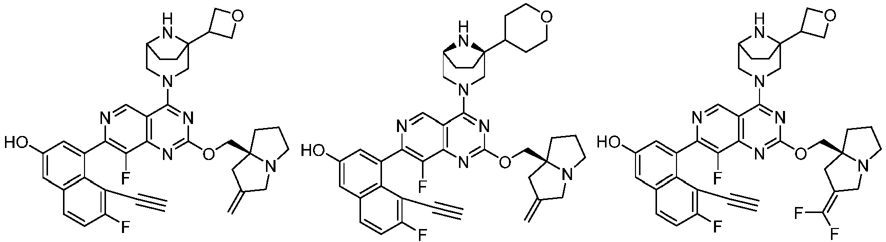

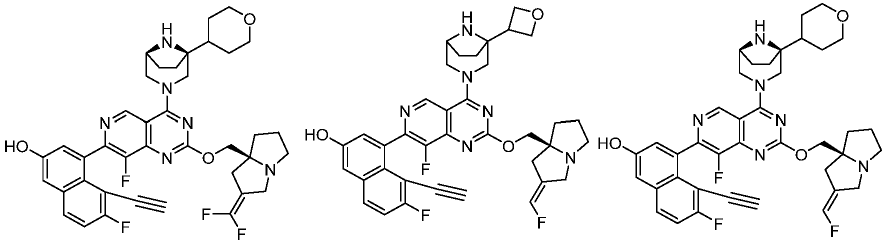

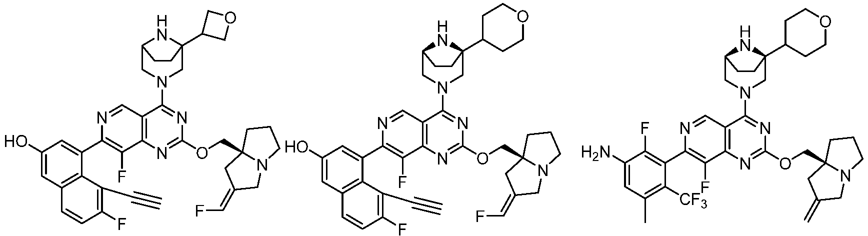

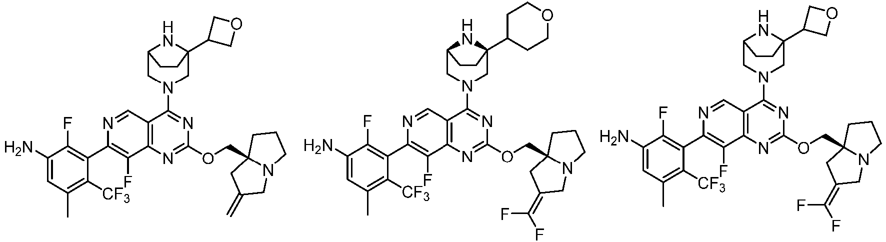

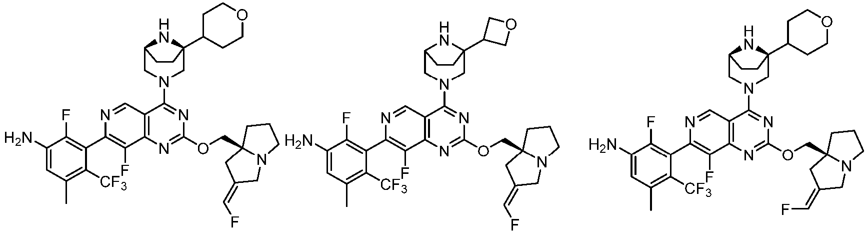

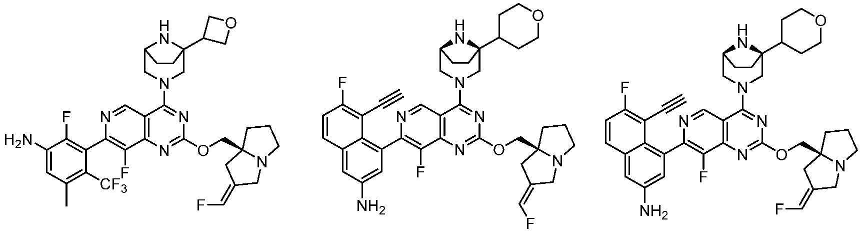

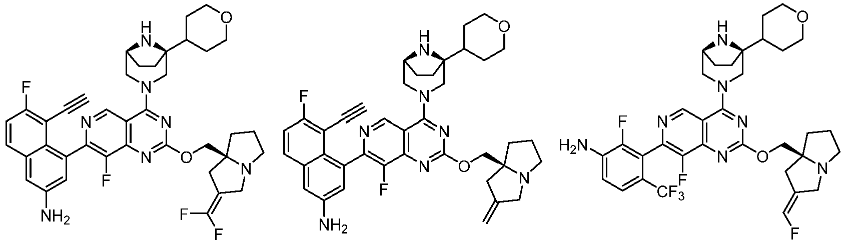

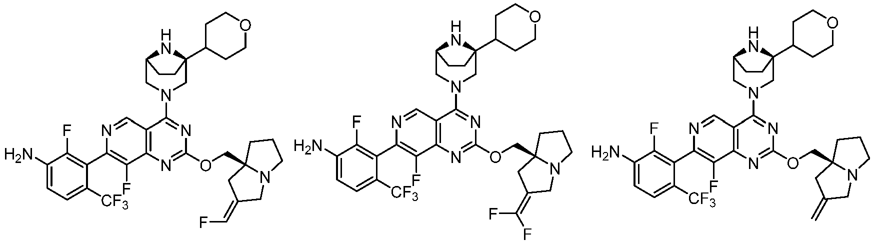

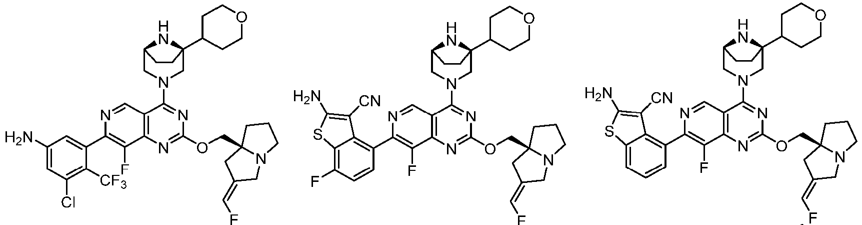

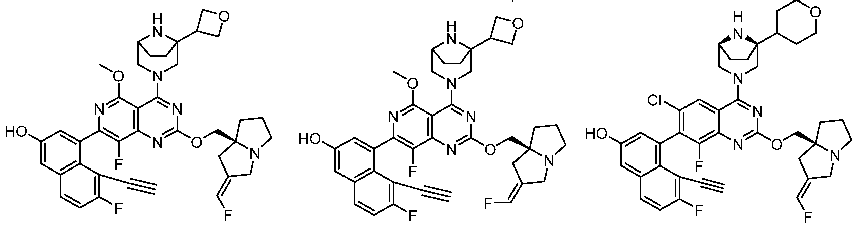

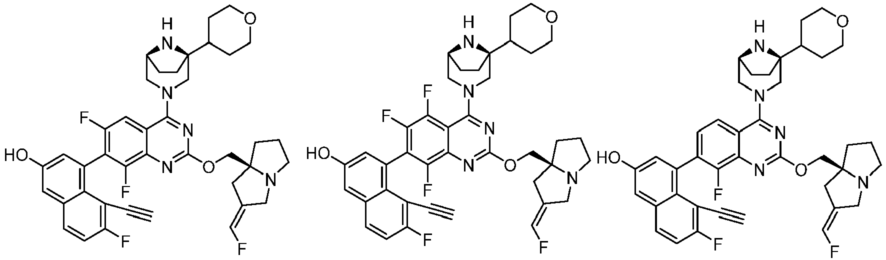

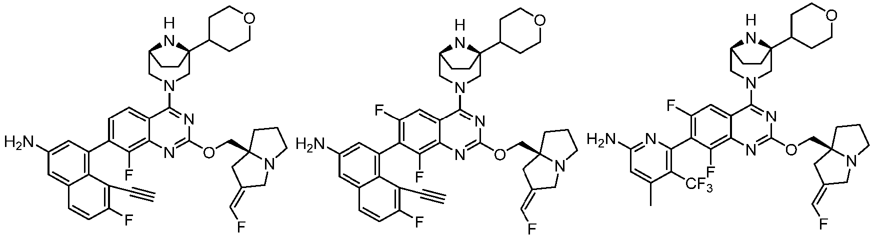

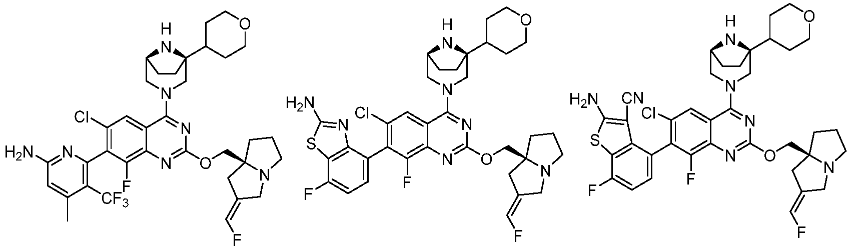

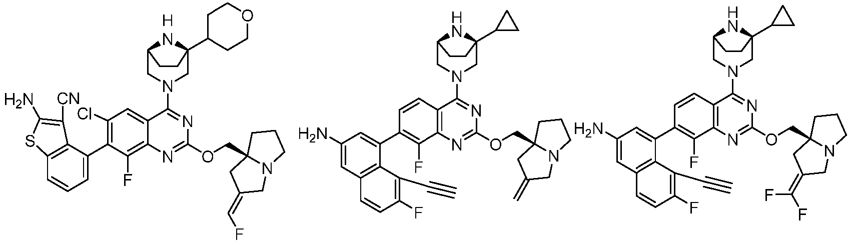

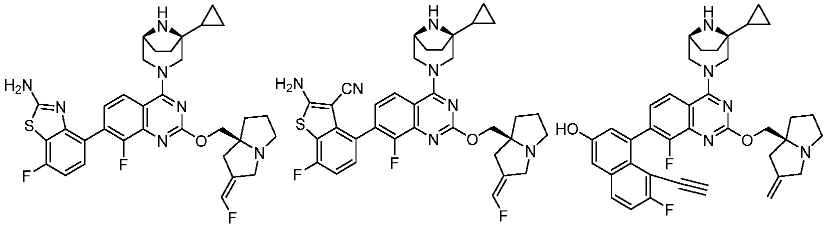

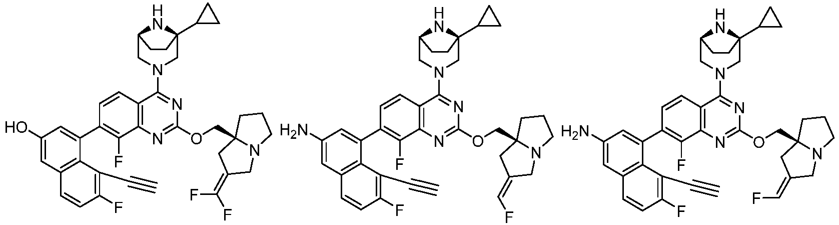

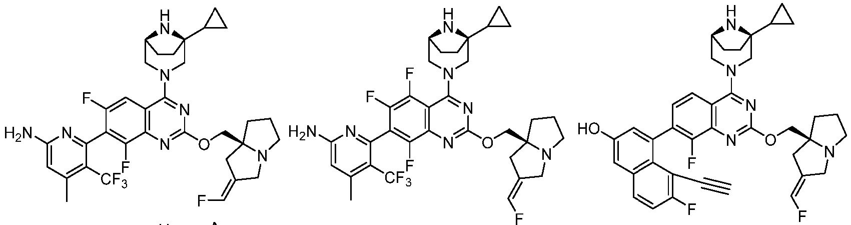

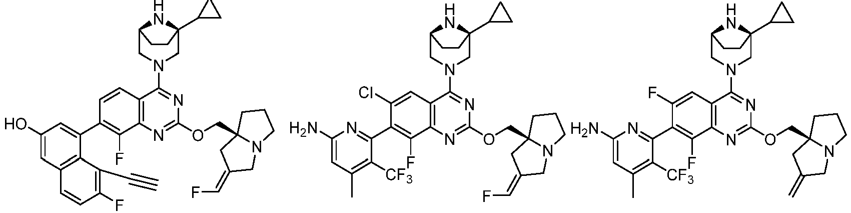

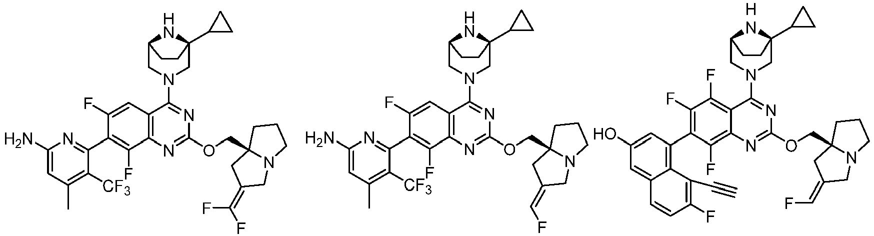

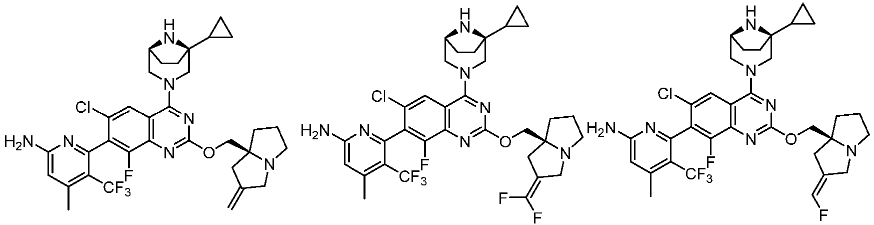

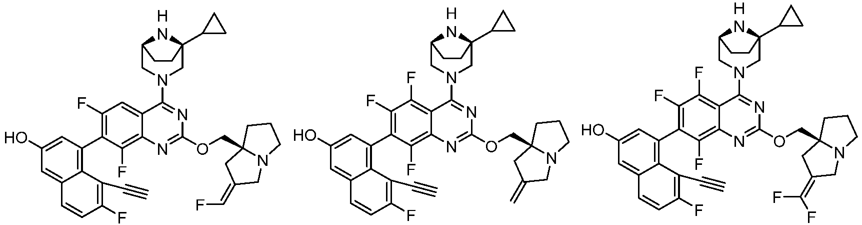

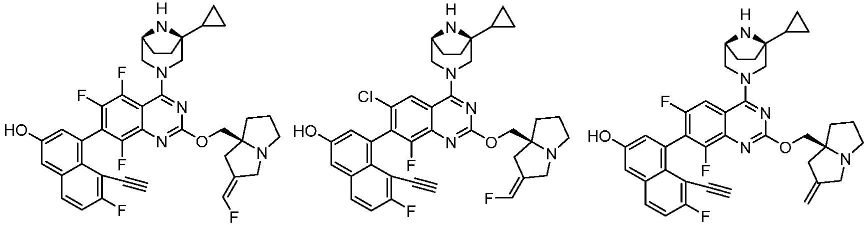

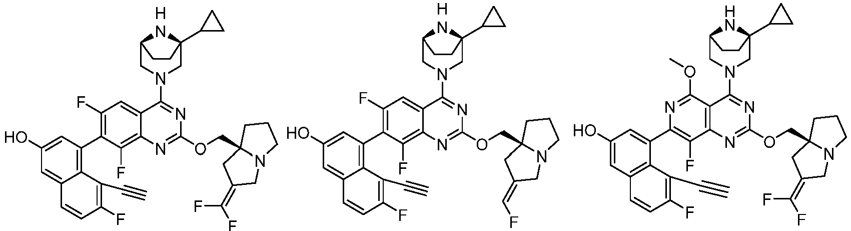

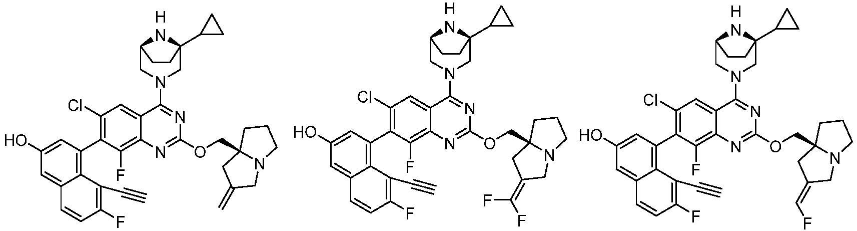

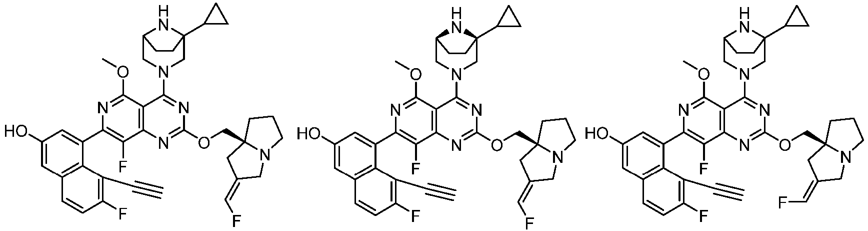

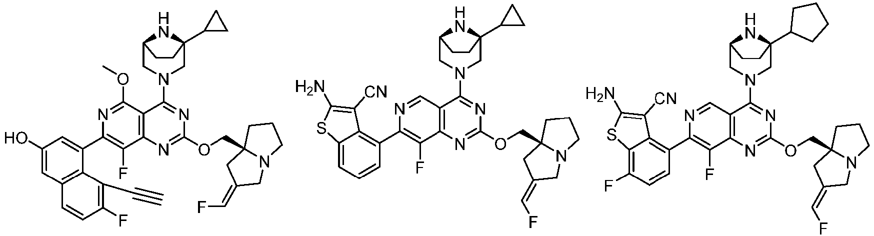

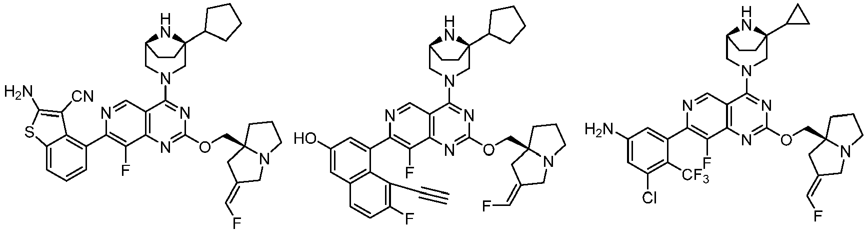

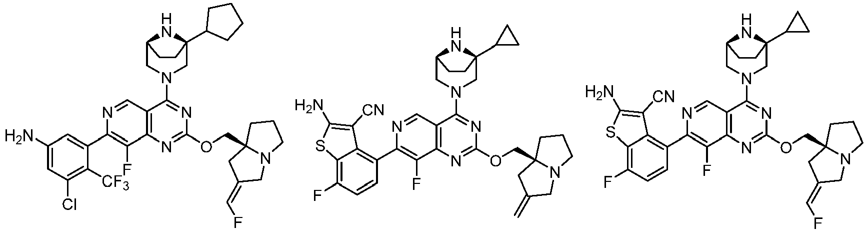

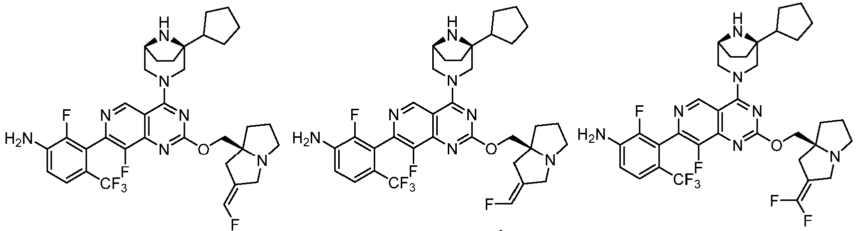

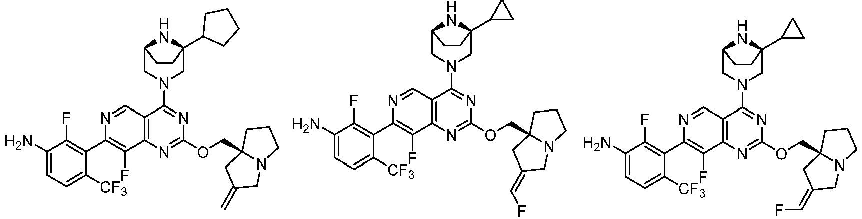

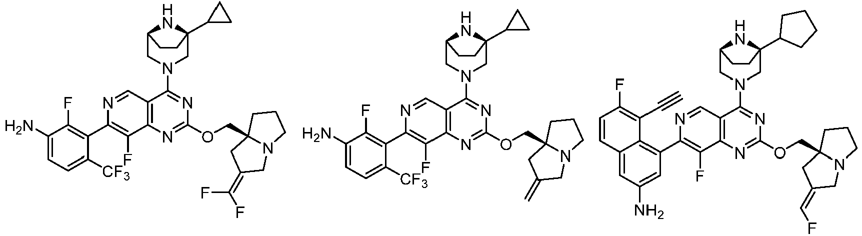

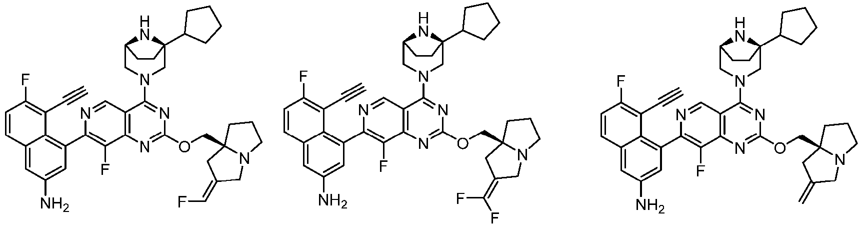

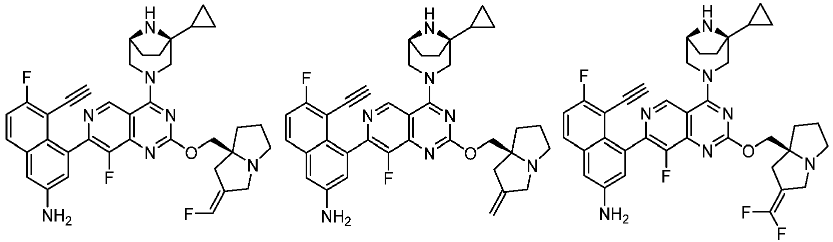

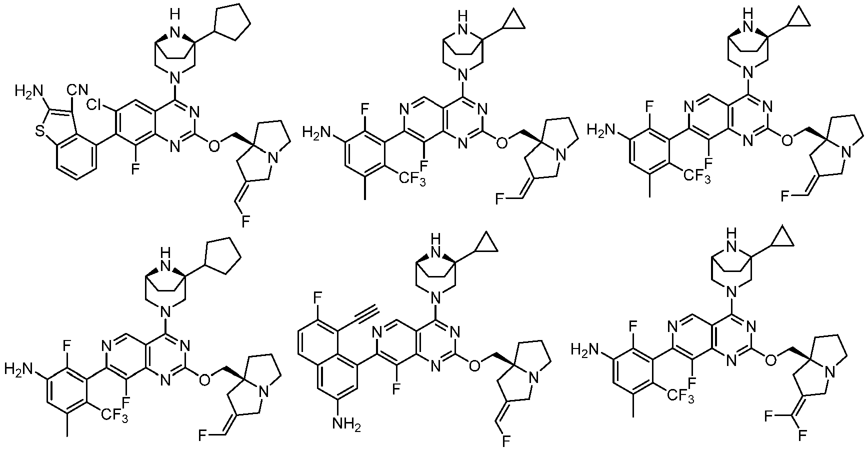

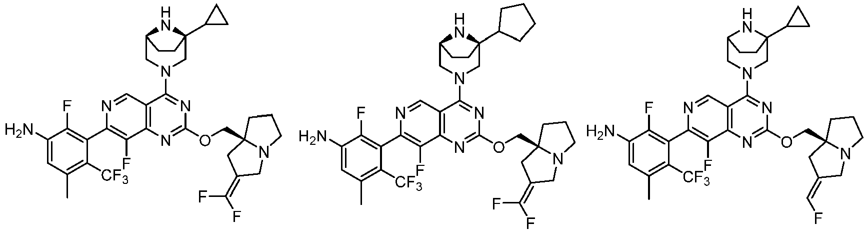

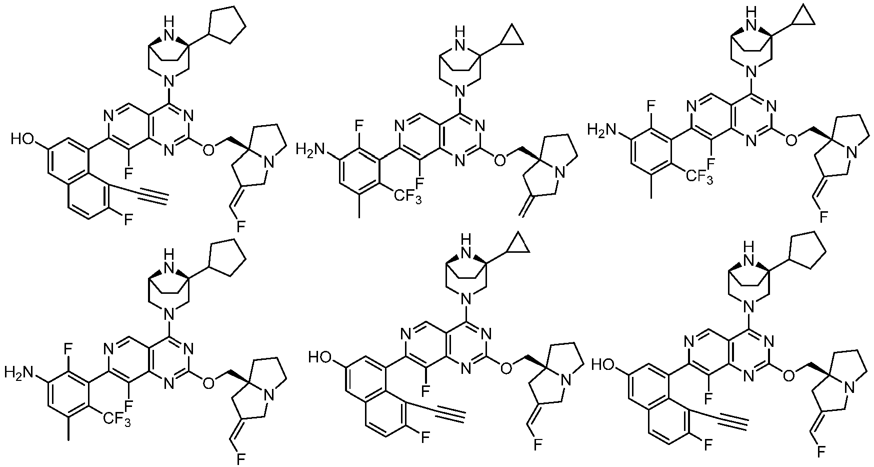

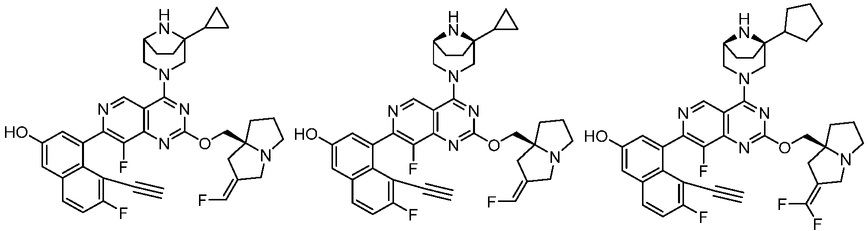

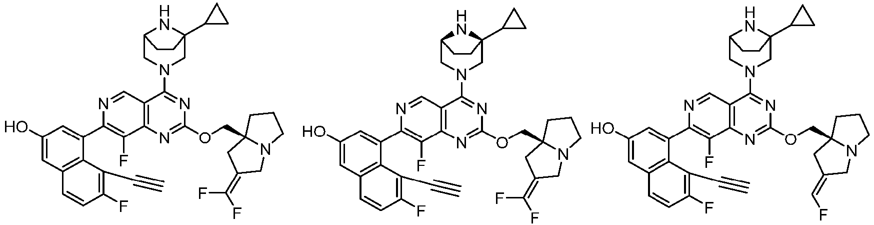

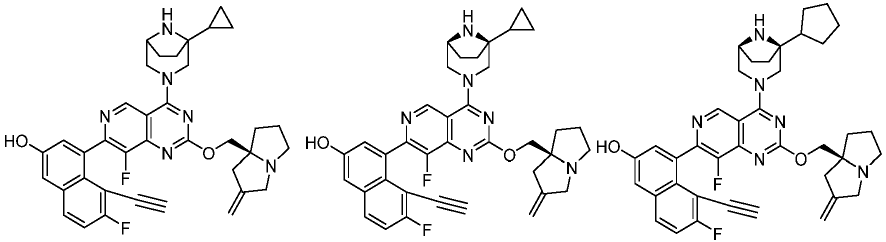

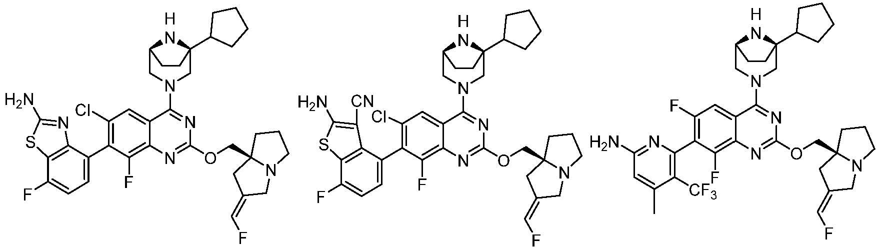

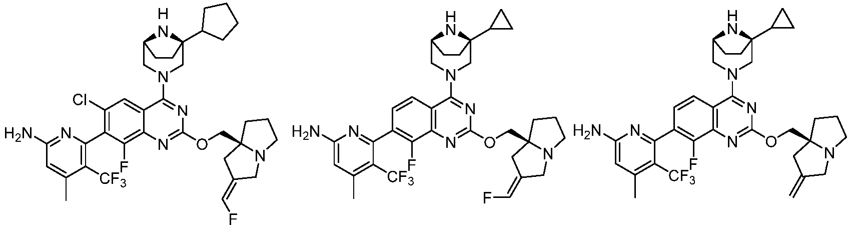

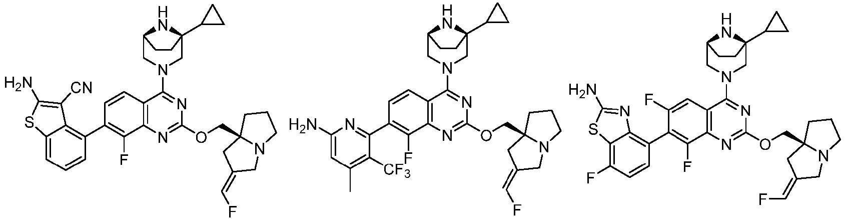

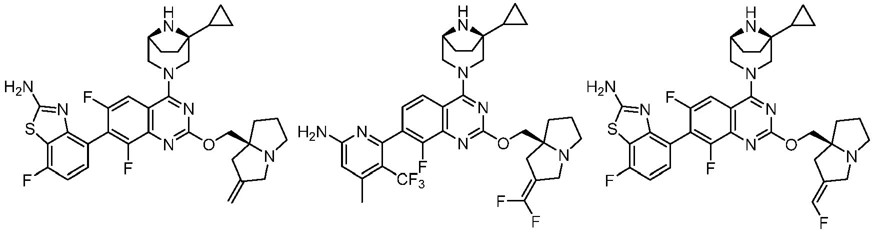

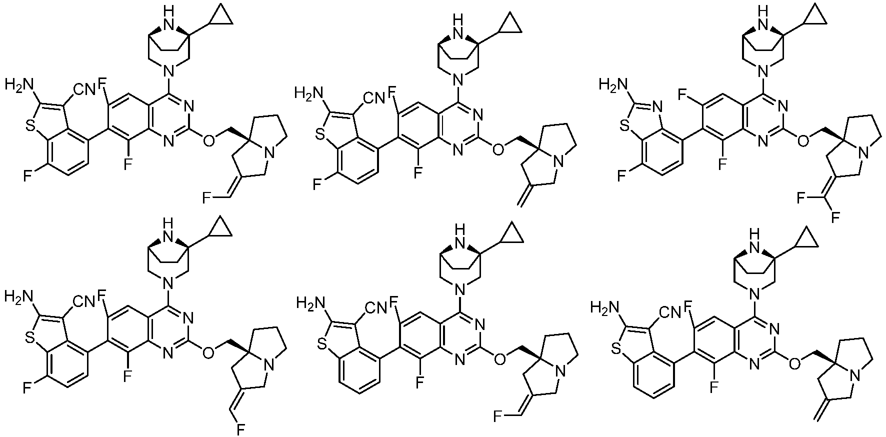

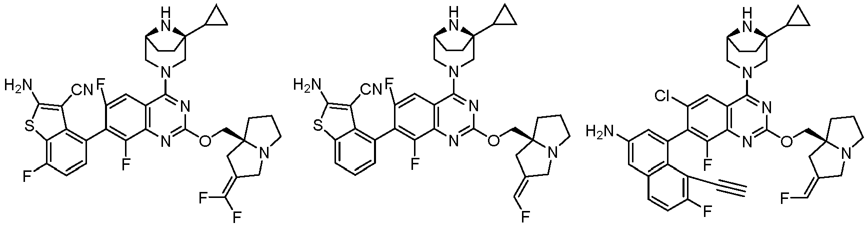

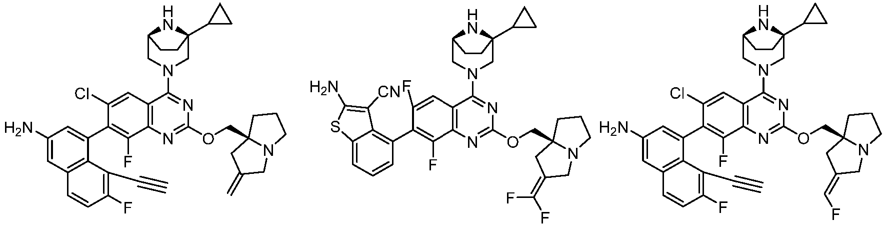

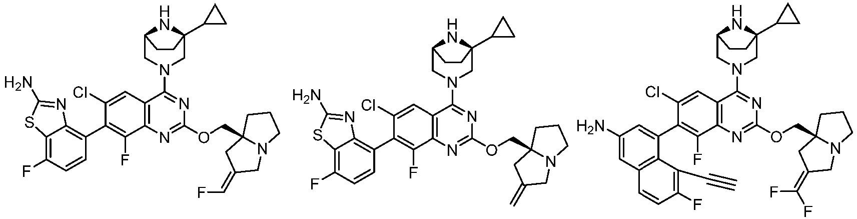

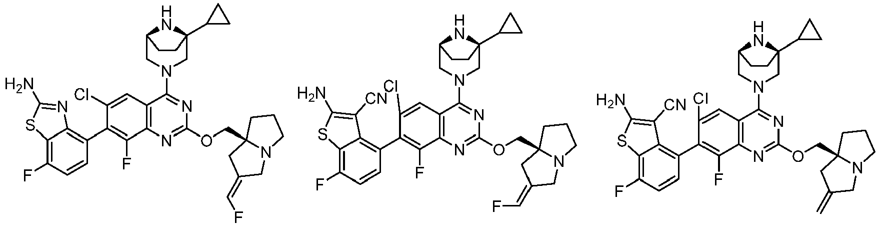

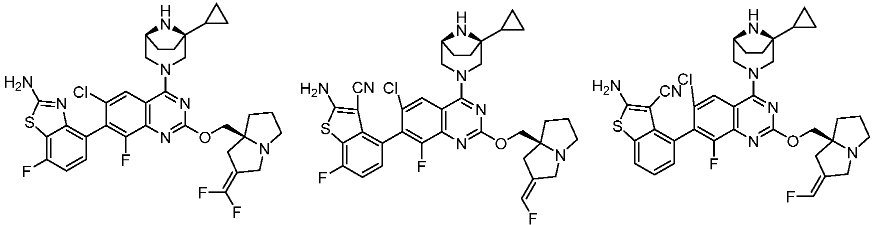

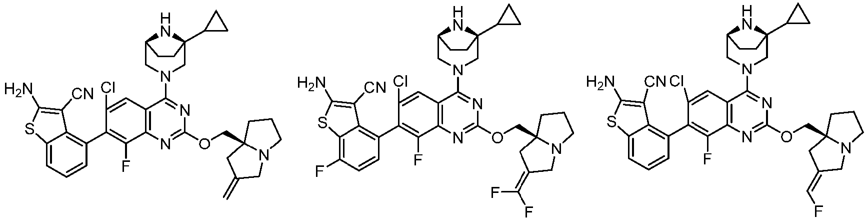

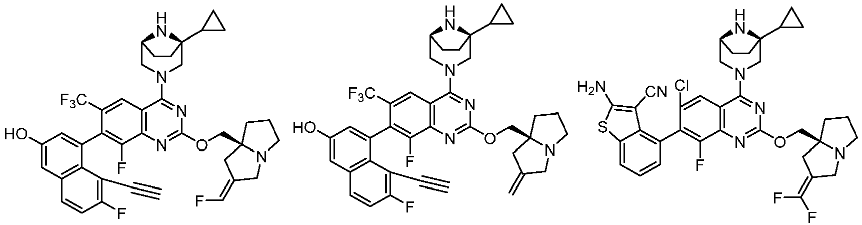

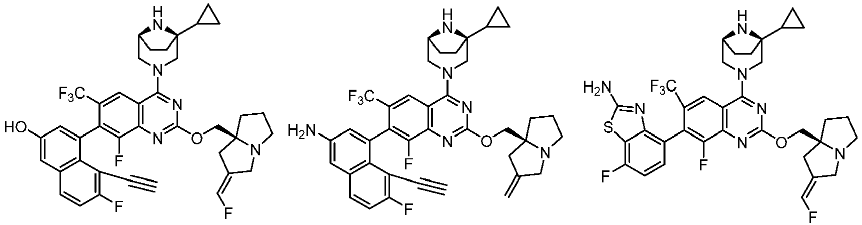

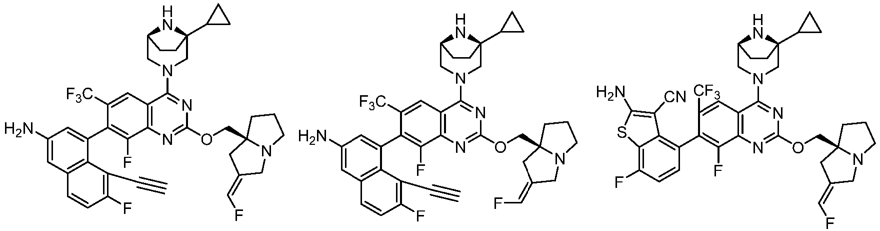

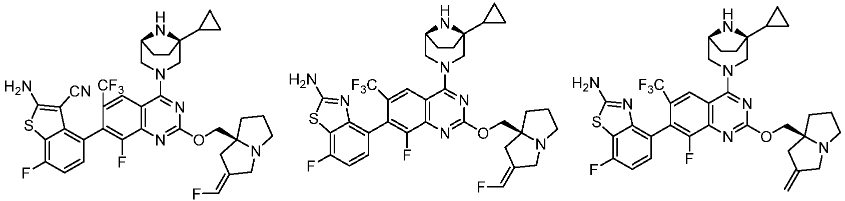

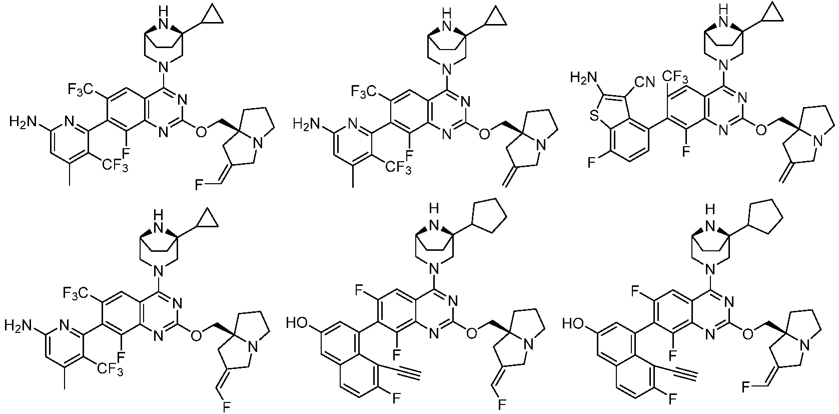

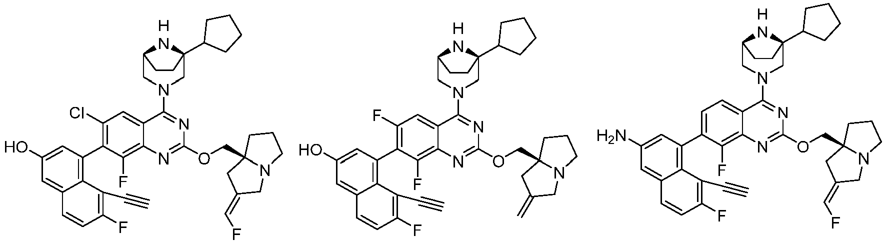

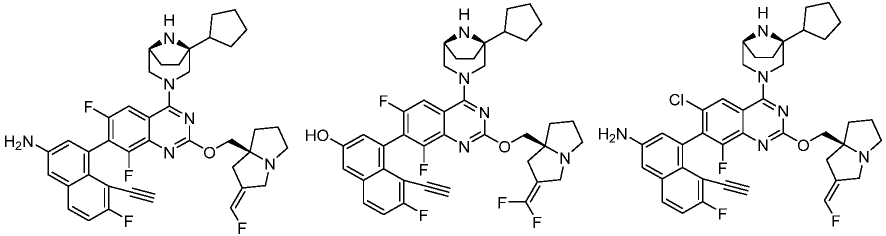

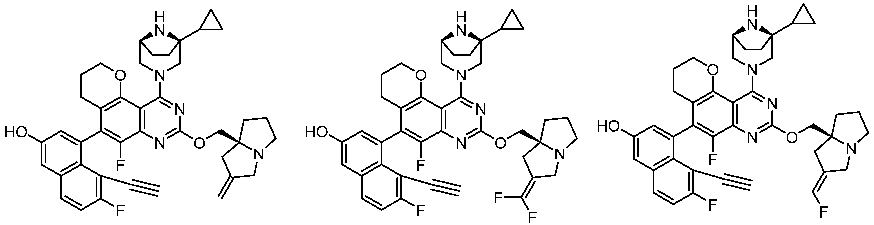

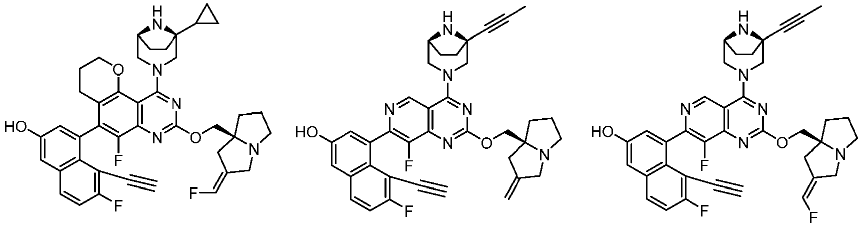

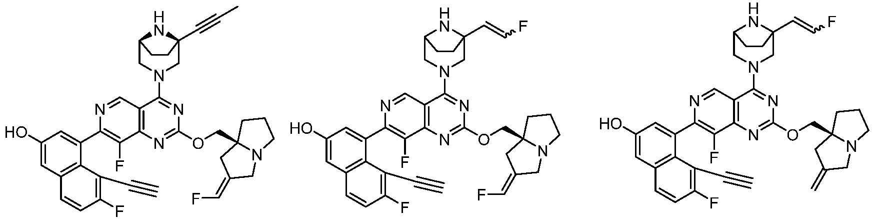

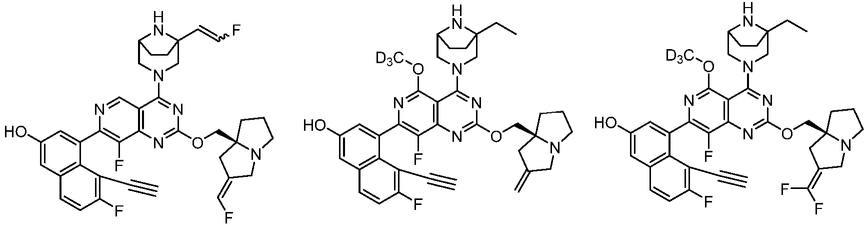

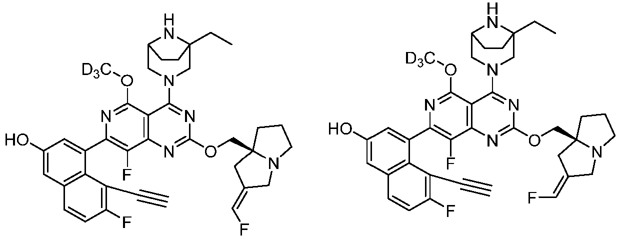

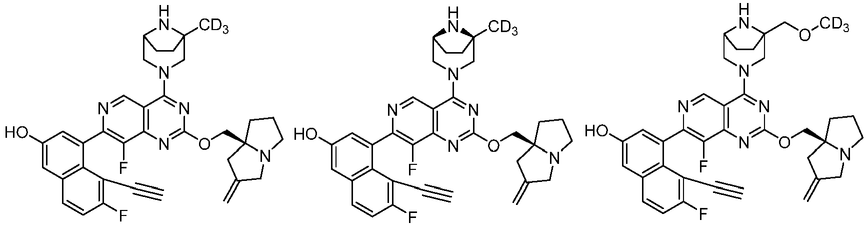

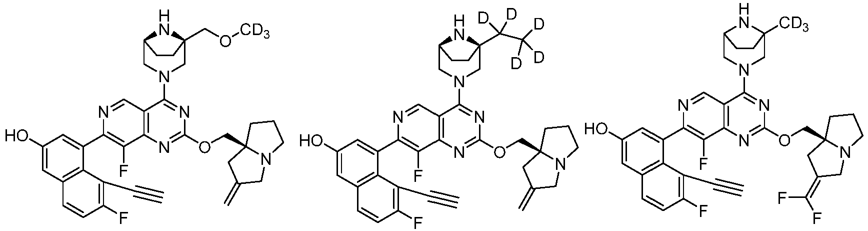

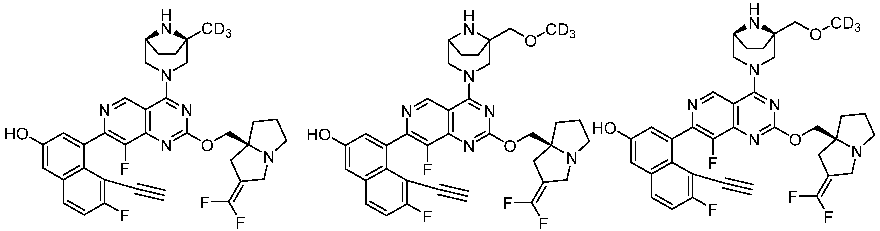

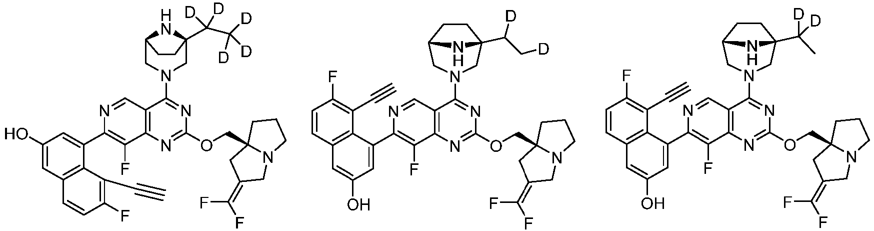

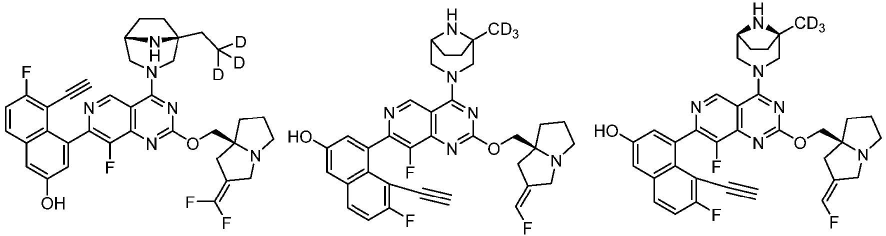

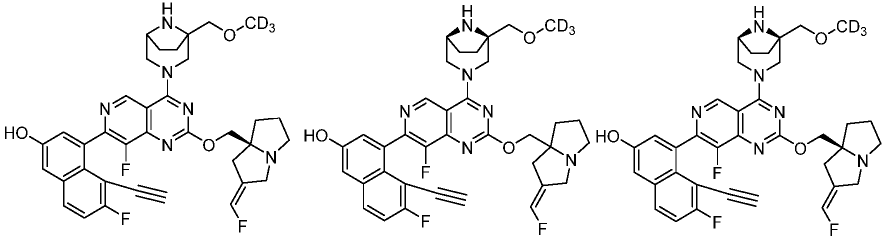

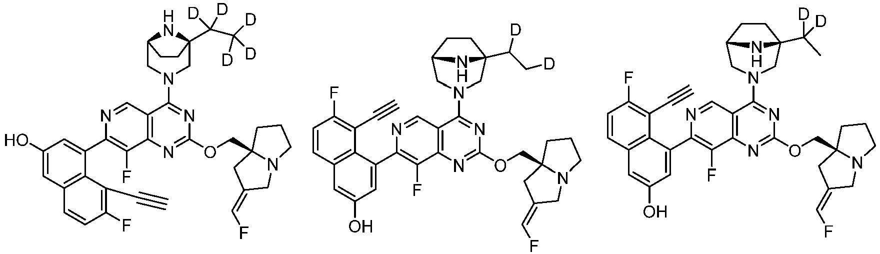

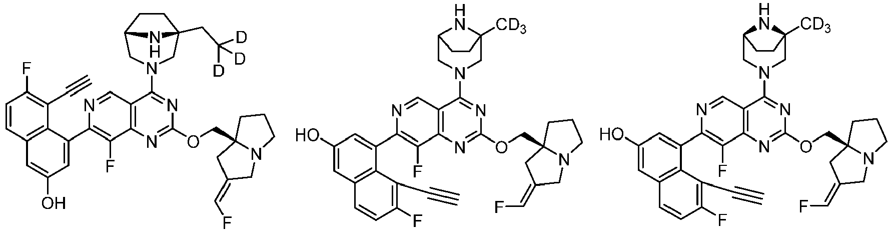

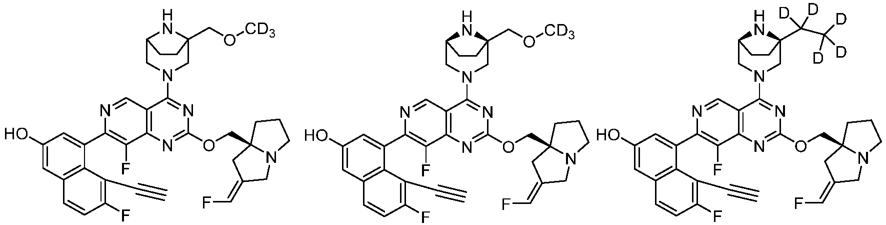

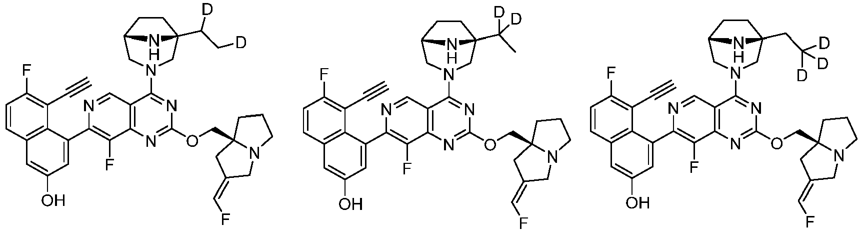

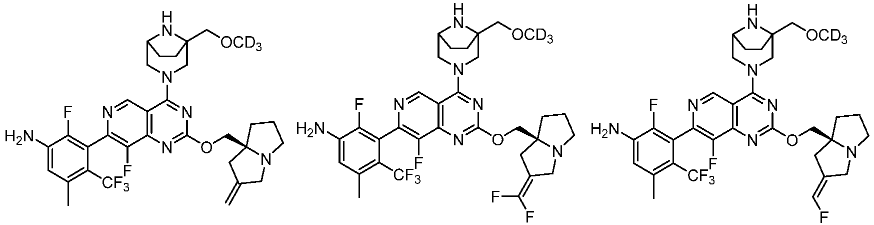

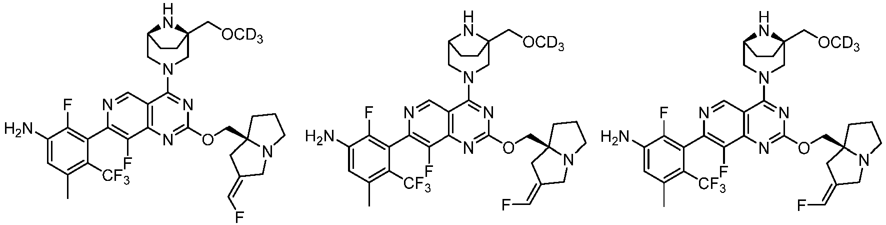

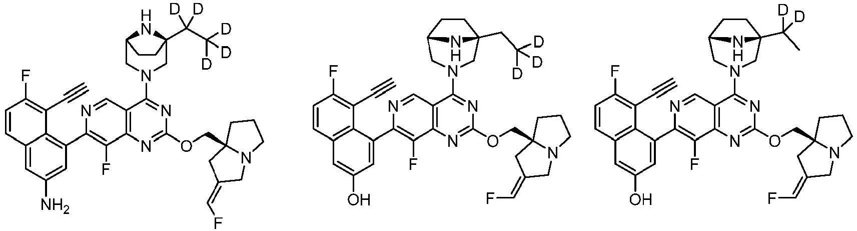

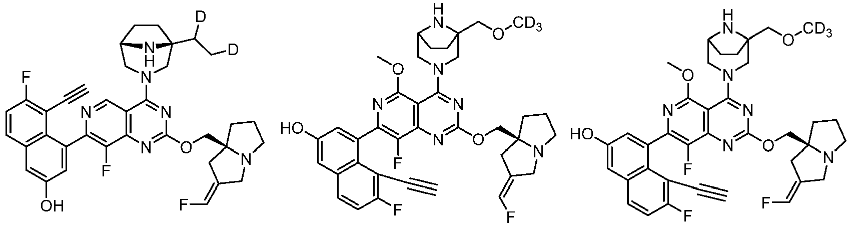

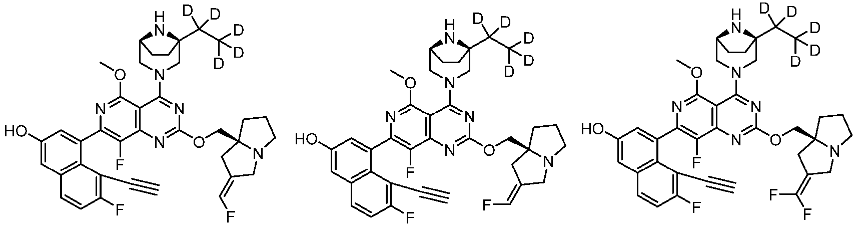

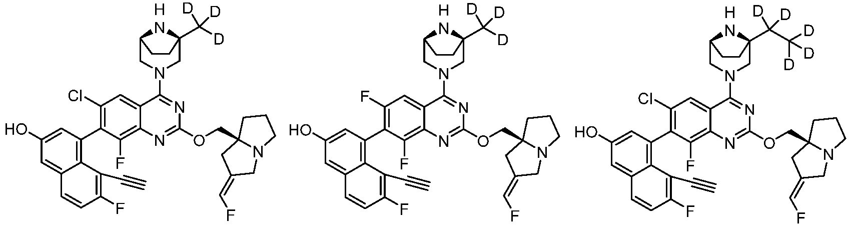

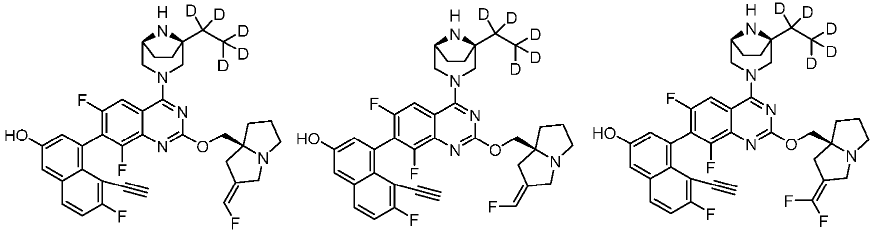

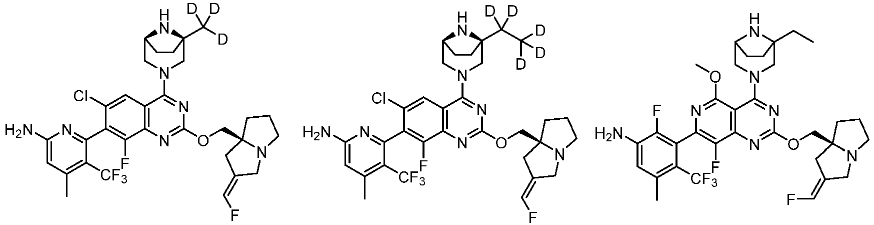

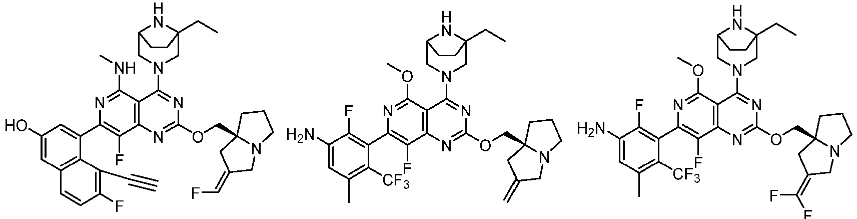

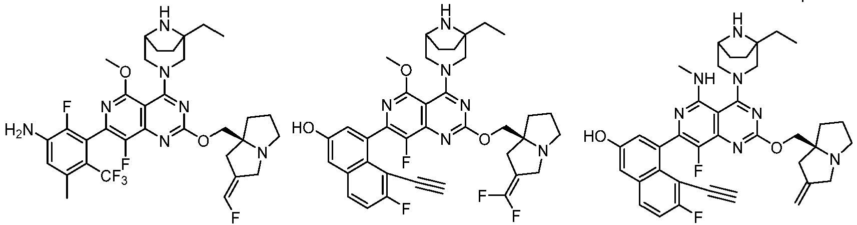

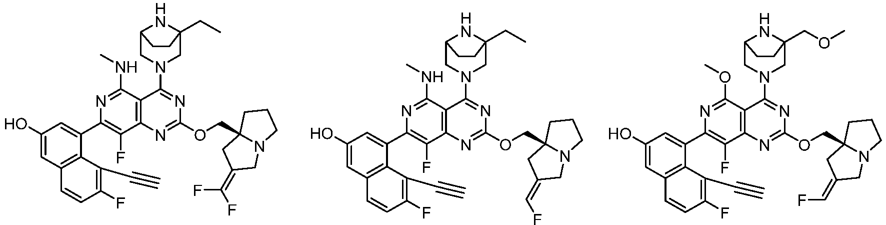

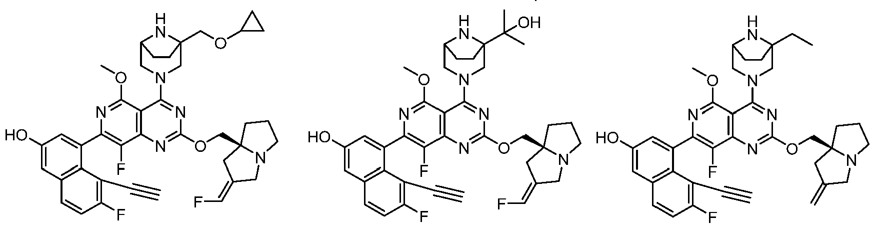

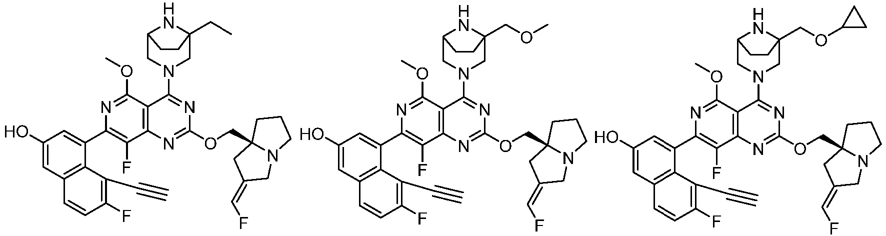

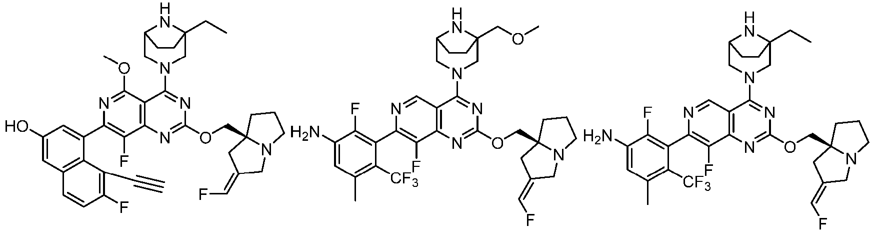

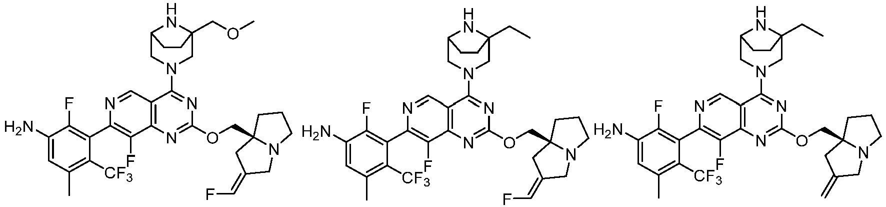

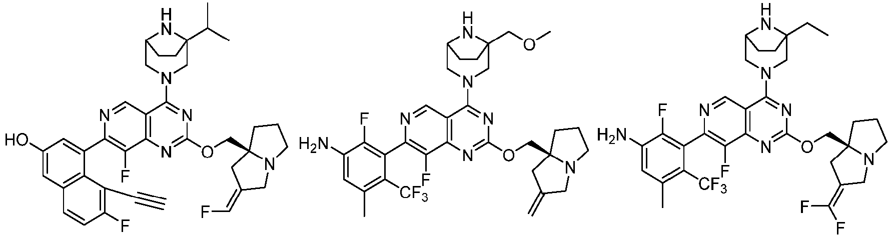

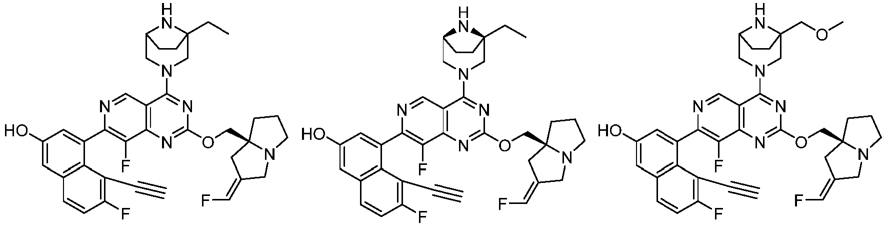

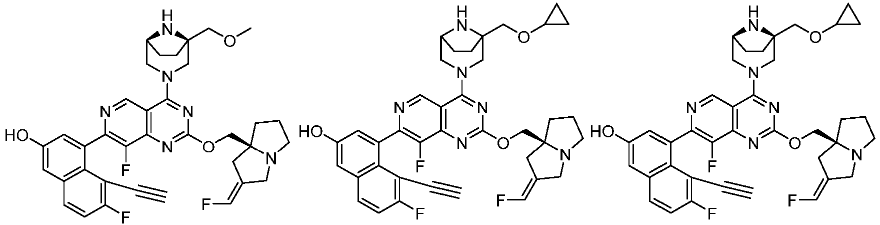

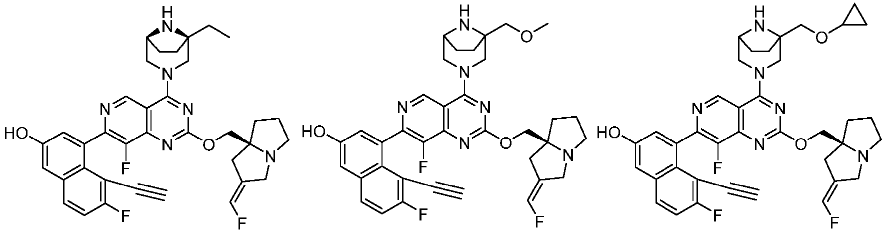

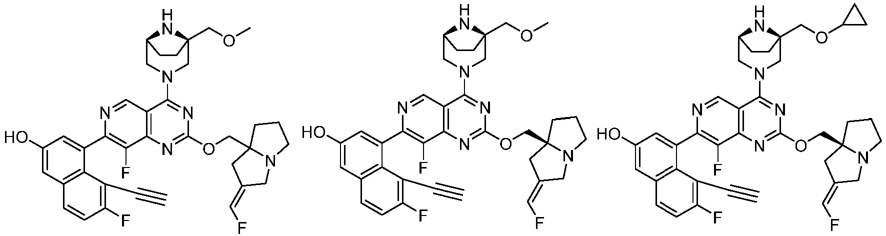

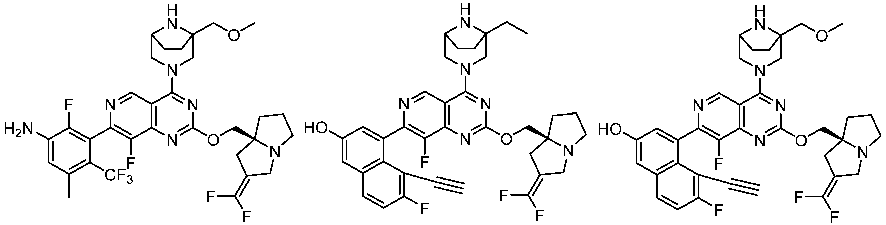

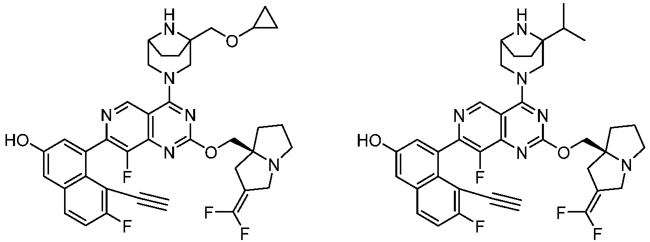

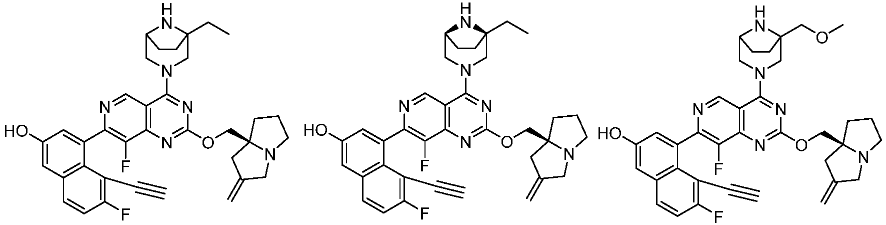

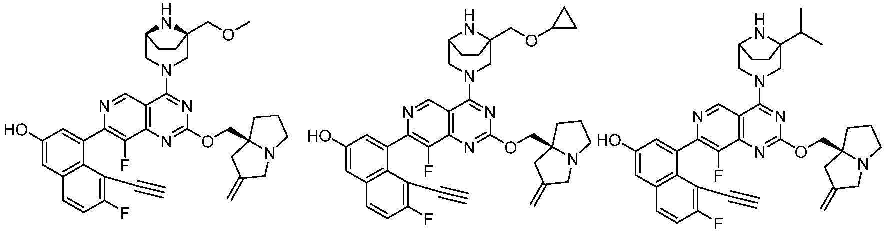

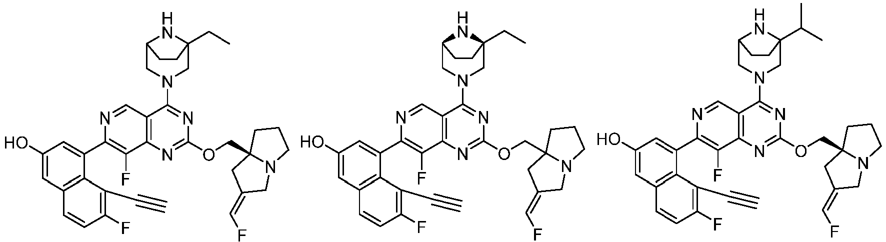

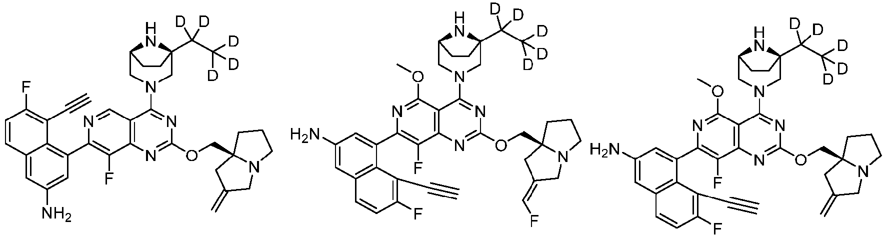

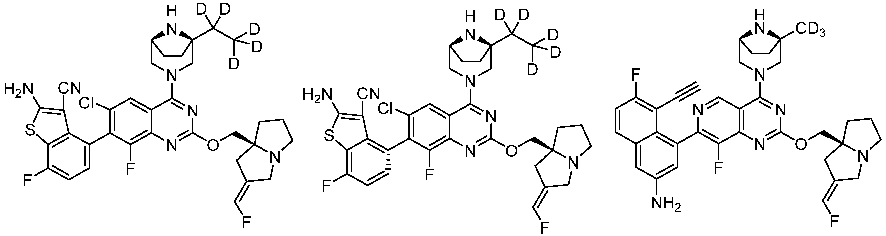

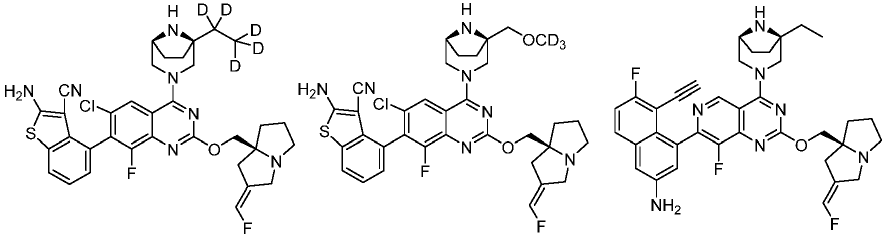

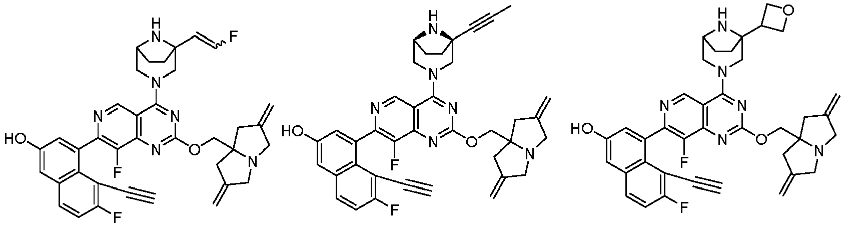

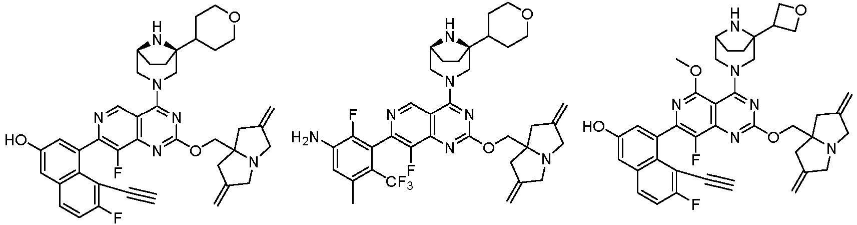

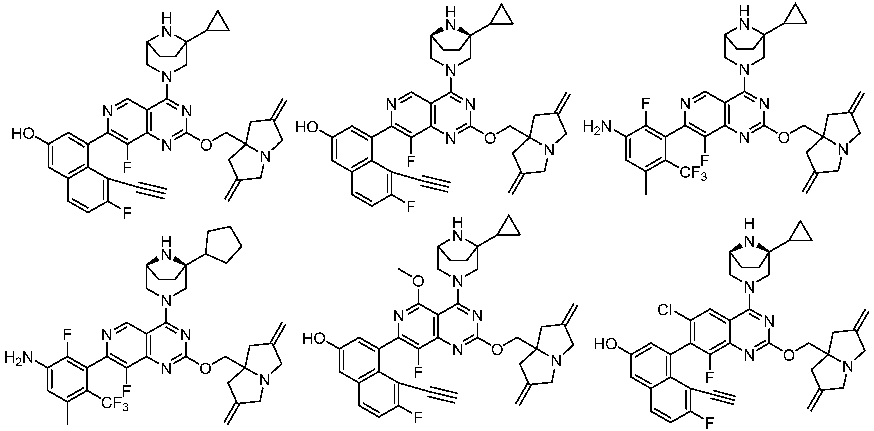

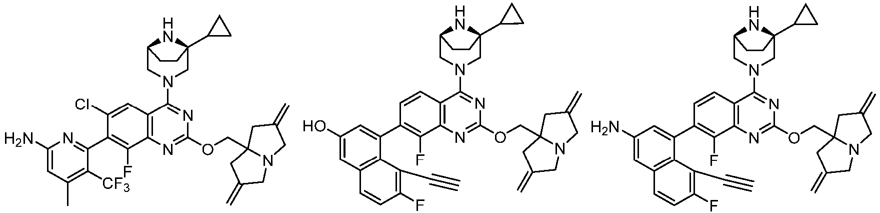

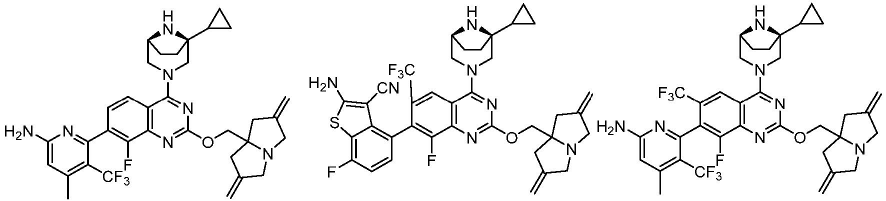

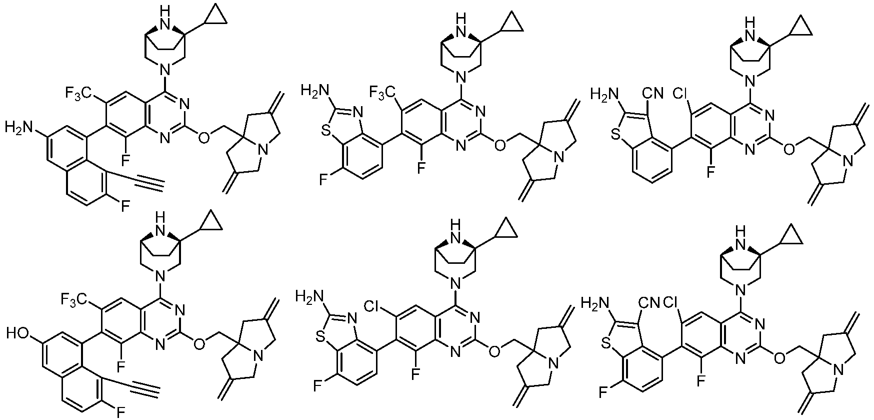

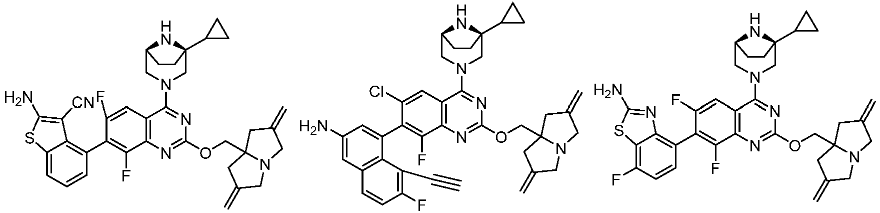

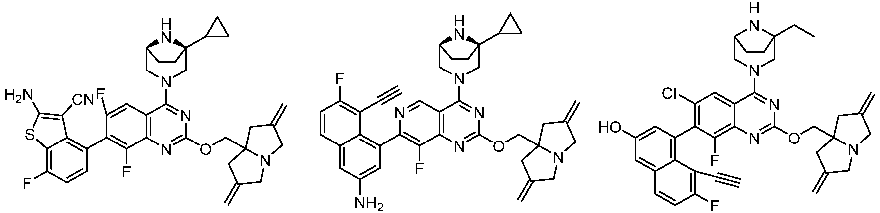

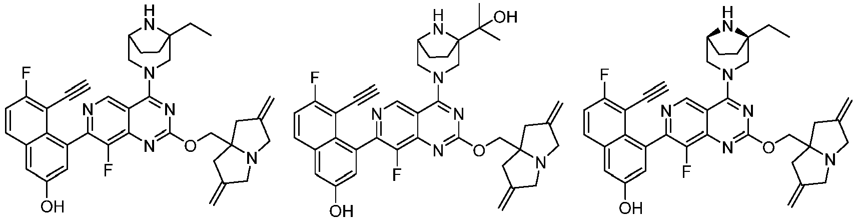

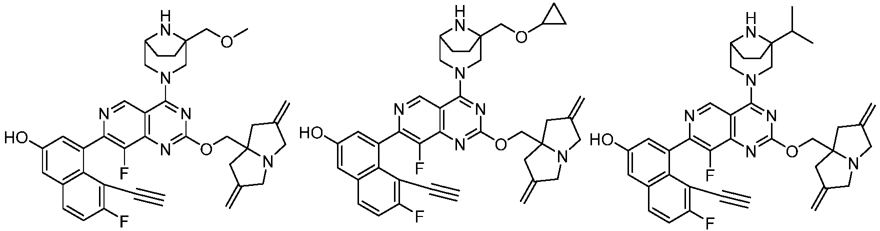

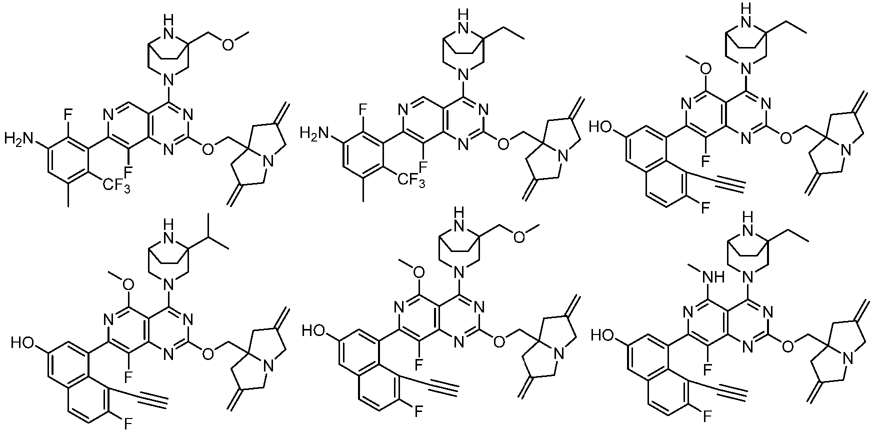

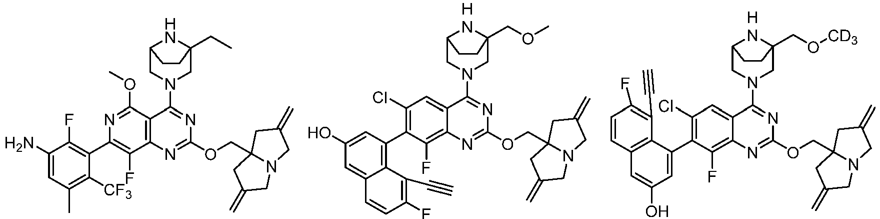

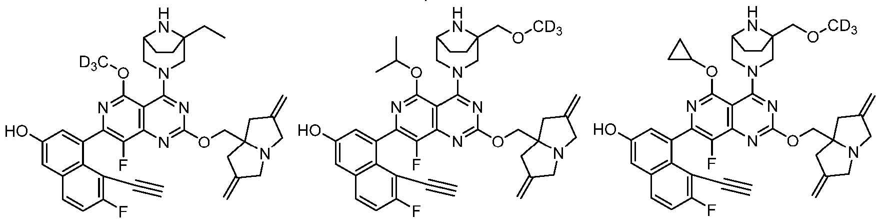

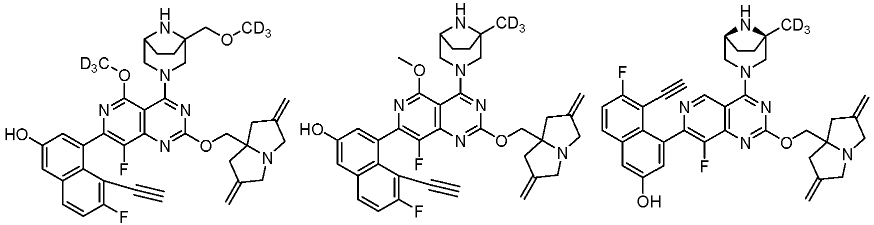

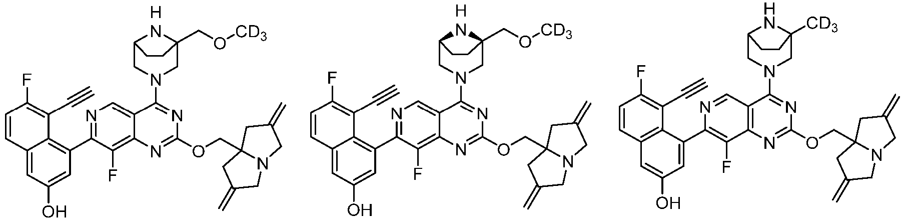

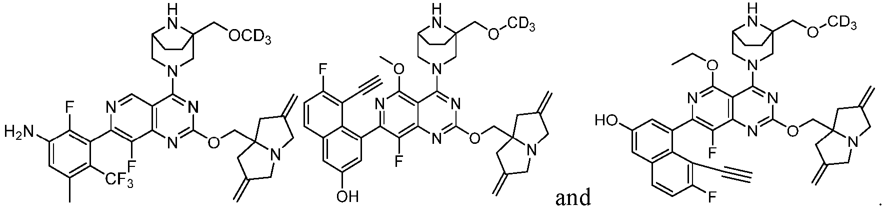

The second aspect of the present invention provides a process for preparing the compound of formula (I), the stereoisomer or pharmaceutically acceptable salt thereof, comprising the following step:

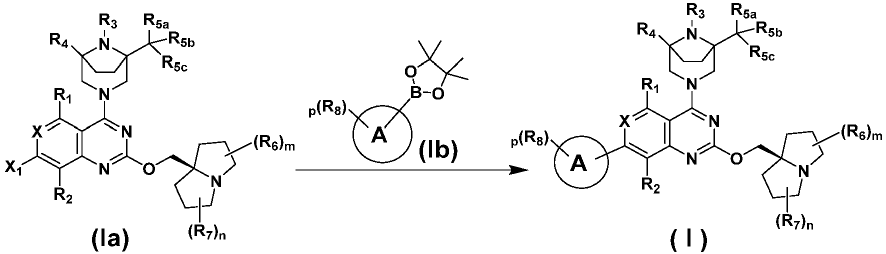

The compound of formula (Ia) or the acid salt thereof reacts with the compound of formula (Ib) or the acid salt thereof to form the compound of formula (I) or the acid salt thereof; or

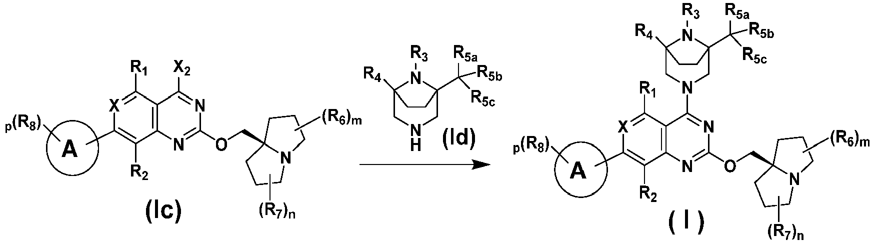

The compound of formula (Ic) or the acid salt thereof reacts with the compound of formula (Id) or the acid salt thereof to form the compound of formula (I) or the acid salt thereof, wherein, X₁ and X₂ are each independently fluorine, chlorine or bromine, and X, R₁, R₂, R₃, R₄, R₅ₐ, R_{5b}, R_{5c}, R₆, R₇, R₈, p, m and n are defined as in the compound of formula (I).

The third aspect of the present invention provides a pharmaceutical composition comprising the compound of formula (I), the stereoisomer or pharmaceutically acceptable salt thereof, and a pharmaceutically acceptable carrier.

The present invention also relates to the use of the compound of formula (I), the stereoisomer or pharmaceutically acceptable salt thereof in the preparation of a medicament for treating and/or preventing cancers or tumors related to KRAS G12D.

The present invention also relates to the use of the compound of formula (I), the stereoisomer or pharmaceutically acceptable salt thereof in the preparation of a medicament for treating and/or preventing KRAS G12D-related sarcoma (angiosarcoma, fibrosarcoma, rhabdomyosarcoma, liposarcoma), myxoma, rhabdomyoma, fibroma, lipoma, teratoma; bronchial carcinoma (squamous cell carcinoma, undifferentiated small cell carcinoma, undifferentiated large cell carcinoma, adenocarcinoma), alveolar (bronchiolar carcinoma) carcinoma, bronchial adenoma, lymphoma, chondromatoid hamartoma, mesothelioma; esophageal carcinoma (squamous cell carcinoma, adenocarcinoma, leiomyosarcoma, lymphoma), gastric carcinoma (lymphoma, leiomyosarcoma), pancreatic carcinoma (ductal adenocarcinoma, insulinoma, glucagonoma, gastrinoma, carcinoid tumor, vasoactive intestinal peptide tumor), small intestine cancer (adenocarcinoma, lymphoma, carcinoid tumor, Kaposi's sarcoma, leiomyoma, hemangioma, lipoma, neurofibroma, fibroma), colorectal carcinoma (adenocarcinoma, tubular adenoma, villous adenoma, hamartoma, leiomyoma); kidney cancer (adenocarcinoma, Wilms' tumor (nephroblastoma), lymphoma, leukemia), bladder cancer and urethral cancer (squamous cell carcinoma, transitional cell carcinoma, adenocarcinoma), prostate cancer (adenocarcinoma, sarcoma), testicular cancer (seminoma, teratoma, embryonal carcinoma, terato-epithelial cancer, choriocarcinoma, sarcoma, mesenchymal cell carcinoma, fibroma, fibroadenoma, adenomatoid tumor, lipoma); liver cancer (hepatocellular carcinoma), cholangiocarcinoma, hepatoblastoma, angiosarcoma, hepatocellular adenoma, hemangioma; gallbladder cancer, ampullary carcinoma, cholangiocarcinoma; osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochondroma (osteochondral exostosis), benign chondroma, chondroblastoma, chondromyxoid fibroma, osteoid osteoma and giant cell tumor; skull cancer (osteoma, hemangioma, granuloma, xanthoma, osteitis deformans), meningeal carcinomatosis (meningioma, meningeal sarcoma, gliomatosis), brain cancer (astrocytoma, medulloblastoma, glioma, ependymoma, blastoma (pineal tumor), glioblastoma multiforme, oligodendroglioma, neurilemmoma, retinoblastoma, congenital tumor, spinal neurofibroma, meningioma, glioma, sarcoma); uterine cancer (endometrial cancer (serous cystadenocarcinoma, mucinous cystadenocarcinoma, unclassified cancer), granulosa-theca cell tumor, Sertoli-Leydig cell tumor, dysgerminoma, malignant teratoma, vulvar cancer (squamous cell carcinoma, intraepithelial carcinoma, adenocarcinoma, fibrosarcoma, melanoma), vaginal cancer (clear cell carcinoma, squamous cell carcinoma, botryoid sarcoma (embryonal rhabdomyosarcoma)), fallopian tube (carcinoma); hematologic cancer (myelogenous leukemia (acute and chronic), acute lymphoblastic leukemia, chronic lymphocytic leukemia, myeloproliferative disease, multiple myeloma, myelodysplastic syndrome), Hodgkin's disease, non-Hodgkin's lymphoma (malignant lymphoma); malignant melanoma, basal cell carcinoma, squamous cell carcinoma, Kaposi's sarcoma, dysplastic nevus, lipoma, hemangioma, dermatofibroma, keloid, psoriasis, adrenal tumor or neuroblastoma.

The present invention also relates to the compound of formula (I), the stereoisomer or pharmaceutically acceptable salt thereof, for use as a drug.

The present invention also relates to the compound of formula (I), the stereoisomer or pharmaceutically acceptable salt thereof, for the treatment and/or prevention of cancers or tumors related to KRAS G12D.

The present invention also relates to the use of the compound of formula (I), the stereoisomer or pharmaceutically acceptable salt thereof for treating and/or preventing KRAS G12D-related sarcoma (angiosarcoma, fibrosarcoma, rhabdomyosarcoma, liposarcoma), myxoma, rhabdomyoma, fibroma, lipoma, teratoma; bronchial carcinoma (squamous cell carcinoma, undifferentiated small cell carcinoma, undifferentiated large cell carcinoma, adenocarcinoma), alveolar (bronchiolar carcinoma) carcinoma, bronchial adenoma, lymphoma, chondromatoid hamartoma, mesothelioma; esophageal carcinoma (squamous cell carcinoma, adenocarcinoma, leiomyosarcoma, lymphoma), gastric carcinoma (lymphoma, leiomyosarcoma), pancreatic carcinoma (ductal adenocarcinoma, insulinoma, glucagonoma, gastrinoma, carcinoid tumor, vasoactive intestinal peptide tumor), small intestine cancer (adenocarcinoma, lymphoma, carcinoid tumor, Kaposi's sarcoma, leiomyoma, hemangioma, lipoma, neurofibroma, fibroma), colorectal carcinoma (adenocarcinoma, tubular adenoma, villous adenoma, hamartoma, leiomyoma); kidney cancer (adenocarcinoma, Wilms' tumor (nephroblastoma), lymphoma, leukemia), bladder cancer and urethral cancer (squamous cell carcinoma, transitional cell carcinoma, adenocarcinoma), prostate cancer (adenocarcinoma, sarcoma), testicular cancer (seminoma, teratoma, embryonal carcinoma, terato-epithelial cancer, choriocarcinoma, sarcoma, mesenchymal cell carcinoma, fibroma, fibroadenoma, adenomatoid tumor, lipoma); liver cancer (hepatocellular carcinoma), cholangiocarcinoma, hepatoblastoma, angiosarcoma, hepatocellular adenoma, hemangioma; gallbladder cancer, ampullary carcinoma, cholangiocarcinoma; osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochondroma (osteochondral exostosis), benign chondroma, chondroblastoma, chondromyxoid fibroma, osteoid osteoma and giant cell tumor; skull cancer (osteoma, hemangioma, granuloma, xanthoma, osteitis deformans), meningeal carcinomatosis (meningioma, meningeal sarcoma, gliomatosis), brain cancer (astrocytoma, medulloblastoma, glioma, ependymoma, blastoma (pineal tumor), glioblastoma multiforme, oligodendroglioma, neurilemmoma, retinoblastoma, congenital tumor, spinal neurofibroma, meningioma, glioma, sarcoma); uterine cancer (endometrial cancer (serous cystadenocarcinoma, mucinous cystadenocarcinoma, unclassified cancer), granulosa-theca cell tumor, Sertoli-Leydig cell tumor, dysgerminoma, malignant teratoma), vulvar cancer (squamous cell carcinoma, intraepithelial carcinoma, adenocarcinoma, fibrosarcoma, melanoma), vaginal cancer (clear cell carcinoma, squamous cell carcinoma, botryoid sarcoma (embryonal rhabdomyosarcoma)), fallopian tube (carcinoma); hematologic cancer (myelogenous leukemia (acute and chronic), acute lymphoblastic leukemia, chronic lymphocytic leukemia, myeloproliferative disease, multiple myeloma, myelodysplastic syndrome), Hodgkin's disease, non-Hodgkin's lymphoma (malignant lymphoma); malignant melanoma, basal cell carcinoma, squamous cell carcinoma, Kaposi's sarcoma, dysplastic nevus, lipoma, hemangioma, dermatofibroma, keloid, psoriasis, adrenal tumor or neuroblastoma.

The present invention also relates to a method for treating and/or preventing cancers or tumors related to KRAS G12D, comprising administering to a patient in need a therapeutically effective amount of the compound of formula (I), the stereoisomer or pharmaceutically acceptable salt thereof.

The present invention also relates to a method for treating and/or preventing KRAS G12D-related sarcoma (angiosarcoma, fibrosarcoma, rhabdomyosarcoma, liposarcoma), myxoma, rhabdomyoma, fibroma, lipoma, teratoma; bronchial carcinoma (squamous cell carcinoma, undifferentiated small cell carcinoma, undifferentiated large cell carcinoma, adenocarcinoma), alveolar (bronchiolar carcinoma) carcinoma, bronchial adenoma, lymphoma, chondromatoid hamartoma, mesothelioma; esophageal carcinoma (squamous cell carcinoma, adenocarcinoma, leiomyosarcoma, lymphoma), gastric carcinoma (lymphoma, leiomyosarcoma), pancreatic carcinoma (ductal adenocarcinoma, insulinoma, glucagonoma, gastrinoma, carcinoid tumor, vasoactive intestinal peptide tumor), small intestine cancer (adenocarcinoma, lymphoma, carcinoid tumor, Kaposi's sarcoma, leiomyoma, hemangioma, lipoma, neurofibroma, fibroma), colorectal carcinoma (adenocarcinoma, tubular adenoma, villous adenoma, hamartoma, leiomyoma); kidney cancer (adenocarcinoma, Wilms' tumor (nephroblastoma), lymphoma, leukemia), bladder cancer and urethral cancer (squamous cell carcinoma, transitional cell carcinoma, adenocarcinoma), prostate cancer (adenocarcinoma, sarcoma), testicular cancer (seminoma, teratoma, embryonal carcinoma, terato-epithelial cancer, choriocarcinoma, sarcoma, mesenchymal cell carcinoma, fibroma, fibroadenoma, adenomatoid tumor, lipoma); liver cancer (hepatocellular carcinoma), cholangiocarcinoma, hepatoblastoma, angiosarcoma, hepatocellular adenoma, hemangioma; gallbladder cancer, ampullary carcinoma, cholangiocarcinoma; osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochondroma (osteochondral exostosis), benign chondroma, chondroblastoma, chondromyxoid fibroma, osteoid osteoma and giant cell tumor; skull cancer (osteoma, hemangioma, granuloma, xanthoma, osteitis deformans), meningeal carcinomatosis (meningioma, meningeal sarcoma, gliomatosis), brain cancer (astrocytoma, medulloblastoma, glioma, ependymoma, blastoma (pineal tumor), glioblastoma multiforme, oligodendroglioma, neurilemmoma, retinoblastoma, congenital tumor, spinal neurofibroma, meningioma, glioma, sarcoma); uterine cancer (endometrial cancer (serous cystadenocarcinoma, mucinous cystadenocarcinoma, unclassified cancer), granulosa-theca cell tumor, Sertoli-Leydig cell tumor, dysgerminoma, malignant teratoma), vulvar cancer (squamous cell carcinoma, intraepithelial carcinoma, adenocarcinoma, fibrosarcoma, melanoma), vaginal cancer (clear cell carcinoma, squamous cell carcinoma, botryoid sarcoma (embryonal rhabdomyosarcoma)), fallopian tube (carcinoma); hematologic cancer (myelogenous leukemia (acute and chronic), acute lymphoblastic leukemia, chronic lymphocytic leukemia, myeloproliferative disease, multiple myeloma, myelodysplastic syndrome), Hodgkin's disease, non-Hodgkin's lymphoma (malignant lymphoma); malignant melanoma, basal cell carcinoma, squamous cell carcinoma, Kaposi's sarcoma, dysplastic nevus, lipoma, hemangioma, dermatofibroma, keloid, psoriasis, adrenal tumor or neuroblastoma, comprising administering to a patient in need a therapeutically effective amount of the compound of formula (I), the stereoisomer or pharmaceutically acceptable salt thereof.

### DETAILED DESCRIPTION OF EMBODIMENTS

After an extensive and intensive research, the inventors of the present invention develop KRAS G12D inhibitors with the structure of formula (I) for the first time. The series of compounds of the present invention can be widely applied in the preparation of a medicament for the treatment and/or prevention of cancers or tumors related to KRAS G12D, and are expected to be developed into a new generation of KRAS G12D inhibitors. The present invention is achieved on this basis.

Detailed description: unless otherwise stated or specified, the following terms used in the specification and claims have the following meanings.

"Alkyl" refers to linear or branched saturated aliphatic alkyl groups, preferably including a linear or branched alkyl having 1 to 10 or 1 to 6 carbon atoms or 1 to 4 carbon atoms, including but not limited to methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or various branched isomers thereof, etc. "C₁₋₁₀ alkyl" means a linear or branched alkyl containing 1 to 10 carbon atoms, "C₁₋₄ alkyl" means a linear or branched alkyl containing 1 to 4 carbon atoms, "C₁₋₃ alkyl" means a linear or branched alkyl containing 1 to 3 carbon atoms, "C₀₋₈ alkyl" means a linear or branched alkyl containing 0 to 8 carbon atoms, "C₀₋₄ alkyl" means a linear or branched alkyl containing 0 to 4 carbon atoms.

Alkyl can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Alkylene" refers to a group of the formula "=R", wherein "R" is a group obtained after two hydrogen atoms are removed from the "alkyl" that is defined as above. Preferably, it includes a group which is obtained after two hydrogen atoms are removed from a linear or branched alkyl containing 1 to 10 or 1 to 6 carbon atoms or 1 to 4 carbon atoms, including but not limited to methylene ( = CH₂), ethylene ( = CHCH₃), hexylene (= CH(CH₂)₄CH₃), etc. For example, in the following compound: 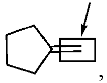 the group within the rectangle box indicated by the arrow is methylene.

Alkylene can be optionally substituted or unsubstituted, and when it is substituted, the substituent is preferably one or more (preferably 1, 2, 3 or 4) of the groups independently selected from the group consisting of deuterium, halogen, cyano, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Cycloalkyl" or "carbocyclic ring" refers to monocyclic or polycyclic hydrocarbon substituents that are saturated or partially unsaturated, wherein the partially unsaturated cyclic hydrocarbon refers to a cyclic hydrocarbon that may contain one or more (preferably 1, 2 or 3) double bonds, but none of the rings have a fully conjugated π-electron system. Cycloalkyl may be monocyclic cycloalkyl or polycyclic cycloalkyl, preferably including a cycloalkyl containing 3 to 12 or 3 to 8 or 3 to 6 carbon atoms. For example, "C₃₋₁₂ cycloalkyl" means a cycloalkyl having 3 to 12 carton atoms, "C₃₋₁₀ cycloalkyl" means a cycloalkyl having 3 to 10 carton atoms, "C₃₋₈ cycloalkyl" means a cycloalkyl having 3 to 8 carton atoms, and "C₃₋₆ cycloalkyl" means a cycloalkyl having 3 to 6 carton atoms, wherein:
monocyclic cycloalkyl includes but is not limited to cyclopropyl, cyclobutyl, cyclopentyl, cyclopentenyl, cyclohexyl, cyclohexenyl, cyclohexadienyl, cycloheptyl, cycloheptarienyl, cyclooctyl, etc.

Polycyclic cycloalkyl includes spirocycloalkyl, fused cycloalkyl and bridged cycloalkyl. "Spirocycloalkyl" refers to a polycyclic group in which a carton atom (called spiro-atom) is shared among monocyclic rings, wherein these rings may contain one or more (preferably 1, 2 or 3) double bonds, but none of them have a fully conjugated π-electron system. According to the number of the spiro-atoms shared among the rings, the spirocycloalkyl may be monospirocycloalkyl, bispirocycloalkyl or polyspirocycloalkyl, including but not limited to:

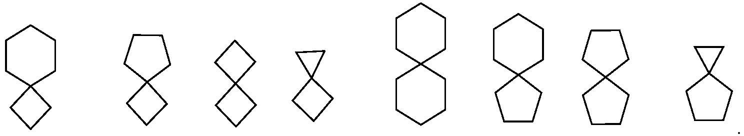

"Fused cycloalkyl" refers to an all-carbon polycyclic group in which each ring shares a pair of adj acent carbon atoms with other rings in the system, wherein one or more of the rings may contain one or more (preferably 1, 2 or 3) double bonds, but none of them have a fully conjugated π-electron system. According to the number of formed rings, the fused cycloalkyl may be bicyclic, tricyclic, tetracyclic or polycyclic, including but not limited to:

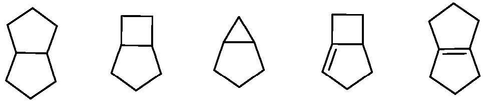

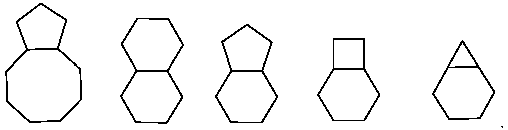

"Bridged cycloalkyl" refers to an all-carbon polycyclic group in which any two rings share two carbon atoms that are not directly connected to each other, wherein these rings may contain one or more (preferably 1, 2 or 3) double bonds, but none of them have a fully conjugated π-electron system. According to the number of formed rings, the bridged cycloalkyl may be bicyclic, tricyclic, tetracyclic or polycyclic, including but not limited to:

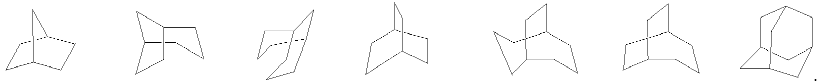

The cycloalkyl ring may be fused to an aryl, heteroaryl or heterocycloalkyl ring, wherein the ring attached to the parent structure is cycloalkyl, which includes, but is not limited to, indenyl, tetrahydronaphthyl, benzocycloheptyl, etc.

Cycloalkyl can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R_{15,} -C₀₋₈ alkyl-C(O)NR₁₆R₁₇ and -C₀₋₈ alkyl-N(R₁₆)-C(O)R₁₅.

"Cycloalkylene" refers to a group of the formula " = Rₐ", wherein "Rₐ" is a group obtained after two hydrogen atoms are removed from the "cycloalkyl" that is defined as above. Preferably, it includes a group which is obtained after two hydrogen atoms are removed from cycloalkyl containing 3 to 12 or 3 to 8 carbon atoms or 3 to 6 carbon atoms, including but not limited to cyclopropylene 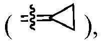 cyclobutylene ( 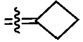 ), cyclopentylene ( 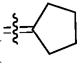 ), etc. For example, in the following compound: 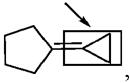 the group within the rectangle box indicated by the arrow is cyclopropylene.

Cycloalkylene can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Heterocyclyl" or "heterocycle" refers to a monocyclic or polycyclic hydrocarbon substituent that is saturated or partially unsaturated, wherein the partially unsaturated cyclic hydrocarbon refers to a cyclic hydrocarbon that may contain one or more (preferably 1, 2 or 3) double bonds, but none of the rings have a fully conjugated π-electron system, wherein one or more (preferably 1, 2, 3 or 4) of the rings atoms in heterocyclyl are heteroatoms selected from N, O, N•O or S(O)ᵣ (wherein r is an integer of 0, 1 or 2), excluding ring portions of -O-O-, -O-S- or -S-S-, and the remaining ring atoms are carton atoms. Preferably, it includes heterocyclyl containing 3 to 12 or 3 to 10 or 3 to 8 or 3 to 6 ring atoms. For example, "3-6 membered heterocyclyl" means a heterocyclyl containing 3 to 6 ring atoms, "3-8 membered heterocyclyl" means a heterocyclyl containing 3 to 8 ring atoms, "3-10 membered heterocyclyl" means a heterocyclyl containing 3 to 10 ring atoms, "4-8 membered heterocyclyl" means a heterocyclyl containing 4 to 8 ring atoms, "4-10 membered heterocyclyl" means a heterocyclyl containing 4 to 10 ring atoms, "5-8 membered heterocyclyl" means a heterocyclyl containing 5 to 8 ring atoms, and "3-12 membered heterocyclyl" means a heterocyclyl containing 3 to 12 ring atoms.

Monocyclic heterocyclyl includes but is not limited to pyrrolidinyl, piperidinyl, piperazinyl, morpholinyl, thiomorpholinyl, homopiperazinyl, oxacyclobutyl, tetrahydrofuranyl, etc.

Polycyclic heterocyclyl includes spiroheterocyclyl, fused heterocyclyl and bridged heterocyclyl. "Spiroheterocyclyl" refers to a polycyclic heterocyclyl group in which an atom (called spiro-atom) is shared among monocyclic rings, wherein one or more (preferably 1, 2, 3 or 4) of the ring atoms are heteroatoms selected from N, O, N•O or S(O)ᵣ (wherein r is an integer of 0, 1 or 2), and the remaining ring atoms are carbon atoms. These rings may contain one or more (preferably 1, 2 or 3) double bonds, but none of them have a fully conjugated π-electronic system. According to the number of sprio-atoms shared among the rings, spiroheterocyclyl may be monospiroheterocyclyl, bispiroheterocyclyl or polyspiroheterocyclyl. Spiroheterocyclyl includes but is not limited to:

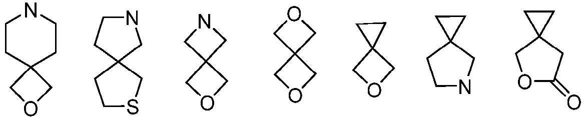

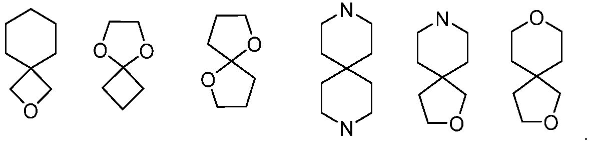

"Fused heterocyclyl" refers to a polycyclic heterocyclyl in which each ring shares a pair of adj acent atoms with the other rings in the system, where one or more (preferably 1, 2, 3 or 4) of the rings may contain one or more (preferably 1, 2 or 3) double bonds, but none of them have a fully conjugated π-electron system, where one or more (preferably 1, 2, 3 or 4) of the ring atoms are heteroatoms selected from N, O, N•O or S(O)ᵣ (where r is an integer of 0, 1 or 2), and the remaining ring atoms are carbon atoms. According to the number of formed rings, the fused heterocyclyl may be bicyclic, tricyclic, tetracyclic or polycyclic, including but not limited to:

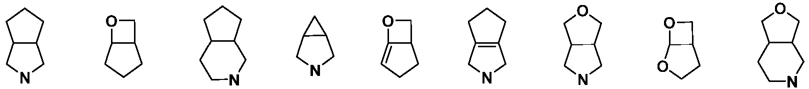

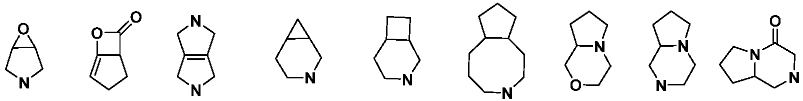

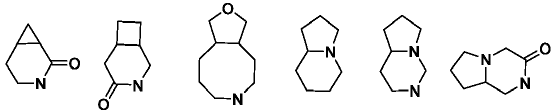

"Bridged heterocyclyl" refers to a polycyclic heterocyclyl in which any two rings share two carton atoms that are not directly attached to each other, wherein these rings may contain one or more (preferably 1, 2, or 3) double bonds, but none of them have a fully conjugated π-electron system, wherein one or more (preferably 1, 2, 3, or 4) of the ring atoms are heteroatoms selected from N, O, N atom, or S(O)ᵣ (wherein r is an integer of 0, 1 or 2), and the remaining ring atoms are carbon atoms. According to the number of formed rings, the bridged heterocyclyl may be bicyclic, tricyclic, tetracyclic or polycyclic, including but not limited to:

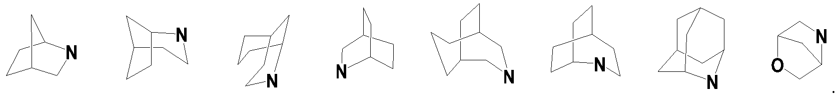

The heterocyclyl ring may be fused to an aryl, heteroaryl or cycloalkyl ring, wherein the ring attached to the parent structure is heterocyclyl, which includes but is not limited to:

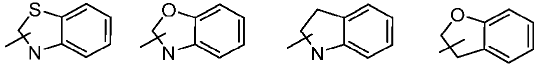

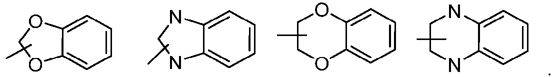

Heterocyclyl can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Heterocycloalkylene" refers to a group of the formula " = R_{b}", wherein "R_{b}" is a group obtained after two hydrogen atoms are removed from the "heterocycloalkyl" that is defined as above. Preferably, it includes a group which is obtained after two hydrogen atoms are removed from heterocycloalkyl containing 3 to 12 or 3 to 8 carbon atoms or 3 to 6 carbon atoms, including but not limited to oxacyclopropylene ( 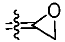 ), azacyclopropylene ( 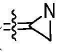 ), etc. For example, in the following compound: 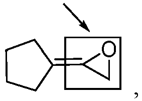 the group within the rectangle box indicated by the arrow is oxacyclopropylene.

Heterocycloalkylene can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Aryl" or "aromatic ring" means an all-carbon monocyclic or fused-polycyclic group (i.e., rings that share a pair of adjacent carbon atoms) and a polycyclic group having a conjugated π-electron system (i.e., rings with adjacent pairs of carbon atoms), preferably including all-carbon aryl containing 6-10 or 6-8 carbon atoms. For example, "C₆₋₁₀ aryl" means an all-carbon aryl containing 6-10 carbon atoms, including but not limited to phenyl and naphthyl, and "C₆₋₈ aryl" means an all-carbon aryl containing 6-8 carbon atoms. The aryl ring may be fused to an heteroaryl, heterocyclyl or cycloalkyl ring, wherein the ring attached to the parent structure is aryl ring, including but not limited to:

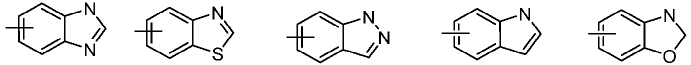

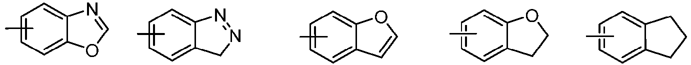

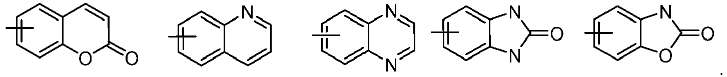

"Aryl" can be substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Heteroaryl" refers to a heteroaromatic system containing one or more (preferably 1, 2, 3 or 4) of the heteroatoms, where the heteroatoms include heteroatoms selected from N, O, N systems and S(O)r (where r is an integer of 0, 1 or 2), preferably including a heteroaromatic system containing 5-10 or 5-8 or 5-6 ring atoms. For example, "5-8 membered heteroaryl" means a heteroaromatic system containing 5-8 ring atoms, and "5-10 membered heteroaryl" means a heteroaromatic system containing 5-10 ring atoms, including but not limited to furyl, thiophenyl, pyridyl, pyrrolyl, N-alkylpyrrolyl, pyrimidinyl, pyrazinyl, imidazolyl, tetrazolyl, etc. The heteroaryl ring may be fused to an aryl, heterocyclyl or cycloalkyl ring, wherein the ring attached to the parent structure is heteroaryl ring, including but not limited to:

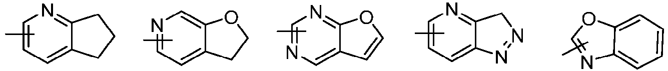

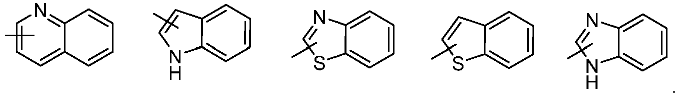

"Heteroaryl" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Alkenyl" refers to an alkyl defined as above consisting of at least two carbon atoms and at least one carbon-carbon double bond, preferably including a linear or branched alkenyl containing 2-10 or 2-4 carbon atoms. For example, "C₂₋₁₀ alkenyl" means a linear or branched alkenyl containing 2-10 carbon atoms, and "C₂₋₄ alkenyl" means a linear or branched alkenyl containing 2-4 carbon atoms. The alkenyl includes, but is not limited to, vinyl, 1-propenyl, 2-propenyl, 1-,2-, or 3-butenyl, etc.

"Alkenyl" can be substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Alkynyl" refers to an alkyl defined as above consisting of at least two carbon atoms and at least one carbon-carbon triple bond, preferably including a linear or branched alkynyl containing 2-10 or 2-4 carbon atoms. For example, "C₂₋₁₀ alkynyl" means a linear or branched alkynyl containing 2-10 carbon atoms, and "C₂₋₄ alkynyl" means a linear or branched alkynyl containing 2-4 carbon atoms. The alkynyl includes, but is not limited to, ethynyl, 1-propynyl, 2-propynyl, 1-,2-, or 3-butynyl, etc.

"Alkynyl" can be substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Alkoxy" refers to an -O-alkyl group, wherein the alkyl is defined as above. For example, "C₁₋₁₀ alkoxy" means an alkyloxy containing 1-10 carbon atoms, "C₁₋₄ alkoxy" means an alkyloxy containing 1-4 carbon atoms, and "C₁₋₂ alkoxy" means an alkyloxy containing 1-2 carbon atoms, including but not limited to, methoxy, ethoxy, propoxy, butoxy, etc.

"Alkoxy" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Cycloalkoxy" or "cycloalkyloxy" refers to an -O-cycloalkyl group, wherein cycloalkyl is defined as above. For example, "C₃₋₁₂ cycloalkoxy" means a cycloalkyloxy containing 3-12 carbon atoms, and "C₃₋₆ cycloalkoxy" means a cycloalkyloxy containing 3-6 carbon atoms, including but not limited to cyclopropoxy, cyclobutoxy, cyclopentoxy, cyclohexoxy, etc.

"Cycloalkoxy" or "cycloalkyloxy" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Heterocycloxy" or "heterocyclyloxy" refers to an -O-heterocyclyl group, wherein the heterocyclyl is defined as above, including but not limited to, azetidyloxy, oxetanyloxy, azetidyloxy, nitrogen, oxetanyloxy, etc.

"Heterocycloxy" or "heterocyclyloxy" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"C₁₋₁₀ alkanoyl" refers to a monovalent atomic group which is obtained after a hydroxy is removed from C₁₋₁₀ alkyl acid, and is also generally referred to as "C₀₋₉ alkyl-C(O)-". For example, "C₁ alkyl-C(O)-" refers to acetyl; "C₂ alkyl-C(O)-" refers to propanoyl; "C₃ alkyl-C(O)-" refers to butanoyl or isobutanoyl.

"-C₀₋₈ alkyl-S(O)(=N-R₁₀)R₁₁" refers to the sulfur atom in -S(O)(=N-R₁₀)R₁₁ attached to C₀₋₈ alkyl, wherein the C₀₋₈ alkyl is defined as above.

"-C₀₋₈ alkyl-N=S(O)R₁₁R₁₂" refers to the nitrogen atom in -N=S(O)R₁₁R₁₂ attached to C₀₋₈ alkyl, wherein the C₀₋₈ alkyl is defined as above.

"-C₀₋₈ alkyl-N=SR₁₁R₁₂" refers to the nitrogen atom in -N=SR₁₁R₁₂ attached to C₀₋₈ alkyl, wherein the C₀₋₈ alkyl is defined as above.

"-C₀₋₈ alkyl-O-S(O)₂R₁₃" refers to the oxygen atom in -O-S(O)₂R₁₃ attached to C₀₋₈ alkyl, wherein the C₀₋₈ alkyl is defined as above.

"-C₀₋₈ alkyl-S(O)ᵣR₁₃" refers to the sulfur atom in -S(O)ᵣR₁₃ attached to C₀₋₈ alkyl, wherein the C₀₋₈ alkyl is defined as above.

"-C₀₋₈ alkyl-O-R₁₄" refers to the oxygen atom in -O-R₁₄ attached to C₀₋₈ alkyl, wherein the C₀₋₈ alkyl is defined as above.

"-C₀₋₈ alkyl-C(O)OR₁₄" refers to the carbonyl in -C(O)OR₁₄ attached to C₀₋₈ alkyl, wherein the C₀₋₈ alkyl is defined as above.

"-C₀₋₈ alkyl-C(O)SR₁₄" refers to the carbonyl in -C(O)SR₁₄ attached to C₀₋₈ alkyl, wherein the C₀₋₈ alkyl is defined as above.

"-C₀₋₈ alkyl-S-C(O)R₁₅" refers to the sulfur atom in -S-C(O)R₁₅ attached to C₀₋₈ alkyl, wherein the C₀₋₈ alkyl is defined as above.

"-C₀₋₈ alkyl-C(O)R₁₅" refers to the carbonyl in -C(O)R₁₅ attached to C₀₋₈ alkyl, wherein the C₀₋₈ alkyl is defined as above.

"-C₀₋₈ alkyl-O-C(O)R₁₅" refers to the oxygen atom in -O-C(O)R₁₅ attached to C₀₋₈ alkyl, wherein the C₀₋₈ alkyl is defined as above.

"-C₀₋₈ alkyl-P(O)(R₁₅)₂" refers to the phosphorus atom in -P(O)(R₁₅)₂ attached to C₀₋₈ alkyl, wherein the C₀₋₈ alkyl is defined as above.

"-C₀₋₈ alkyl-NR₁₆R₁₇" refers to the nitrogen atom in -NR₁₆R₁₇ attached to C₀₋₈ alkyl, wherein the C₀₋₈ alkyl is defined as above.

"-C₀₋₈ alkyl-C(=NR₁₆)R₁₅" refers to the carbon atom in -C(=NR₁₆)R₁₅ attached to C₀₋₈ alkyl, wherein the C₀₋₈ alkyl is defined as above.

"-C₀₋₈ alkyl-N(R₁₆)-C(=NR₁₇)R₁₅" refers to the nitrogen atom in -N(R₁₆)-C(=NR₁₇)R₁₅ attached to C₀₋₈ alkyl, wherein the C₀₋₈ alkyl is defined as above.

"-C₀₋₈ alkyl-C(O)NR₁₆R₁₇" refers to the carbonyl in -C(O)NR₁₆R₁₇ attached to C₀₋₈ alkyl, wherein the C₀₋₈ alkyl is defined as above.

"-C₀₋₈ alkyl-N(R₁₆)-C(O)R₁₅" refers to the nitrogen atom in -N(R₁₆)-C(O)R₁₅ attached to C₀₋₈ alkyl, wherein the C₀₋₈ alkyl is defined as above.

"C₁₋₁₀ haloalkyl" refers to an alkyl having 1-10 carbon atoms in which hydrogens on the alkyl are optionally substituted by a fluorine, chlorine, bromine and iodine atom, including but not limited to difluoromethyl, dichloromethyl, dibromomethyl, trifluoromethyl, trichloromethyl, tribromomethyl, etc.

"C₁₋₁₀ haloalkoxy" refers to an alkoxy having 1-10 carbon atoms in which hydrogens on the alkyl are optionally substituted by a fluorine, chlorine, bromine and iodine atom, including but not limited to difluoromethoxy, dichloromethoxy, dibromomethoxy, trifluoromethoxy, trichloromethoxy, tribromomethoxy, etc.

"C₁₋₁₀ deuterioalkyl" refers to an alkyl having 1-10 carbon atoms in which hydrogens on the alkyl are optionally substituted by a deuterium atom, including but not limited to monodeuterium, dideuterium, trideuterium, etc.

"Halogen" refers to fluorine, chlorine, bromine or iodine.

The term "optional" or "optionally" means that the event or circumstance subsequently described may, but not necessarily, occur, and that the description includes instances where the event or circumstance occurs or does not occur, i.e., both substituted or unsubstituted instances. For example, "heterocyclyl group optionally substituted by alkyl" means that alkyl may be, but not necessarily, present, and that the description includes instances where the heterocyclyl group is and is not substituted by alkyl.

The term "substituted" means that one or more "hydrogen atoms" in a group are each independently substituted by a corresponding number of substituents. It goes without saying that a substituent is only in its possible chemical position and consistent with the valence bond theory in chemistry, and those skilled in the art will be able to determine (by experiments or theories) possible or impossible substitution without undue efforts. For example, it may be unstable when an amino or hydroxy having a free hydrogen is bound to a carbon atom having an unsaturated bond (such as olefin).

"Stereoisomer" refers to isomers produced by different spatial arrangements of atoms in the molecules. They can be divided into cis-trans isomers and enantiomers and can also be divided into two categories: enantiomers and diastereomers. A stereoisomer produced by the rotation of a single bond is referred to as a conformational stereo-isomer, sometimes also referred to as a rotamer. A stereoisomer produced by bond length, bond angle, double bonds in the molecule, ring and other reasons is referred to as a configuration stereo-isomer, which can be divided into two categories. An isomer produced by the inability of a double bond or a single bond of ring carbon atoms to rotate freely becomes a geometric isomer, also known as a cis-trans isomer, which can be divided into Z and E configurations. For example: cis-2-butene and trans-2-butene are a pair of geometric isomers. Stereoisomers with different optical properties produced by the absence of anti-axial symmetry in the molecules are called optical isomers, which can be divided into R and S configurations. "Stereoisomers" as described in the present invention, if not specifically indicated, are to be understood to include one or more of the enantiomers, configurational isomers, and conformational isomers described above.

"Pharmacologically acceptable salt" in the present invention refers to pharmaceutically acceptable acid addition salts, including inorganic and organic acid salts, which may be prepared by methods known in the art.

"Pharmaceutical composition" refers to a mixture containing one or more of the compounds described herein or a physiologically/pharmaceutically acceptable salt or pro-drug thereof, and other chemical components, for example physiologically/pharmaceutically acceptable carriers and excipients. The purpose of the pharmaceutical composition is to promote the administration to an organism, which facilitates the absorption of the active ingredient, thereby exerting biological activities.

**The present invention is further explained in detail below with reference to examples, which are not intended to limit the present invention, and the present invention is not merely limited to the contents of the examples.**

The compound structure of the present invention is determined by nuclear magnetic resonance (NMR) or/and liquid chromatography-mass spectrometry (LC-MS). The NMR chemical shift (δ) is given in parts per million (ppm). The NMR determination is conducted by using a Bruker AVANCE-400/500 nuclear magnetic resonance apparatus, with hexadeuterodimethyl sulfoxide (DMSO-*d₆*), tetradeuteromethanol (CD₃OD) and deuterated chloroform (CDCl₃) as determination solvents, and tetramethylsilane (TMS) as internal standard.

The LC-MS determination is conducted by using an Agilent 6120 mass spectrometer. The HPLC determination is conducted by using an Agilent 1200 DAD high pressure liquid chromatography (Sunfire C18 150 × 4.6 mm chromatographic column) and a Waters 2695-2996 high pressure liquid chromatography (Gimini C18 150 × 4.6 mm chromatographic column).

Yantai Yellow Sea HSGF254 or Qingdao GF254 silica gel plate is adopted as a thin layer chromatography (TLC) silica gel plate. The specification adopted by the TLC is 0.15-0.20 mm, and the specification adopted by the thin layer chromatography for the separation and purification of products is 0.4-0.5 mm. The Yantai Yellow Sea silica gel of 200-300 mesh is generally utilized as a carrier in column chromatography.

Starting materials in the examples of the present invention are known and commercially available, or may be synthesized by using or according to methods known in the art.

Unless otherwise stated, all reactions of the present invention are carried out under a dry nitrogen or argon atmosphere with continuous magnetic stirring, wherein the solvent is a dry solvent and the reaction temperature is in degree centigrade (°C).

### I. Preparation of Intermediates

### Intermediate 1-1: Preparation of (2,6-dimethylenetetrahydro-1H-pyrrolizin-7a(5H)-yl)methanol

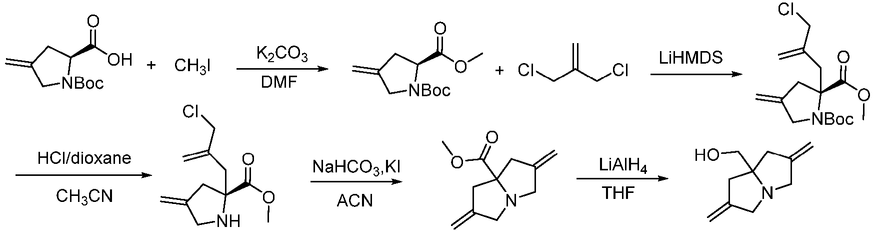

### Step 1: Synthesis of 1-(tert-butyl) 2-methyl (S)-4-methylenepyrrolidine-1,2-dicarboxylate

(S)-1-(tert-butoxycarbonyl)-4-methylenepyrrolidine-2-carboxylic acid (24 g, 105.61 mmol) was dissolved in DMF (240 mL), K₂CO₃ (21.89 g, 158.41 mmol) was added at 0°C, and the mixture was stirred for 1 hour. Then, CH₃I (29.98 g, 211.21 mmol) was added and the mixture was reacted at room temperature for 18 hours. After the reaction was completed, the solution was diluted with ethyl acetate, and the organic phase was separated and washed with saturated brine once. The organic phase was dried and filtered, the filtrate was concentrated, and the remaining was separated by using a fast silica gel column [0-50% ethyl acetate: petroleum ether] to obtain 1-(tert-butyl) 2-methyl (S)-4-methylenepyrrolidine-1,2-dicarboxylate (23.6 g, yield: 92.6%). ESI-MS: 242.3 [M+1]⁺.

### Step 2: Synthesis of 1-(tert-butyl) 2-methyl (R)-2-(2-(chloromethyl)allyl)-4-methylenepyrrolidine-1,2-dicarboxylate

1-(tert-butyl) 2-methyl (S)-4-methylenepyrrolidine-1,2-dicarboxylate (11.8 g, 48.9 mmol) was dissolved in dry THF (150 mL), LiHMDS/THF (73.36 mL, 73.36 mmol, 1 M) was added at -78°C, and the mixture was reacted at -78°C for 1 hour. 3-chloro-2-(chloromethyl)prop-1-ene (30.56 g, 244.52 mmol) was dissolved in dry THF (100 mL) solution and it was slowly added dropwise to the reaction system. After the addition, the mixture was warmed to room temperature and reacted for 18 hours. After the reaction was completed, the solution was diluted with ethyl acetate, and the organic phase was separated and washed with saturated brine once. The organic phase was dried, filtered and concentrated, and the resulting residue was separated by silica gel column chromatography to obtain 1-(tert-butyl) 2-methyl (R)-2-(2-(chloromethyl)allyl)-4-methylenepyrrolidine-1,2-dicarboxylate (10.3 g, yield: 63.9%). ESI-MS: 330.4 [M+1]⁺.

### Step 3: Synthesis of methyl (R)-2-(2-(chloromethyl)allyl)-4-methylenepyrroli dine-2-carboxylate

1-(tert-butyl) 2-methyl (R)-2-(2-(chloromethyl)allyl)-4-methylenepyrrolidine-1,2-dicarboxylate (20.7 g, 62.76 mmol) was dissolved in dry dichloromethane (100 mL), hydrochloric acid/dioxane solution (50 ml, 200 mmol) was added at 0°C, and the mixture was reacted at 0°C for 3 hours. After LCMS showed that the reaction was completed, it was concentrated to obtain methyl (R)-2-(2-(chloromethyl)allyl)-4-met hylenepyrrolidine-2-carboxylate (14.42 g, yield: 100%). ESI-MS: 230.3 [M+1]⁺.

### Step 4: Synthesis of methyl 2,6-dimethylenetetrahydro-1H-pyrrolizin-7a(5H) -carboxylate

Methyl (R)-2-(2-(chloromethyl)allyl)-4-methylenepyrrolidine-2-carboxylate (14.4 2 g, 62.78 mmol) was dissolved in acetonitrile (800 mL), and NaHCO₃ (26.37 g, 31 3.89 mmol) and KI (1.04 g, 6.28 mmol) were added in an ice-water bath. The mixt ure was reacted at room temperature for 2 hours. After the reaction was completed, the solution was diluted with ethyl acetate, and the organic phase was separated an d washed with saturated brine once. The organic phase was dried and filtered, the f iltrate was concentrated, and the remaining was separated by using a fast silica gel column [0-50% ethyl acetate: petroleum ether] to obtain methyl 2,6-dimethylenetetra hydro-1H-pyrrolizin-7a(5H)-carboxylate (11.3 g, yield: 93.16%). ESI-MS: 194.4 [M +1]⁺.

### Step 5: Synthesis of (2,6-dimethylenetetrahydro-1H-pyrrolizin-7a(5H)-yl)me thanol

Methyl 2,6-dimethylenetetrahydro-1H-pyrrolizin-7a(5H)-carboxylate (4 g, 20.7 0 mmol) was dissolved in dry tetrahydrofuran (80 mL), and lithium aluminum tetra hydride solution (16.60 ml, 41.40 mmol, 2.5 M in THF) was added at 0°C. The mi xture was reacted at 0°C for 2 hours. After the reaction was completed, 25 mL of water was added and the mixture was stirred for 30 minutes. 75 mL of 2 N sodium hydroxide solution was added and the mixture was stirred for 30 minutes. Then, 2 5 ml of water was added, and the mixture was stirred for 1 hour and diluted with dichloromethane. The organic phase was separated and washed once with saturated brine. The organic phase was dried and filtered, and the filtrate was concentrated to obtain (2,6-dimethylenetetrahydro-1H-pyrrolizin-7a(5H)-yl)methanol (3.1 g, yield: 9 0.64%). ESI-MS: 166.12 [M+1]⁺.

### Intermediate 1-2: Preparation of (S,Z)-(2-(fluoromethylene)tetrahydro-1H-pyrrolizin-7a(5H)-yl)methanol and

### Intermediate 1-3 (S,E)-(2-(fluoromethylene)tetrahydro-1H-pyrrolizin-7a(5H)-yl)methanol

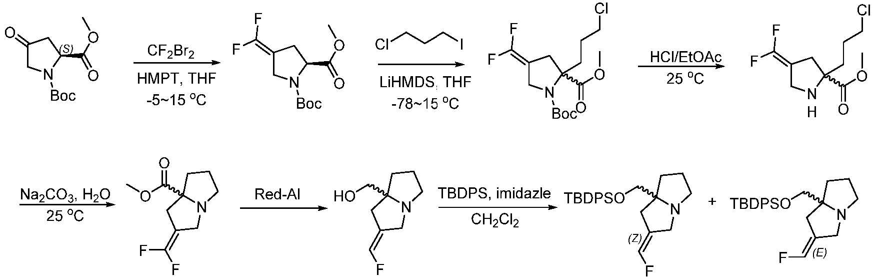

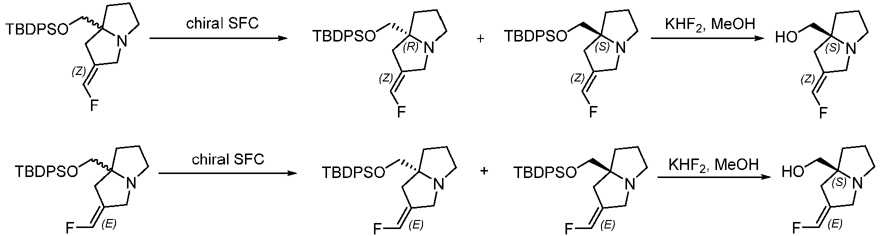

### Step 1: Synthesis of 1-(tert-butyl) 2-methyl (S)-4-(difluoromethylene)pyrrolidine-1,2-dicarboxylate

1-(tert-butyl) 2-methyl (S)-4-oxopyrrolidine-1,2-dicarboxylate (40 g, 164 mmol) and CF₂Br₂ (75.9 g, 362 mmol) were dissolved in THF (800 mL), HMPT (133 m L, 723 mmol) was added dropwise to the mixture at -5°C, and the reaction solution was stirred at 25°C for 15 hours. The reaction mixture was extracted with water (1 L) and ethyl acetate (1 L*3). The organic phase was combined, dried over anhy drous magnesium sulfate, filtered and concentrated. The crude product was separate d by fast silica gel column to obtain 1-(tert-butyl) 2-methyl (S)-4-(difluoromethylen e)pyrrolidine-1,2-dicarboxylate (24 g, 86.6 mmol, yield: 52.6 %).

¹H NMR (400 MHz, CDCl₃) *δ* 4.57- 4.29 (m, 1H), 4.16 - 3.94 (m, 2H), 3.67 (s, 3H), 3.05 - 2.76(m, 1H), 2.59 (br d, *J* =16.4 Hz, 1H), 1.44 - 1.33 (m, 9H).

### Step 2: Synthesis of 1-(tert-butyl) 2-methyl 2-(3-chloropropyl)-4-(difluoromethylene)pyrrolidine-1,2-dicarboxylate

1-(tert-butyl) 2-methyl (S)-4-(difluoromethylene)pyrrolidine-1,2-dicarboxylate (28 g, 100 mmol) was dissolved in THF (400 mL), LiHMDS (1 M n-hexane solution, 111 mL, 111 mmol) was added dropwise to the solution at -78°C, and the reaction solution was stirred at -78°C for 0.5 hours. Then, 1-chloro-3-iodo-propane (16.3 mL, 151 mmol) was added dropwise to the reaction solution, and the reaction solution was stirred at 25°C for 17.5 hours. The reaction was quenched by adding water (500 mL) at 0°C, and the mixture was extracted with water (1 L) and ethyl acetate (1 L*3). The organic phase was combined, dried over anhydrous magnesium sulfate and filtered, and the filtrate was concentrated to obtain 1-(tert-butyl) 2-methyl 2-(3-chloropropyl)-4-(difluoromethylene)pyrrolidine-1,2-dicarboxylate (35.7 g), which was used directly in the next step.

### Step 3: Synthesis of methyl 2-(3-chloropropyl)-4-(difluoromethylene)pyrrolidine-2-carboxylate

1-(tert-butyl) 2-methyl 2-(3-chloropropyl)-4-(difluoromethylene)pyrrolidine-1,2-dicarboxylate (35 g, 98.9 mmol) was dissolved in hydrochloric acid/dioxane (494 mL, 4 N), and the reaction solution was stirred at 25°C for 3 hours. The reaction solution was concentrated under reduced pressure to remove the solvent to obtain methyl 2-(3-chloropropyl)-4-(difluoromethylene)pyrrolidine-2-carboxylate (25 g, 98.5 mmol), which was used directly in the next step.

### Step 4: Synthesis of methyl 2-(difluoromethylene)tetrahydro-1H-pyrrolizin-7a(5H)-carboxylate

Methyl 2-(3-chloropropyl)-4-(difluoromethylene)pyrrolidine-2-carboxylate (25 g, 98.5 mmol) was dissolved in water (300 mL). Na₂CO₃ (73.1 g, 690 mmol) was added to the mixture, and the reaction solution was stirred at 25°C for 18 hours. The reaction mixture was extracted with water (1 L) and ethyl acetate (1 L*3). The organic phase was combined, dried over anhydrous magnesium sulfate, filtered and concentrated. The crude product was separated by fast silica gel column to obtain methyl 2-(difluoromethylene)tetrahydro-1H-pyrrolizin-7a(5H)-carboxylate (14 g, 64.4 mmol, yield: 65.4%).

¹H NMR (400 MHz, CDCl₃) *δ* 3.80 - 3.61 (m, 4H), 3.31 (dd, *J* = 1.8,14.4 Hz, 1H), 3.18 (td, *J* = 5.3,10.0 Hz, 1H), 2.97 (tdd, *J* = 1.5, 3.0, 16.0 Hz, 1H), 2.63 - 2.50 (m, 1H), 2.45 - 2.37 (m, 1H), 2.36 - 2.28 (m, 1H), 1.91 - 1.73(m, 3H).

### Step 5: Synthesis of (Z or E)-(2-(fluoromethylene)tetrahydro-1H-pyrrolizin-7a(5H)-yl)methanol

Methyl 2-(difluoromethylene)tetrahydro-1H-pyrrolizin-7a(5H)-carboxylate (7.5 g, 34.5 mmol) was dissolved in 2-methyltetrahydrofuran (300 mL). Red-Al (49.9 g, 173 mmol) was added dropwise to the solution at -15°C, and the reaction solution was stirred at 25°C for 18 hours. The reaction mixture was quenched by adding water (500 mL) at 0°C, and the mixture was extracted with water (500 mL) and ethyl acetate (500 mL*3). The organic phase was combined, dried over anhydrous magnesium sulfate, filtered and concentrated to obtain (Z or E)-(2-(fluoromethylene)tetrahydro-1H-pyrrolizin-7a(5H)-yl)methanol (5.91 g, 34.5 mmol), which was used directly in the next step.

### Step 6: Synthesis of (Z)-7a-(((tert-butyldiphenylsilyl)oxy)methyl)-2-(fluorom ethylene)hexahydro-1H-pyrrolizine and (E)-7a-(((tert-butyldiphenylsilyl)oxy)meth yl)-2-(fluoromethylene)hexahydro-1H-pyrrolizine

(Z or E)-(2-(fluoromethylene)tetrahydro-1H-pyrrolizin-7a(5H)-yl)methanol (11.8 g, 68.9 mmol) and imidazole (9.29 mL, 138 mmol) were dissolved in dichloromethane (200 mL). TBDPSCl (26.8 mL, 103 mmol) was added to the mixture in batches at 0°C, and the reaction solution was stirred at 25°C for 2 hours. The reaction mixture was extracted with water (200 mL) and ethyl acetate (300 mL*3). The organic phase was combined, dried over anhydrous magnesium sulfate, filtered and concentrated. The residue was separated by fast silica gel column to obtain (Z)-7a-(((tert-butyldiphenylsilyl)oxy)methyl)-2-(fluoromethylene)hexahydro-1H-pyrroli zine (10 g, yield: 35.4%) and (E)-7a-(((tert-butyldiphenylsilyl)oxy)methyl)-2-(fluoromethylene)hexahydro-1H-pyrroli zine (8 g, yield: 28.3%), respectively. The Z and E configurations were further confirmed by 2D NMR.

### (Z)-7a-(((tert-butyldiphenylsilyl)oxy)methyl)-2-(fluoromethylene)hexahydro-1 H-pyrrolizine:

¹H NMR (400 MHz, CDCl₃) *δ* 7.68 -7.62 (m, 4H), 7.43 - 7.37 (m, 6H), 6.53 - 6.26 (m, 1H), 3.76 (br d, *J* = 6.2 Hz, 1H), 3.56 - 3.32 (m, 3H), 3.13 (br s, 1H), 2.65 - 2.51 (m, 2H), 2.21 (br d, *J=* 15.8Hz, 1H), 2.01 (dt, *J=* 3.7, 8.3 Hz, 1H), 1.89 - 1.66 (m,3H), 1.07 (s, 9H).

### (E)-7a-(((tert-butyldiphenylsilyl)oxy)methyl)-2-(fluoromethylene)hexahydro-1 H-pyrrolizine:

¹H NMR (400 MHz, CDCl₃) *δ* 7.67 (td, *J* = 1.5, 7.8 Hz, 4H), 7.45 - 7.32 (m, 1H), 7.45 - 7.32 (m, 1H), 7.45 - 7.32 (m, 4H), 6.68 - 6.38 (m, 1H), 3.59 (br d, *J* = 13.4 Hz, 1H), 3.50 - 3.32 (m, 2H), 3.18 (br d, *J* = 13.9 Hz, 1H), 3.05 (br s, 1H), 2.85 (br d, *J* = 16.3 Hz, 1H), 2.62 - 2.49 (m, 1H), 2.34 (br d, *J* = 16.3 Hz, 1H), 2.08 - 1.96 (m, 1H), 1.82 - 1.59 (m, 3H), 1.07 (s, 9H).

### Step 7: Synthesis of (S,Z)-7a-(((tert-butyldiphenylsilyl)oxy)methyl)-2-(fluoro methylene)hexahydro-1H-pyrrolizine and (S,E)-7a-(((tert-butyldiphenylsilyl)oxy) methyl)-2-(fluoromethylene)hexahydro-1H-pyrrolizine

(Z)-7a-(((tert-butyldiphenylsilyl)oxy)methyl)-2-(fluoromethylene)hexahydro-1H-p yrrolizine (12 g, 24.4 mmol) was separated by chiral SFC (chiral column: Phenomenex-Cellulose-2 (250 mm*50 mm, 10 µm), mobile phase: CO₂-MeOH (0.1% NH₃•H₂O)) to obtain **Peak 1** (4.6 g, yield: 38.3%, ee value: 99.22%) and **Peak 2** (4.4 g, yield: 36.6%, ee value: 99.66%). Subsequent activity verification showed that Peak 1 was the desired S configuration.

### Step 8: Synthesis of (S,Z)-(2-(fluoromethylene)tetrahydro-1H-pyrrolizin-7a (5H)-yl)methanol and (S,E)-(2-(fluoromethylene)tetrahydro-1H-pyrrolizin-7a(5H) -yl)methanol

The **Peak 1** (2 g, 4.88 mmol) was dissolved in anhydrous methanol (25 mL). Under nitrogen protection, KHF₂ (7.63 g, 97.7 mmol) was added at 25°C and the mixture was reacted at 25°C for 2 hours. The reaction solution was filtered, concentrated under reduced pressure to remove the solvent, and the residue was separated by fast silica gel column to obtain (S,Z)-(2-(fluoromethylene)tetrahydro-1H-pyrrolizin-7a(5H)-yl)methanol (0.6 g, yield: 71.8%).

¹H NMR (400 MHz, CDCl₃) δ 6.66 - 6.35 (m, 1H), 3.75 (br d, *J* = 15.2 Hz, 1H), 3.51 - 3.40 (m, 1H), 3.35 - 3.23 (m, 2H), 3.17 - 3.06 (m, 1H), 2.67 (td, *J* = 7.4, 10.0 Hz, 1H), 2.47 - 2.36 (m, 1H), 2.34 - 2.24 (m, 1H), 2.01 - 1.71 (m, 5H).

(S,E)-(2-(fluoromethylene)tetrahydro-1H-pyrrolizin-7a(5H)-yl)methanol can be synthesized with reference to the synthesis of the compound with Z configuration.

### Intermediate 1-3: Preparation of (S)-(2-(difluoromethylene)tetrahydro-1H-pyrrolizin-7a(5H)-yl)methanol

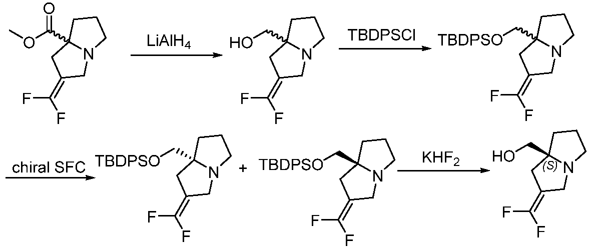

### Step 1: Synthesis of (2-(difluoromethylene)tetrahydro-1H-pyrrolizin-7a(5H)-yl)methanol

Methyl 2-(difluoromethylene)tetrahydro-1H-pyrrolizin-7a(5H)-carboxylate (7 g, 32.2 mmol) was dissolved in anhydrous THF (100 mL), and LiAlH₄ (1.83 g, 48.3 mmol) was slowly added in batches at 0°C. After the addition, the mixture was reacted at low temperature for 1 hour. After the reaction was completed, the reaction solution was quenched by adding dropwise water (1.5 mL), 15% aqueous NaOH solution (4.5 mL) and water (1.5 mL) in sequence, and filtered. The mixture was extracted with water and ethyl acetate, and the organic phase was washed with brine, dried over magnesium sulfate, and concentrated under reduced pressure to obtain (2-(difluoromethylene)tetrahydro-1H-pyrrolizin-7a(5H)-yl)methanol (5 g, yield: 82.0%).

¹H NMR (400MHz, CDCl₃) δ 3.64 (td, *J* = 3.7, 14.6 Hz, 1H), 3.40 -3.26 (m, 3H), 3.18 - 3.06 (m, 1H), 2.83 (br s, 1H), 2.65 (td, *J* = 7.5, 10.1 Hz, 1H), 2.48 - 2.28(m, 2H), 2.02 - 1.68 (m, 4H).

### Step 2: Synthesis of 7a-(((tert-butyldiphenylsilyl)oxy)methyl)-2-(difluoromet hylene)hexahydro-1H-pyrrolidine

(2-(difluoromethylene)tetrahydro-1H-pyrrolizin-7a(5H)-yl)methanol (1.5 g, 7.93 mmol) and imidazole (1.08 g, 15.8 mmol) were dissolved in CH₂Cl₂ (20 mL). Under nitrogen protection, TBDPSCl (3.08 mL, 11.9 mmol) was added at 0°C. The mixture was reacted at room temperature for 2 hours. The reaction solution was filtered, concentrated under reduced pressure to remove the solvent, and the residue was separated by fast silica gel column to obtain 7a-(((tert-butyldiphenylsilyl)oxy)methyl)-2-(difluoromethylene)hexahydro-1H-pyrrolidi ne (3 g, purity: 70%, yield: 61.9%).

### Step 3: Synthesis of (S)-7a-(((tert-butyldiphenylsilyl)oxy)methyl)-2-(difluoro methylene)hexahydro-1H-pyrrolidine and (R)-7a-(((tert-butyldiphenylsilyl)oxy)m ethyl)-2-(difluoromethylene)hexahydro-1H-pyrrolidine

7a-(((tert-butyldiphenylsilyl)oxy)methyl)-2-(difluoromethylene)hexahydro-1H-pyr rolidine (3 g) was separated by chiral (chiral column: DAICEL CHIRALPAK AD (250 mm*30 mm, 10 µm), mobile phase: CO₂-MeOH (0.1% NH₃•H₂O)) to obtain (S)-7a-(((tert-butyldiphenylsilyl)oxy)methyl)-2-(difluoromethylene)hexahydro-1H-pyrr olidine (Peak 1, 1 g, yield: 33.3%, ee value: 95.68%) and (R)-7a-(((tert-butyldiphenylsilyl)oxy)methyl)-2-(difluoromethylene)hexahydro-1H-pyrr olidine (Peak 2, 1 g, yield: 33.3%, ee value: 96.96%).

### Step 4: Synthesis of (S)-(2-(difluoromethylene)tetrahydro-1H-pyrrolizin-7a (5H)-yl)methanol

(S)-7a-(((tert-butyldiphenylsilyl)oxy)methyl)-2-(difluoromethylene)hexahydro-1H-pyrrolidine (1.0 g, 2.11 mmol) was dissolved in anhydrous methanol (15 mL), KHF₂ (1.64 g, 21.1 mmol) was added, and the mixture was reacted at room temperature for 5 hours. The reaction solution was filtered, concentrated under reduced pressure to remove the solvent, and the residue was separated by fast silica gel column to obtain (S)-(2-(difluoromethylene)tetrahydro-1H-pyrrolizin-7a(5H)-yl)methanol (300 mg, yield: 71.6%).

¹H NMR (400 MHz, CDCl₃) δ 3.70 - 3.61 (m, 1H), 3.43 - 3.30 (m, 3H), 3.11 (ddd, *J* = 4.3, 6.6, 10.4 Hz, 1H), 3.05 - 2.93 (m, 1H), 2.66 (td, *J* = 7.6, 10.0 Hz, 1H), 2.49 - 2.33 (m, 2H), 2.06 - 1.77 (m, 4H).

### Intermediate 1-4: Preparation of (S)-(2-methylenetetrahydro-1H-pyrrolizin-7a(5H)-yl)methanol

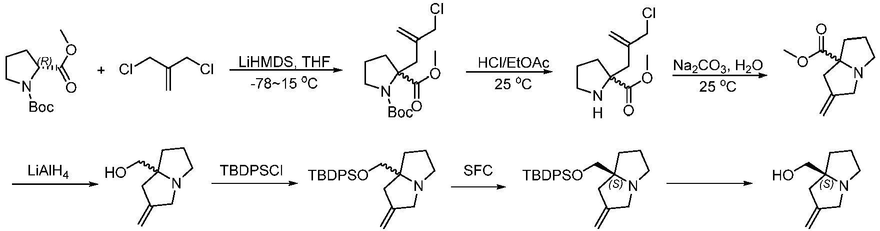

Refer to the corresponding steps of Intermediates 1-1 and 1-2 and select the corresponding raw materials for preparation.

### Intermediate 2-1: Preparation of 2-((2,6-dimethylenetetrahydro-1H-pyrroliz in-7a(5H)-yl)methoxy)-7-(8-ethynyl-7-fluoro-3-(methoxymethoxy)naphthalen-1-yl)-8-fluoropyrido[4,3-d]pyrimidin-4-ol

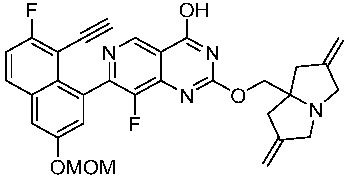

### Step 1: Synthesis of 2,7-dichloro-8-fluoro-4-(2-(trimethylsilyl)ethoxy)pyrido [4,3-d]pyrimidine

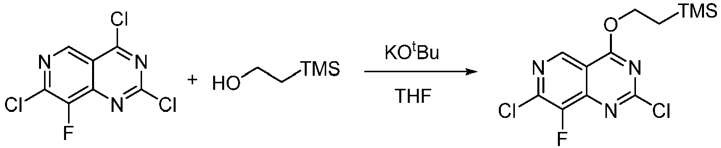

2,4,7-trichloro-8-fluoropyrido[4,3-d]pyrimidine (6.0 g, 23.77 mmol) was dissolved in THF (60 mL), and 2-(trimethylsilyl)ethan-1-ol (2.75 g, 23.292 mmol) and potassium tert-butoxide (29.23 mL, 29.23 mmol, 1 M THF solution) were added in sequence at -60°C. The mixture was stirred at -60°C for 2 hours. TLC showed that the reaction was completed. The reaction was filtered through a celite pad, diluted with water (100 mL), and extracted with ethyl acetate (100 mL*3). The organic phase was washed with saturated brine and dried over anhydrous sodium sulfate, filtered and concentrated. The residue was separated by fast column chromatography to obtain 2,7-dichloro-8-fluoro-4-(2-(trimethylsilyl)ethoxy)pyrido[4,3-d]pyrimidine (3.90 g, 11.668 mmol, yield: 49.1%).

### Step 2: Synthesis of 7-chloro-2-((2,6-dimethylenetetrahydro-1H-pyrrolizin-7a(5H)-yl)methoxy)-8-fluor o-4-(2-(trimethylsilyl)ethoxy)pyrido[4,3-d]pyrimidine

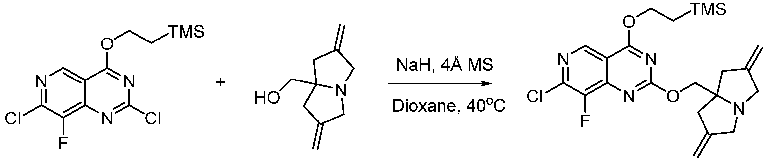

(2,6-dimethylenetetrahydro-1H-pyrrolizin-7a(5H)-yl)methanol (3.86 g, 23.34 mmol) was dissolved in 1,4-dioxane (100 mL), 60% NaH (0.93 g, 23.34 mmol) was added under cooling in an ice-water bath, and the mixture was stirred at room temperature for 10 minutes. 2,7-dichloro-8-fluoro-4-(2-(trimethylsilyl)ethoxy)pyrido[4,3-d]pyrimidine (3.9 g, 11.67 mmol) was dissolved in 1,4-dioxane (100 mL) and slowly added to the reaction system. The mixture was reacted at room temperature for 2 hours. After the reaction was completed, the reaction solution was quenched by adding water (200 mL), and then it was extracted with EtOAc (200 mL). The organic phase was washed with saturated brine, dried over anhydrous sodium sulfate, filtered, concentrated, and separated by column chromatography to obtain 7-chloro-2-((2,6-dimethylenetetrahydro-1H-pyrrolizin-7a(5H)-yl)methoxy)-8-fluoro-4-( 2-(trimethylsilyl)ethoxy)pyrido[4,3-d]pyrimidine (2.347 g, 5.069 mmol, yield: 43.5%). ESI-MS: 463.2 [M+1]⁺.

### Step 3: Synthesis of 2-((2,6-dimethylenetetrahydro-1H-pyrrolizin-7a(5H)-yl)methoxy)-8-fluoro-7-(7-flu oro-3-(methoxymethoxy)-8-((triisopropylsilyl)ethynyl)naphthalen-1-yl)-4-(2-(trime thylsilyl)ethoxy)pyrido[4,3-d]pyrimidine

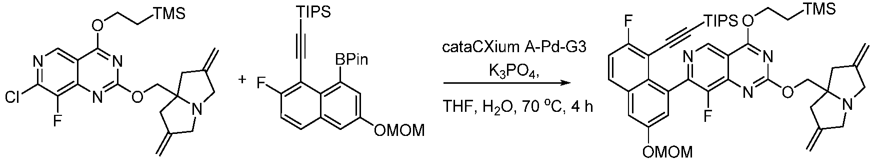

((2-fluoro-6-(methoxymethoxy)-8-(4,4,5,5-tetramethyl-1,3,2-dioxaborolan-2-yl)na phthalen-1-yl)ethynyl)triisopropylsilane (3.90 g, 7.60 mmol) was added to THF (50 mL) and H₂O (5 mL), then 7-chloro-2-((2,6-dimethylenetetrahydro-1H-pyrrolizin-7a(5H)-yl)methoxy)-8-fluoro-4-( 2-(trimethylsilyl)ethoxy)pyrido[4,3-d]pyrimidine (2.347 g, 5.07 mmol), K₃PO₄ (3.23 g, 15.21 mmol) and CataCXiµm A-Pd-G3 (3.15 mg, 0.01 mmol) were added. Under nitrogen protection, the mixture was heated and reacted at 70°C for 2 hours. After the reaction was completed, the reaction solution was cooled to room temperature, concentrated, and separated by column chromatography to obtain 2-((2,6-dimethylenetetrahydro-1H-pyrrolizin-7a(5H)-yl)methoxy)-8-fluoro-7-(7-fluoro-3-(methoxymethoxy)-8-((triisopropylsilyl)ethynyl)naphthalen-1-yl)-4-(2-(trimethylsilyl )ethoxy)pyrido[4,3-d]pyrimidine (3.48 g, 4.28 mmol, yield: 84.5%).

### Step 4: Synthesis of 2-((2,6-dimethylenetetrahydro-1H-pyrrolizin-7a(5H)-yl)methoxy)-7-(8-ethynyl-7-fl uoro-3-(methoxymethoxy)naphthalen-1-yl)-8-fluoropyrido[4,3-d]pyrimidin-4-ol

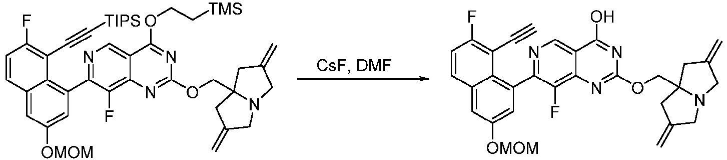

2-((2,6-dimethylenetetrahydro-1H-pyrrolizin-7a(5H)-yl)methoxy)-8-fluoro-7-(7-fl uoro-3-(methoxymethoxy)-8-((triisopropylsilyl)ethynyl)naphthalen-1-yl)-4-(2-(trimethy lsilyl)ethoxy)pyrido[4,3-d]pyrimidine (3.48 g, 4.28 mmol) was dissolved in DMF (50 mL), and CsF (6.50 g, 42.80 mmol) was added. The mixture was heated and reacted at 50°C for 2 hours. After the reaction was completed, the reaction solution was cooled to room temperature, filtered, concentrated, and separated by reversed-phase column chromatography to obtain 2-((2,6-dimethylenetetrahydro-1H-pyrrolizin-7a(5H)-yl)methoxy)-7-(8-ethynyl-7-fluoro -3-(methoxymethoxy)naphthalen-1-yl)-8-fluoropyrido[4,3-d]pyrimidin-4-ol (1.66 g, 2.97 mmol, yield: 69.5%). ESI-MS: 557.2 [M+1]⁺.

Intermediates **2-2** to **2-5** can be prepared by selecting the corresponding raw materials with reference to the synthesis methods of Intermediate 2-1.

| **Interme diate No.** | **Structural Formula** | **Chemical Name** | **MS: *m*/*z* [M+1]⁺** |
|---|---|---|---|
| **2-2** | 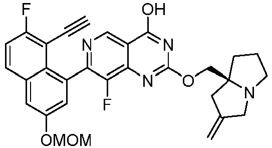 | (S)-7-(8-ethynyl-7-fluoro-3-(metho xymethoxy)naphthalen-1-yl)-8-fluo ro-2-((2-methylenetetrahydro-1H-p yrrolizin-7a(5H)-yl)methoxy)pyrido [4,3-d]pyrimidin-4-ol | 545.2 |
| **2-3** | 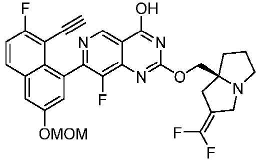 | (S)-2-((2-(difluoromethylene)tetrah ydro-1H-pyrrolizin-7a(5H)-yl)meth oxy)-7-(8-ethynyl-7-fluoro-3-(meth oxymethoxy)naphthalen-1-yl)-8-flu oropyrido[4,3-d]pyrimidin-4-ol | 581.2 |
| **2-4** | 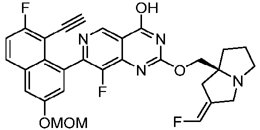 | (S,E)-7-(8-ethynyl-7-fluoro-3-(met hoxymethoxy)naphthalen-1-yl)-8-fl uoro-2-((2-(fluoromethylene)tetrah ydro-1H-pyrrolizin-7a(5H)-yl)meth oxy)pyrido[4,3-d]pyrimidin-4-ol | 563.4 |
| **2-5** | 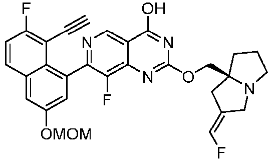 | (S,Z)-7-(8-ethynyl-7-fluoro-3-(met hoxymethoxy)naphthalen-1-yl)-8-fl uoro-2-((2-(fluoromethylene)tetrah ydro-1H-pyrrolizin-7a(5H)-yl)meth oxy)pyrido[4,3-d]pyrimidin-4-ol | 563.4 |

### Intermediate 3-1: Preparation of tert-butyl 1-(methyl-d₃)-3,8-diazabicyclo[3. 2.1]octan-8-carboxylate

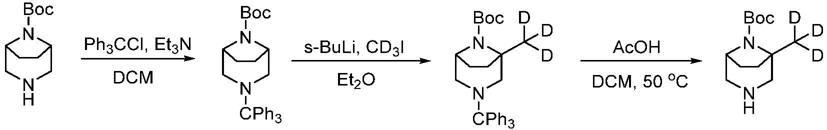

### Step 1: Synthesis of tert-butyl 3-triphenylmethyl-3,8-diazabicyclo[3.2.1]octa n-8-carboxylate

At room temperature, tert-butyl 3,8-diazabicyclo[3.2.1]octan-8-carboxylate (7.0 g, 33.0 mmol) was dissolved in 100 mL dichloromethane, and triethylamine (4.6 mL, 33.0 mmol) and (chloromethanetriyl)tribenzene (9.2 g, 33.0 mmol) were added in sequence. After being stirred at room temperature for 24 hours, the solution was added with 2.8 g (chloromethanetriyl)tribenzene. It was again stirred at room temperature for 24 hours, and quenched by adding dropwise saturated sodium carbonate aqueous solution. Then, it was left to stand for stratification. The aqueous phase was extracted with dichloromethane (50 mL* 2) and the organic phase was combined. The solution was washed sequentially with water (10 mL) and saturated sodium chloride aqueous solution (10 mL), dried over anhydrous sodium sulfate and concentrated, and the resulting crude product was separated by using a fast silica gel column [eluent: petroleum ether: ethyl acetate = 1: 1] to obtain tert-butyl 3-triphenylmethyl-3,8-diazabicyclo[3.2.1]octan-8-carboxylate (13.0 g, yield: 86%). ESI-MS: 243 [M+1]⁺.

### Step 2: Synthesis of tert-butyl 1-(methyl-d₃)-3-triphenylmethyl-3,8-diazabic yclo[3.2.1]octan-8-carboxylate

Under nitrogen protection, sec-butyllithium solution (3.6 mL, 4.6 mmol, 1.3 M) was added dropwise to anhydrous ether suspension (20 mL) of tert-butyl 3-triphenylmethyl-3,8-diazabicyclo[3.2.1]octan-8-carboxylate (900.0 mg, 2.0 mmol) and N,N,N',N'-tetramethylethylenediamine (526 mg, 4.6 mmol). The temperature of the reaction solution was maintained at around -30°C. After the dropwise addition, the reaction solution was stirred at 0°C for half an hour, and then deuterated iodomethane (287mg, 2.0 mmol) was added dropwise. After the dropwise addition, the reaction solution was further stirred at 0°C for 15 minutes. Then, saturated aqueous ammonium chloride solution (10 mL) was added dropwise, the mixture was allowed to stand for stratification, the aqueous phase was extracted with dichloromethane (10 mL), and the organic phase was combined, washed with saturated aqueous sodium bicarbonate solution (10 mL), water (10 mL) and saturated aqueous sodium chloride solution (10 mL) in sequence, dried over anhydrous sodium sulfate, filtered and concentrated. The resulting crude product was separated by fast silica gel column to obtain tert-butyl 1-(methyl-*d*₃)-3-triphenylmethyl-3,8-diazabicyclo[3.2.1]octan-8-carboxylate (888.0 mg, yield: 77%). ESI-MS: 243 [M+1]⁺.

### Step 3: Synthesis of tert-butyl 1-(methyl-d₃)-3,8-diazabicyclo[3.2.1]octan-8-c arboxylate

Tert-butyl 1-(methyl-*d*₃)-3-triphenylmethyl-3,8-diazabicyclo[3.2.1]octan-8-carbox ylate (880 mg, 1.87 mmol) was dissolved in dichloromethane (5 mL), 5 mL acetic acid was added, and the solution was heated to 50°C and reacted for 1 hour, and w as then spun dry to obtain tert-butyl 1-(methyl-*d*₃)-3,8-diazabicyclo[3.2.1]octan-8-car boxylate, which was used directly for the next reaction. ESI-MS: 230.4 [M+1]⁺.

### Intermediate 3-2: Preparation of tert-butyl (1S,5S)-1-cyclopropyl-3,8-diazabicyclo[3.2.1]octan-8-carboxylate

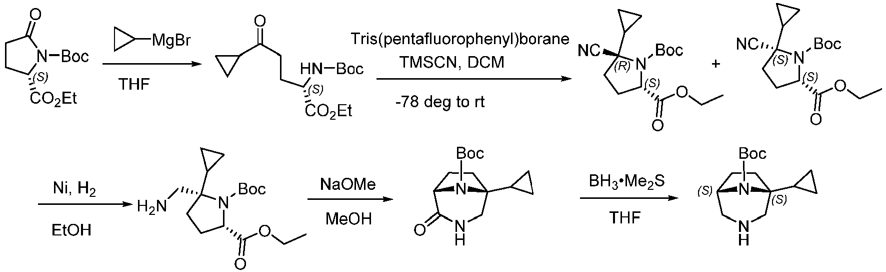

### Step 1: Synthesis of ethyl (S)-2-((tert-butoxycarbonyl)amino)-5-cyclopropyl-5-oxopentanoate

1-(tert-butyl) 2-ethyl (S)-5-oxopyrrolidine-1,2-dicarboxylate (25.0 g, 97.17 mmol) was dissolved in dry THF (250 mL), and the reaction solution was cooled to -78°C in a dry ice-acetone bath. Under nitrogen atmosphere, cPrMgBr/THF solution (106.9 mL, 106.9 mmol, 1 M) was added dropwise through a constant pressure dropping funnel. After the addition, the mixture was stirred and reacted at -78°C for 3 hours. The reaction was quenched with saturated ammonium chloride solution (250 mL) under cooling, and the mixture was extracted with ethyl acetate (200 mL*2). The organic phase was combined and concentrated, and the residue was separated by column chromatography [PE/EA=15%] to obtain ethyl (S)-2-((tert-butoxycarbonyl)amino)-5-cyclopropyl-5-oxopentanoate (11.0 g, yield: 38%). MS m/z (ESI): 244.0 [M-56+H]⁺.

¹H NMR (400 MHz, CDCl₃) δ 5.11 (d, *J* = 8.4 Hz, 1H), 4.31 - 4.23 (m, 1H), 4.19 (q, *J* = 7.1 Hz, 2H), 2.78 - 2.58 (m, 2H), 2.19 - 2.08 (m, 1H), 1.98 - 1.85 (m, 2H), 1.45 (s, 9H), 1.28 (t, *J* = 7.2 Hz, 3H), 1.05 - 1.00 (m, 2H), 0.90 - 0.85 (m, 2H).

### Step 2: Synthesis of 1-(tert-butyl) 2-ethyl (2S,5S)-5-cyano-5-cyclopropylpyr rolidine-1,2-dicarboxylate

Ethyl (S)-2-((tert-butoxycarbonyl)amino)-5-cyclopropyl-5-oxopentanoate (11.0 g, 36.74 mmol) was dissolved in dichloromethane (200 mL), and the reaction solution was cooled to -78°C in a dry ice-acetone bath. Under nitrogen atmosphere, a mixed solution of tri(pentafluorophenyl)borane (2.82 g, 5.51 mmol) and TMSCN (9.80 mL, 73.49 mmol) dissolved in dichloromethane (20 mL) was added dropwise through a constant pressure dropping funnel. After the addition, the mixture was stirred and reacted at -78°C for half an hour. The reaction solution was slowly warmed to room temperature and stirred overnight at room temperature. The reaction was quenched with saturated sodium bicarbonate solution (200 mL) and extracted with dichloromethane (200 mL*2). The organic phase was combined and concentrated, and the residue was separated by column chromatography [PE/EA=10-13%] to obtain 1-(tert-butyl) 2-ethyl (2S,5R)-5-cyano-5-cyclopropylpyrrolidine-1,2-dicarboxylate (3.75 g, yield: 33%, isomer 1) and 1-(tert-butyl) 2-ethyl (2S,5S)-5-cyano-5-cyclopropylpyrrolidine-1,2-dicarboxylate (5.0 g, yield: 44%, isomer 2).

### 1-(tert-butyl) 2-ethyl (2S,5R)-5-cyano-5-cyclopropylpyrrolidine-1,2-dicarboxy late (isomer 1)

MS m/z (ESI): 253.2 [M-56+H]⁺.

¹H NMR (400 MHz, CDCl₃) δ 4.57 - 4.37 (m, 1H), 4.20 (q, *J* = 7.1 Hz, 2H), 2.43 - 2.17 (m, 3H), 2.12 - 1.96 (m, 1H), 1.64 - 1.40 (m, 10H), 1.29 (t, *J* = 7.1 Hz, 3H), 0.95-0.86 (m, 1H), 0.84 - 0.77 (m, 1H), 0.63 - 0.55 (m, 1H), 0.52 - 0.43 (m, 1H).

### 1-(tert-butyl) 2-ethyl (2S,5S)-5-cyano-5-cyclopropylpyrrolidine-1,2-dicarboxy late (isomer 2)

MS m/z (ESI): 253.2 [M-56+H]⁺.

¹H NMR (400 MHz, CDCl₃) δ 4.48 (dd, *J* = 8.7, 1.9 Hz, 0.6H), 4.38 (dd, *J* = 8.6, 2.4 Hz, 0.4H), 4.29 - 4.11 (m, 2H), 2.62-2.51 (m, 0.6H), 2.49 - 2.43 (m, 0.4H), 2.35 - 2.13 (m, 1.6H), 2.12 - 2.00 (m, 1.4H), 1.55 (s, 6H), 1.44 (s, 3H), 1.40 - 1.26 (m, 4H), 0.91 - 0.83 (m, 1H), 0.82 - 0.73 (m, 1H), 0.63 - 0.52 (m, 1H), 0.50 - 0.43 (m, 1H).

### Step 3: Synthesis of 1-(tert-butyl) 2-ethyl (2S,5S)-5-(aminomethyl)-5-cyclop ropylpyrrolidine-1,2-dicarboxylate

1-(tert-butyl) 2-ethyl (2S,5S)-5-cyano-5-cyclopropylpyrrolidine-1,2-dicarboxylate (4.90 g, 15.89 mmol) was dissolved in ethanol (60 mL), and Raney nickel (1.2 g) was added. Under hydrogen atmosphere, the mixture was stirred at room temperature for 40 hours. The reaction solution was filtered through celite and washed with methanol (30 mL). The filtrate was concentrated, and the residue was separated by column chromatography [MeOH/DCM=5%] to obtain 1-(tert-butyl) 2-ethyl (2S,5S)-5-(aminomethyl)-5-cyclopropylpyrrolidine-1,2-dicarboxylate (3.51 g, yield: 71%). ESI-MS: 313.2 [M+1]⁺.

¹H NMR (400 MHz, CDCl₃) δ 4.40 (dd, *J* = 9.4, 3.1 Hz, 0.39H), 4.29 (dd, *J* = 9.5, 2.7 Hz, 0.61H), 4.26 - 4.10 (m, 2H), 3.57 (d, *J* = 13.4 Hz, 0.61H), 3.14 (d, *J* = 13.4 Hz, 0.39H), 2.88 (d, *J* = 13.4 Hz, 0.61H), 2.71 (d, *J* = 13.5 Hz, 0.39H), 2.69 (brs, 2H), 2.25 - 2.09 (m, 1H), 1.99 - 1.77 (m, 2H), 1.50 (s, 3H), 1.48 - 1.44 (m, 1H), 1.40 (s, 6H), 1.37 - 1.32 (m, 1H), 1.28 (q, *J* = 7.1 Hz, 3H), 0.56 - 0.35 (m, 2H), 0.35 - 0.18 (m, 2H).

### Step 4: Synthesis of tert-butyl (1S,5S)-1-cyclopropyl-4-oxo-3,8-diazabicyclo [3.2.1]octan-8-carboxylate

1-(tert-butyl) 2-ethyl (2S,5S)-5-(aminomethyl)-5-cyclopropylpyrrolidine-1,2-dicar boxylate (3.50 g, 11.20 mmol) was dissolved in methanol (60 mL). Sodium methoxi de (0.91 g, 16.80 mmol) was slowly added under cooling in an ice bath and the mi xture was stirred overnight at room temperature. The reaction solution was concentr ated, and the residue was separated by column chromatography [PE/EA=60%] to ob tain tert-butyl (1S,5S)-1-cyclopropyl-4-oxo-3,8-diazabicyclo[3.2.1]octan-8-carboxylate (2.60 g, yield: 87%). MS m/z (ESI): 211.2 [M-56+H]⁺.

¹H NMR (400 MHz, CDCl₃) δ 6.07 (s, 1H), 4.41 (d, *J* = 6.4 Hz, 1H), 3.86 (d, *J* = 11.2 Hz, 1H), 3.06 (dd, *J=* 11.2, 2.6 Hz, 1H), 2.03 - 1.91 (m, 2H), 1.84 - 1.75 (m, 1H), 1.60 - 1.50 (m, 2H), 1.47 (s, 9H), 0.62 - 0.50 (m, 2H), 0.47 - 0.37 (m, 1H), 0.29 - 0.24 (m, 1H).

### Step 5: Synthesis of tert-butyl (1S,5S)-1-cyclopropyl-3,8-diazabicyclo[3.2.1]o ctan-8-carboxylate

Tert-butyl (1S,5S)-1-cyclopropyl-4-oxo-3,8-diazabicyclo[3.2.1]octan-8-carboxylat e (2.30 g, 8.64 mmol) was dissolved in dry tetrahydrofuran (20 mL), borane dimeth yl sulfide solution (21.59 mL, 43.18 mmol) was slowly added dropwise under cooli ng in an ice bath, and the mixture was stirred at 0°C for 1 hour. The reaction solut ion was warmed to room temperature and stirred overnight. The reaction was quenc hed by slowly adding methanol (20 mL) and the mixture was stirred and reacted at 50°C for 18 hours. The reaction solution was directly concentrated by rotary evapo ration, and the residue was separated by column chromatography [MeOH/DCM=5%] to obtain tert-butyl (1S,5S)-1-cyclopropyl-3,8-diazabicyclo[3.2.1]octan-8-carboxylate (1.10 g, yield: 50%). ESI-MS: 253.2 [M+1]⁺.

¹H NMR (400 MHz, CDCl₃) δ 4.26 (d, *J* = 7.3 Hz, 1H), 3.11 (d, *J* = 12.3 Hz, 1H), 3.04 (d, *J* = 12.5 Hz, 1H), 2.74 - 2.66 (m, 2H), 1.95 - 1.83 (m, 1H), 1.76 - 1.68 (m, 1H), 1.66 - 1.59 (m, 1H), 1.48 (s, 9H), 1.47- 1.33 (m, 2H), 0.50 - 0.44 (m, 2H), 0.38 - 0.33 (m, 1H), 0.28 - 0.23 (m, 1H).

Intermediates **3-3** to **3-12** can be prepared by selecting the corresponding raw materials with reference to the synthesis methods of Intermediate **3-1** or **3-2.**

| **Intermed iate No.** | **Structural Formula** | **Chemical Name** | **ESI-MS: [M+1]⁺** |
|---|---|---|---|
| **3-3** | 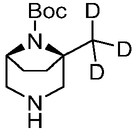 | tert-butyl (1R,5S)-1-(methyl-*d*₃)-3,8-diazabic yclo[3.2.1]octan-8-carboxylate | 230.4 |
| **3-4** | 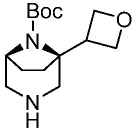 | tert-butyl (1S,5S)-1-(oxetan-3-yl)-3,8-diazabi cyclo[3.2.1]octan-8-carboxylate | 269.4 |
| **3-5** | 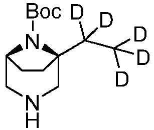 | tert-butyl (1R,5S)-1-(ethyl-*d*₅)-3,8-diazabicyc lo[3.2.1]octan-8-carboxylate | 246.4 |
| **3-6** | 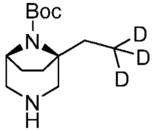 | tert-butyl (1R,5S)-1-(ethyl-2,2,2-*d*₃)-3,8-diaz abicyclo[3.2.1]octan-8-carboxylate | 244.4 |
| **3-7** | 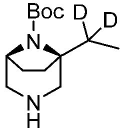 | tert-butyl (1R,5S)-1-(ethyl-1,1-*d*₂)-3,8-diazab icyclo[3.2.1]octan-8-carboxylate | 243.4 |
| **3-8** | 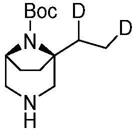 | tert-butyl (1R,5S)-1-(ethyl-1,2-*d*₂)-3,8-diazab icyclo[3.2.1]octan-8-carboxylate | 243.4 |
| **3-9** | 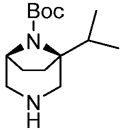 | tert-butyl (1S,5S)-1-isopropyl-3,8-diazabicycl o[3.2.1]octan-8-carboxylate | 255.4 |
| **3-10** | 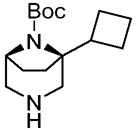 | tert-butyl (1S,5S)-1-cyclobutyl-3,8-diazabicy clo[3.2.1]octan-8-carboxylate | 267.4 |
| **3-11** | 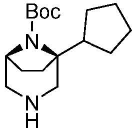 | tert-butyl (1S,5S)-1-cyclopentyl-3,8-diazabic yclo[3.2.1]octan-8-carboxylate | 281.4 |
| **3-12** | 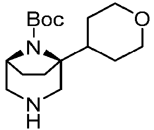 | tert-butyl (1S,5S)-1-(tetrahydro-2H-pyran-4-yl)-3,8-diazabicyclo[3.2.1]octan-8-carboxylate | 297.4 |

### Intermediate 3-13: Preparation of tert-butyl 1-(methoxymethyl)-3,8-diazabicyclo[3.2.1]octan-8-carboxylate

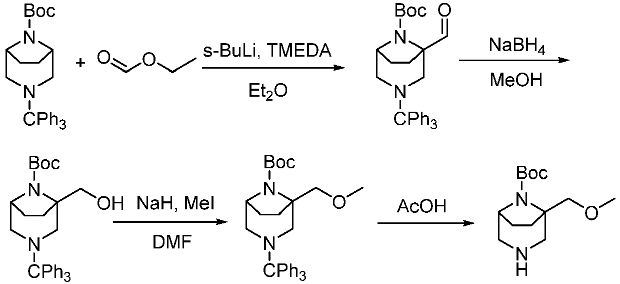

### Step 1: Synthesis of tert-butyl 1-formyl-3-triphenylmethyl-3,8-diazabicyclo[3.2.1]octan-8-carboxylate

Under nitrogen protection, sec-butyllithium solution (14.4 mL, 18.7 mmol, 1.3 M) was slowly added dropwise into anhydrous ether (40 mL) suspension of tert-butyl 3-triphenylmethyl-3,8-diazabicyclo[3.2.1]octan-8-carboxylate (5.0 g, 11.0 mmol) and TMEDA (2.2 g, 18.7 mmol). The reaction temperature was maintained at around 0°C. After the dropwise addition, the reaction solution was stirred at 0°C for 1.5 hours, and then ethyl formate (2.4 g, 33.0 mmol) was added dropwise. After the dropwise addition, the reaction solution was further stirred at 0°C for 15 minutes. Then, saturated aqueous ammonium chloride solution (20 mL) was added dropwise, the mixture was allowed to stand for stratification, the aqueous phase was extracted with dichloromethane (10 mL), and the organic phase was combined, washed with saturated aqueous sodium bicarbonate solution (10 mL), water (10 mL) and saturated aqueous sodium chloride solution (10 mL) in sequence, dried over anhydrous sodium sulfate and concentrated. The resulting crude product was separated by fast silica gel column to obtain tert-butyl 1-formyl-3-triphenylmethyl-3,8-diazabicyclo[3.2.1]octan-8-carboxylate (1.9 g, yield: 36%). ESI-MS: 483.2 [M+1]⁺.

### Step 2: Synthesis of tert-butyl 1-(hydroxymethyl)-3-triphenylmethyl-3,8-diazabicyclo[3.2.1]octan-8-carboxylate

Tert-butyl 1-formyl-3-triphenylmethyl-3,8-diazabicyclo[3.2.1]octan-8-carboxylate (560.0 mg, 1.2 mmol) was dissolved in methanol (10 mL), NaBH₄ (78.0 mg, 2.3 mmol) was added under the ice bath, and then the solution was stirred at room temperature for 1 hour until the reaction was completed. The solution was quenched by adding water (15 mL) and ethyl acetate (15 mL), and stirred at room temperature for 5 minutes. It was then left to stand for stratification. The aqueous phase was extracted with ethyl acetate (10 mL*2), and the organic phase was combined, washed with saturated sodium bicarbonate aqueous solution (10 mL), water (10 mL) and saturated sodium chloride aqueous solution (10 mL) in sequence, dried over anhydrous sodium sulfate, filtered and concentrated to obtain tert-butyl 1-(hydroxymethyl)-3-triphenylmethyl-3,8-diazabicyclo[3.2.1]octan-8-carboxylate (560.0 mg, yield: 91%). ESI-MS: 485.2 [M+1]⁺.

### Step 3: Synthesis of tert-butyl 1-(methoxymethyl)-3-triphenylmethyl-3,8-diazabicyclo[3.2.1]octan-8-carboxylate

Tert-butyl 1-(hydroxymethyl)-3-triphenylmethyl-3,8-diazabicyclo[3.2.1]octan-8-ca rboxylate (700 mg, 1.44 mmol) was dissolved in DMF (10 mL) solution, and sodiu m hydrogen (69.3 mg, 1.73 mmol) was slowly added at 0°C. After the reaction sol ution was stirred and reacted at 0°C for 2 hours, methyl iodide (270 µL, 4.33 mmo l) was added. After the addition, the reaction lasted for 2 hours at 0°C. After the re action was completed, water (20 mL) was added for quenching and the aqueous ph ase was extracted with ethyl acetate (20 mL*2). The combined organic phase was washed with saturated brine, dried over anhydrous magnesium sulfate, filtrated and concentrated under reduced pressure to remove the solvent. The remaining was sepa rated with a rapid silica gel column to obtain tert-butyl 1-(methoxymethyl)-3-triphen ylmethyl-3,8-diazabicyclo[3.2.1]octan-8-carboxylate (440 mg, yield: 61.1%).

### Step 4: Synthesis of tert-butyl 1-(methoxymethyl)-3,8-diazabicyclo[3.2.1]octan-8-carboxylate

Tert-butyl 1-(methoxymethyl)-3-triphenylmethyl-3,8-diazabicyclo[3.2.1]octan-8-carboxylate (440 mg, 0.88 mmol) was dissolved in HOAc (5 mL). The mixture was reacted at 25°C for 2 hours. It was concentrated under reduced pressure to remove the solvent, to obtain tert-butyl 1-(methoxymethyl)-3,8-diazabicyclo[3.2.1]octan-8-carboxylate (220 mg, yield: 97.3 %). ESI-MS: 256 [M+1]⁺.

### Intermediate 3-14: Preparation of tert-butyl (1S,5R)-1-((methoxy-d₃)methyl)-3,8-diazabicyclo[3.2.1]octan-8-carboxylate

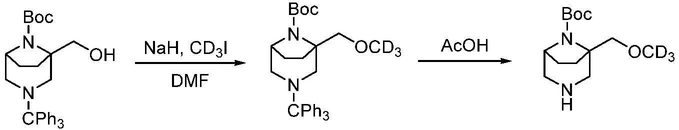

Intermediate 3-14 can be prepared by referring to Intermediate 3-13.

### Intermediate 3-14-1 and Intermediate 3-14-2: Preparation of tert-butyl (1S, 5R)-1-((methoxy-d₃)methyl)-3,8-diazabicyclo[3.2.1]octan-8-carboxylate and tert-b utyl (1R,5S)-1-((methoxy-d₃)methyl)-3,8-diazabicyclo[3.2.1]octan-8-carboxylate

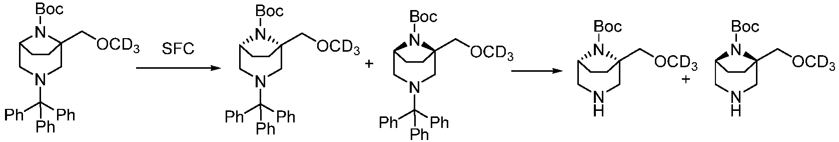

### Step 1: Synthesis of tert-butyl (1S,5R)-1-((methoxy-d₃)methyl)-3-triphenylm ethyl-3,8-diazabicyclo[3.2.1]octan-8-carboxylate and tert-butyl (1R,5S)-1-((metho xy-d₃)methyl)-3-triphenylmethyl-3,8-diazabicyclo[3.2.1]octan-8-carboxylate

(±)-tert-butyl (1S,5R)-1-((methoxy-*d*₃)methyl)-3-triphenylmethyl-3,8-diazabicyclo [3.2.1]octan-8-carboxylate (4 g, 7.97 mmol) was separated by SFC (chiral column: REGIS (s,s) WHELK-O1 (250 mm*50 mm, 10 µm); mobile phase: CO₂-EtOH (0.1% NH₃•H₂O)) to obtain Peak 1 tert-butyl (1S,5R)-1-((methoxy-*d*₃)methyl)-3-triphenyl methyl-3,8-diazabicyclo[3.2.1]octan-8-carboxylate (assumed configuration) (1.83 g, y ield: 45.8%, purity 97.2%, ESI-MS 524 [M+23]⁺) and Peak 2 tert-butyl (1R,5S)-1-((methoxy-*d*₃)methyl)-3-triphenylmethyl-3,8-diazabicyclo[3.2.1]octan-8-carboxylate (a ssumed configuration) (1.81 g, yield: 45.3%, purity 97.0%), respectively. ESI-MS: 5 24 [M+23]⁺.

### Peak 1: tert-butyl (1S,5R)-1-((methoxy-d₃)methyl)-3-triphenylmethyl-3,8-dia zabicyclo[3.2.1]octan-8-carboxylate

¹H NMR (400MHz, CDCl₃) δ 7.51 (br s, 5H), 7.32 - 7.24 (m, 7H), 7.19 - 7.13 (m, 3H), 4.14 (br s, 1H), 3.64 (br s, 2H), 3.08 (d, *J* = 11.0 Hz, 1H), 2.95 (br d, *J* = 11.0 Hz, 1H), 2.61 - 2.49 (m, 1H), 2.24 (dt, *J* = 3.4, 10.4 Hz, 1H), 2.17 - 1.97 (m, 2H), 1.84 (br d, *J =* 11.0 Hz, 1H), 1.75 (br d, *J* = 9.0 Hz, 1H), 1.19 (s, 9H).

### Peak 2: tert-butyl (1R,5S)-1-((methoxy-d₃)methyl)-3-triphenylmethyl-3,8-dia zabicyclo[3.2.1]octan-8-carboxylate

¹H NMR (400MHz, CDCl₃) δ 7.51 (br s, 5H), 7.31 - 7.25 (m, 7H), 7.19 - 7.14 (m, 3H), 4.14 (br s, 1H), 3.64 (br s, 2H), 3.08 (d, *J* = 11.0 Hz, 1H), 2.96 (br d, *J* = 11.4 Hz, 1H), 2.62 - 2.50 (m, 1H), 2.33 - 2.18 (m, 1H), 2.15 - 1.99 (m, 2H), 1.84 (br d, *J* = 11.0 Hz, 1H), 1.75 (br d, *J* = 8.4 Hz, 1H), 1.19 (s, 9H).

### Step 2: Synthesis of tert-butyl (1S,5R)-1-((methoxy-d₃)methyl)-3,8-diazabicy clo[3.2.1]octan-8-carboxylate or tert-butyl (1R,5S)-1-((methoxy-d₃)methyl)-3,8-dia zabicyclo[3.2.1]octan-8-carboxylate

Refer to Step 4 of Intermediate 3-13 and select the corresponding single chiral raw materials for preparation.

### Intermediate 3-15: Preparation of tert-butyl 1-vinyl-3,8-diazabicyclo[3.2.1]octan-8-carboxylate

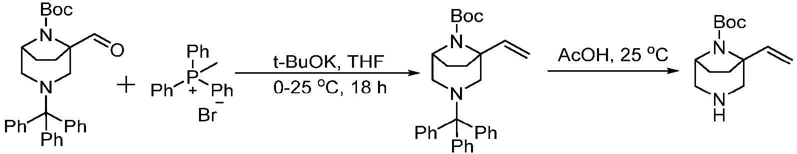

### Step 1: Synthesis of tert-butyl 3-triphenylmethyl-1-vinyl-3,8-diazabicyclo[3.2.1]octan-8-carboxylate

Methyl triphenylphosphane bromide (3.66 g, 10.3 mmol) was dissolved in anhy drous tetrahydrofuran (30 mL), and potassium tert-butoxide (1.27 g, 11.3 mmol) wa s added. The solution was stirred for 10 minutes at 0°C, and tetrahydrofuran solutio n (10 mL) of tert-butyl 1-formyl-3-triphenylmethyl-3,8-diazabicyclo[3.2.1]octan-8-car boxylate (1.65 g, 3.42 mmol) was slowly added. The mixture was reacted at 25°C f or 2 hours. TLC detected that the reaction was complete. The reaction solution was diluted with 30 mL water, and extracted with 30 mL ethyl acetate twice. The orga nic phase was washed with 20 mL saturated brine once, dried over anhydrous sodiu m sulfate and concentrated under reduced pressure to remove the solvent. The rema ining was separated by using a fast silica gel column to obtain tert-butyl 3-triphenyl methyl-1-vinyl-3,8-diazabicyclo[3.2.1]octan-8-carboxylate (1.19 g, yield: 72.4%).

¹H NMR (400 MHz, CDCl₃) δ 7.50 (br s, 5H), 7.33-7.25 (m, 7H), 7.19-7.09 (m, 3H), 6.16 (dd, *J* =11.2, 17.9 Hz, 1H), 4.93 (d, *J* = 11.3 Hz, 1H), 4.81 (d, *J* = 18.1 Hz, 1H), 4.16 (d, *J =* 5.5 Hz, 1H), 3.19 (d, *J* = 11.0 Hz, 1H), 2.96 (d, *J* = 11.0 Hz, 1H), 2.58-2.46 (m, 1H), 2.36-2.22 (m, 1H), 2.13-1.89 (m, 2H), 1.92 (d, *J* = 11.0 Hz, 1H), 1.84 (d, *J* = 11.0 Hz, 1H), 1.15 (s, 9H).

### Step 2: Synthesis of tert-butyl 1-vinyl-3,8-diazabicyclo[3.2.1]octan-8-carbox ylate

Tert-butyl 3-triphenylmethyl-1-vinyl-3,8-diazabicyclo[3.2.1]octan-8-carboxylate (550 mg, 1.14 mmol) was dissolved in acetic acid (10 mL). The mixture was reacted overnight at 25°C. It was concentrated under reduced pressure to remove the solvent, to obtain tert-butyl 1-vinyl-3,8-diazabicyclo[3.2.1]octan-8-carboxylate (540 mg, crude product). The crude product can be directly used in the next reaction without separation. ESI-MS: 183 [M+1-56]⁺.

### Intermediate 3-16 can be prepared by selecting the corresponding raw materials with reference to the synthesis method of Intermediate 3-15:

| **Intermed iate No.** | **Structural Formula** | **Chemical Name** | **ESI-MS: [M+1]⁺** |
|---|---|---|---|
| **3-16** | 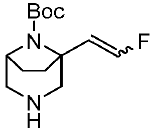 | tert-butyl 1-(2-fluorovinyl)-3,8-diazabicyclo [3.2.1]octan-8-carboxylate | 257 |

### Intermediate 3-17: Preparation of tert-butyl 1-ethyl-3,8-diazabicyclo [3.2.1]octan-8-carboxylate

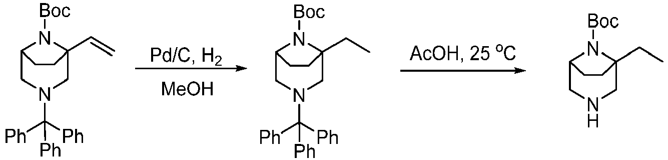

### Step 1: Synthesis of tert-butyl 1-ethyl-3-triphenylmethyl-3,8-diazabicyclo[3.2.1]octan-8-carboxylate

Tert-butyl 3-triphenylmethyl-1-vinyl-3,8-diazabicyclo[3.2.1]octan-8-carboxylate (550 mg, 1.14 mmol) was dissolved in anhydrous methanol solution (20 mL), and wet Pd/C (200 mg, purity: 10%) was added under nitrogen protection. The hydroge n substitution was carried out three times, and the mixture was reacted at 25°C for 3 hours under a hydrogen balloon atmosphere. TLC confirmed that the reaction was complete. The mixture was filtered by diatomite, and the filtrate was concentrated under reduced pressure to remove the solvent, to obtain tert-butyl 1-ethyl-3-tripheny lmethyl-3,8-diazabicyclo[3.2.1]octan-8-carboxylate (550 mg, yield: 100%).

### Step 2: Synthesis of tert-butyl 1-ethyl-3,8-diazabicyclo[3.2.1]octan-8-carbox ylate

Tert-butyl 1-ethyl-3-triphenylmethyl-3,8-diazabicyclo[3.2.1]octan-8-carboxylate (550 mg, 1.13 mmol) was dissolved in HOAc (10 mL). The mixture was reacted overnight at 25°C. It was concentrated under reduced pressure to remove the solvent to obtain tert-butyl 1-ethyl-3,8-diazabicyclo[3.2.1]octan-8-carboxylate (540 mg, crude product). ESI-MS: 241 [M+1]⁺.

### Intermediate 3-17-1 and Intermediate 3-17-2: Preparation of tert-butyl (1S,5R)-1-ethyl-3,8-diazabicyclo[3.2.1]octan-8-carboxylate and tert-butyl (1R,5S)-1-ethyl-3,8-diazabicyclo[3.2.1]octan-8-carboxylate

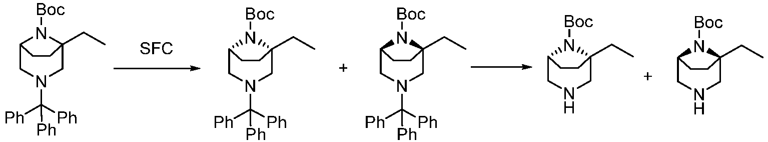

### Step 1: Synthesis of tert-butyl (1S,5R)-1-ethyl-3-triphenylmethyl-3,8-diazabicyclo[3.2.1]octan-8-carboxylate and tert-butyl (1R,5S)-1-ethyl-3-triphenylmethyl-3,8-diazabicyclo[3.2.1]octan-8-carboxylate

Tert-butyl 1-ethyl-3,8-diazabicyclo[3.2.1]octan-8-carboxylate (4 g, 7.97 mmol) was separated by SFC (chiral column: REGIS (s,s) WHELK-O1 (250 mm*50 mm, 10 µm); mobile phase: CO₂-EtOH (0.1% NH₃•H₂O)) to obtain Peak 1 tert-butyl (1S, 5R)-1-ethyl-3-triphenylmethyl-3,8-diazabicyclo[3.2.1]octan-8-carboxylate (assumed co nfiguration) (1.83 g, yield: 45.8%, purity 97.2%, ESI-MS 524 [M+23]⁺) and Peak 2 tert-butyl (1R,5S)-1-ethyl-3-triphenylmethyl-3,8-diazabicyclo[3.2.1]octan-8-carboxyla te (assumed configuration) (1.81 g, yield: 45.3%, purity 97.0%), respectively. ESI-MS 524 [M+23]⁺.

### Peak 1: tert-butyl (1S,5R)-1-ethyl-3-triphenylmethyl-3,8-diazabicyclo[3.2.1]o ctan-8-carboxylate

¹H NMR (400MHz, CDCl₃) δ 7.51 (br s, 5H), 7.32 - 7.24 (m, 7H), 7.19 - 7.13 (m, 3H), 4.14 (br s, 1H), 3.64 (br s, 2H), 3.08 (d, *J* = 11.0 Hz, 1H), 2.95 (br d, *J* = 11.0 Hz, 1H), 2.61 - 2.49 (m, 1H), 2.24 (dt, *J* = 3.4, 10.4 Hz, 1H), 2.17 - 1.97 (m, 2H), 1.84 (br d, *J =* 11.0 Hz, 1H), 1.75 (br d, *J* = 9.0 Hz, 1H), 1.19 (s, 9H).

### Peak 2: tert-butyl (1R,5S)-1-ethyl-3-triphenylmethyl-3,8-diazabicyclo[3.2.1]o ctan-8-carboxylate

¹H NMR (400MHz, CDCl₃) δ 7.51 (br s, 5H), 7.31 - 7.25 (m, 7H), 7.19 - 7.14 (m, 3H), 4.14 (br s, 1H), 3.64 (br s, 2H), 3.08 (d, *J* = 11.0 Hz, 1H), 2.96 (br d, *J* = 11.4 Hz, 1H), 2.62 - 2.50 (m, 1H), 2.33 - 2.18 (m, 1H), 2.15 - 1.99 (m, 2H), 1.84 (br d, *J =* 11.0 Hz, 1H), 1.75 (br d, *J* = 8.4 Hz, 1H), 1.19 (s, 9H).

### Step 2: Synthesis of tert-butyl (1S,5R)-1-ethyl-3,8-diazabicyclo[3.2.1]octan-8 -carboxylate and tert-butyl (1R,5S)-1-ethyl-3,8-diazabicyclo[3.2.1]octan-8-carbox ylate

Refer to Step 2 of Intermediate 3-17 and select the corresponding single chiral raw materials for preparation.

### Intermediate 3-18: Preparation of tert-butyl (1S,5R)-1-(2-hydroxypropan-2-yl)-3,8-diazabicyclo[3.2.1]octan-8-carboxylate

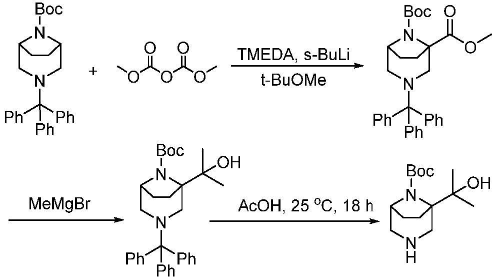

Refer to Intermediate 3-1 and select the corresponding raw materials for preparation.

### Intermediate 4-1: Preparation of (2-((tert-butoxycarbonyl)amino)-7-fluorob enzo[d]thiazol-4-yl)boronic acid

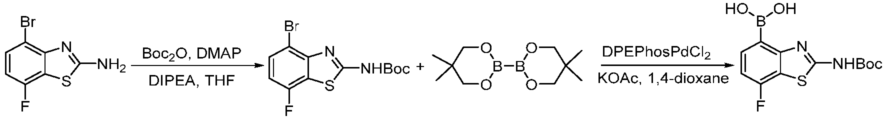

### Step 1: Synthesis of tert-butyl (4-bromo-7-fluorobenzo[d]thiazol-2-yl)carba mate

4-bromo-7-fluorobenzo[d]thiazol-2-amine (2.9 g, 11.74 mmol), DMAP (110 mg, 0.94 mmol) and DIPEA (2.28 g, 17.6 mmol) were dissolved in a mixed solution of THF (36 mL) and DMF (3 mL), Boc₂O (2.82 g, 12.91 mmol) was added at 0°C, and the mixture was stirred at room temperature for 18 hours. The reaction was completed as monitored by LCMS. After the reaction was completed, the reaction solution was diluted with ethyl acetate, and the organic phase was separated and washed with saturated brine. The organic phase was dried and filtered, the filtrate was concentrated, and the resulting residue was separated by fast silica gel column chromatography to obtain tert-butyl (4-bromo-7-fluorobenzo[d]thiazol-2-yl)carbamate (3.14 g, yield: 77.2%). ESI-MS: 348.0 [M+1]⁺.

### Step 2: Synthesis of (2-((tert-butoxycarbonyl)amino)-7-fluorobenzo[d]thiazol -4-yl)boronic acid

Tert-butyl (4-bromo-7-fluorobenzo[d]thiazol-2-yl)carbamate (2.0 g, 5.76 mmol), KOAc (1.70 g, 17.28 mmol), bis(neopentylglycol)diboron (4.94 g, 21.89 mmol) were dissolved in 1,4-dioxane (40 mL), DPEPhosPdCl₂ (310 mg, 0.574 mmol) was added, the nitrogen substitution was carried out three times, and the mixture was heated and stirred at 100°C for 18 hours. The reaction was completed as monitored by LCMS. After the reaction was completed, the reaction solution was concentrated, and the resulting residue was separated by fast silica gel column chromatography to obtain (2-((tert-butoxycarbonyl)amino)-7-fluorobenzo[d]thiazol-4-yl)boronic acid (1.01 g, yield: 56%). ESI-MS: 313.1 [M+1]⁺.

### Intermediate 4-2: Preparation of tert-butyl (3-cyano-4-(5,5-dimethyl-1,3,2-dioxaborolan-2-yl)-7-fluorobenzo [b]thiophen-2-yl)c arbamate

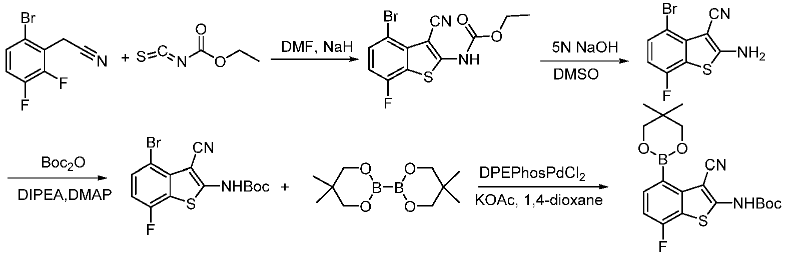

### Step 1: Synthesis of ethyl (4-bromo-3-cyano-7-fluorobenzo[b]thiophen-2-yl)carbamate

2-(6-bromo-2,3-difluorophenyl)acetonitrile (3.8 g, 16.38 mmol) was dissolved in DMF (70 mL), NaH (980 mg, 24.57 mmol, 60%) was added under cooling in an ice bath, and the mixture was stirred at 0°C for 30 minutes. Then, ethoxycarbonyl isothiocyanate (2.58 g, 19.65 mmol) was added, and the mixture was heated and reacted at 100°C for 18 hours. After the reaction was completed as monitored by LCMS, the reaction solution was cooled to room temperature and diluted with ethyl acetate, and the organic phase was separated and washed with saturated brine. The organic phase was dried and filtered, the filtrate was concentrated, and the resulting residue was separated by fast silica gel column chromatography to obtain ethyl (4-bromo-3-cyano-7-fluorobenzo[b]thiophen-2-yl)carbamate (4.78 g, yield: 85.1%). ESI-MS: 344.4 [M+1]⁺.

### Step 2: Synthesis of 2-amino-4-bromo-7-fluorobenzo[b]thiophene-3-carbonitrile

Ethyl (4-bromo-3-cyano-7-fluorobenzo[b]thiophen-2-yl)carbamate (4.78 g, 13.93 mmol) was dissolved in DMSO (19 mL), and aqueous NaOH solution (15.6 mL, 78 mmol, 5 N) was added at 0°C. After the addition, the mixture was heated and stirred at 125°C for 18 hours. The reaction was completed as monitored by LCMS. After the reaction was completed, the reaction solution was diluted with ethyl acetate, and the organic phase was separated and washed with saturated brine. The organic phase was dried and filtered, the filtrate was concentrated, and the resulting residue was separated by fast silica gel column chromatography to obtain 2-amino-4-bromo-7-fluorobenzo[b]thiophene-3-carbonitrile (1.91 g, yield: 50.5%). ESI-MS: 272.3 [M+1]⁺.

### Step 3: Synthesis of tert-butyl (4-bromo-3-cyano-7-fluorobenzo[b]thiophen-2-yl)carbamate

2-amino-4-bromo-7-fluorobenzo[b]thiophene-3-carbonitrile (1.91 g, 7.04 mmol), DMAP (70 mg, 0.56 mmol) and DIPEA (1.37 g, 10.6 mmol) were dissolved in a mixed solution of THF (60 mL) and DMF (5 mL), and Boc₂O (1.69 g, 7.75 mmol) was added under cooling in an ice-water bath. After the addition, the mixture was stirred at room temperature for 18 hours. The reaction was completed as monitored by LCMS. After the reaction was completed, the reaction solution was diluted with ethyl acetate, and the organic phase was separated and washed once with saturated brine. The organic phase was dried and filtered, the filtrate was concentrated, and the resulting residue was separated by fast silica gel column chromatography to obtain tert-butyl (4-bromo-3-cyano-7-fluorobenzo[b]thiophen-2-yl)carbamate (1.69 g, yield: 64.5%). ESI-MS: 372.3 [M+1]⁺.

### Step 4: Synthesis of tert-butyl (3-cyano-4-(5,5-dimethyl-1,3,2-dioxaborolan-2-yl)-7-fluorobenzo[b]thiophen-2-yl)carbamate

Tert-butyl (4-bromo-3-cyano-7-fluorobenzo[b]thiophen-2-yl)carbamate (1.59 g, 4.28 mmol), KOAc (1.26 g, 12.85 mmol) and bis(neopentylglycol)diboron (3.68 g, 16.28 mmol) were dissolved in 1,4-dioxane (40 mL), DPEPhosPdCl₂ (230 mg, 0.43 mmol) was added, the nitrogen substitution was carried out three times, and the mixture was heated and stirred at 95°C for 18 hours. The reaction was completed as monitored by LCMS. After the reaction was completed, the reaction solution was concentrated, and the resulting residue was separated by fast silica gel column chromatography to obtain tert-butyl (3-cyano-4-(5,5-dimethyl-1,3,2-dioxaborolan-2-yl)-7-fluorobenzo[b]thiophen-2-yl)carb amate (1.08 g, yield: 62.4%). ESI-MS: 405.4 [M+1]⁺.

### Intermediate 4-3: Preparation of tert-butyl (3-cyano-4-(5,5-dimethyl-1,3,2-dioxaborolan-2-yl)benzo[b]thiophen-2-yl)carbamate

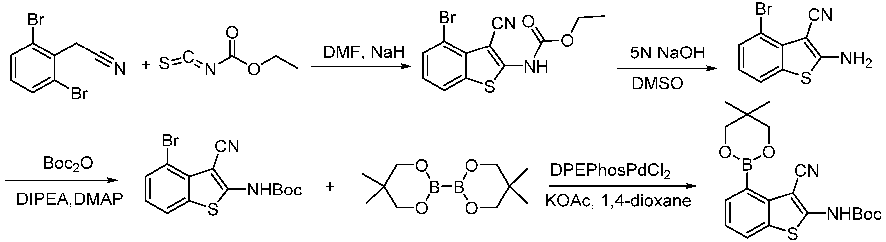

Refer to the synthesis steps of Intermediate 4-2 and select the corresponding raw materials for preparation.

### Intermediate 4-4: Preparation of N-(6-fluoro-4-(4,4,5,5-tetramethyl-1,3,2-dio xaborolan-2-yl)-5-((triisopropylsilyl)ethynyl)naphthalen-2-yl)-1,1-diphenylmethani mine

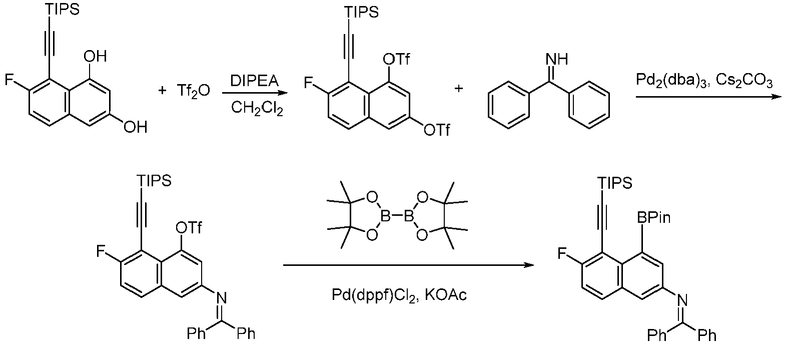

### Step 1: Synthesis of 7-fluoro-8-((triisopropylsilyl)ethynyl)naphthalen-1,3-diy lbis(trifluoromethanesulfonate)

7-fluoro-8-((triisopropylsilyl)ethynyl)naphthalen-1,3-diphenol (2.0 g, 5.58 mmol) was dissolved in CH₂Cl₂ (30 mL), DIPEA (5.53 mL, 33.47 mmol) and Tf₂O (3.7 mL, 22.31 mmol) were added in sequence at 0°C, and the mixture was heated to room temperature and reacted for 4 hours. After the reaction was completed, the reaction solution was diluted with saturated aqueous NaHCO₃ solution (50 mL) and extracted three times with CH₂Cl₂ (30 mL). The organic phase was combined, dried over anhydrous sodium sulfate and filtered, and the filtrate was concentrated. The concentrated residue was separated by fast silica gel column chromatography to obtain 7-fluoro-8-((triisopropylsilyl)ethynyl)naphthalen-1,3-diylbis(trifluoromethanesulfonate) (3.2 g, 5.14 mmol, yield: 92%).

¹H NMR (400 MHz, CDCl₃) δ 7.87 (dd, *J* = 9.1, 5.3 Hz, 1H), 7.80 (d, *J* = 2.4 Hz, 1H), 7.53 - 7.46 (m, 2H), 1.28 - 1.21 (m, 3H), 1.20 - 1.13 (m, 18H).

### Step 2: Synthesis of 3-((diphenylmethylene)amino)-7-fluoro-8-((triisopropyls ilyl)ethynyl)naphthalen-1-yl trifluoromethanesulfonate

7-fluoro-8-((triisopropylsilyl)ethynyl)naphthalen-1,3-diylbis(trifluoromethanesulfo nate) (2.7 g, 4.34 mmol) was dissolved in 1,4-dioxane (20 mL), Cs₂CO₃ (4.24 g, 13.01 mmol), XantPhos (0.5 g, 0.87 mmol) and Pd₂(dba)₃ (0.4 g, 0.43 mmol) were added, the nitrogen balloon substitution was carried out three times, and under nitrogen protection, the mixture was heated to 100°C, stirred and reacted for 5 hours. TLC traced the reaction and showed that the raw materials were completely reacted and new points were generated. The reaction solution was concentrated and the concentrated residue was separated by fast silica gel column chromatography to obtain 3-((diphenylmethylene)amino)-7-fluoro-8-((triisopropylsilyl)ethynyl)naphthalen-1-yl trifluoromethanesulfonate (360 mg, 0.55 mmol, yield: 13%) and some crude products (4.3 g, 1.64 mmol, purity: 25%). ESI-MS 654.5 [M+1]⁺.

### Step 3: Synthesis of N-(6-fluoro-4-(4,4,5,5-tetramethyl-1,3,2-dioxaborolan-2-yl)-5-((triisopropylsilyl)ethynyl)naphthalen-2-yl)-1,1-diphenylmethanimine

3-((diphenylmethylene)amino)-7-fluoro-8-((triisopropylsilyl)ethynyl)naphthalen-1-yl trifluoromethanesulfonate (200 mg, 0.31 mmol) was dissolved in 1,4-dioxane (5 mL), and B₂(Pin)₂ (155 mg, 0.61 mmol), KOAc (90 mg, 0.92 mmol) and Pd(dppf)Cl₂ (45 mg, 0.06 mmol) were added. The nitrogen balloon substitution was carried out three times, and under nitrogen protection, the mixture was heated to 100°C, stirred and reacted for 2 hours. LCMS monitored the reaction and showed that the reaction was completed. The reaction solution was cooled to room temperature and concentrated, and the concentrated residue was separated by fast silica gel column chromatography to obtain N-(6-fluoro-4-(4,4,5,5-tetramethyl-1,3,2-dioxaborolan-2-yl)-5-((triisopropylsilyl)ethyny l)naphthalen-2-yl)-1,1-diphenylmethanimine (100 mg, 0.16 mmol, yield: 52%). ESI-MS 632.6 [M+1]⁺.

### II. Specific Examples

### Example 1: Preparation of 4-(2-((2,6-dimethylenetetrahydro-1H-pyrrolizin-7 a(5H)-yl)methoxy)-8-fluoro-4-(1-((methoxy-d₃)methyl)-3,8-diazabicyclo[3.2.1]octan -3-yl)pyrido[4,3-d]pyrimidin-7-yl)-5-ethynyl-6-fluoronaphthalen-2-ol

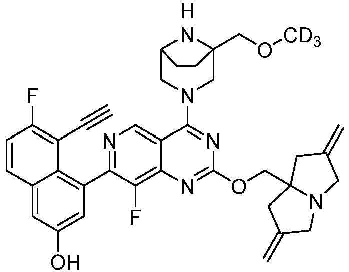

### Step 1: Synthesis of tert-butyl 3-(2,7-dichloro-8-fluoropyrido[4,3-d]pyrimidi n-4-yl)-1-((methoxy-d₃)methyl)-3,8-diazabicyclo[3.2.1]octan-8-carboxylate

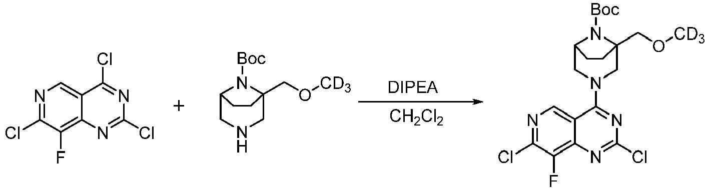

2,4,7-trichloro-8-fluoropyrido[4,3-d]pyrimidine (987.0 mg, 3.91 mmol) was diss olved in CH₂Cl₂ (20 mL), and DIPEA (3.88 g, 30.07 mmol) and tert-butyl (1S,5R)-1-((methoxy-*d*₃)methyl)-3,8-diazabicyclo[3.2.1]octan-8-carboxylate (780 mg, 3.01 m mol) were added at 0°C, and the mixture was reacted at 0°C for 2 hours. After the reaction was completed, the solution was diluted with ethyl acetate, and the organi c phase was separated and washed with saturated brine once. The organic phase wa s dried, filtered and concentrated, and the resulting residue was separated by fast sil ica gel column chromatography to obtain tert-butyl 3-(2,7-dichloro-8-fluoropyrido[4, 3-d]pyrimidin-4-yl)-1-((methoxy-*d*₃)methyl)-3,8-diazabicyclo[3.2.1]octan-8-carboxylat e (715 mg, yield: 50%). ESI-MS: 475.2 [M+1]⁺.

### Step 2: Synthesis of tert-butyl 3-(7-chloro-2-((2,6-dimethylenetetrahydro-1H -pyrrolizin-7a(5H)-yl)methoxy)-8-fluoropyrido[4,3-d]pyrimidin-4-yl)-1-((methoxy-d₃)methyl)-3,8-diazabicyclo[3.2.1]octan-8-carboxylate

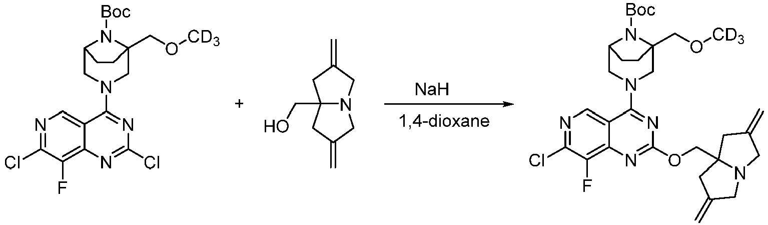

(2,6-dimethylenetetrahydro-1H-pyrrolizin-7a(5H)-yl)methanol (469.27 mg, 2.8 4 mmol) was dissolved in dry 1,4-dioxane (30 mL), 60% NaH (113.60 mg, 2.84 m mol) was added, and the mixture was heated and stirred at 40°C for 30 minutes. T hen, tert-butyl 3-(2,7-dichloro-8-fluoropyrido[4,3-d]pyrimidin-4-yl)-1-((methoxy-*d*₃)m ethyl)-3,8-diazabicyclo[3.2.1]octan-8-carboxylate (675 mg, 1.42 mmol) was added. T he mixture was reacted at 40°C for 2 hours. After the reaction was completed, the reaction solution was diluted with ethyl acetate, the organic phase was separated, w ashed once with saturated brine, dried over anhydrous sodium sulfate, filtered and c oncentrated, and the resulting residue was separated by fast silica gel column chro matography to obtain tert-butyl 3-(7-chloro-2-((2,6-dimethylenetetrahydro-1H-pyrroli zin-7a(5H)-yl)methoxy)-8-fluoropyrido[4,3-d]pyrimidin-4-yl)-1-((methoxy-*d*₃)methyl)-3,8-diazabicyclo[3.2.1]octan-8-carboxylate (544 mg, yield: 63.4%). ESI-MS: 604.4 [M+1]⁺.

### Step 3: Synthesis of tert-butyl 3-(2-((2,6-dimethylenetetrahydro-1H-pyrroliz in-7a(5H)-yl)methoxy)-8-fluoro-7-(7-fluoro-3-(methoxymethoxy)-8-((triisopropylsil yl)ethynyl)naphthalen-1-yl)pyrido[4,3-d]pyrimidin-4-yl)-1-((methoxy-d₃)methyl)-3, 8-diazabicyclo [3.2.1] octan-8-carboxylate

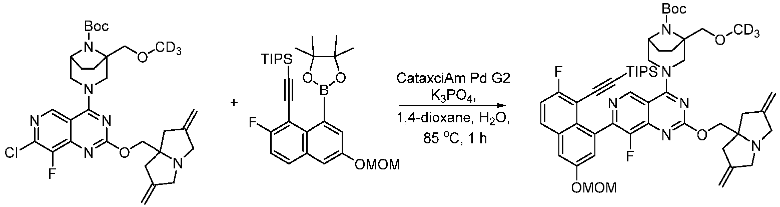

Tert-butyl 3-(7-chloro-2-((2,6-dimethylenetetrahydro-1H-pyrrolizin-7a(5H)-yl)met hoxy)-8-fluoropyrido[4,3-d]pyrimidin-4-yl)-1-((methoxy-*d*₃)methyl)-3,8-diazabicyclo[3. 2.1]octan-8-carboxylate (524 mg, 0.87 mmol), ((2-fluoro-6-(methoxymethoxy)-8-(4,4, 5,5-tetramethyl-1,3,2-dioxaborolan-2-yl)naphthalen-1-yl)ethynyl)triisopropylsilane (66 6.8 mg, 1.30 mmol) and K₃PO₄ (552.34 mg, 2.60 mmol) were dissolved in THF (1 0 mL), water (1 mL), and then CataxciAm Pd G2 (63.2 mg, 0.087 mmol) was add ed. The nitrogen substitution was carried out three times, and the mixture was heate d and reacted at 70°C for 2 hours. After the reaction was completed, the reaction s olution was diluted with ethyl acetate, the organic phase was separated, washed onc e with saturated brine, dried over anhydrous sodium sulfate, filtered and concentrate d, and the resulting residue was separated by fast silica gel column chromatography to obtain tert-butyl 3-(2-((2,6-dimethylenetetrahydro-1H-pyrrolizin-7a(5H)-yl)methox y)-8-fluoro-7-(7-fluoro-3-(methoxymethoxy)-8-((triisopropylsilyl)ethynyl)naphthalen-1 -yl)pyrido[4,3-d]pyrimidin-4-yl)-1-((methoxy-*d*₃)methyl)-3,8-diazabicyclo[3.2.1]octan-8-carboxylate (354 mg, yield: 42.8%). ESI-MS: 954.5 [M+1]⁺.

### Step 4: Synthesis of 4-(2-((2,6-dimethylenetetrahydro-1H-pyrrolizin-7a(5H)-yl)methoxy)-8-fluoro-4-(1-((methoxy-d₃)methyl)-3,8-diazabicyclo[3.2.1]octan-3-yl)p yrido[4,3-d]pyrimidin-7-yl)-6-fluoro-5-((triisopropylsilyl)ethynyl)naphthalen-2-ol

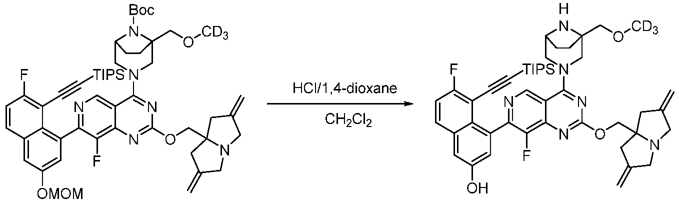

Tert-butyl 3-(2-((2,6-dimethylenetetrahydro-1H-pyrrolizin-7a(5H)-yl)methoxy)-8-fluoro-7-(7-fluoro-3-(methoxymethoxy)-8-((triisopropylsilyl)ethynyl)naphthalen-1-yl)p yrido[4,3-d]pyrimidin-4-yl)-1-((methoxy-*d*₃)methyl)-3,8-diazabicyclo[3.2.1]octan-8-car boxylate (334 mg, 0.35 mmol) was dissolved in CH₂Cl₂ (20 mL), hydrochloric acid/ dioxane solution (5 mL) was added, and the mixture was reacted at 0°C for 2 hour s. After the reaction was completed, the reaction solution was concentrated to obtai n 4-(2-((2,6-dimethylenetetrahydro-1H-pyrrolizin-7a(5H)-yl)methoxy)-8-fluoro-4-(1-((methoxy-*d*₃)methyl)-3,8-diazabicyclo[3.2.1]octan-3-yl)pyrido[4,3-d]pyrimidin-7-yl)-6 -fluoro-5-((triisopropylsilyl)ethynyl)naphthalen-2-ol (283.54 mg, yield: 100%). ESI-MS: 810.4 [M+1]⁺.

### Step 5: Synthesis of 4-(2-((2,6-dimethylenetetrahydro-1H-pyrrolizin-7a(5H)-yl)methoxy)-8-fluoro-4-(1-((methoxy-d₃)methyl)-3,8-diazabicyclo[3.2.1]octan-3-yl)p yrido[4,3-d]pyrimidin-7-yl)-5-ethynyl-6-fluoronaphthalen-2-ol

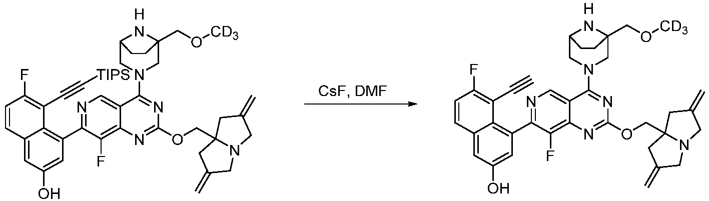

4-(2-((2,6-dimethylenetetrahydro-1H-pyrrolizin-7a(5H)-yl)methoxy)-8-fluoro-4-(1 -((methoxy-*d*₃)methyl)-3,8-diazabicyclo[3.2.1]octan-3-yl)pyrido[4,3-d]pyrimidin-7-yl)-6-fluoro-5-((triisopropylsilyl)ethynyl)naphthalen-2-ol (283.54 mg, 0.35 mmol) was di ssolved in DMF (10 mL), CsF (5.32 g, 35.0 mmol) was added, and the mixture wa s heated and stirred at 50°C for 2 hours. The reaction was completed as monitored by LCMS and the reaction solution was filtered. After the concentration, it was sep arated by reversed-phase column chromatography to obtain 4-(2-((2,6-dimethylenetet rahydro-1H-pyrrolizin-7a(5H)-yl)methoxy)-8-fluoro-4-(1-((methoxy-*d*₃)methyl)-3,8-dia zabicyclo[3.2.1]octan-3-yl)pyrido[4,3-d]pyrimidin-7-yl)-5-ethynyl-6-fluoronaphthalen-2-ol (134.5 mg, yield: 58.8% ) ESI-MS: 654.3 [M+1]⁺.

¹H NMR (400 MHz, MeOH-*d*₄) δ 9.00 (d, *J* = 4.3 Hz, 1H), 7.85 (dd, *J* = 9.1, 5.7 Hz, 1H), 7.38 - 7.27 (m, 2H), 7.21 (d, *J* = 1.9 Hz, 1H), 5.01 (d, *J* = 7.3 Hz, 4H), 4.71 - 4.53 (m, 2H), 4.33 - 4.30 (m, 2H), 3.76 (d, *J* = 14.9 Hz, 2H), 3.70 - 3.62 (m, 2H), 3.58 (dd, *J=* 12.3, 8.2 Hz, 1H), 3.51 - 3.48 (m, 2H), 3.37 - 3.35 (m, 2H), 3.31 (s, 1H), 2.78 (d, *J=* 16.5 Hz, 2H), 2.58 (d, *J* = 16.4 Hz, 2H), 1.97 - 1.89 (m, 1H), 1.83 - 1.69 (m, 2H), 1.65 - 1.58 (m, 1H).

### Example 2: Preparation of 4-(2-((2,6-dimethylenetetrahydro-1H-pyrrolizin-7 a(5H)-yl)methoxy)-8-fluoro-4-(1-(2-hydroxypropan-2-yl)-3,8-diazabicyclo[3.2.1]oct an-3-yl)pyrido[4,3-d]pyrimidin-7-yl)-5-ethynyl-6-fluoronaphthalen-2-ol

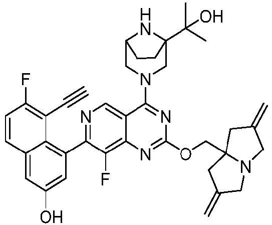

### Step 1: Synthesis of tert-butyl 3-(2-((2,6-dimethylenetetrahydro-1H-pyrroliz in-7a(5H)-yl)methoxy)-7-(8-ethynyl-7-fluoro-3-(methoxymethoxy)naphthalen-1-yl)-8-fluoropyrido[4,3-d]pyrimidin-4-yl)-1-(2-hydroxypropan-2-yl)-3,8-diazabicyclo[3. 2.1]octan-8-carboxylate

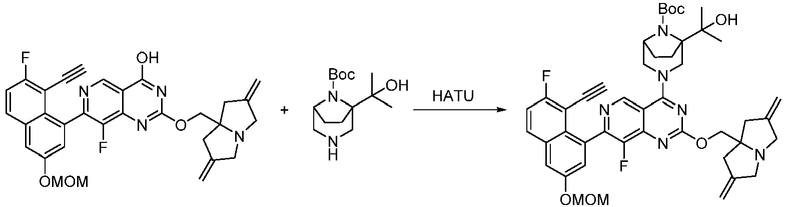

2-((2,6-dimethylenetetrahydro-1H-pyrrolizin-7a(5H)-yl)methoxy)-7-(8-ethynyl-7-f luoro-3-(methoxymethoxy)naphthalen-1-yl)-8-fluoropyrido[4,3-d]pyrimidin-4-ol (19 0 mg, 0.34 mmol) was dissolved in CH₂Cl₂ (10 mL), DIPEA (132 mg, 1.024 mmol) and HATU (156 mg, 0.410 mmol) were added, and the mixture was stirred at roo m temperature for 10 minutes. Then, tert-butyl (1S,5R)-1-(2-hydroxypropan-2-yl)-3,8 -diazabicyclo[3.2.1]octan-8-carboxylate (101.5 mg, 0.376 mmol) was added, and the mixture was reacted at room temperature for 2 hours. After the reaction was compl eted, the reaction solution was concentrated and separated by column chromatograp hy to obtain tert-butyl 3-(2-((2,6-dimethylenetetrahydro-1H-pyrrolizin-7a(5H)-yl)meth oxy)-7-(8-ethynyl-7-fluoro-3-(methoxymethoxy)naphthalen-1-yl)-8-fluoropyrido[4,3-d] pyrimidin-4-yl)-1-(2-hydroxypropan-2-yl)-3,8-diazabicyclo[3.2.1]octan-8-carboxylate (180 mg, yield: 65.2%). ESI-MS: 809.4 [M+1]⁺.

### Step 2: Synthesis of 4-(2-((2,6-dimethylenetetrahydro-1H-pyrrolizin-7a(5H)-yl)methoxy)-8-fluoro-4-(1-(2-hydroxypropan-2-yl)-3,8-diazabicyclo [3.2.1] octan-3-yl) pyrido[4,3-d]pyrimidin-7-yl)-5-ethynyl-6-fluoronaphthalen-2-ol

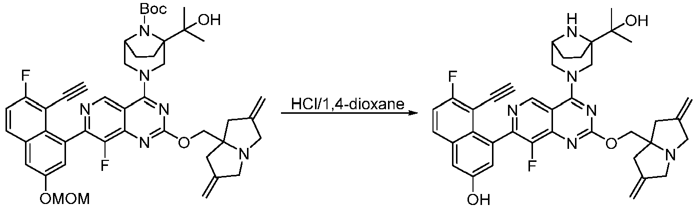

Refer to Step 4 of Example 1 for synthesis. ESI-MS: 665.4 [M+1]⁺.

¹H NMR (400 MHz, MeOH-*d*₄) δ 9.01 (s, 1H), 7.89 - 7.83 (m, 1H), 7.37 - 7.27 (m, 2H), 7.21 (dd, J = 7.7, 2.5 Hz, 1H), 5.02 (d, J = 8.4 Hz, 4H), 4.61 (s, 1H), 4.53 (d, J = 1 3.6 Hz, 1H), 4.42 - 4.32 (m, 2H), 3.86 - 3.67 (m, 4H), 3.51 (dd, J = 28.5, 12.4 Hz, 1H), 3.40 - 3.32 (m, 3H), 2.78 (d, J = 16.6 Hz, 2H), 2.59 (dd, J = 16.4, 7.1 Hz, 2H), 1.98 - 1.8 1 (m, 2H), 1.80 - 1.64 (m, 2H), 1.37 (d, J = 1.9 Hz, 3H), 1.34 (d, J = 5.1 Hz, 3H).

**Examples 3 to 172 and 355 to 357 and reference compounds 1 to 4 can be prepared by selecting the corresponding intermediates with reference to the route of Example 1 or 2.**

| **Example No.** | **Structural Formula** | **Chemical Name** | **ESI-MS: [M+1]⁺** |
|---|---|---|---|
| **3** | 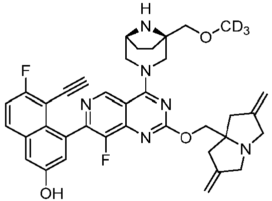 | 4-(2-((2,6-dimethylenetetrahyd ro-1H-pyrrolizin-7a(5H)-yl)me thoxy)-8-fluoro-4-((1R,5S)-1-( (methoxy-*d*₃)methyl)-3,8-diaza bicyclo[3.2.1]octan-3-yl)pyrid o[4,3-d]pyrimidin-7-yl)-5-ethy nyl-6-fluoronaphthalen-2-ol | 654.3 |
| **4** | 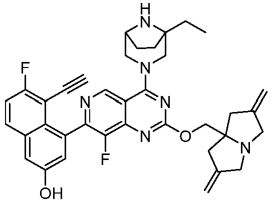 | 4-(2-((2,6-dimethylenetetrahyd ro-1H-pyrrolizin-7a(5H)-yl)me thoxy)-4-(1-ethyl-3,8-diazabic yclo[3.2.1]octan-3-yl)-8-fluoro pyrido[4,3-d]pyrimidin-7-yl)-5 -ethynyl-6-fluoronaphthalen-2-ol | 635.4 |
| **5** | 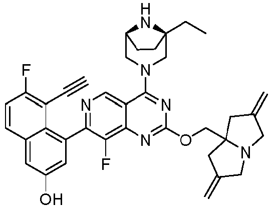 | 4-(2-((2,6-dimethylenetetrahyd ro-1H-pyrrolizin-7a(5H)-yl)me thoxy)-4-((1R,5S)-1-ethyl-3,8-diazabicyclo[3.2.1]octan-3-yl)-8-fluoropyrido[4,3-d]pyrimidin -7-yl)-5-ethynyl-6-fluoronapht halen-2-ol | 635.4 |
| **6** | 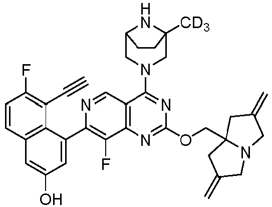 | 4-(2-((2,6-dimethylenetetrahyd ro-1H-pyrrolizin-7a(5H)-yl)me thoxy)-8-fluoro-4-(1-(methyl-*d* ₃)-3,8-diazabicyclo[3.2.1]octan -3-yl)pyrido[4,3-d]pyrimidin-7 -yl)-5-ethynyl-6-fluoronaphtha len-2-ol | 624.4 |
| **7** | 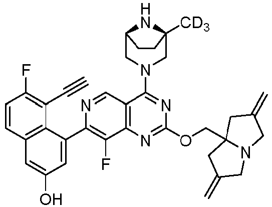 | 4-(2-((2,6-dimethylenetetrahyd ro-1H-pyrrolizin-7a(5H)-yl)me thoxy)-8-fluoro-4-((1R,5S)-1-( methyl-*d*₃)-3,8-diazabicyclo[3. 2.1]octan-3-yl)pyrido[4,3-d]py rimidin-7-yl)-5-ethynyl-6-fluor onaphthalen-2-ol | 624.4 |
| **8** | 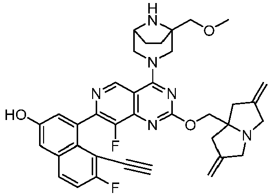 | 4-(2-((2,6-dimethylenetetrahyd ro-1H-pyrrolizin-7a(5H)-yl)me thoxy)-8-fluoro-4-(1-(methoxy methyl)-3,8-diazabicyclo[3.2.1 ]octan-3-yl)pyrido[4,3-d]pyrim idin-7-yl)-5-ethynyl-6-fluorona phthalen-2-ol | 651.4 |
| **9** | 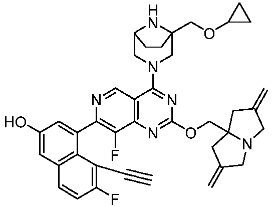 | 4-(4-(1-(cyclopropyloxymethyl )-3,8-diazabicyclo[3.2.1]octan-3-yl)-2-((2,6-dimethylenetetrah ydro-1H-pyrrolizin-7a(5H)-yl) methoxy)-8-fluoropyrido[4,3-d ]pyrimidin-7-yl)-5-ethynyl-6-fl uoronaphthalen-2-ol | 677.4 |
| **10** | 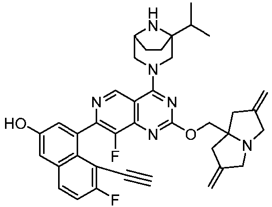 | 4-(2-((2,6-dimethylenetetrahyd ro-1H-pyrrolizin-7a(5H)-yl)me thoxy)-8-fluoro-4-(1-isopropyl -3,8-diazabicyclo[3.2.1]octan-3-yl)pyrido[4,3-d]pyrimidin-7-yl)-5-ethynyl-6-fluoronaphthal en-2-ol | 649.4 |
| **11** | 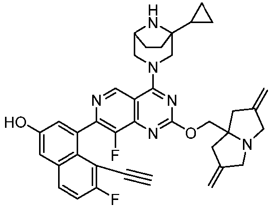 | 4-(4-(1-cyclopropyl-3,8-diazab icyclo[3.2.1]octan-3-yl)-2-((2, 6-dimethylenetetrahydro-1H-p yrrolizin-7a(5H)-yl)methoxy)-8-fluoropyrido[4,3-d]pyrimidin -7-yl)-5-ethynyl-6-fluoronapht halen-2-ol | 647.4 |
| **12** | 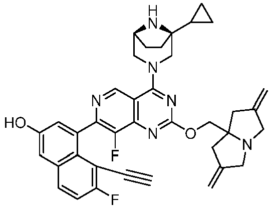 | 4-(4-((1S,5S)-1-cyclopropyl-3, 8-diazabicyclo[3.2.1]octan-3-y l)-2-((2,6-dimethylenetetrahydr o-1H-pyrrolizin-7a(5H)-yl)met hoxy)-8-fluoropyrido[4,3-d]pyr imidin-7-yl)-5-ethynyl-6-fluor onaphthalen-2-ol | 647.4 |
| **13** | 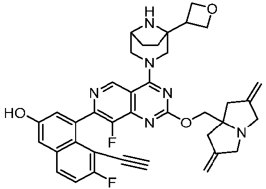 | 4-(2-((2,6-dimethylenetetrahyd ro-1H-pyrrolizin-7a(5H)-yl)me thoxy)-8-fluoro-4-(1-(oxetan-3 -yl)-3,8-diazabicyclo[3.2.1]oct an-3-yl)pyrido[4,3-d]pyrimidin -7-yl)-5-ethynyl-6-fluoronapht halen-2-ol | 663.4 |
| **14** | 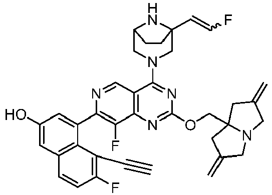 | 4-(2-((2,6-dimethylenetetrahyd ro-1H-pyrrolizin-7a(5H)-yl)me thoxy)-8-fluoro-4-(1-(2-fluoro vinyl)-3,8-diazabicyclo[3.2.1]o ctan-3-yl)pyrido[4,3-d]pyrimid in-7-yl)-5-ethynyl-6-fluoronap hthalen-2-ol | 651.4 |
| **15** | 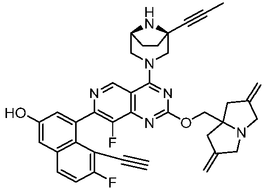 | 4-(2-((2,6-dimethylenetetrahyd ro-1H-pyrrolizin-7a(5H)-yl)me thoxy)-8-fluoro-4-((1S,5S)-1-( prop-1-yn-1-yl)-3,8-diazabicyc lo[3.2.1]octan-3-yl)pyrido[4,3-d]pyrimidin-7-yl)-5-ethynyl-6-fluoronaphthalen-2-ol | 645.4 |
| **16** | 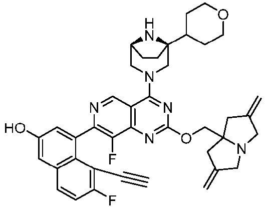 | 4-(2-((2,6-dimethylenetetrahyd ro-1H-pyrrolizin-7a(5H)-yl)me thoxy)-8-fluoro-4-((1S,5S)-1-(t etrahydro-2H-pyran-4-yl)-3,8-diazabicyclo[3.2.1]octan-3-yl) pyrido[4,3-d]pyrimidin-7-yl)-5 -ethynyl-6-fluoronaphthalen-2-ol | 691.4 |
| **17** | 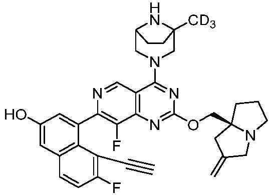 | 5-ethynyl-6-fluoro-4-(8-fluoro-4-(1-(methyl-***d*₃**)-3,8-diazabicy clo[3.2.1]octan-3-yl)-2-(((S)-2-methylenetetrahydro-1H-pyrro lizin-7a(5H)-yl)methoxy)pyrid o[4,3-d]pyrimidin-7-yl)naphth alen-2-ol | 612.4 |
| **18** | 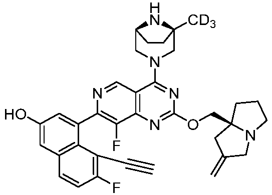 | 5-ethynyl-6-fluoro-4-(8-fluoro-4-((1R,5S)-1-(methyl-***d*₃**)-3,8-d iazabicyclo[3.2.1]octan-3-yl)-2 -(((S)-2-methylenetetrahydro-1 H-pyrrolizin-7a(5H)-yl)metho xy)pyrido[4,3-d]pyrimidin-7-yl )naphthalen-2-ol | 612.4 |
| **19** | 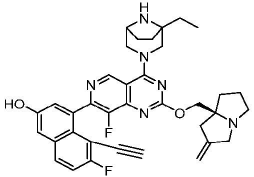 | 4-(4-(1-ethyl-3,8-diazabicyclo[ 3.2.1]octan-3-yl)-8-fluoro-2-((( S)-2-methylenetetrahydro-1H-pyrrolizin-7a(5H)-yl)methoxy) pyrido[4,3-d]pyrimidin-7-yl)-5 -ethynyl-6-fluoronaphthalen-2-ol | 623.4 |
| **20** | 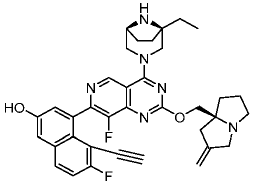 | 4-(4-((1R,5S)-1-ethyl-3,8-diaz abicyclo[3.2.1]octan-3-yl)-8-fl uoro-2-(((S)-2-methylenetetrah ydro-1H-pyrrolizin-7a(5H)-yl) methoxy)pyrido[4,3-d]pyrimid in-7-yl)-5-ethynyl-6-fluoronap hthalen-2-ol | 623.4 |
| **21** | 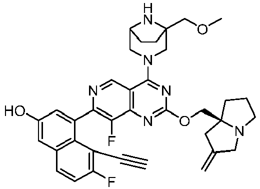 | 5-ethynyl-6-fluoro-4-(8-fluoro-4-(1-(methoxymethyl)-3,8-diaz abicyclo[3.2.1]octan-3-yl)-2-(( (S)-2-methylenetetrahydro-1H-pyrrolizin-7a(5H)-yl)methoxy) pyrido[4,3-d]pyrimidin-7-yl)na phthalen-2-ol | 639.4 |
| **22** | 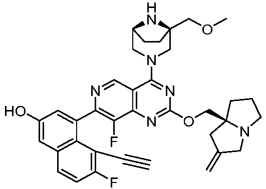 | 5-ethynyl-6-fluoro-4-(8-fluoro-4-((1R,5 S)-1-(methoxymethyl) -3,8-diazabicyclo[3.2.1]octan-3-yl)-2-(((S)-2-methylenetetra hydro-1H-pyrrolizin-7a(5H)-yl )methoxy)pyrido[4,3-d]pyrimi din-7-yl)naphthalen-2-ol | 639.4 |
| **23** | 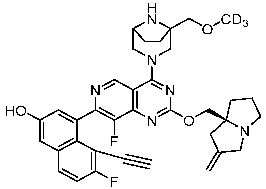 | 5-ethynyl-6-fluoro-4-(8-fluoro-4-(1-((methoxy-***d*₃**)methyl)-3,8 -diazabicyclo[3.2.1]octan-3-yl) -2-(((S)-2-methylenetetrahydro -1H-pyrrolizin-7a(5H)-yl)meth oxy)pyrido[4,3-d]pyrimidin-7-yl)naphthalen-2-ol | 642.4 |
| **24** | 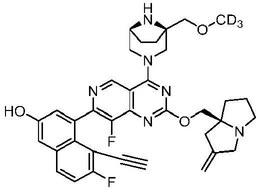 | 5-ethynyl-6-fluoro-4-(8-fluoro-4-((1R,5S)-1-((methoxy-***d*₃**)me thyl)-3,8-diazabicyclo[3.2.1]oc tan-3-yl)-2-(((S)-2-methylenet etrahydro-1H-pyrrolizin-7a(5H )-yl)methoxy)pyrido[4,3-d]pyri midin-7-yl)naphthalen-2-ol | 642.4 |
| **25** | 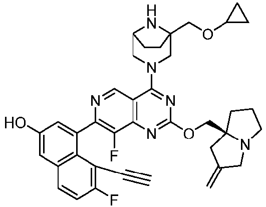 | 4-(4-(1-(cyclopropyloxymethyl )-3,8-diazabicyclo[3.2.1]octan-3-yl)-8-fluoro-2-(((S)-2-methyl enetetrahydro-1H-pyrrolizin-7 a(5H)-yl)methoxy)pyrido[4,3-d]pyrimidin-7-yl)-5-ethynyl-6-fluoronaphthalen-2-ol | 665.4 |
| **26** | 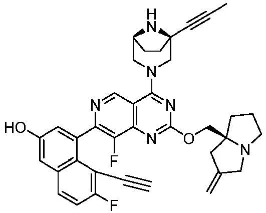 | 4-(4-((1S,5S)-1-cyclopentyl-3,8-diazabicyclo[3.2.1]octan-3-yl)-8 -fluoro-2-(((S)-2-methylenetetra hydro-1H-pyrrolizin-7a(5H)-yl) methoxy)pyrido[4,3-d]pyrimidin -7-yl)-5-ethynyl-6-fluoronaphtha len-2-ol | 633.4 |
| **27** | 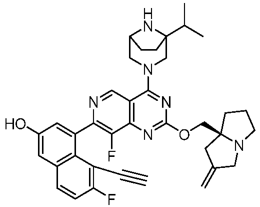 | 5-ethynyl-6-fluoro-4-(8-fluoro-4-(1-isopropyl-3,8-diazabicycl o[3.2.1]octan-3-yl)-2-(((S)-2-methylenetetrahydro-1H-pyrro lizin-7a(5H)-yl)methoxy)pyrid o[4,3-d]pyrimidin-7-yl)naphth alen-2-ol | 637.4 |
| **28** | 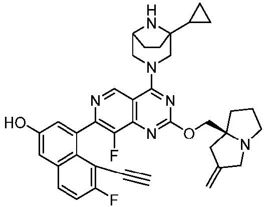 | 4-(4-(1-cyclopropyl-3,8-diazab icyclo[3.2.1]octan-3-yl)-8-fluo ro-2-(((S)-2-methylenetetrahyd ro-1H-pyrrolizin-7a(5H)-yl)me thoxy)pyrido[4,3-d]pyrimidin-7-yl)-5-ethynyl-6-fluoronaphth alen-2-ol | 635.4 |
| **29** | 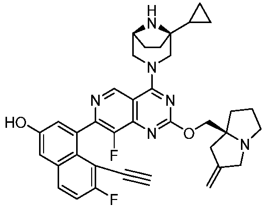 | 4-(4-((1S,5S)-1-cyclopropyl-3, 8-diazabicyclo[3.2.1]octan-3-y l)-8-fluoro-2-(((S)-2-methylen etetrahydro-1H-pyrrolizin-7a(5 H)-yl)methoxy)pyrido[4,3-d]p yrimidin-7-yl)-5-ethynyl-6-flu oronaphthalen-2-ol | 635.4 |
| **30** | 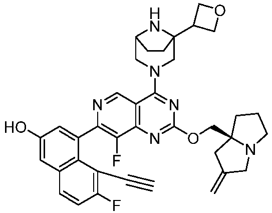 | 5-ethynyl-6-fluoro-4-(8-fluoro-2-(((S)-2-methylenetetrahydro-1H-pyrrolizin-7a(5H)-yl)meth oxy)-4-(1-(oxetan-3-yl)-3,8-dia zabicyclo[3.2.1]octan-3-yl)pyri do[4,3-d]pyrimidin-7-yl)napht halen-2-ol | 651.4 |
| **31** | 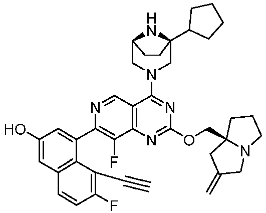 | 4-(4-((1S,5S)-1-cyclopentyl-3,8 -diazabicyclo[3.2.1]octan-3-yl)-8-fluoro-2-(((S)-2-methylenetet rahydro-1H-pyrrolizin-7a(5H)-yl)methoxy)pyrido[4,3-d]pyrim idin-7-yl)-5-ethynyl-6-fluorona phthalen-2-ol | 663.4 |
| **32** | 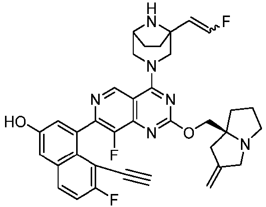 | 5-ethynyl-6-fluoro-4-(8-fluoro-4-(1-(2-fluorovinyl)-3,8-diazab icyclo[3.2.1]octan-3-yl)-2-(((S )-2-methylenetetrahydro-1H-p yrrolizin-7a(5H)-yl)methoxy)p yrido[4,3-d]pyrimidin-7-yl)nap hthalen-2-ol | 639.4 |
| **33** | 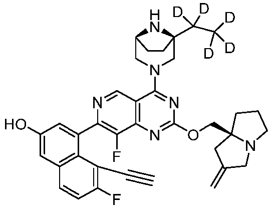 | 4-(4-((1R,5S)-1-(ethyl-ds)-3,8-diazabicyclo[3.2.1]octan-3-yl)-8-fluoro-2-(((S)-2-methylenete trahydro-1H-pyrrolizin-7a(5H) -yl)methoxy)pyrido[4,3-d]pyri midin-7-yl)-5-ethynyl-6-fluoro naphthalen-2-ol | 628.4 |
| **34** | 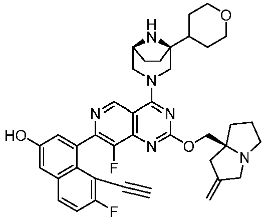 | 5-ethynyl-6-fluoro-4-(8-fluoro-2-(((S)-2-methylenetetrahydro-1H-pyrrolizin-7a(5H)-yl)meth oxy)-4-((1S,5S)-1-(tetrahydro-2H-pyran-4-yl)-3,8-diazabicycl o[3.2.1]octan-3-yl)pyrido[4,3-d]pyrimidin-7-yl)naphthalen-2 -ol | 679.4 |
| **35** | 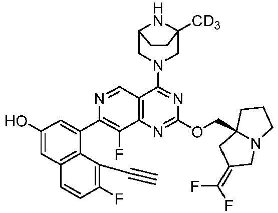 | 4-(2-(((S)-2-(difluoromethylen e)tetrahydro-1H-pyrrolizin-7a( 5H)-yl)methoxy)-8-fluoro-4-(1 -(methyl-***d*₃**)-3,8-diazabicyclo[ 3.2.1]octan-3-yl)pyrido[4,3-d] pyrimidin-7-yl)-5-ethynyl-6-fl uoronaphthalen-2-ol | 648.4 |
| **36** | 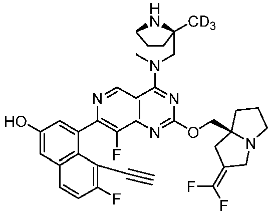 | 4-(2-(((S)-2-(difluoromethylen e)tetrahydro-1H-pyrrolizin-7a( 5H)-yl)methoxy)-8-fluoro-4-(( 1R,5S)-1-(methyl-***d*₃**)-3,8-diaz abicyclo[3.2.1]octan-3-yl)pyri do[4,3-d]pyrimidin-7-yl)-5-eth ynyl-6-fluoronaphthalen-2-ol | 648.4 |
| **37** | 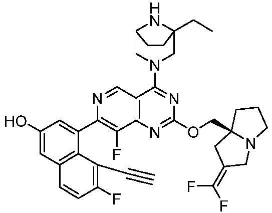 | 4-(2-(((S)-2-(difluoromethylen e)tetrahydro-1H-pyrrolizin-7a( 5H)-yl)methoxy)-4-(1-ethyl-3, 8-diazabicyclo[3.2.1]octan-3-y l-8-fluoropyrido[4,3-d]pyrimi din-7-yl)-5-ethynyl-6-fluorona phthalen-2-ol | 659.4 |
| **38** | 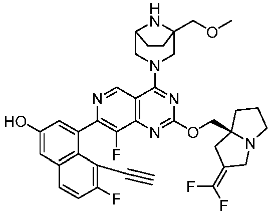 | 4-(2-(((S)-2-(difluoromethylen e)tetrahydro-1H-pyrrolizin-7a( 5H)-yl)methoxy)-8-fluoro-4-(1 -(methoxymethyl)-3,8-diazabic yclo[3.2.1]octan-3-yl)pyrido[4, 3-d]pyrimidin-7-yl)-5-ethynyl-6-fluoronaphthalen-2-ol | 675.4 |
| **39** | 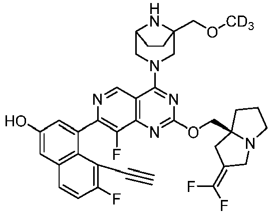 | 4-(2-(((S)-2-(difluoromethylen e)tetrahydro-1H-pyrrolizin-7a( 5H)-yl)methoxy)-8-fluoro-4-(1 -((methoxy-***d*₃**)methyl)-3,8-dia zabicyclo[3.2.1]octan-3-yl)pyri do[4,3-d]pyrimidin-7-yl)-5-eth ynyl-6-fluoronaphthalen-2-ol | 678.4 |
| **40** | 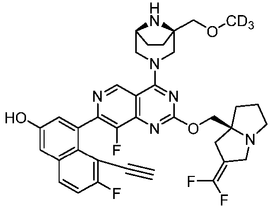 | 4-(2-(((S)-2-(difluoromethylen e)tetrahydro-1H-pyrrolizin-7a( 5H)-yl)methoxy)-8-fluoro-4-(( 1,5S)-1-((methoxy-***d*₃**)methyl )-3,8-diazabicyclo[3.2.1]octan-3-yl)pyrido[4,3-d]pyrimidin-7-yl)-5-ethynyl-6-fluoronaphthal en-2-ol | 678.4 |
| **41** | 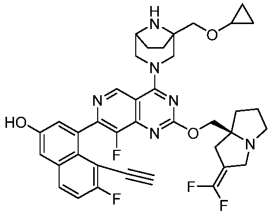 | 4-(4-(1-(cyclopropyloxymethyl )-3,8-diazabicyclo[3.2.1]octan-3-yl)-2-(((S)-2-(difluoromethyl ene)tetrahydro-1H-pyrrolizin-7 a(5H)-yl)methoxy)-8-fluoropyr ido[4,3-d]pyrimidin-7-yl)-5-et hynyl-6-fluoronaphthalen-2-ol | 701.4 |
| **42** | 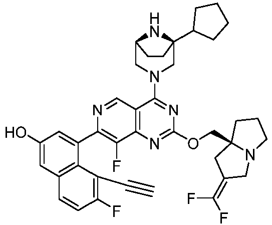 | 4-(4-((1S,5S)-1-cyclopentyl-3,8-diazabicyclo[3.2.1]octan-3-yl)-2 -(((S)-2-(difluoromethylene)tetra hydro-1H-pyrrolizin-7a(5H)-yl) methoxy)-8-fluoropyrido[4,3-d] pyrimidin-7-yl)-5-ethynyl-6-fluo ronaphthalen-2-ol | 699.4 |
| **43** | 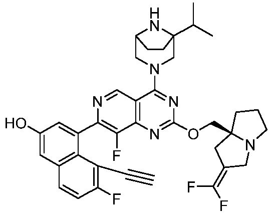 | 4-(2-(((S)-2-(difluoromethylen e)tetrahydro-1H-pyrrolizin-7a( 5H)-yl)methoxy)-8-fluoro-4-(1 -isopropyl-3,8-diazabicyclo[3. 2.1]octan-3-yl)pyrido[4,3-d]py rimidin-7-yl)-5-ethynyl-6-fluor onaphthalen-2-ol | 673.4 |
| **44** | 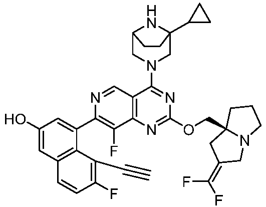 | 4-(4-(1-cyclopropyl-3,8-diazab icyclo[3.2.1]octan-3-yl)-2-(((S )-2-(difluoromethylene)tetrahy dro-1H-pyrrolizin-7a(5H)-yl)m ethoxy)-8-fluoropyrido[4,3-d]p yrimidin-7-yl)-5-ethynyl-6-flu oronaphthalen-2-ol | 671.4 |
| **45** | 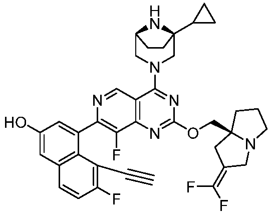 | 4-(4-((1S,5S)-1-cyclopropyl-3, 8-diazabicyclo[3.2.1]octan-3-y l)-2-(((S)-2-(difluoromethylene )tetrahydro-1H-pyrrolizin-7a(5 H)-yl)methoxy)-8-fluoropyrido [4,3-d]pyrimidin-7-yl)-5-ethyn yl-6-fluoronaphthalen-2-ol | 671.4 |
| **46** | 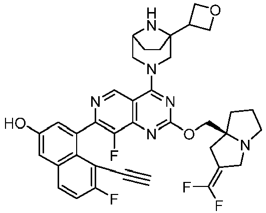 | 4-(2-(((S)-2-(difluoromethylen e)tetrahydro-1H-pyrrolizin-7a( 5H)-yl)methoxy)-8-fluoro-4-(1 -(oxetan-3-yl)-3,8-diazabicyclo [3.2.1]octan-3-yl)pyrido[4,3-d] pyrimidin-7-yl)-5-ethynyl-6-fl uoronaphthalen-2-ol | 687.4 |
| **47** | 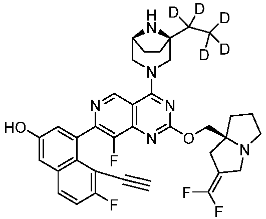 | 4-(2-(((S)-2-(difluoromethylen e)tetrahydro-1H-pyrrolizin-7a( 5H)-yl)methoxy)-4-((1R,5S)-1 -(ethyl-*d*₅)-3,8-diazabicyclo[3. 2.1]octan-3-yl)-8-fluoropyrido[ 4,3-d]pyrimidin-7-yl)-5-ethyny l-6-fluoronaphthalen-2-ol | 664.4 |
| **48** | 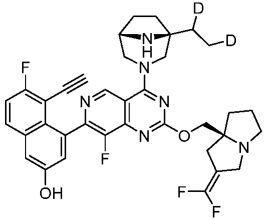 | 4-(2-(((S)-2-(difluoromethylen e)tetrahydro-1H-pyrrolizin-7a( 5H)-yl)methoxy)-4-((1R,5S)-1 -(ethyl-1,2-*d*₂)-3,8-diazabicycl o[3.2.1]octan-3-yl)-8-fluoropyr ido[4,3-d]pyrimidin-7-yl)-5-et hynyl-6-fluoronaphthalen-2-ol | 661.4 |
| **49** | 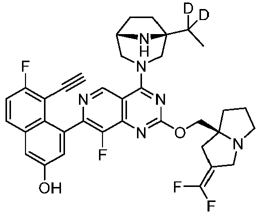 | 4-(2-(((S)-2-(difluoromethylen e)tetrahydro-1H-pyrrolizin-7a( 5H)-yl)methoxy)-4-((1R,5S)-1 -(ethyl-1,1-*d*₂)-3,8-diazabicycl o[3.2.1]octan-3-yl)-8-fluoropyr ido[4,3-d]pyrimidin-7-yl)-5-et hynyl-6-fluoronaphthalen-2-ol | 661.4 |
| **50** | 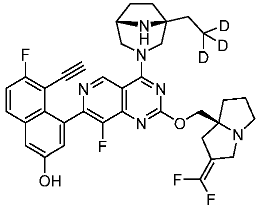 | 4-(2-(((S)-2-(difluoromethylen e)tetrahydro-1H-pyrrolizin-7a( 5H)-yl)methoxy)-4-((1R,5S)-1 -(ethyl-2,2,2-*d*₃)-3,8-diazabicy clo[3.2.1]octan-3-yl)-8-fluorop yrido[4,3-d]pyrimidin-7-yl)-5-ethynyl-6-fluoronaphthalen-2-ol | 662.4 |
| **51** | 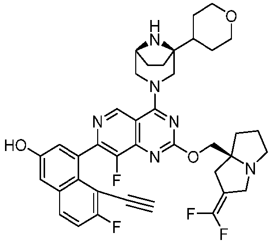 | 4-(2-(((S)-2-(difluoromethylene )tetrahydro-1H-pyrrolizin-7a(5 H)-yl)methoxy)-8-fluoro-4-((1 S ,5S)-1-(tetrahydro-2H-pyran-4-yl)-3,8-diazabicyclo[3.2.1]octa n-3-yl)pyrido[4,3-d]pyrimidin-7-yl)-5-ethynyl-6-fluoronaphth alen-2-ol | 715.4 |
| **52** | 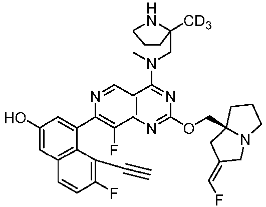 | 5-ethynyl-6-fluoro-4-(8-fluoro-2-(((S,Z)-2-(fluoromethylene)t etrahydro-1H-pyrrolizin-7a(5H )-yl)methoxy)-4-(1-(methyl-***d*₃**) -3,8-diazabicyclo[3.2.1]octan-3-yl)pyrido[4,3-d]pyrimidin-7-yl)naphthalen-2-ol | 630.4 |
| **53** | 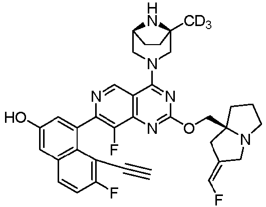 | 5-ethynyl-6-fluoro-4-(8-fluoro-2-(((S,Z)-2-(fluoromethylene)t etrahydro-1H-pyrrolizin-7a(5H )-yl)methoxy)-4-((1R,5S)-1-(m ethyl-***d*₃**)-3,8-diazabicyclo[3.2. 1octan-3-yl)pyrido[4,3-d]pyri midin-7-yl)naphthalen-2-ol | 630.4 |
| **54** | 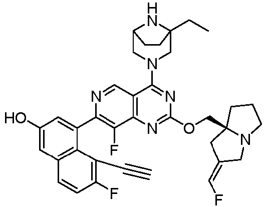 | 4-(4-(1-ethyl-3,8-diazabicyclo[ 3.2.1]octan-3-yl)-8-fluoro-2-((( S,Z)-2-(fluoromethylene)tetrah ydro-1H-pyrrolizin-7a(5H)-yl) methoxy)pyrido[4,3-d]pyrimid in-7-yl)-5-ethynyl-6-fluoronap hthalen-2-ol | 641.4 |
| **55** | 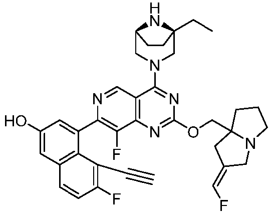 | 4-(4-((1R,5S)-1-ethyl-3,8-diaz abicyclo[3.2.1]octan-3-yl)-8-fl uoro-2-(((Z)-2-(fluoromethyle ne)tetrahydro-1H-pyrrolizin-7a (5H)-yl)methoxy)pyrido[4,3-d] pyrimidin-7-yl)-5-ethynyl-6-fl uoronaphthalen-2-ol | 641.4 |
| **56** | 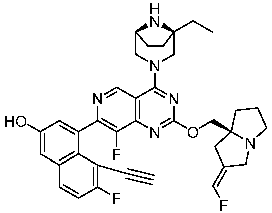 | 4-(4-((1R,5S)-1-ethyl-3,8-diaz abicyclo[3.2.1]octan-3-yl)-8-fl uoro-2-(((S,Z)-2-(fluoromethyl ene)tetrahydro-1H-pyrrolizin-7 a(SH)-yl)methoxy)pyrido[4,3-d]pyrimidin-7-yl)-5-ethynyl-6-fluoronaphthalen-2-ol | 641.4 |
| **57** | 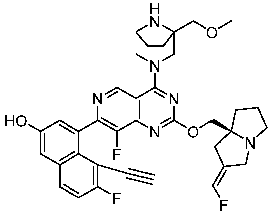 | 5-ethynyl-6-fluoro-4-(8-fluoro-2-(((S,Z)-2-(fluoromethylene)t etrahydro-1H-pyrrolizin-7a(5H )-yl)methoxy)-4-(1-(methoxym ethyl)-3,8-diazabicyclo[3.2.1]o ctan-3-yl)pyrido[4,3-d]pyrimid in-7-yl)naphthalen-2-ol | 657.4 |
| **58** | 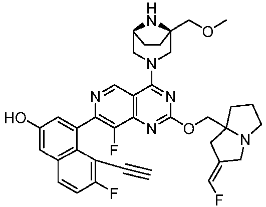 | 5-ethynyl-6-fluoro-4-(8-fluoro-2-(((Z)-2-(fluoromethylene)tetr ahydro-1H-pyrrolizin-7a(5H)-yl)methoxy)-4-((1R,5S)-1-(met hoxymethyl)-3,8-diazabicyclo[ 3.2.1]octan-3-yl)pyrido[4,3-d] pyrimidin-7-yl)naphthalen-2-ol | 657.4 |
| **59** | 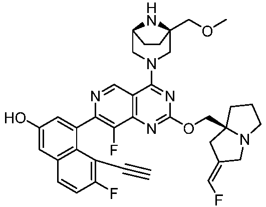 | 5-ethynyl-6-fluoro-4-(8-fluoro-2-(((S,Z)-2-(fluoromethylene)t etrahydro-1H-pyrrolizin-7a(5H )-yl)methoxy)-4-((1R,5S)-1-(m ethoxymethyl)-3,8-diazabicycl o[3.2.1]octan-3-yl)pyrido[4,3-d]pyrimidin-7-yl)naphthalen-2 -ol | 657.4 |
| **60** | 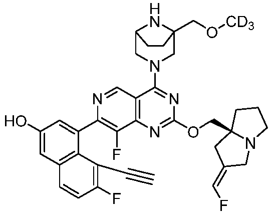 | 5-ethynyl-6-fluoro-4-(8-fluoro-2-(((S,Z)-2-(fluoromethylene)t etrahydro-1H-pyrrolizin-7a(5H )-yl)methoxy)-4-(1-((methoxy-***d*₃**)methyl)-3,8-diazabicyclo[3. 2.1]octan-3-yl)pyrido[4,3-d]py rimidin-7-yl)naphthalen-2-ol | 660.4 |
| **61** | 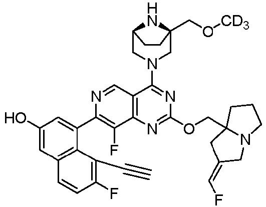 | 5-ethynyl-6-fluoro-4-(8-fluoro-2-(((Z)-2-(fluoromethylene)tetr ahydro-1H-pyrrolizin-7a(5H)-yl)methoxy)-4-((1R,5S)-1-((m ethoxy-***d*₃**)methyl)-3,8-diazabic yclo[3.2.1]octan-3-yl)pyrido[4, 3-d]pyrimidin-7-yl)naphthalen -2-ol | 660.4 |
| **62** | 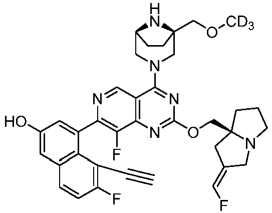 | 5-ethynyl-6-fluoro-4-(8-fluoro-2-(((S,Z)-2-(fluoromethylene)t etrahydro-1H-pyrrolizin-7a(5H )-yl)methoxy)-4-((1R,5S)-1-(( methoxy-***d*₃**)methyl)-3,8-diaza bicyclo[3.2.1]octan-3-yl)pyrid o[4,3-d]pyrimidin-7-yl)naphth alen-2-ol | 660.4 |
| **63** | 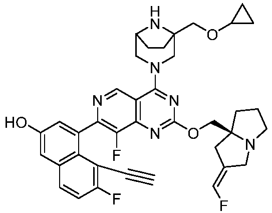 | 4-(4-(1-(cyclopropyloxymethyl )-3,8-diazabicyclo[3.2.1]octan-3-yl)-8-fluoro-2-(((S,Z)-2-(fluo romethylene)tetrahydro-1H-py rrolizin-7a(5H)-yl)methoxy)py rido[4,3-d]pyrimidin-7-yl)-5-et hynyl-6-fluoronaphthalen-2-ol | 683.4 |
| **64** | 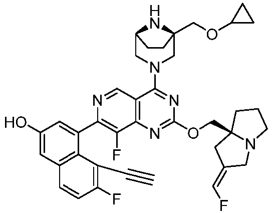 | 4-(4-((1R,5S)-1-(cyclopropylo xymethyl)-3,8-diazabicyclo[3. 2.1]octan-3-yl)-8-fluoro-2-(((S ,Z)-2-(fluoromethylene)tetrahy dro-1H-pyrrolizin-7a(5H)-yl)m ethoxy)pyrido[4,3-d]pyrimidin -7-yl)-5-ethynyl-6-fluoronapht halen-2-ol | 683.4 |
| **65** | 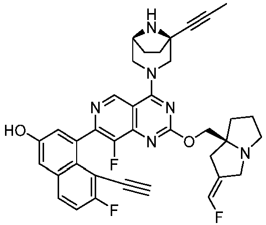 | 5-ethynyl-6-fluoro-4-(8-fluoro-2-(((S,Z)-2-(fluoromethylene)t etrahydro-1H-pyrrolizin-7a(5H )-yl)methoxy)-4-((1S,5S)-1-(pr op-1-yn-1-yl)-3,8-diazabicyclo [3.2.1]octan-3-yl)pyrido[4,3-d] pyrimidin-7-yl)naphthalen-2-ol | 651.4 |
| **66** | 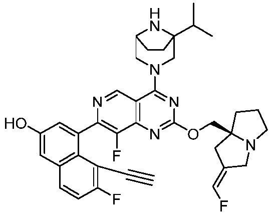 | 5-ethynyl-6-fluoro-4-(8-fluoro-2-(((S,Z)-2-(fluoromethylene)t etrahydro-1H-pyrrolizin-7a(5H )-yl)methoxy)-4-(1-isopropyl-3 ,8-diazabicyclo[3.2.1]octan-3-yl)pyrido[4,3-d]pyrimidin-7-yl )naphthalen-2-ol | 655.4 |
| **67** | 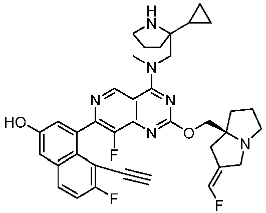 | 4-(4-(1-cyclopropyl-3,8-diazab icyclo[3.2.1]octan-3-yl)-8-fluo ro-2-(((S,Z)-2-(fluoromethylen e)tetrahydro-1H-pyrrolizin-7a( 5H)-yl)methoxy)pyrido[4,3-d] pyrimidin-7-yl)-5-ethynyl-6-fl uoronaphthalen-2-ol | 653.4 |
| **68** | 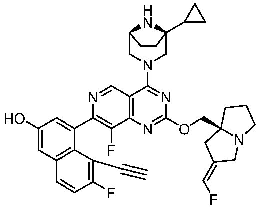 | 4-(4-(1-cyclopropyl-3,8-diazab icyclo[3.2.1]octan-3-yl)-8-fluo ro-2-(((S,Z)-2-(fluoromethylen e)tetrahydro-1H-pyrrolizin-7a( 5H)-yl)methoxy)pyrido[4,3-d] pyrimidin-7-yl)-5-ethynyl-6-fl uoronaphthalen-2-ol | 653.4 |
| **69** | 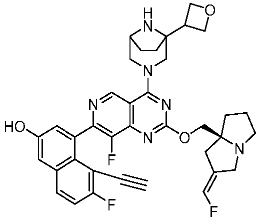 | 5-ethynyl-6-fluoro-4-(8-fluoro-2-(((S,Z)-2-(fluoromethylene)t etrahydro-1H-pyrrolizin-7a(5H )-yl)methoxy)-4-(1-(oxetan-3-yl)-3,8-diazabicyclo[3.2.1]octa n-3-yl)pyrido[4,3-d]pyrimidin-7-yl)naphthalen-2-ol | 669.4 |
| **70** | 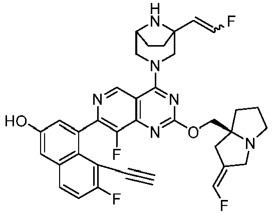 | 5-ethynyl-6-fluoro-4-(8-fluoro-2-(((S,Z)-2-(fluoromethylene)t etrahydro-1H-pyrrolizin-7a(5H )-yl)methoxy)-4-(1-(2-fluorovi nyl)-3,8-diazabicyclo[3.2.1]oct an-3-yl)pyrido[4,3-d]pyrimidin -7-yl)naphthalen-2-ol | 657.4 |
| **71** | 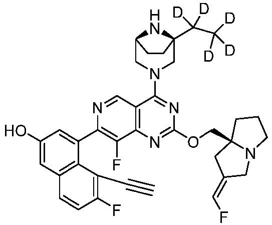 | 4-(4-((1R,5S)-1-(ethyl-*d*₅)-3,8-diazabicyclo[3.2.1]octan-3-yl)-8-fluoro-2-(((S,Z)-2-(fluoromet hylene)tetrahydro-1H-pyrrolizi n-7a(5H)-yl)methoxy)pyrido[4, 3-d]pyrimidin-7-yl)-5-ethynyl-6-fluoronaphthalen-2-ol | 646.4 |
| **72** | 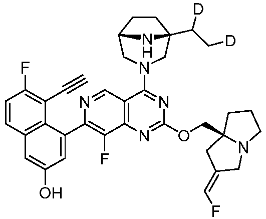 | 4-(4-((1R,5S)-1-(ethyl-1,2-*d*₂)-3,8-diazabicyclo[3.2.1]octan-3 -yl)-8-fluoro-2-(((S,Z)-2-(fluor omethylene)tetrahydro-1H-pyr rolizin-7a(5H)-yl)methoxy)pyr ido[4,3-d]pyrimidin-7-yl)-5-et hynyl-6-fluoronaphthalen-2-ol | 643.4 |
| **73** | 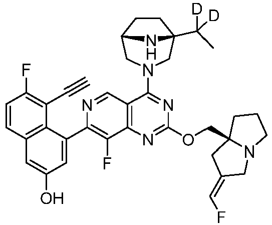 | 4-(4-((1R,5S)-1-(ethyl-1,1-*d*₂)-3,8-diazabicyclo[3.2.1]octan-3 -yl)-8-fluoro-2-(((S,Z)-2-(fluor omethylene)tetrahydro-1H-pyr rolizin-7a(5H)-yl)methoxy)pyr ido[4,3-d]pyrimidin-7-yl)-5-et hynyl-6-fluoronaphthalen-2-ol | 643.4 |
| **74** | 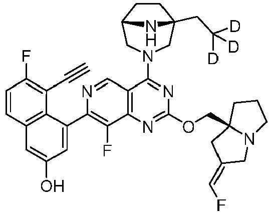 | 4-(4-((1R,5S)-1-(ethyl-2,2,2-*d*₃ )-3,8-diazabicyclo[3.2.1]octan-3-yl)-8-fluoro-2-(((S,Z)-2-(fluo romethylene)tetrahydro-1H-py rrolizin-7a(5H)-yl)methoxy)py rido[4,3-d]pyrimidin-7-yl)-5-et hynyl-6-fluoronaphthalen-2-ol | 644.4 |
| **75** | 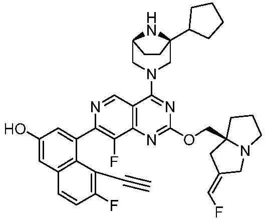 | 4-(4-((1S,5S)-1-cyclopentyl-3,8-diazabicyclo[3.2.1]octan-3-yl)-8 -fluoro-2-(((S,Z)-2-(fluoromethy lene)tetrahydro-1H-pyrrolizin-7a (5H)-yl)methoxy)pyrido[4,3-d]p yrimidin-7-yl)-5-ethynyl-6-fluor onaphthalen-2-ol | 681.4 |
| **76** | 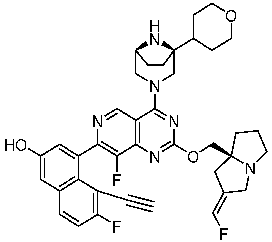 | 5-ethynyl-6-fluoro-4-(8-fluoro-2-(((S,Z)-2-(fluoromethylene)t etrahydro-1H-pyrrolizin-7a(5H )-yl)methoxy)-4-((1S,5S)-1-(te trahydro-2H-pyran-4-yl)-3,8-di azabicyclo[3.2.1]octan-3-yl)py rido[4,3-d]pyrimidin-7-yl)naph thalen-2-ol | 697.4 |
| **77** | 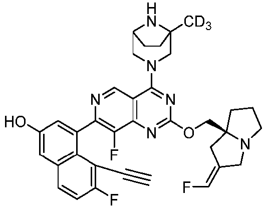 | 5-ethynyl-6-fluoro-4-(8-fluoro-2-(((S,E)-2-(fluoromethylene)t etrahydro-1H-pyrrolizin-7a(5H )-yl)methoxy)-4-(1-(methyl-***d*₃**) -3,8-diazabicyclo[3.2.1]octan-3-yl)pyrido[4,3-d]pyrimidin-7-yl)naphthalen-2-ol | 630.4 |
| **78** | 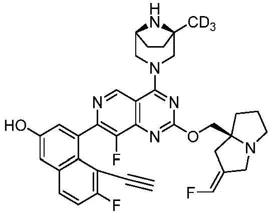 | 5-ethynyl-6-fluoro-4-(8-fluoro-2-(((S,E)-2-(fluoromethylene)t etrahydro-1H-pyrrolizin-7a(5H )-yl)methoxy)-4-((1R,5S)-1-(m ethyl-***d*₃**)-3,8-diazabicyclo[3.2. 1octan-3-yl)pyrido[4,3-d]pyri midin-7-yl)naphthalen-2-ol | 630.4 |
| **79** | 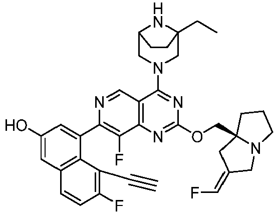 | 4-(4-(1-ethyl-3,8-diazabicyclo[ 3.2.1]octan-3-yl)-8-fluoro-2-((( S,E)-2-(fluoromethylene)tetrah ydro-1H-pyrrolizin-7a(5H)-yl) methoxy)pyrido[4,3-d]pyrimid in-7-yl)-5-ethynyl-6-fluoronap hthalen-2-ol | 641.4 |
| **80** | 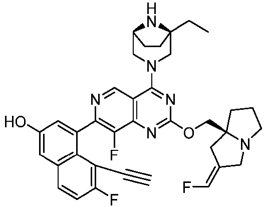 | 4-(4-((1R,5S)-1-ethyl-3,8-diaz abicyclo[3.2.1]octan-3-yl)-8-fl uoro-2-(((S,E)-2-(fluoromethyl ene)tetrahydro-1H-pyrrolizin-7 a(5H)-yl)methoxy)pyrido[4,3-d]pyrimidin-7-yl)-5-ethynyl-6-fluoronaphthalen-2-ol | 641.4 |
| **81** | 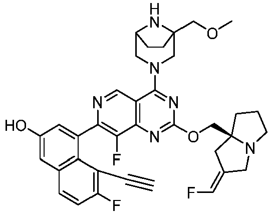 | 5-ethynyl-6-fluoro-4-(8-fluoro-2-(((S,E)-2-(fluoromethylene)t etrahydro-1H-pyrrolizin-7a(5H )-yl)methoxy)-4-(1-(methoxym ethyl)-3,8-diazabicyclo[3.2.1]o ctan-3-yl)pyrido[4,3-d]pyrimid in-7-yl)naphthalen-2-ol | 657.4 |
| **82** | 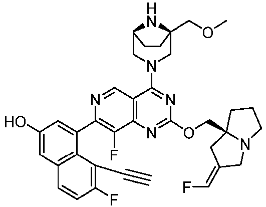 | 5-ethynyl-6-fluoro-4-(8-fluoro-2-(((S,E)-2-(fluoromethylene)t etrahydro-1H-pyrrolizin-7a(5H )-yl)methoxy)-4-((1R,5S)-1-(m ethoxymethyl)-3,8-diazabicycl o[3.2.1]octan-3-yl)pyrido[4,3-d]pyrimidin-7-yl)naphthalen-2 -ol | 657.4 |
| **83** | 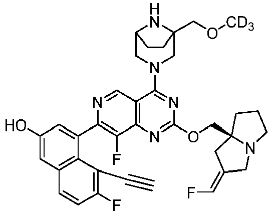 | 5-ethynyl-6-fluoro-4-(8-fluoro-2-(((S,E)-2-(fluoromethylene)t etrahydro-1H-pyrrolizin-7a(5H )-yl)methoxy)-4-(1-((methoxy-***d*₃**)methyl)-3,8-diazabicyclo[3. 2.1]octan-3-yl)pyrido[4,3-d]py rimidin-7-yl)naphthalen-2-ol | 660.4 |
| **84** | 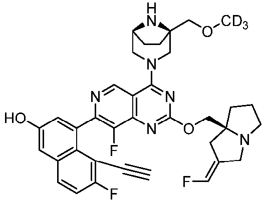 | 5-ethynyl-6-fluoro-4-(8-fluoro-2-(((S,E)-2-(fluoromethylene)t etrahydro-1H-pyrrolizin-7a(5H )-yl)methoxy)-4-((1R,5S)-1-(( methoxy-***d*₃**)methyl)-3,8-diaza bicyclo[3.2.1]octan-3-yl)pyrid o[4,3-d]pyrimidin-7-yl)naphth alen-2-ol | 660.4 |
| **85** | 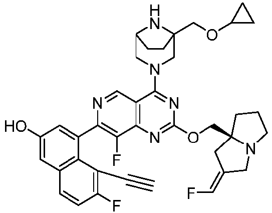 | 4-(4-(1-(cyclopropyloxymethyl )-3,8-diazabicyclo[3.2.1]octan-3-yl)-8-fluoro-2-(((S,E)-2-(fluo romethylene)tetrahydro-1H-py rrolizin-7a(5H)-yl)methoxy)py rido[4,3-d]pyrimidin-7-yl)-5-et hynyl-6-fluoronaphthalen-2-ol | 683.4 |
| **86** | 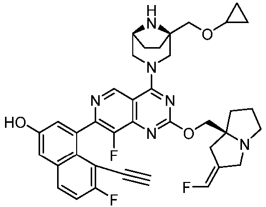 | 4-(4-((1R,5S)-1-(cyclopropylo xymethyl)-3,8-diazabicyclo[3. 2.1]octan-3-yl)-8-fluoro-2-(((S ,E)-2-(fluoromethylene)tetrahy dro-1H-pyrrolizin-7a(5H)-yl)m ethoxy)pyrido[4,3-d]pyrimidin -7-yl)-5-ethynyl-6-fluoronapht halen-2-ol | 683.4 |
| **87** | 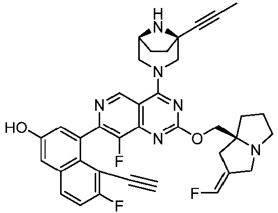 | 5-ethynyl-6-fluoro-4-(8-fluoro-2-(((S,E)-2-(fluoromethylene)t etrahydro-1H-pyrrolizin-7a(5H )-yl)methoxy)-4-((1S,5S)-1-(pr op-1-yn-1-yl)-3,8-diazabicyclo [3.2.1]octan-3-yl)pyrido[4,3-d] pyrimidin-7-yl)naphthalen-2-ol | 651.4 |
| **88** | 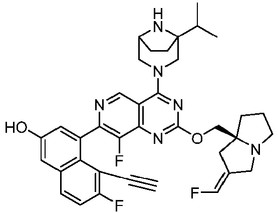 | 5-ethynyl-6-fluoro-4-(8-fluoro-2-(((S,E)-2-(fluoromethylene)t etrahydro-1H-pyrrolizin-7a(5H )-yl)methoxy)-4-(1-isopropyl-3 ,8-diazabicyclo[3.2.1]octan-3-yl)pyrido[4,3-d]pyrimidin-7-yl )naphthalen-2-ol | 655.4 |
| **89** | 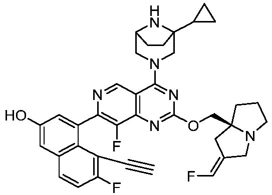 | 4-(4-(1-cyclopropyl-3,8-diazab icyclo[3.2.1]octan-3-yl)-8-fluo ro-2-(((S,E)-2-(fluoromethylen e)tetrahydro-1H-pyrrolizin-7a( 5H)-yl)methoxy)pyrido[4,3-d] pyrimidin-7-yl)-5-ethynyl-6-fl uoronaphthalen-2-ol | 653.4 |
| **90** | 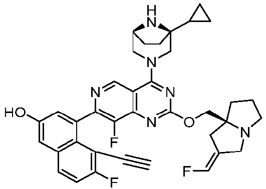 | 4-(4-((1S,5S)-1-cyclopropyl-3, 8-diazabicyclo[3.2.1]octan-3-y l)-8-fluoro-2-(((S,E)-2-(fluoro methylene)tetrahydro-1H-pyrr olizin-7a(5H)-yl)methoxy)pyri do[4,3-d]pyrimidin-7-yl)-5-eth ynyl-6-fluoronaphthalen-2-ol | 653.4 |
| **91** | 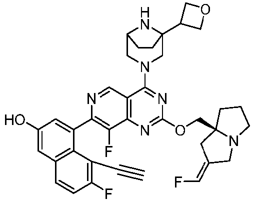 | 5-ethynyl-6-fluoro-4-(8-fluoro-2-(((S,E)-2-(fluoromethylene)t etrahydro-1H-pyrrolizin-7a(5H )-yl)methoxy)-4-(1-(oxetan-3-yl)-3,8-diazabicyclo[3.2.1]octa n-3-yl)pyrido[4,3-d]pyrimidin-7-yl)naphthalen-2-ol | 669.4 |
| **92** | 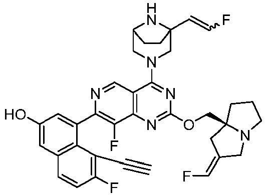 | 5-ethynyl-6-fluoro-4-(8-fluoro-2-(((S,E)-2-(fluoromethylene)t etrahydro-1H-pyrrolizin-7a(5H )-yl)methoxy)-4-(1-(2-fluorovi nyl)-3,8-diazabicyclo[3.2.1]oct an-3-yl)pyrido[4,3-d]pyrimidin -7-yl)naphthalen-2-ol | 657.4 |
| **93** | 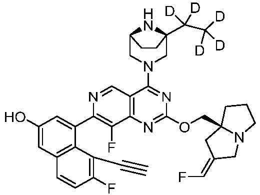 | 4-(4-((1R,5S)-1-(ethyl-ds)-3,8-diazabicyclo[3.2.1]octan-3-yl)-8-fluoro-2-(((S,E)-2-(fluorome thylene)tetrahydro-1H-pyrroliz in-7a(5H)-yl)methoxy)pyrido[ 4,3-d]pyrimidin-7-yl)-5-ethyny l-6-fluoronaphthalen-2-ol | 646.4 |
| **94** | 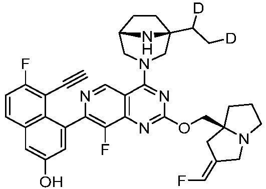 | 4-(4-((1R,5S)-1-(ethyl-1,2-*d*₂)-3,8-diazabicyclo[3.2.1]octan-3 -yl)-8-fluoro-2-(((S,E)-2-(fluor omethylene)tetrahydro-1H-pyr rolizin-7a(5H)-yl)methoxy)pyr ido[4,3-d]pyrimidin-7-yl)-5-et hynyl-6-fluoronaphthalen-2-ol | 643.4 |
| **95** | 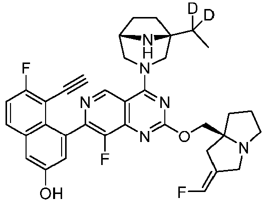 | 4-(4-((1R,5S)-1-(ethyl-1,1-*d*₂)-3,8-diazabicyclo[3.2.1]octan-3 -yl)-8-fluoro-2-(((S,E)-2-(fluor omethylene)tetrahydro-1H-pyr rolizin-7a(5H)-yl)methoxy)pyr ido[4,3-d]pyrimidin-7-yl)-5-et hynyl-6-fluoronaphthalen-2-ol | 643.4 |
| **96** | 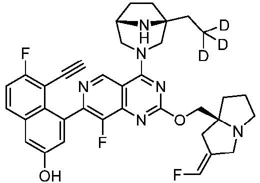 | 4-(4-((1R,5S)-1-(ethyl-2,2,2-*d*₃ )-3,8-diazabicyclo[3.2.1]octan-3-yl)-8-fluoro-2-(((S,E)-2-(fluo romethylene)tetrahydro-1H-py rrolizin-7a(5H)-yl)methoxy)py rido[4,3-d]pyrimidin-7-yl)-5-et hynyl-6-fluoronaphthalen-2-ol | 644.4 |
| **97** | 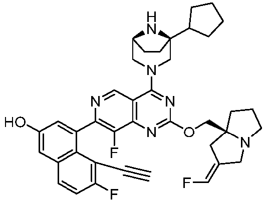 | 4-(4-((1S,5S)-1-cyclopentyl-3,8-diazabicyclo[3.2.1]octan-3-yl)-8 -fluoro-2-(((S,E)-2-(fluoromethy lene)tetrahydro-1H-pyrrolizin-7a (5H)-yl)methoxy)pyrido[4,3-d]p yrimidin-7-yl)-5-ethynyl-6-fluor onaphthalen-2-ol | 681.4 |
| **98** | 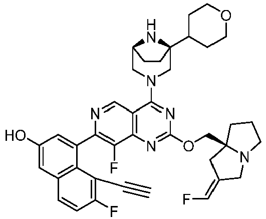 | 5-ethynyl-6-fluoro-4-(8-fluoro-2-(((S,E)-2-(fluoromethylene)t etrahydro-1H-pyrrolizin-7a(5H )-yl)methoxy)-4-((1S,5S)-1-(te trahydro-2H-pyran-4-yl)-3,8-di azabicyclo[3.2.1]octan-3-yl)py rido[4,3-d]pyrimidin-7-yl)naph thalen-2-ol | 697.4 |
| **99** | 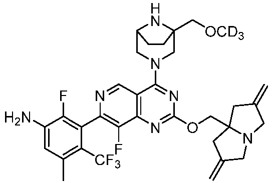 | 3-(2-((2,6-dimethylenetetrahyd ro-1H-pyrrolizin-7a(5H)-yl)me thoxy)-8-fluoro-4-(1-((methox y-***d*₃**)methyl)-3,8-diazabicyclo[ 3.2.1]octan-3-yl)pyrido[4,3-d] pyrimidin-7-yl)-2-fluoro-5-met hyl-4-(trifluoromethyl)aniline | 661.4 |
| **100** | 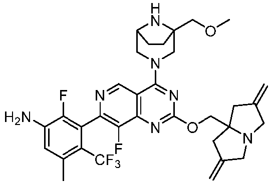 | 3-(2-((2,6-dimethylenetetrahyd ro-1H-pyrrolizin-7a(5H)-yl)me thoxy)-8-fluoro-4-(1-(methoxy methyl)-3,8-diazabicyclo[3.2.1 ]octan-3-yl)pyrido[4,3-d]pyrim idin-7-yl)-2-fluoro-5-methyl-4-(trifluoromethyl)aniline | 658.4 |
| **101** | 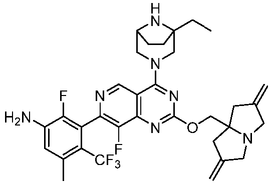 | 3-(2-((2,6-dimethylenetetrahyd ro-1H-pyrrolizin-7a(5H)-yl)me thoxy)-4-(1-ethyl-3,8-diazabic yclo[3.2.1]octan-3-yl)-8-fluoro pyrido[4,3-d]pyrimidin-7-yl)-2 -fluoro-5-methyl-4-(trifluorom ethyl)aniline | 642.4 |
| **102** | 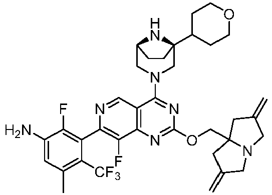 | 3-(2-((2,6-dimethylenetetrahyd ro-1H-pyrrolizin-7a(5H)-yl)me thoxy)-8-fluoro-4-((1S,5S)-1-(t etrahydro-2H-pyran-4-yl)-3,8-diazabicyclo[3.2.1]octan-3-yl) pyrido[4,3-d]pyrimidin-7-yl)-2 -fluoro-5-methyl-4-(trifluorom ethyl)aniline | 698.4 |
| **103** | 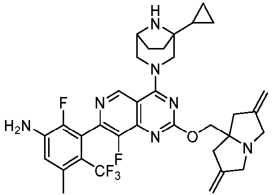 | 3-(4-(1-cyclopropyl-3,8-diazab icyclo[3.2.1]octan-3-yl)-2-((2, 6-dimethylenetetrahydro-1H-p yrrolizin-7a(5H)-yl)methoxy)-8-fluoropyrido[4,3-d]pyrimidin -7-yl)-2-fluoro-5-methyl-4-(trif luoromethyl)aniline | 654.4 |
| **104** | 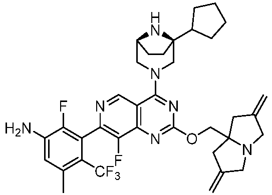 | 3-(4-((1S,5S)-1-cyclopentyl-3,8 -diazabicyclo[3.2.1]octan-3-yl)-2-((2,6-dimethylenetetrahydro-1H-pyrrolizin-7a(5H)-yl)metho xy)-8-fluoropyrido[4,3-d]pyrim idin-7-yl)-2-fluoro-5-methyl-4-(trifluoromethyl)aniline | 682.4 |
| **105** | 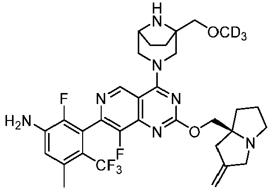 | 2-fluoro-3-(8-fluoro-4-(1-((met hoxy-***d*₃**)methyl)-3,8-diazabicy clo[3.2.1]octan-3-yl)-2-(((S)-2-methylenetetrahydro-1H-pyrro lizin-7a(5H)-yl)methoxy)pyrid o[4,3-d]pyrimidin-7-yl)-5-met hyl-4-(trifluoromethyl)aniline | 649.4 |
| **106** | 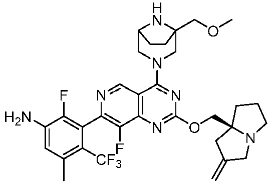 | 2-fluoro-3-(8-fluoro-4-(1-(met hoxymethyl)-3,8-diazabicyclo[ 3.2.1]octan-3-yl)-2-(((S)-2-met hylenetetrahydro-1H-pyrrolizi n-7a(5H)-yl)methoxy)pyrido[4 ,3-d]pyrimidin-7-yl)-5-methyl-4-(trifluoromethyl)aniline | 646.4 |
| **107** | 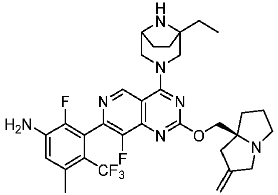 | 3-(4-(1-ethyl-3,8-diazabicyclo[ 3.2.1]octan-3-yl)-8-fluoro-2-((( S)-2-methylenetetrahydro-1H-pyrrolizin-7a(5H)-yl)methoxy) pyrido[4,3-d]pyrimidin-7-yl)-2 -fluoro-5-methyl-4-(trifluorom ethyl)aniline | 630.4 |
| **108** | 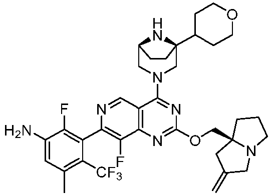 | 2-fluoro-3-(8-fluoro-2-(((S)-2-methylenetetrahydro-1H-pyrrol izin-7a(5H)-yl)methoxy)-4-((1S ,5S)-1-(tetrahydro-2H-pyran-4-yl)-3,8-diazabicyclo[3.2.1]octa n-3-yl)pyrido[4,3-d]pyrimidin-7-yl)-5-methyl-4-(trifluorometh yl)aniline | 686.4 |
| **109** | 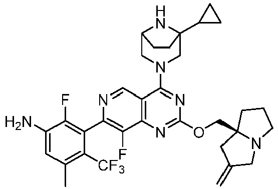 | 3-(4-(1-cyclopropyl-3,8-diazab icyclo[3.2.1]octan-3-yl)-8-fluo ro-2-(((S)-2-methylenetetrahyd ro-1H-pyrrolizin-7a(5H)-yl)me thoxy)pyrido[4,3-d]pyrimidin-7-yl)-2-fluoro-5-methyl-4-(trifl uoromethyl)aniline | 642.4 |
| **110** | 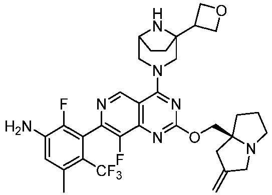 | 2-fluoro-3-(8-fluoro-2-(((S)-2-methylenetetrahydro-1H-pyrro lizin-7a(5H)-yl)methoxy)-4-(1-(oxetan-3-yl)-3,8-diazabicyclo[ 3.2.1]octan-3-yl)pyrido[4,3-d] pyrimidin-7-yl)-5-methyl-4-(tri fluoromethyl)aniline | 658.4 |
| **111** | 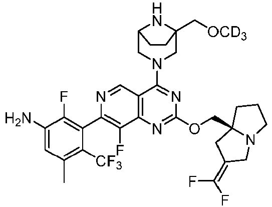 | 3-(2-(((S)-2-(difluoromethylen e)tetrahydro-1H-pyrrolizin-7a( 5H)-yl)methoxy)-8-fluoro-4-(1 -((methoxy-***d*₃**)methyl)-3,8-dia zabicyclo[3.2.1]octan-3-yl)pyri do[4,3-d]pyrimidin-7-yl)-2-flu oro-5-methyl-4-(trifluorometh yl)aniline | 685.4 |
| **112** | 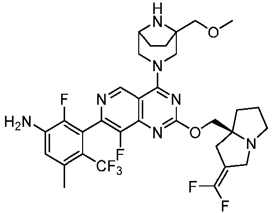 | 3-(2-(((S)-2-(difluoromethylen e)tetrahydro-1H-pyrrolizin-7a( 5H)-yl)methoxy)-8-fluoro-4-(1 -(methoxymethyl)-3,8-diazabic yclo[3.2.1]octan-3-yl)pyrido[4, 3-d]pyrimidin-7-yl)-2-fluoro-5 -methyl-4-(trifluoromethyl)anil ine | 682.4 |
| **113** | 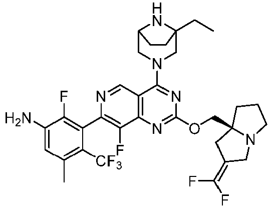 | 3-(2-(((S)-2-(difluoromethylen e)tetrahydro-1H-pyrrolizin-7a( 5H)-yl)methoxy)-4-(1-ethyl-3, 8-diazabicyclo[3.2.1]octan-3-y 1)-8-fluoropyrido[4,3-d]pyrimi din-7-yl)-2-fluoro-5-methyl-4-(trifluoromethyl)aniline | 666.4 |
| **114** | 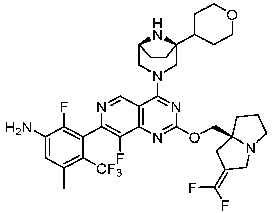 | 3-(2-(((S)-2-(difluoromethylene )tetrahydro-1H-pyrrolizin-7a(5 H)-yl)methoxy)-8-fluoro-4-((1S ,5S)-1-(tetrahydro-2H-pyran-4-yl)-3,8-diazabicyclo[3.2.1]octa n-3-yl)pyrido[4,3-d]pyrimidin-7-yl)-2-fluoro-5-methyl-4-(trifl uoromethyl)aniline | 722.4 |
| **115** | 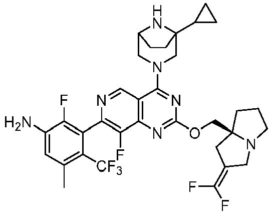 | 3-(4-(1-cyclopropyl-3,8-diazab icyclo[3.2.1]octan-3-yl)-2-(((S )-2-(difluoromethylene)tetrahy dro-1H-pyrrolizin-7a(5H)-yl)m ethoxy)-8-fluoropyrido[4,3-d]p yrimidin-7-yl)-2-fluoro-5-meth yl-4-(trifluoromethyl)aniline | 678.4 |
| **116** | 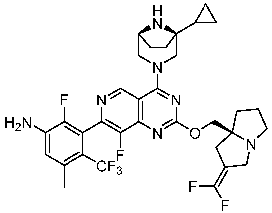 | 3-(4-((1S,5 S)-1-cyclopropyl-3, 8-diazabicyclo[3.2.1]octan-3-y l)-2-(((S)-2-(difluoromethylene )tetrahydro-1H-pyrrolizin-7a(5 H)-yl)methoxy)-8-fluoropyrido [4,3-d]pyrimidin-7-yl)-2-fluoro -5-methyl-4-(trifluoromethyl)a niline | 678.4 |
| **117** | 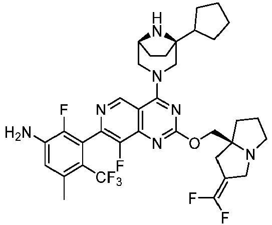 | 3-(4-((1S,5S)-1-cyclopentyl-3,8 -diazabicyclo[3.2.1]octan-3-yl)-2-(((S)-2-(difluoromethylene)te trahydro-1H-pyrrolizin-7a(5H)-yl)methoxy)-8-fluoropyrido[4,3 -d]pyrimidin-7-yl)-2-fluoro-5-methyl-4-(trifluoromethyl)anili ne | 706.4 |
| **118** | 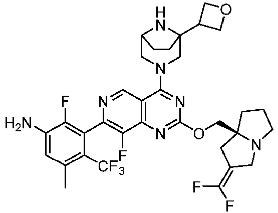 | 3-(2-(((S)-2-(difluoromethylen e)tetrahydro-1H-pyrrolizin-7a( 5H)-yl)methoxy)-8-fluoro-4-(1 -(oxetan-3-yl)-3,8-diazabicyclo [3.2.1]octan-3-yl)pyrido[4,3-d] pyrimidin-7-yl)-2-fluoro-5-met hyl-4-(trifluoromethyl)aniline | 694.4 |
| **119** | 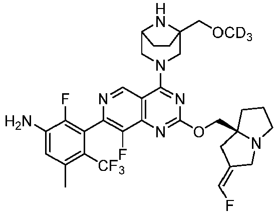 | 2-fluoro-3-(8-fluoro-2-(((S,Z)-2-(fluoromethylene)tetrahydro-1H-pyrrolizin-7a(5H)-yl)meth oxy)-4-(1-((methoxy-***d*₃**)methy l)-3,8-diazabicyclo[3.2.1]octan -3-yl)pyrido[4,3-d]pyrimidin-7 -yl)-5-methyl-4-(trifluorometh yl)aniline | 667.4 |
| **120** | 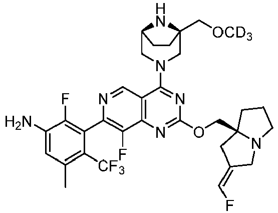 | 2-fluoro-3-(8-fluoro-2-(((S,Z)-2-(fluoromethylene)tetrahydro-1H-pyrrolizin-7a(5H)-yl)meth oxy)-4-((1R,5S)-1-((methoxy-***d*₃**)methyl)-3,8-diazabicyclo[3. 2.1]octan-3-yl)pyrido[4,3-d]py rimidin-7-yl)-5-methyl-4-(trifl uoromethyl)aniline | 667.4 |
| **121** | 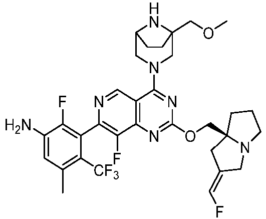 | 2-fluoro-3-(8-fluoro-2-(((S,Z)-2-(fluoromethylene)tetrahydro-1H-pyrrolizin-7a(5H)-yl)meth oxy)-4-(1-(methoxymethyl)-3, 8-diazabicyclo[3.2.1]octan-3-y l)pyrido[4,3-d]pyrimidin-7-yl)-5-methyl-4-(trifluoromethyl)an iline | 664.4 |
| **122** | 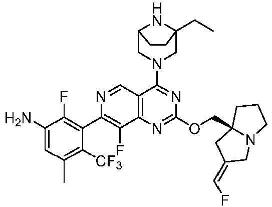 | 3-(4-(1-ethyl-3,8-diazabicyclo[ 3.2.1]octan-3-yl)-8-fluoro-2-((( S,Z)-2-(fluoromethylene)tetrah ydro-1H-pyrrolizin-7a(5H)-yl) methoxy)pyrido[4,3-d]pyrimidi n-7-yl)-2-fluoro-5-methyl-4-(tri fluoromethyl)aniline | 648.4 |
| **123** | 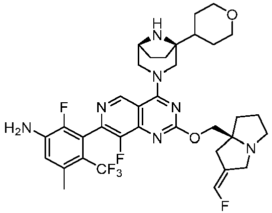 | 2-fluoro-3-(8-fluoro-2-(((S,Z)-2-(fluoromethylene)tetrahydro-1H -pyrrolizin-7a(5H)-yl)methoxy)-4-((1S,5S)-1-(tetrahydro-2H-pyr an-4-yl)-3,8-diazabicyclo[3.2.1] octan-3-yl)pyrido[4,3-d]pyrimidi n-7-yl)-5-methyl-4-(trifluoromet hyl)aniline | 704.4 |
| **124** | 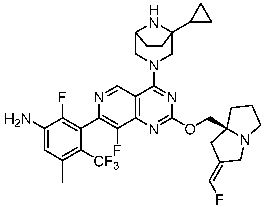 | 3-(4-(1-cyclopropyl-3,8-diazab icyclo[3.2.1]octan-3-yl)-8-fluo ro-2-(((S,Z)-2-(fluoromethylen e)tetrahydro-1H-pyrrolizin-7a( 5H)-yl)methoxy)pyrido[4,3-d] pyrimidin-7-yl)-2-fluoro-5-met hyl-4-(trifluoromethyl)aniline | 660.4 |
| **125** | 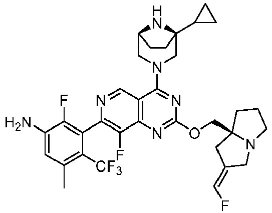 | 3-(4-((1S,5S)-1-cyclopropyl-3, 8-diazabicyclo[3.2.1]octan-3-y l)-8-fluoro-2-(((S,Z)-2-(fluoro methylene)tetrahydro-1H-pyrr olizin-7a(5H)-yl)methoxy)pyri do[4,3-d]pyrimidin-7-yl)-2-flu oro-5-methyl-4-(trifluorometh yl)aniline | 660.4 |
| **126** | 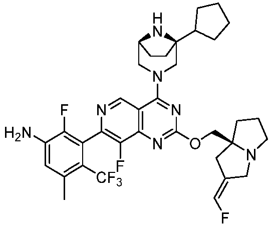 | 3-(4-((1S,5S)-1-cyclopentyl-3,8 -diazabicyclo[3.2.1]octan-3-yl)-8-fluoro-2-(((S,Z)-2-(fluoromet hylene)tetrahydro-1H-pyrrolizi n-7a(5H)-yl)methoxy)pyrido[4, 3-d]pyrimidin-7-yl)-2-fluoro-5-methyl-4-(trifluoromethyl)anili ne | 688.4 |
| **127** | 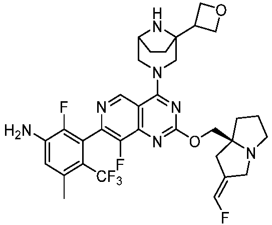 | 2-fluoro-3-(8-fluoro-2-(((S,Z)-2-(fluoromethylene)tetrahydro-1H-pyrrolizin-7a(5H)-yl)meth oxy)-4-(1-(oxetan-3-yl)-3,8-dia zabicyclo[3.2.1]octan-3-yl)pyri do[4,3-d]pyrimidin-7-yl)-5-me thyl-4-(trifluoromethyl)aniline | 676.4 |
| **128** | 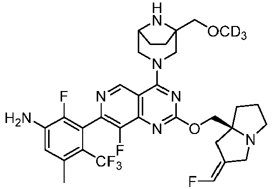 | 2-fluoro-3-(8-fluoro-2-(((S,E)-2-(fluoromethylene)tetrahydro-1H-pyrrolizin-7a(5H)-yl)meth oxy)-4-(1-((methoxy-***d*₃**)methy l)-3,8-diazabicyclo[3.2.1]octan -3-yl)pyrido[4,3-d]pyrimidin-7 -yl)-5-methyl-4-(trifluorometh yl)aniline | 667.4 |
| **129** | 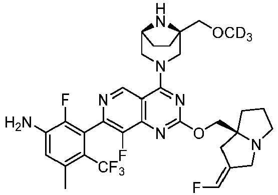 | 2-fluoro-3-(8-fluoro-2-(((S,E)-2-(fluoromethylene)tetrahydro-1H-pyrrolizin-7a(5H)-yl)meth oxy)-4-((1R,5S)-1-((methoxy-***d*₃**)methyl)-3,8-diazabicyclo[3. 2.1]octan-3-yl)pyrido[4,3-d]py rimidin-7-yl)-5-methyl-4-(trifl uoromethyl)aniline | 667.4 |
| **130** | 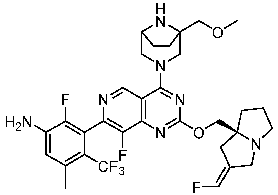 | 2-fluoro-3-(8-fluoro-2-(((S,E)-2-(fluoromethylene)tetrahydro-1H-pyrrolizin-7a(5H)-yl)meth oxy)-4-(1-(methoxymethyl)-3, 8-diazabicyclo[3.2.1]octan-3-y l)pyrido[4,3-d]pyrimidin-7-yl)-5-methyl-4-(trifluoromethyl)an iline | 664.4 |
| **131** | 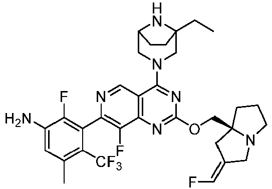 | 3-(4-(1-ethyl-3,8-diazabicyclo[ 3.2.1]octan-3-yl)-8-fluoro-2-((( S,E)-2-(fluoromethylene)tetrah ydro-1H-pyrrolizin-7a(5H)-yl) methoxy)pyrido[4,3-d]pyrimid in-7-yl)-2-fluoro-5-methyl-4-(t rifluoromethyl)aniline | 648.4 |
| **132** | 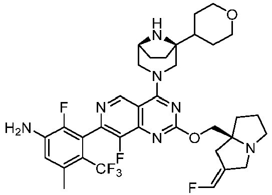 | 2-fluoro-3-(8-fluoro-2-(((S,E)-2 -(fluoromethylene)tetrahydro-1 H-pyrrolizin-7a(5H)-yl)methox y)-4-((1S,5S)-1-(tetrahydro-2H -pyran-4-yl)-3,8-diazabicyclo[3 .2.1]octan-3-yl)pyrido[4,3-d]py rimidin-7-yl)-5-methyl-4-(triflu oromethyl)aniline | 704.4 |
| **133** | 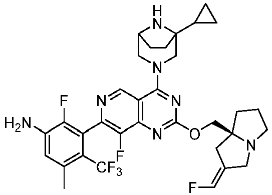 | 3-(4-(1-cyclopropyl-3,8-diazab icyclo[3.2.1]octan-3-yl)-8-fluo ro-2-(((S,E)-2-(fluoromethylen e)tetrahydro-1H-pyrrolizin-7a( 5H)-yl)methoxy)pyrido[4,3-d] pyrimidin-7-yl)-2-fluoro-5-met hyl-4-(trifluoromethyl)aniline | 660.4 |
| **134** | 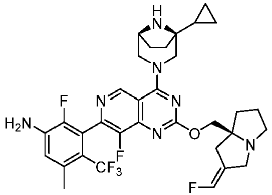 | 3-(4-((1S,5S)-1-cyclopropyl-3, 8-diazabicyclo[3.2.1]octan-3-y l)-8-fluoro-2-(((S,E)-2-(fluoro methylene)tetrahydro-1H-pyrr olizin-7a(5H)-yl)methoxy)pyri do[4,3-d]pyrimidin-7-yl)-2-flu oro-5-methyl-4-(trifluorometh yl)aniline | 660.4 |
| **135** | 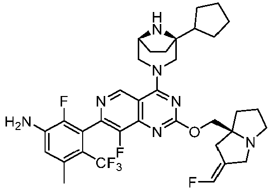 | 3-(4-((1S,5S)-1-cyclopentyl-3,8 -diazabicyclo[3.2.1]octan-3-yl)-8-fluoro-2-(((S,E)-2-(fluoromet hylene)tetrahydro-1H-pyrrolizi n-7a(5H)-yl)methoxy)pyrido[4, 3-d]pyrimidin-7-yl)-2-fluoro-5-methyl-4-(trifluoromethyl)anili ne | 688.4 |
| **136** | 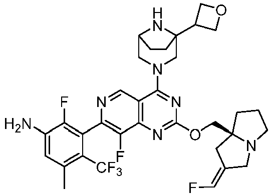 | 2-fluoro-3-(8-fluoro-2-(((S,E)-2-(fluoromethylene)tetrahydro-1H-pyrrolizin-7a(5H)-yl)meth oxy)-4-(1-(oxetan-3-yl)-3,8-dia zabicyclo[3.2.1]octan-3-yl)pyri do[4,3-d]pyrimidin-7-yl)-5-me thyl-4-(trifluoromethyl)aniline | 676.4 |
| **137** | 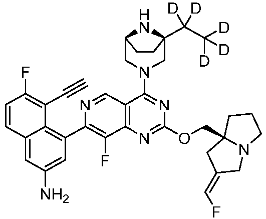 | 4-(4-((1R,5S)-1-(ethyl-ds)-3,8-diazabicyclo[3.2.1]octan-3-yl)-8-fluoro-2-(((S,Z)-2-(fluorome thylene)tetrahydro-1H-pyrroliz in-7a(5H)-yl)methoxy)pyrido[ 4,3-d]pyrimidin-7-yl)-5-ethyny l-6-fluoronaphthalen-2-amine | 645.4 |
| **138** | 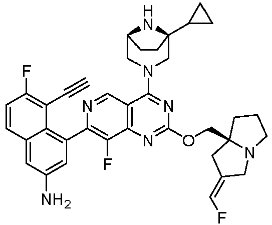 | 4-(4-((1S,5S)-1-cyclopropyl-3, 8-diazabicyclo[3.2.1]octan-3-y l)-8-fluoro-2-(((S,Z)-2-(fluoro methylene)tetrahydro-1H-pyrr olizin-7a(5H)-yl)methoxy)pyri do[4,3-d]pyrimidin-7-yl)-5-eth ynyl-6-fluoronaphthalen-2-ami ne | 652.4 |
| **139** | 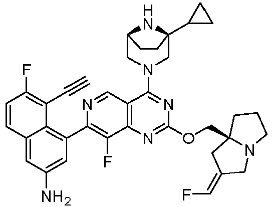 | 4-(4-((1S,5S)-1-cyclopropyl-3, 8-diazabicyclo[3.2.1]octan-3-y l)-8-fluoro-2-(((S,E)-2-(fluoro methylene)tetrahydro-1H-pyrr olizin-7a(5H)-yl)methoxy)pyri do[4,3-d]pyrimidin-7-yl)-5-eth ynyl-6-fluoronaphthalen-2-ami ne | 652.4 |
| **140** | 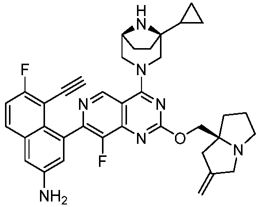 | 4-(4-((1S,5S)-1-cyclopropyl-3, 8-diazabicyclo[3.2.1]octan-3-y l)-8-fluoro-2-(((S)-2-methylen etetrahydro-1H-pyrrolizin-7a(5 H)-yl)methoxy)pyrido[4,3-d]p yrimidin-7-yl)-5-ethynyl-6-flu oronaphthalen-2-amine | 634.4 |
| **141** | 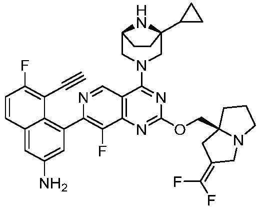 | 4-(4-((1S,5S)-1-cyclopropyl-3, 8-diazabicyclo[3.2.1]octan-3-y l)-2-(((S)-2-(difluoromethylene )tetrahydro-1H-pyrrolizin-7a(5 H)-yl)methoxy)-8-fluoropyrido [4,3-d]pyrimidin-7-yl)-5-ethyn yl-6-fluoronaphthalen-2-amine | 670.4 |
| **142** | 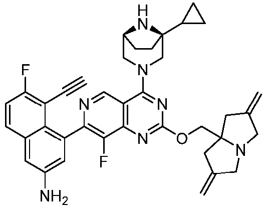 | 4-(4-((1S,5S)-1-cyclopropyl-3,8-diazabicyclo[3.2.1]octan-3-yl)-2 -((2,6-dimethylenetetrahydro-1H -pyrrolizin-7a(5H)-yl)methoxy)-8-fluoropyrido[4,3-d]pyrimidin-7-yl)-5-ethynyl-6-fluoronaphthal en-2-amine | 646.4 |
| **143** | 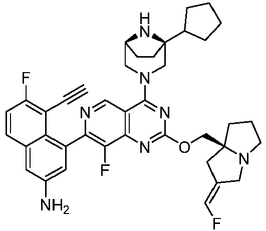 | 4-(4-((1S,5S)-1-cyclopentyl-3,8 -diazabicyclo[3.2.1]octan-3-yl)-8-fluoro-2-(((S,Z)-2-(fluoromet hylene)tetrahydro-1H-pyrrolizi n-7a(5H)-yl)methoxy)pyrido[4, 3-d]pyrimidin-7-yl)-5-ethynyl-6-fluoronaphthalen-2-amine | 680.4 |
| **144** | 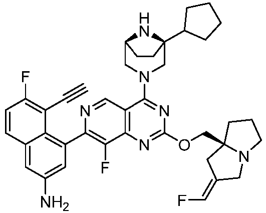 | 4-(4-((1S,5S)-1-cyclopentyl-3,8 -diazabicyclo[3.2.1]octan-3-yl)-8-fluoro-2-(((S,E)-2-(fluoromet hylene)tetrahydro-1H-pyrrolizi n-7a(5H)-yl)methoxy)pyrido[4, 3-d]pyrimidin-7-yl)-5-ethynyl-6-fluoronaphthalen-2-amine | 680.4 |
| **145** | 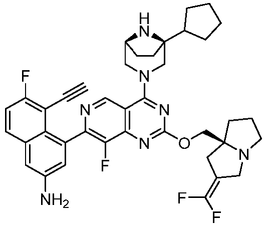 | 4-(4-((1S,5S)-1-cyclopentyl-3,8 -diazabicyclo[3.2.1]octan-3-yl)-2-(((S)-2-(difluoromethylene)te trahydro-1H-pyrrolizin-7a(5H)-yl)methoxy)-8-fluoropyrido[4,3 -d]pyrimidin-7-yl)-5-ethynyl-6-fluoronaphthalen-2-amine | 698.4 |
| **146** | 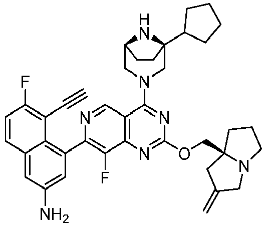 | 4-(4-((1S,5S)-1-cyclopentyl-3,8 -diazabicyclo[3.2.1]octan-3-yl)-8-fluoro-2-(((S)-2-methylenetet rahydro-1H-pyrrolizin-7a(5H)-yl)methoxy)pyrido[4,3-d]pyrim idin-7-yl)-5-ethynyl-6-fluorona phthalen-2-amine | 662.4 |
| **147** | 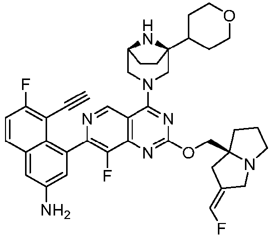 | 5-ethynyl-6-fluoro-4-(8-fluoro-2-(((S,Z)-2-(fluoromethylene)t etrahydro-1H-pyrrolizin-7a(5H )-yl)methoxy)-4-((IS,5S)-I-(te trahydro-2H-pyran-4-yl)-3,8-di azabicyclo[3.2.1]octan-3-yl)py rido[4,3-d]pyrimidin-7-yl)naph thalen-2-amine | 696.4 |
| **148** | 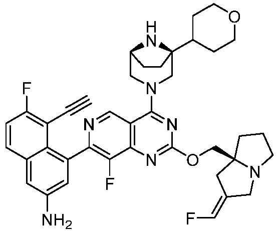 | 5-ethynyl-6-fluoro-4-(8-fluoro-2-(((S,E)-2-(fluoromethylene)t etrahydro-1H-pyrrolizin-7a(5H )-yl)methoxy)-4-((IS,5S)-I-(te trahydro-2H-pyran-4-yl)-3,8-di azabicyclo[3.2.1]octan-3-yl)py rido[4,3-d]pyrimidin-7-yl)naph thalen-2-amine | 696.4 |
| **149** | 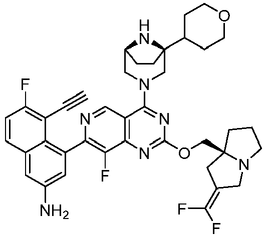 | 4-(2-(((S)-2-(difluoromethylene )tetrahydro-1H-pyrrolizin-7a(5 H)-yl)methoxy)-8-fluoro-4-((1S ,5S)-1-(tetrahydro-2H-pyran-4-yl)-3,8-diazabicyclo[3.2.1]octa n-3-yl)pyrido[4,3-d]pyrimidin-7-yl)-5-ethynyl-6-fluoronaphth alen-2-amine | 714.4 |
| **150** | 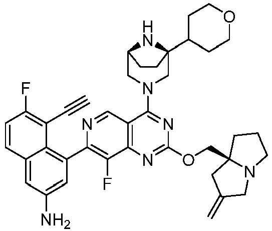 | 5-ethynyl-6-fluoro-4-(8-fluoro-2-(((S)-2-methylenetetrahydro-1H-pyrrolizin-7a(5H)-yl)meth oxy)-4-((1S,5S)-1-(tetrahydro-2H-pyran-4-yl)-3,8-diazabicycl o[3.2.1]octan-3-yl)pyrido[4,3-d]pyrimidin-7-yl)naphthalen-2 -amine | 678.4 |
| **151** | 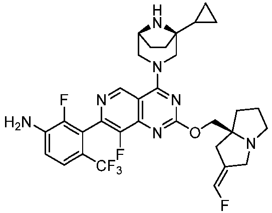 | 3-(4-((1S,5S)-1-cyclopropyl-3, 8-diazabicyclo[3.2.1]octan-3-y l)-8-fluoro-2-(((S,Z)-2-(fluoro methylene)tetrahydro-1H-pyrr olizin-7a(5H)-yl)methoxy)pyri do[4,3-d]pyrimidin-7-yl)-2-flu oro-4-(trifluoromethyl)aniline | 646.4 |
| **152** | 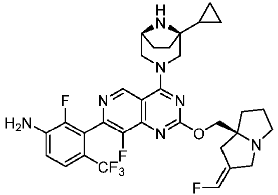 | 3-(4-((1S,5S)-1-cyclopropyl-3, 8-diazabicyclo[3.2.1]octan-3-y l)-8-fluoro-2-(((S,E)-2-(fluoro methylene)tetrahydro-1H-pyrr olizin-7a(5H)-yl)methoxy)pyri do[4,3-d]pyrimidin-7-yl)-2-flu oro-4-(trifluoromethyl)aniline | 646.4 |
| **153** | 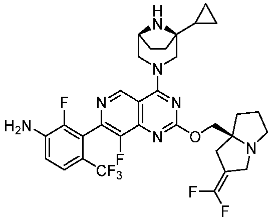 | 3-(4-((1S,5S)-1-cyclopropyl-3, 8-diazabicyclo[3.2.1]octan-3-y l)-2-(((S)-2-(difluoromethylene )tetrahydro-1H-pyrrolizin-7a(5 H)-yl)methoxy)-8-fluoropyrido [4,3-d]pyrimidin-7-yl)-2-fluoro -4-(trifluoromethyl)aniline | 664.4 |
| **154** | 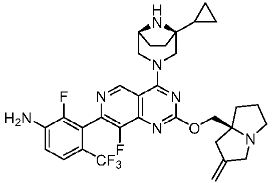 | 3-(4-((1S,5S)-1-cyclopropyl-3, 8-diazabicyclo[3.2.1]octan-3-y l)-8-fluoro-2-(((S)-2-methylen etetrahydro-1H-pyrrolizin-7a(5 H)-yl)methoxy)pyrido[4,3-d]p yrimidin-7-yl)-2-fluoro-4-(trifl uoromethyl)aniline | 628.4 |
| **155** | 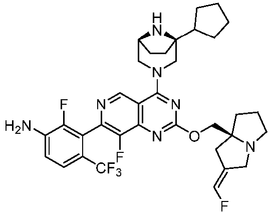 | 3-(4-((1S,5S)-1-cyclopentyl-3,8 -diazabicyclo[3.2.1]octan-3-yl)-8-fluoro-2-(((S,Z)-2-(fluoromet hylene)tetrahydro-1H-pyrrolizi n-7a(5H)-yl)methoxy)pyrido[4, 3-d]pyrimidin-7-yl)-2-fluoro-4-(trifluoromethyl)aniline | 674.4 |
| **156** | 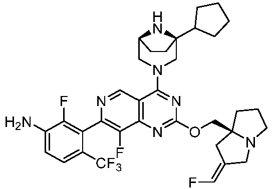 | 3-(4-((1S,5S)-1-cyclopentyl-3,8 -diazabicyclo[3.2.1]octan-3-yl)-8-fluoro-2-(((S,E)-2-(fluoromet hylene)tetrahydro-1H-pyrrolizi n-7a(5H)-yl)methoxy)pyrido[4, 3-d]pyrimidin-7-yl)-2-fluoro-4-(trifluoromethyl)aniline | 674.4 |
| **157** | 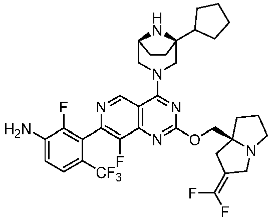 | 3-(4-((1S,5S)-1-cyclopentyl-3,8 -diazabicyclo[3.2.1]octan-3-yl)-2-(((S)-2-(difluoromethylene)te trahydro-1H-pyrrolizin-7a(5H)-yl)methoxy)-8-fluoropyrido[4,3 -d]pyrimidin-7-yl)-2-fluoro-4-(t rifluoromethyl)aniline | 692.4 |
| **158** | 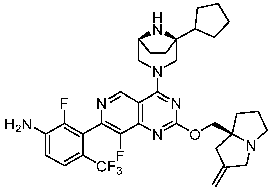 | 3-(4-((1S,5S)-1-cyclopentyl-3,8 -diazabicyclo[3.2.1]octan-3-yl)-8-fluoro-2-(((S)-2-methylenetet rahydro-1H-pyrrolizin-7a(5H)-yl)methoxy)pyrido[4,3-d]pyrim idin-7-yl)-2-fluoro-4-(trifluoro methyl)aniline | 656.4 |
| **159** | 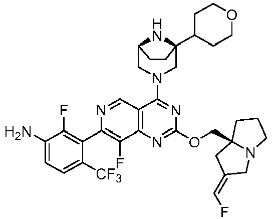 | 2-fluoro-3-(8-fluoro-2-(((S,Z)-2-(fluoromethylene)tetrahydro-1H-pyrrolizin-7a(5H)-yl)meth oxy)-4-((1S,5S)-1-(tetrahydro-2H-pyran-4-yl)-3,8-diazabicycl o[3.2.1]octan-3-yl)pyrido[4,3-d]pyrimidin-7-yl)-4-(trifluoro methyl)aniline | 690.4 |
| **160** | 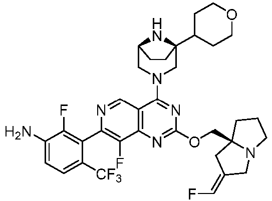 | 2-fluoro-3-(8-fluoro-2-(((S,E)-2-(fluoromethylene)tetrahydro-1H-pyrrolizin-7a(5H)-yl)meth oxy)-4-((1S,5S)-1-(tetrahydro-2H-pyran-4-yl)-3,8-diazabicycl o[3.2.1]octan-3-yl)pyrido[4,3-d]pyrimidin-7-yl)-4-(trifluoro methyl)aniline | 690.4 |
| **161** | 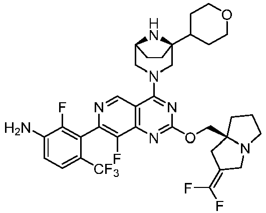 | 3-(2-(((S)-2-(difluoromethylene )tetrahydro-1H-pyrrolizin-7a(5 H)-yl)methoxy)-8-fluoro-4-((1S ,5S)-1-(tetrahydro-2H-pyran-4-yl)-3,8-diazabicyclo[3.2.1]octa n-3-yl)pyrido[4,3-d]pyrimidin-7-yl)-2-fluoro-4-(trifluorometh yl)aniline | 708.4 |
| **162** | 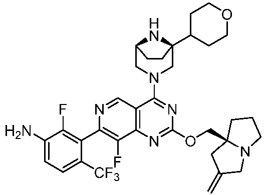 | 2-fluoro-3-(8-fluoro-2-(((S)-2-methylenetetrahydro-1H-pyrrol izin-7a(5H)-yl)methoxy)-4-((1S ,5S)-1-(tetrahydro-2H-pyran-4-yl)-3,8-diazabicyclo[3.2.1]octa n-3-yl)pyrido[4,3-d]pyrimidin-7-yl)-4-(trifluoromethyl)aniline | 672.4 |
| **163** | 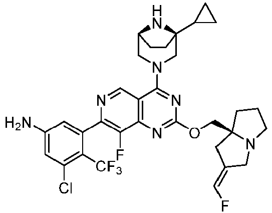 | 3-chloro-5-(4-((1S,5S)-1-cyclo propyl-3,8-diazabicyclo[3.2.1] octan-3-yl)-8-fluoro-2-(((S,Z)-2-(fluoromethylene)tetrahydro-1H-pyrrolizin-7a(5H)-yl)meth oxy)pyrido[4,3-d]pyrimidin-7-yl)-4-(trifluoromethyl)aniline | 662.4 |
| **164** | 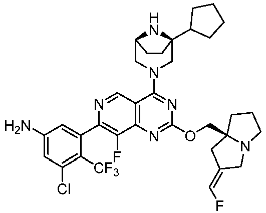 | 3-chloro-5-(4-((1S,5S)-1-cyclo pentyl-3,8-diazabicyclo[3.2.1] octan-3-yl)-8-fluoro-2-(((S,Z)-2-(fluoromethylene)tetrahydro-1H-pyrrolizin-7a(5H)-yl)meth oxy)pyrido[4,3-d]pyrimidin-7-yl)-4-(trifluoromethyl)aniline | 670.4 |
| **165** | 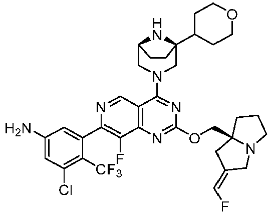 | 3-chloro-5-(8-fluoro-2-(((S,Z)-2-(fluoromethylene)tetrahydro-1H-pyrrolizin-7a(5H)-yl)meth oxy)-4-((1S,5S)-1-(tetrahydro-2H-pyran-4-yl)-3,8-diazabicycl o[3.2.1]octan-3-yl)pyrido[4,3-d]pyrimidin-7-yl)-4-(trifluoro methyl)aniline | 706.4 |
| **166** | 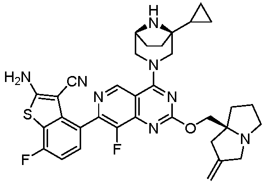 | 2-amino-4-(4-((1S,5S)-1-cyclo propyl-3,8-diazabicyclo[3.2.1] octan-3-yl)-8-fluoro-2-(((S)-2-methylenetetrahydro-1H-pyrro lizin-7a(5H)-yl)methoxy)pyrid o[4,3-d]pyrimidin-7-yl)-7-fluor obenzo[b]thiophene-3-carbonit rile | 641.4 |
| **167** | 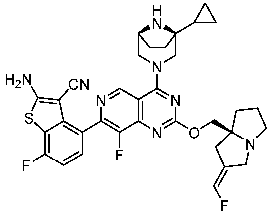 | 2-amino-4-(4-((1S,5S)-1-cyclo propyl-3,8-diazabicyclo[3.2.1] octan-3-yl)-8-fluoro-2-(((S,Z)-2-(fluoromethylene)tetrahydro-1H-pyrrolizin-7a(5H)-yl)meth oxy)pyrido[4,3-d]pyrimidin-7-yl)-7-fluorobenzo[b]thiophene-3-carbonitrile | 659.4 |
| **168** | 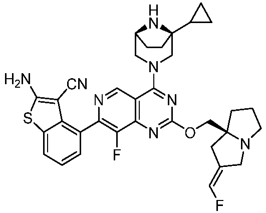 | 2-amino-4-(4-((1S,5S)-1-cyclo propyl-3,8-diazabicyclo[3.2.1] octan-3-yl)-8-fluoro-2-(((S,Z)-2-(fluoromethylene)tetrahydro-1H-pyrrolizin-7a(5H)-yl)meth oxy)pyrido[4,3-d]pyrimidin-7-yl)benzo[b]thiophene-3-carbon itrile | 641.4 |
| **169** | 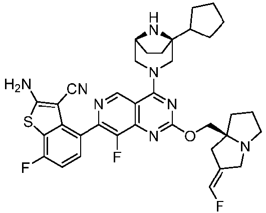 | 2-amino-4-(4-((1S,5S)-1-cyclo pentyl-3,8-diazabicyclo[3.2.1] octan-3-yl)-8-fluoro-2-(((S,Z)-2-(fluoromethylene)tetrahydro-1H-pyrrolizin-7a(5H)-yl)meth oxy)pyrido[4,3-d]pyrimidin-7-yl)-7-fluorobenzo[b]thiophene-3-carbonitrile | 687.4 |
| **170** | 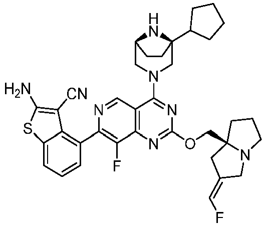 | 2-amino-4-(4-((1S,5S)-1-cyclo pentyl-3,8-diazabicyclo[3.2.1] octan-3-yl)-8-fluoro-2-(((S,Z)-2-(fluoromethylene)tetrahydro-1H-pyrrolizin-7a(5H)-yl)meth oxy)pyrido[4,3-d]pyrimidin-7-yl)benzo[b]thiophene-3-carbon itrile | 669.4 |
| **171** | 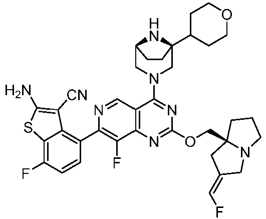 | 2-amino-7-fluoro-4-(8-fluoro-2 -(((S,Z)-2-(fluoromethylene)tet rahydro-1H-pyrrolizin-7a(5H)-yl)methoxy)-4-((1S,5S)-1-(tetr ahydro-2H-pyran-4-yl)-3,8-dia zabicyclo[3.2.1]octan-3-yl)pyri do[4,3-d]pyrimidin-7-yl)benzo [b]thiophene-3-carbonitrile | 703.4 |
| **172** | 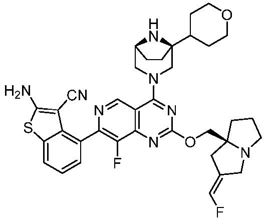 | 2-amino-4-(8-fluoro-2-(((S,Z)-2-(fluoromethylene)tetrahydro-1H-pyrrolizin-7a(5H)-yl)meth oxy)-4-((1S,5S)-1-(tetrahydro-2H-pyran-4-yl)-3,8-diazabicycl o[3.2.1]octan-3-yl)pyrido[4,3-d]pyrimidin-7-yl)benzo[b]thio phene-3-carbonitrile | 685.4 |
| **355** | 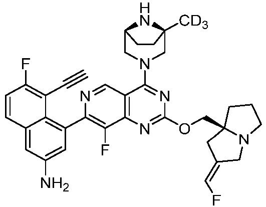 | 5-ethynyl-6-fluoro-4-(8-fluoro-2-(((S,Z)-2-(fluoromethylene)t etrahydro-1H-pyrrolizin-7a(5H )-yl)methoxy)-4-((1R,5S)-1-(m ethyl-*d₃*)-3,8-diazabicyclo[3.2. 1octan-3-yl)pyrido[4,3-d]pyri midin-7-yl)naphthalen-2-amin e | 629.4 |
| **356** | 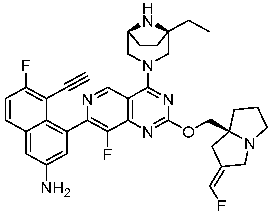 | 4-(4-((1R,5S)-1-ethyl-3,8-diaz abicyclo[3.2.1]octan-3-yl)-8-fl uoro-2-(((S,Z)-2-(fluoromethyl ene)tetrahydro-1H-pyrrolizin-7 a(SH)-yl)methoxy)pyrido[4,3-d]pyrimidin-7-yl)-5-ethynyl-6-fluoronaphthalen-2-amine | 640.4 |
| **357** | 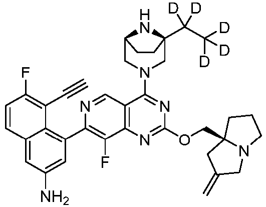 | 4-(4-((1R,5S)-1-(ethyl-*d₅*)-3,8-diazabicyclo[3.2.1]octan-3-yl)-8-fluoro-2-(((S)-2-methylenete trahydro-1H-pyrrolizin-7a(5H) -yl)methoxy)pyrido[4,3-d]pyri midin-7-yl)-5-ethynyl-6-fluoro naphthalen-2-amine | 627.4 |
| **Reference Compound 1** | 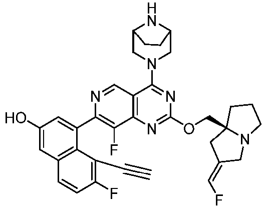 | 4-(4-((1R,5S)-3,8-diazabicyclo [3.2.1]octan-3-yl)-8-fluoro-2-(( (S,Z)-2-(fluoromethylene)tetra hydro-1H-pyrrolizin-7a(5H)-yl )methoxy)pyrido[4,3-d]pyrimi din-7-yl)-5-ethynyl-6-fluorona phthalen-2-ol | 613.6 |
| **Reference Compound 2** | 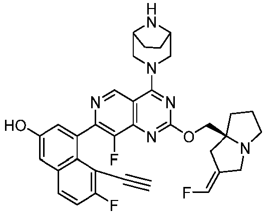 | 4-(4-((1R,5S)-3,8-diazabicyclo [3.2.1]octan-3-yl)-8-fluoro-2-(( (S,E)-2-(fluoromethylene)tetra hydro-1H-pyrrolizin-7a(5H)-yl )methoxy)pyrido[4,3-d]pyrimi din-7-yl)-5-ethynyl-6-fluorona phthalen-2-ol | 613.6 |
| **Reference Compoun d 3** | 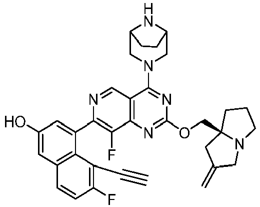 | 4-(4-((1R,5S)-3,8-diazabicyclo [3.2.1]octan-3-yl)-8-fluoro-2-(( (S)-2-methylenetetrahydro-1H-pyrrolizin-7a(5H)-yl)methoxy) pyrido[4,3-d]pyrimidin-7-yl)-5 -ethynyl-6-fluoronaphthalen-2-ol | 595.6 |
| **Reference Compoun d 4** | 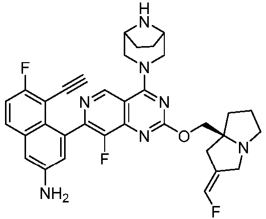 | 4-(4-((1R,5S)-3,8-diazabicyclo [3.2.1]octan-3-yl)-8-fluoro-2-(( (S,Z)-2-(fluoromethylene)tetra hydro-1H-pyrrolizin-7a(5H)-yl )methoxy)pyrido[4,3-d]pyrimi din-7-yl)-5-ethynyl-6-fluorona phthalen-2-amine | 612.6 |

### Example 173: Preparation of 4-(2-((2,6-dimethylenetetrahydro-1H-pyrrolizin-7a(5H)-yl)methoxy)-8-fluoro-5-me thoxy-4-((1S,5R)-1-((methoxy-d₃)methyl)-3,8-diazabicyclo[3.2.1]octan-3-yl)pyrido[ 4,3-d] pyrimidin-7-yl)-5-ethynyl-6-fluoronaphthalen-2-ol

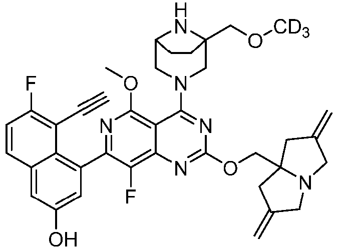

### Step 1: Synthesis of 7-chloro-8-fluoro-5-methoxy-2-(methylthio)pyrido[4,3-d]pyrimidin-4(3H)-one

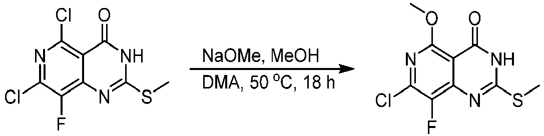

5,7-dichloro-8-fluoro-2-(methylthio)pyrido[4,3-d]pyrimidin-4(3H)-one (1.0 g, 3.57 mmol) was dissolved in DMF (50 mL), a methanol solution of sodium methoxide (1.29 g, 7.14 mmol) was added at 0°C, and the mixture was heated and reacted at 50°C for 2 hours. After the reaction was completed, the solution was diluted with ethyl acetate, and the organic phase was separated and washed with saturated brine once. The organic phase was dried and filtered, the filtrate was concentrated, and the resulting residue was separated by fast silica gel column chromatography to obtain 7-chloro-8-fluoro-5-methoxy-2-(methylthio)pyrido[4,3-d]pyrimidin-4(3H)-one (597 mg, yield: 60.7%). ESI-MS: 276.2 [M+1]⁺.

### Step 2: Synthesis of 4,7-dichloro-8-fluoro-5-methoxy-2-(methylthio)pyrido [4,3-d] pyrimidin

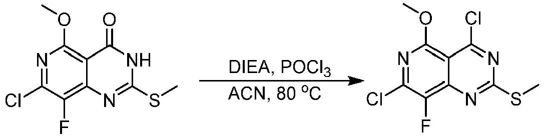

7-chloro-8-fluoro-5-methoxy-2-(methylthio)pyrido[4,3-d]pyrimidin-4(3H)-one (597 mg, 2.17 mmol) was dissolved in acetonitrile (10 mL), DIPEA (1.12 g, 8.66 mmol) and POCl₃ (664 mg, 4.33 mmol) was added at 0°C, and the mixture was heated and reacted at 80°C for 2 hours. After the reaction was completed, the reaction solution was concentrated to obtain 4,7-dichloro-8-fluoro-5-methoxy-2-(methylthio)pyrido[4,3-d]pyrimidine (637 mg, yield: 100%). ESI-MS: 294.2 [M+1]⁺.

### Step 3: Synthesis of tert-butyl (1S,5R)-3-(7-chloro-8-fluoro-5-methoxy-2-(methylthio)pyrido[4,3-d]pyrimidin-4-yl )-1-((methoxy-d₃)methyl)-3,8-diazabicyclo[3.2.1]octan-8-carboxylate

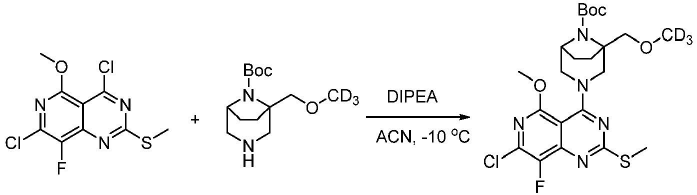

4,7-dichloro-8-fluoro-5-methoxy-2-(methylthio)pyrido[4,3-d]pyrimidine (637 mg, 2.17 mmol) was dissolved in acetonitrile (10 mL), DIPEA (1.40 g, 10.83 mmol) and tert-butyl (1S,5R)-1-((methoxy-***d*₃**)methyl)-3,8-diazabicyclo[3.2.1]octan-8-carboxylate (618 mg, 2.38 mmol) were added at 0°C, and the mixture was reacted at 0°C for 2 hours. After the reaction was completed, the solution was diluted with ethyl acetate, and the organic phase was separated and washed with saturated brine once. The organic phase was dried and filtered, the filtrate was concentrated, and the resulting residue was separated by fast silica gel column chromatography to obtain tert-butyl (1S,5R)-3-(7-chloro-8-fluoro-5-methoxy-2-(methylthio)pyrido[4,3-d]pyrimidin-4-yl)-1-((methoxy-***d*₃**)methyl)-3,8-diazabicyclo[3.2.1]octan-8-carboxylate (927 mg, yield: 82.8%). ESI-MS: 517.2 [M+1]⁺.

### Step 4: Synthesis of 8-fluoro-7-(7-fluoro-3-(methoxymethoxy)-8-((triisopropylsilyl)ethynyl)naphthalen-1-yl)-5-methoxy-4-((1S,5R)-1-((methoxy-d₃)methyl)-3,8-diazabicyclo[3.2.1]octan-3-yl)-2-(methylthio)pyrido[4,3-d]pyrimidine

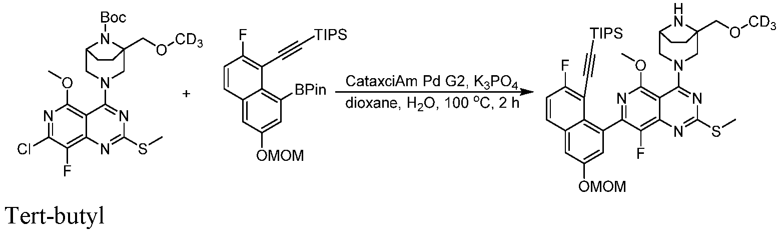

(1S,5R)-3-(7-chloro-8-fluoro-5-methoxy-2-(methylthio)pyrido[4,3-d]pyrimidin-4-yl)-1-((methoxy-*d*₃)methyl)-3,8-diazabicyclo[3.2.1]octan-8-carboxylate (927 mg, 1.79 mmol), ((2-fluoro-6-(methoxymethoxy)-8-(4,4,5,5-tetramethyl-1,3,2-dioxaborolan-2-yl)naphtha len-1-yl)ethynyl)triisopropylsilane (919 mg, 1.79 mmol) and potassium phosphate (1.52 g, 7.17 mmol) were dissolved in 1,4-dioxane (20 mL) and water (2 mL), CataxciAm Pd G2 (240 mg, 0.36 mmol) was added, the nitrogen substitution was carried out three times, and the mixture was heated and reacted at 100°C for 2 hours. After the reaction was completed, the solution was diluted with ethyl acetate, and the organic phase was separated and washed with saturated brine once. The organic phase was dried and filtered, the filtrate was concentrated, and the resulting residue was separated by fast silica gel column chromatography to obtain tert-butyl (1S,5R)-3-(8-fluoro-7-(7-fluoro-3-(methoxymethoxy)-8-((triisopropylsilyl)ethynyl)naph thalen-1-yl)-5-methoxy-2-(methylthio)pyrido[4,3-d]pyrimidin-4-yl)-1-((methoxy-*d*₃)me thyl)-3,8-diazabicyclo[3.2.1]octan-8-carboxylate (923 mg, yield: 59.6%). ESI-MS: 767.4 [M+1]⁺.

### Step 5: Synthesis of tert-butyl (1S,5R)-3-(8-fluoro-7-(7-fluoro-3-(methoxymethoxy)-8-((triisopropylsilyl)ethynyl)n aphthalen-1-yl)-5-methoxy-2-(methylsulfinyl)pyrido[4,3-d]pyrimidin-4-yl)-1-((met hoxy-d₃)methyl)-3,8-diazabicyclo[3.2.1]octan-8-carboxylate

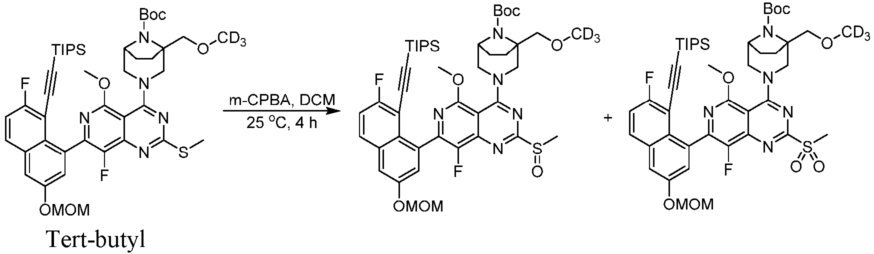

(1S,5R)-3-(8-fluoro-7-(7-fluoro-3-(methoxymethoxy)-8-((triisopropylsilyl)ethynyl)naph thalen-1-yl)-5-methoxy-2-(methylthio)pyrido[4,3-d]pyrimidin-4-yl)-1-((methoxy-*d*₃)me thyl)-3,8-diazabicyclo[3.2.1]octan-8-carboxylate (923 mg, 1.06 mmol) was dissolved in CH₂Cl₂ (50 mL), *m*-CPBA (303 mg, 1.49 mmol) was added at 0°C, and the mixture was reacted at room temperature for 1 hour. After the reaction was completed, the solution was diluted with ethyl acetate, and the organic phase was separated and washed with saturated brine once. The organic phase was dried and filtered, the filtrate was concentrated, and the resulting residue was separated by fast silica gel column chromatography to obtain tert-butyl (1S,5R)-3-(8-fluoro-7-(7-fluoro-3-(methoxymethoxy)-8-((triisopropylsilyl)ethynyl)naph thalen-1-yl)-5-methoxy-2-(methylsulfinyl)pyrido[4,3-d]pyrimidin-4-yl)-1-((methoxy-*d*₃ )methyl)-3,8-diazabicyclo[3.2.1]octan-8-carboxylate and tert-butyl (1S,5R)-3-(8-fluoro-7-(7-fluoro-3-(methoxymethoxy)-8-((triisopropylsilyl)ethynyl)naph thalen-1-yl)-5-methoxy-2-(methanesulfonyl)pyrido[4,3-d]pyrimidin-4-yl)-1-((methoxy-***d*₃**)methyl)-3,8-diazabicyclo[3.2.1]octan-8-carboxylate (950 mg, mixture). ESI-MS: 883.4 [M+1]⁺, 899.4 [M+1]⁺.

### Step 6: Synthesis of tert-butyl (1S,5R)-3-(2-((2,6-dimethylenetetrahydro-1H-pyrrolizin-7a(5H)-yl)methoxy)-8-fluo ro-7-(7-fluoro-3-(methoxymethoxy)-8-((triisopropylsilyl)ethynyl)naphthalen-1-yl)-5 -methoxypyrido[4,3-d]pyrimidin-4-yl)-1-((methoxy-d₃)methyl)-3,8-diazabicyclo[3.2 .1] octan-8-carboxylate

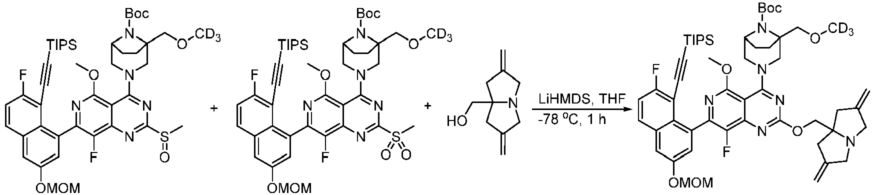

(2,6-dimethylenetetrahydro-1H-pyrrolizin-7a(5H)-yl)methanol (261 mg, 1.58 mmol) was dissolved in dry THF (20 mL), 1 M LiHMDS/THF solution (1.26 mL, 1.26 mmol) was added under cooling in an ice-water bath, the mixture was reacted for 30 minutes, and the crude product obtained in the previous step (930 mg, 1.053 mmol) was added. The mixture was reacted at 0°C for 1 hour. After the reaction was completed, the solution was diluted with ethyl acetate, and the organic phase was separated and washed with saturated brine once. The organic phase was dried and filtered, the filtrate was concentrated, and the resulting residue was separated by fast silica gel column chromatography to obtain tert-butyl (1S,5R)-3-(2-((2,6-dimethylenetetrahydro-1H-pyrrolizin-7a(5H)-yl)methoxy)-8-fluoro-7-(7-fluoro-3-(methoxymethoxy)-8-((triisopropylsilyl)ethynyl)naphthalen-1-yl)-5-meth oxypyrido[4,3-d]pyrimidin-4-yl)-1-((methoxy-*d*₃)methyl)-3,8-diazabicyclo[3.2.1]octan-8-carboxylate (272 mg, yield: 26.2%). ESI-MS: 984.5 [M+1]⁺.

### Step 7: Synthesis of 4-(2-((2,6-dimethylenetetrahydro-1H-pyrrolizin-7a(5H)-yl)methoxy)-8-fluoro-5-me thoxy-4-((1S,5R)-1-((methoxy-d₃)methyl)-3,8-diazabicyclo[3.2.1]octan-3-yl)pyrido[ 4,3-d]pyrimidin-7-yl)-6-fluoro-5-((triisopropylsilyl)ethynyl)naphthalen-2-ol

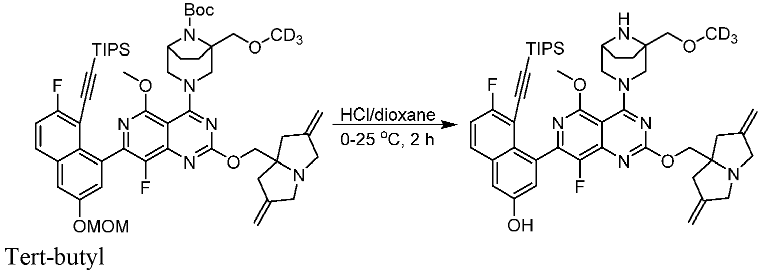

(1S,5R)-3-(2-((2,6-dimethylenetetrahydro-1H-pyrrolizin-7a(5H)-yl)methoxy)-8-fluoro-7-(7-fluoro-3-(methoxymethoxy)-8-((triisopropylsilyl)ethynyl)naphthalen-1-yl)-5-meth oxypyrido[4,3-d]pyrimidin-4-yl)-1-((methoxy-*d*₃)methyl)-3,8-diazabicyclo[3.2.1]octan-8-carboxylate (262 mg, 0.27 mmol) was dissolved in CH₂Cl₂ (4 mL), hydrochloric acid/dioxane solution (1 mL, 1 N) was added under cooling in an ice-water bath, and the mixture was reacted at 0°C for 2 hours. After the reaction was completed, the reaction solution was concentrated to obtain 4-(2-((2,6-dimethylenetetrahydro-1H-pyrrolizin-7a(5H)-yl)methoxy)-8-fluoro-5-methox y-4-((1S,5R)-1-((methoxy-*d*₃)methyl)-3,8-diazabicyclo[3.2.1]octan-3-yl)pyrido[4,3-d]p yrimidin-7-yl)-6-fluoro-5-((triisopropylsilyl)ethynyl)naphthalen-2-ol (223.6 mg, yield: 100%). ESI-MS: 840.4 [M+1]⁺.

### Step 8: Synthesis of 4-(2-((2,6-dimethylenetetrahydro-1H-pyrrolizin-7a(5H)-yl)methoxy)-8-fluoro-5-me thoxy-4-((1S,5R)-1-((methoxy-d₃)methyl)-3,8-diazabicyclo[3.2.1]octan-3-yl)pyrido[ 4,3-d]pyrimidin-7-yl)-5-ethynyl-6-fluoronaphthalen-2-ol

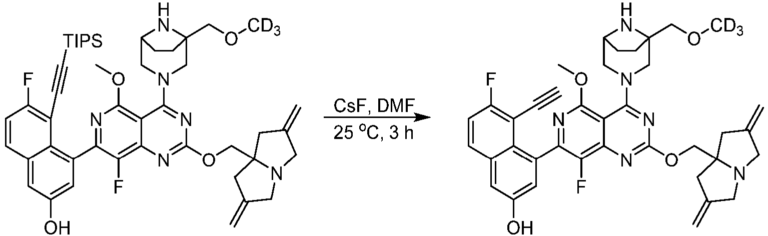

4-(2-((2,6-dimethylenetetrahydro-1H-pyrrolizin-7a(5H)-yl)methoxy)-8-fluoro-5-m ethoxy-4-((1S,5R)-1-((methoxy-*d*₃)methyl)-3,8-diazabicyclo[3.2.1]octan-3-yl)pyrido[4, 3-d]pyrimidin-7-yl)-6-fluoro-5-((triisopropylsilyl)ethynyl)naphthalen-2-ol (223.6 mg, 0.27 mmol) was dissolved in DMF (10 mL), CsF (4.04 g, 26.62 mmol) was added, and the mixture was heated to 50°C and stirred for 2 hours. The reaction was completed as monitored by LCMS, and the reaction solution was filtered, concentrated and separated by reversed-phase column chromatography [eluent: 0-80% acetonitrile: water] to obtain 4-(2-((2,6-dimethylenetetrahydro-1H-pyrrolizin-7a(5H)-yl)methoxy)-8-fluoro-5-methox y-4-((1S,5R)-1-((methoxy-***d*₃**)methyl)-3,8-diazabicyclo[3.2.1]octan-3-yl)pyrido[4,3-d]p yrimidin-7-yl)-5-ethynyl-6-fluoronaphthalen-2-ol (61.6 mg, yield: 33.8%) ESI-MS: 684.4 [M+1]⁺.

¹H NMR (400 MHz, MeOH-*d*₄) δ 8.71 (dd, *J* = 9.1, 5.9 Hz, 1H), 8.24 - 8.12 (m, 2H), 8.03 (dd, *J =* 6.2, 2.4 Hz, 1H), 5.74 (d, *J* = 8.8 Hz, 4H), 4.70 (s, 3H), 4.58 - 4.30 (m, 6H), 4.26 - 4.09 (m, 4H), 4.06 - 3.93 (m, 5H), 3.44 (d, *J* = 16.3 Hz, 2H), 2.60 - 2.48 (m, 2H), 2.43 - 2.13 (m, 3H).

**Examples 174 to 219 and 358 to 359 can be prepared by selecting the corresponding intermediates with reference to the route of Example 173.**

| **Example No.** | **Structural Formula** | **Chemical Name** | **ESI-MS: [M+1]⁺** |
|---|---|---|---|
| **174** | 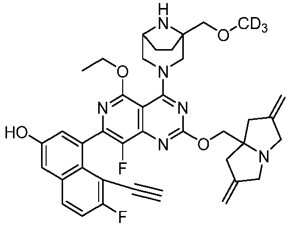 | 4-(2-((2,6-dimethylenetetra hydro-1H-pyrrolizin-7a(5H) -yl)methoxy)-5-ethoxy-8-fl uoro-4-(1-((methoxy-***d*₃**)met hyl)-3,8-diazabicyclo[3.2.1] octan-3-yl)pyrido[4,3-d]pyr imidin-7-yl)-5-ethynyl-6-flu oronaphthalen-2-ol | 698.4 |
| **175** | 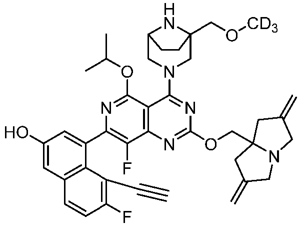 | 4-(2-((2,6-dimethylenetetra hydro-1H-pyrrolizin-7a(5H) -yl)methoxy)-8-fluoro-5-iso propoxy-4-(1-((methoxy-*d*₃) methyl)-3,8-diazabicyclo[3. 2.1]octan-3-yl)pyrido[4,3-d] pyrimidin-7-yl)-5-ethynyl-6 -fluoronaphthalen-2-ol | 712.4 |
| **176** | 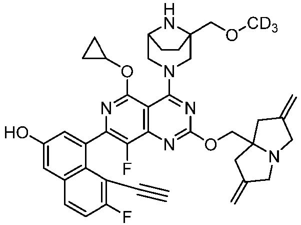 | 4-(5-cyclopropyloxy-2-((2,6 -dimethylenetetrahydro-1H-pyrrolizin-7a(5H)-yl)metho xy)-8-fluoro-4-(1-((methoxy -*d*₃)methyl)-3,8-diazabicycl o[3.2.1]octan-3-yl)pyrido[4, 3-d]pyrimidin-7-yl)-5-ethyn yl-6-fluoronaphthalen-2-ol | 710.4 |
| **177** | 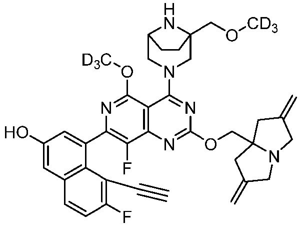 | 4-(2-((2,6-dimethylenetetra hydro-1H-pyrrolizin-7a(5H) -yl)methoxy)-8-fluoro-5-(m ethoxy-*d*₃)-4-(1-((methoxy-*d*₃)methyl)-3,8-diazabicyclo [3.2.1]octan-3-yl)pyrido[4,3 -d]pyrimidin-7-yl)-5-ethyny l-6-fluoronaphthalen-2-ol | 687.4 |
| **178** | 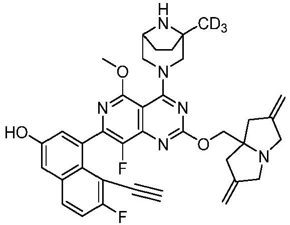 | 4-(2-((2,6-dimethylenetetra hydro-1H-pyrrolizin-7a(5H) -yl)methoxy)-8-fluoro-5-me thoxy-4-(1-(methyl-***d*₃**)-3,8-diazabicyclo[3.2.1]octan-3-yl)pyrido[4,3-d]pyrimidin-7 -yl)-5-ethynyl-6-fluoronaph thalen-2-ol | 654.4 |
| **179** | 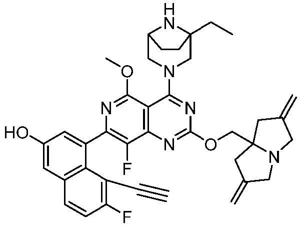 | 4-(2-((2,6-dimethylenetetra hydro-1H-pyrrolizin-7a(5H) -yl)methoxy)-4-(1-ethyl-3,8 -diazabicyclo[3.2.1]octan-3-yl)-8-fluoro-5-methoxypyri do[4,3-d]pyrimidin-7-yl)-5-ethynyl-6-fluoronaphthalen-2-ol | 665.4 |
| **180** | 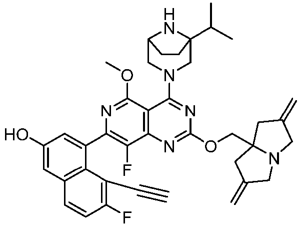 | 4-(2-((2,6-dimethylenetetra hydro-1H-pyrrolizin-7a(5H) -yl)methoxy)-8-fluoro-4-(1-isopropyl-3,8-diazabicyclo[ 3.2.1]octan-3-yl)-5-methox ypyrido[4,3-d]pyrimidin-7-yl)-5-ethynyl-6-fluoronapht halen-2-ol | 679.4 |
| **181** | 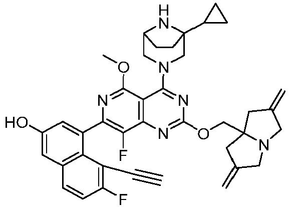 | 4-(4-(1-cyclopropyl-3,8-dia zabicyclo[3.2.1]octan-3-yl)-2-((2,6-dimethylenetetrahyd ro-1H-pyrrolizin-7a(5H)-yl) methoxy)-8-fluoro-5-metho xypyrido[4,3-d]pyrimidin-7 -yl)-5-ethynyl-6-fluoronaph thalen-2-ol | 677.4 |
| **182** | 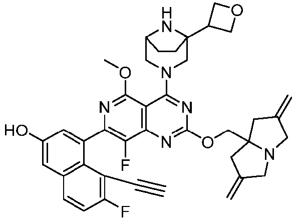 | 4-(2-((2,6-dimethylenetetrah ydro-1H-pyrrolizin-7a(5H)-yl)methoxy)-8-fluoro-5-met hoxy-4-(1-(oxetan-3-yl)-3,8 -diazabicyclo[3.2.1]octan-3-yl)pyrido[4,3-d]pyrimidin-7 -yl)-5-ethynyl-6-fluoronapht halen-2-ol | 693.4 |
| **183** | 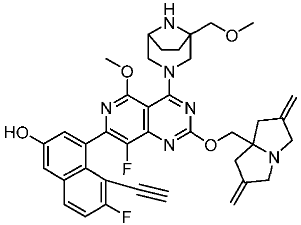 | 4-(2-((2,6-dimethylenetetra hydro-1H-pyrrolizin-7a(5H) -yl)methoxy)-8-fluoro-5-me thoxy-4-(1-(methoxymethyl )-3,8-diazabicyclo[3.2.1]oct an-3-yl)pyrido[4,3-d]pyrimi din-7-yl)-5-ethynyl-6-fluoro naphthalen-2-ol | 681.4 |
| **184** | 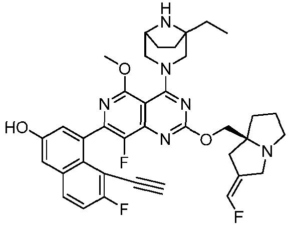 | 4-(4-(1-ethyl-3,8-diazabicycl o[3.2.1]octan-3-yl)-8-fluoro-2-(((S,Z)-2-(fluoromethylene )tetrahydro-1H-pyrrolizin-7a (5H)-yl)methoxy)-5-methoxy pyrido[4,3-d]pyrimidin-7-yl) -5-ethynyl-6-fluoronaphthale n-2-ol | 671.4 |
| **185** | 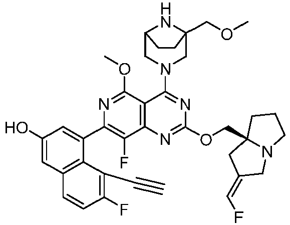 | 5-ethynyl-6-fluoro-4-(8-fluo ro-2-(((S,Z)-2-(fluoromethy lene)tetrahydro-1H-pyrroliz in-7a(5H)-yl)methoxy)-5-m ethoxy-4-(1-(methoxymethy l)-3,8-diazabicyclo[3.2.1]oc tan-3-yl)pyrido[4,3-d]pyrim idin-7-yl)naphthalen-2-ol | 687.4 |
| **186** | 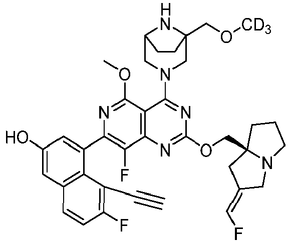 | 5-ethynyl-6-fluoro-4-(8-fluo ro-2-(((S,Z)-2-(fluoromethy lene)tetrahydro-1H-pyrroliz in-7a(5H)-yl)methoxy)-5-m ethoxy-4-(1-((methoxy-***d*₃**) methyl)-3,8-diazabicyclo[3. 2.1]octan-3-yl)pyrido[4,3-d] pyrimidin-7-yl)naphthalen-2-ol | 690.4 |
| **187** | 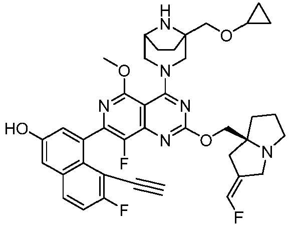 | 4-(4-(1-(cyclopropyloxymet hyl)-3,8-diazabicyclo[3.2.1] octan-3-yl)-8-fluoro-2-(((S, Z)-2-(fluoromethylene)tetra hydro-1H-pyrrolizin-7a(5H) -yl)methoxy)-5-methoxypyr ido[4,3-d]pyrimidin-7-yl)-5 -ethynyl-6-fluoronaphthalen -2-ol | 713.4 |
| **188** | 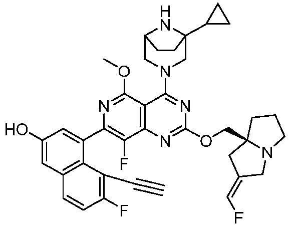 | 4-(4-(1-cyclopropyl-3,8-dia zabicyclo[3.2.1]octan-3-yl)-8-fluoro-2-(((S,Z)-2-(fluoro methylene)tetrahydro-1H-p yrrolizin-7a(5H)-yl)methox y)-5-methoxypyrido[4,3-d]p yrimidin-7-yl)-5-ethynyl-6-f luoronaphthalen-2-ol | 683.4 |
| **189** | 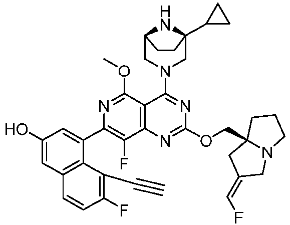 | 4-(4-((1S,5S)-1-cyclopropyl -3,8-diazabicyclo[3.2.1]octa n-3-yl)-8-fluoro-2-(((S,Z)-2-(fluoromethylene)tetrahydro -1H-pyrrolizin-7a(5H)-yl)m ethoxy)-5-methoxypyrido[4, 3-d]pyrimidin-7-yl)-5-ethyn yl-6-fluoronaphthalen-2-ol | 683.4 |
| **190** | 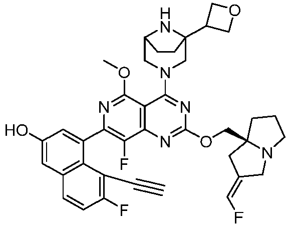 | 5-ethynyl-6-fluoro-4-(8-fluo ro-2-(((S,Z)-2-(fluoromethy lene)tetrahydro-1H-pyrroliz in-7a(5H)-yl)methoxy)-5-m ethoxy-4-(1-(oxetan-3-yl)-3 ,8-diazabicyclo[3.2.1]octan-3-yl)pyrido[4,3-d]pyrimidin -7-yl)naphthalen-2-ol | 699.4 |
| **191** | 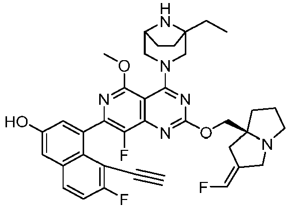 | 4-(4-(1-ethyl-3,8-diazabicycl o[3.2.1]octan-3-yl)-8-fluoro-2-(((S,E)-2-(fluoromethylene )tetrahydro-1H-pyrrolizin-7a (5H)-yl)methoxy)-5-methoxy pyrido[4,3-d]pyrimidin-7-yl) -5-ethynyl-6-fluoronaphthale n-2-ol | 671.4 |
| **192** | 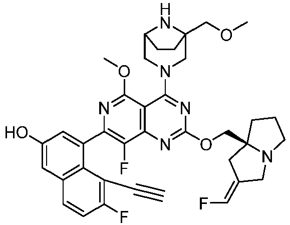 | 5-ethynyl-6-fluoro-4-(8-fluo ro-2-(((S,E)-2-(fluoromethy lene)tetrahydro-1H-pyrroliz in-7a(5H)-yl)methoxy)-5-m ethoxy-4-(1-(methoxymethy l)-3,8-diazabicyclo[3.2.1]oc tan-3-yl)pyrido[4,3-d]pyrim idin-7-yl)naphthalen-2-ol | 687.4 |
| **193** | 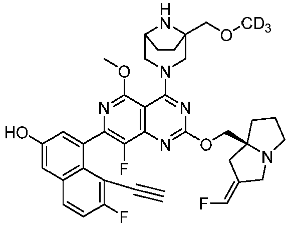 | 5-ethynyl-6-fluoro-4-(8-fluo ro-2-(((S,E)-2-(fluoromethy lene)tetrahydro-1H-pyrroliz in-7a(5H)-yl)methoxy)-5-m ethoxy-4-(1-((methoxy-***d*₃**) methyl)-3,8-diazabicyclo[3. 2.1]octan-3-yl)pyrido[4,3-d] pyrimidin-7-yl)naphthalen-2-ol | 690.4 |
| **194** | 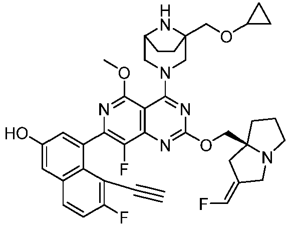 | 4-(4-(1-(cyclopropyloxymet hyl)-3,8-diazabicyclo[3.2.1] octan-3-yl)-8-fluoro-2-(((S, E)-2-(fluoromethylene)tetra hydro-1H-pyrrolizin-7a(5H) -yl)methoxy)-5-methoxypyr ido[4,3-d]pyrimidin-7-yl)-5 -ethynyl-6-fluoronaphthalen -2-ol | 713.4 |
| **195** | 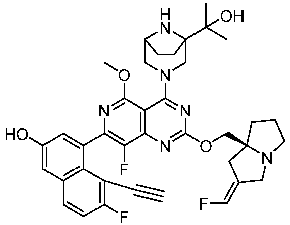 | 5-ethynyl-6-fluoro-4-(8-fluo ro-2-(((S,E)-2-(fluoromethyl ene)tetrahydro-1H-pyrrolizi n-7a(5H)-yl)methoxy)-4-(1-(2-hydroxypropan-2-yl)-3,8-diazabicyclo[3.2.1]octan-3-yl)-5-methoxypyrido[4,3-d] pyrimidin-7-yl)naphthalen-2 -ol | 701.4 |
| **196** | 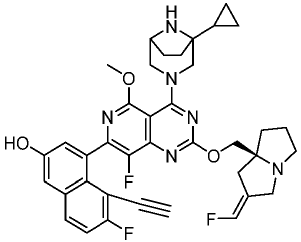 | 4-(4-(1-cyclopropyl-3,8-dia zabicyclo[3.2.1]octan-3-yl)-8-fluoro-2-(((S,E)-2-(fluoro methylene)tetrahydro-1H-p yrrolizin-7a(5H)-yl)methox y)-5-methoxypyrido[4,3-d]p yrimidin-7-yl)-5-ethynyl-6-f luoronaphthalen-2-ol | 683.4 |
| **197** | 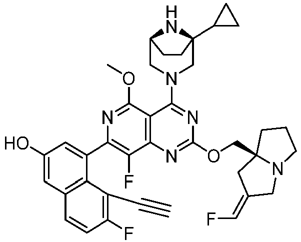 | 4-(4-((1S,5S)-1-cyclopropyl -3,8-diazabicyclo[3.2.1]octa n-3-yl)-8-fluoro-2-(((S,E)-2 -(fluoromethylene)tetrahydr o-1H-pyrrolizin-7a(5H)-yl) methoxy)-5-methoxypyrido [4,3-d]pyrimidin-7-yl)-5-eth ynyl-6-fluoronaphthalen-2-ol | 683.4 |
| **198** | 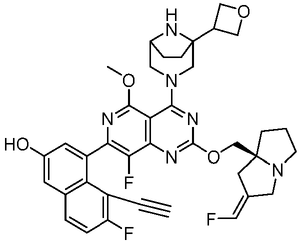 | 5-ethynyl-6-fluoro-4-(8-fluo ro-2-(((S,E)-2-(fluoromethy lene)tetrahydro-1H-pyrroliz in-7a(5H)-yl)methoxy)-5-m ethoxy-4-(1-(oxetan-3-yl)-3 ,8-diazabicyclo[3.2.1]octan-3-yl)pyrido[4,3-d]pyrimidin -7-yl)naphthalen-2-ol | 699.4 |
| **199** | 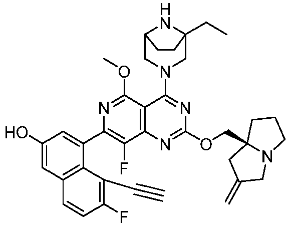 | 4-(4-(1-ethyl-3,8-diazabicycl o[3.2.1]octan-3-yl)-8-fluoro-5-methoxy-2-(((S)-2-methyle netetrahydro-1H-pyrrolizin-7 a(5H)-yl)methoxy)pyrido[4,3 -d]pyrimidin-7-yl)-5-ethynyl-6-fluoronaphthalen-2-ol | 653.4 |
| **200** | 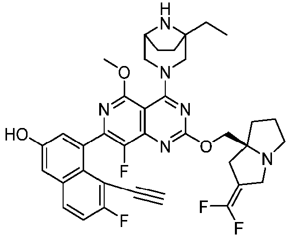 | 4-(2-(((S)-2-(difluoromethyl ene)tetrahydro-1H-pyrrolizi n-7a(5H)-yl)methoxy)-4-(1-ethyl-3,8-diazabicyclo[3.2.1 ]octan-3-yl)-8-fluoro-5-met hoxypyrido[4,3-d]pyrimidin -7-yl)-5-ethynyl-6-fluorona phthalen-2-ol | 689.4 |
| **201** | 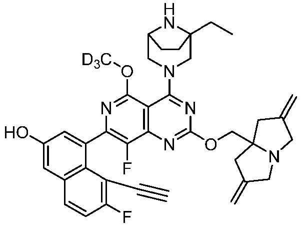 | 4-(2-((2,6-dimethylenetetra hydro-1H-pyrrolizin-7a(5H) -yl)methoxy)-4-(1-ethyl-3,8 -diazabicyclo[3.2.1]octan-3-yl)-8-fluoro-5-(methoxy-***d*₃**) pyrido[4,3-d]pyrimidin-7-yl )-5-ethynyl-6-fluoronaphtha len-2-ol | 668.4 |
| **202** | 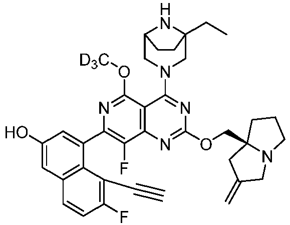 | 4-(4-(1-ethyl-3,8-diazabicycl o[3.2.1]octan-3-yl)-8-fluoro-5-(methoxy-***d*₃**)-2-(((S)-2-met hylenetetrahydro-1H-pyrroliz in-7a(5H)-yl)methoxy)pyrido [4,3-d]pyrimidin-7-yl)-5-ethy nyl-6-fluoronaphthalen-2-ol | 656.4 |
| **203** | 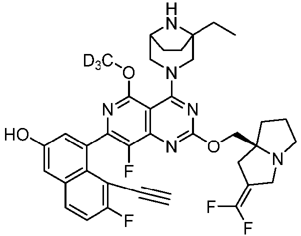 | 4-(2-(((S)-2-(difluoromethyl ene)tetrahydro-1H-pyrrolizi n-7a(5H)-yl)methoxy)-4-(1-ethyl-3,8-diazabicyclo[3.2.1 ]octan-3-yl)-8-fluoro-5-(met hoxy-***d*₃**)pyrido[4,3-d]pyrim idin-7-yl)-5-ethynyl-6-fluor onaphthalen-2-ol | 692.4 |
| **204** | 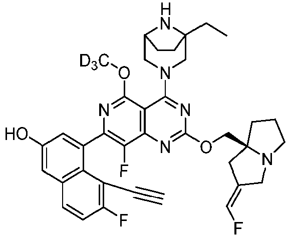 | 4-(4-(1-ethyl-3,8-diazabicycl o[3.2.1]octan-3-yl)-8-fluoro-2-(((S,Z)-2-(fluoromethylene )tetrahydro-1H-pyrrolizin-7a( 5H)-yl)methoxy)-5-(methoxy -***d*₃**)pyrido[4,3-d]pyrimidin-7 -yl)-5-ethynyl-6-fluoronapht halen-2-ol | 674.4 |
| **205** | 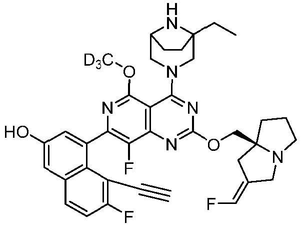 | 4-(4-(1-ethyl-3,8-diazabicycl o[3.2.1]octan-3-yl)-8-fluoro-2-(((S,E)-2-(fluoromethylene )tetrahydro-1H-pyrrolizin-7a( 5H)-yl)methoxy)-5-(methoxy -***d*₃**)pyrido[4,3-d]pyrimidin-7 -yl)-5-ethynyl-6-fluoronapht halen-2-ol | 674.4 |
| **206** | 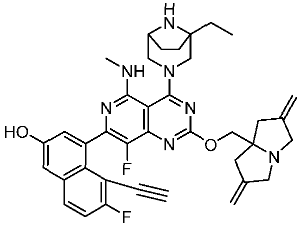 | 4-(2-((2,6-dimethylenetetra hydro-1H-pyrrolizin-7a(5H) -yl)methoxy)-4-(1-ethyl-3,8 -diazabicyclo[3.2.1]octan-3-yl)-8-fluoro-5-(methylamin o)pyrido[4,3-d]pyrimidin-7-yl)-5-ethynyl-6-fluoronapht halen-2-ol | 664.4 |
| **207** | 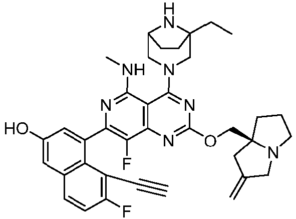 | 4-(4-(1-ethyl-3,8-diazabicycl o[3.2.1]octan-3-yl)-8-fluoro-5-(methylamino)-2-(((S)-2-m ethylenetetrahydro-1H-pyrrol izin-7a(5H)-yl)methoxy)pyri do[4,3-d]pyrimidin-7-yl)-5-et hynyl-6-fluoronaphthalen-2-ol | 652.4 |
| **208** | 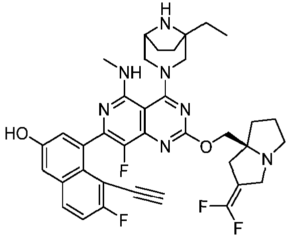 | 4-(2-(((S)-2-(difluoromethyl ene)tetrahydro-1H-pyrrolizi n-7a(5H)-yl)methoxy)-4-(1-ethyl-3,8-diazabicyclo[3.2.1 ]octan-3-yl)-8-fluoro-5-(met hylamino)pyrido[4,3-d]pyri midin-7-yl)-5-ethynyl-6-flu oronaphthalen-2-ol | 688.4 |
| **209** | 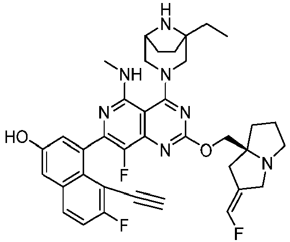 | 4-(4-(1-ethyl-3,8-diazabicycl o[3.2.1]octan-3-yl)-8-fluoro-2-(((S,Z)-2-(fluoromethylene )tetrahydro-1H-pyrrolizin-7a( 5H)-yl)methoxy)-5-(methyla mino)pyrido[4,3-d]pyrimidin -7-yl)-5-ethynyl-6-fluoronap hthalen-2-ol | 670.4 |
| **210** | 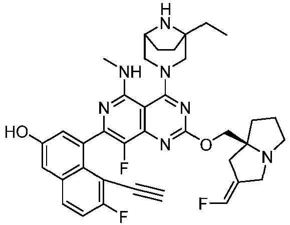 | 4-(4-(1-ethyl-3,8-diazabicycl o[3.2.1]octan-3-yl)-8-fluoro-2-(((S,E)-2-(fluoromethylene )tetrahydro-1H-pyrrolizin-7a (5H)-yl)methoxy)-5-(methyl amino)pyrido[4,3-d]pyrimidi n-7-yl)-5-ethynyl-6-fluorona phthalen-2-ol | 670.4 |
| **211** | 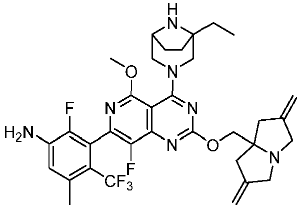 | 3-(2-((2,6-dimethylenetetra hydro-1H-pyrrolizin-7a(5H) -yl)methoxy)-4-(1-ethyl-3,8 -diazabicyclo[3.2.1]octan-3-yl)-8-fluoro-5-methoxypyri do[4,3-d]pyrimidin-7-yl)-2-fluoro-5-methyl-4-(trifluoro methyl)aniline | 672.4 |
| **212** | 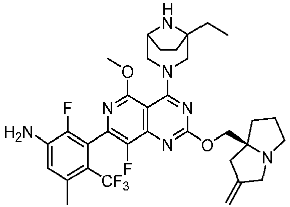 | 3-(4-(1-ethyl-3,8-diazabicycl o[3.2.1]octan-3-yl)-8-fluoro-5-methoxy-2-(((S)-2-methyle netetrahydro-1H-pyrrolizin-7 a(5H)-yl)methoxy)pyrido[4,3 -d]pyrimidin-7-yl)-2-fluoro-5 -methyl-4-(trifluoromethyl)a niline | 660.4 |
| **213** | 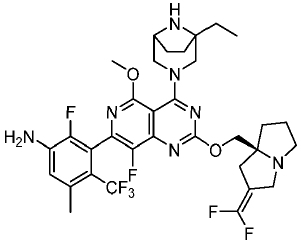 | 3-(2-(((S)-2-(difluoromethyl ene)tetrahydro-1H-pyrrolizi n-7a(5H)-yl)methoxy)-4-(1-ethyl-3,8-diazabicyclo[3.2.1 ]octan-3-yl)-8-fluoro-5-met hoxypyrido[4,3-d]pyrimidin -7-yl)-2-fluoro-5-methyl-4-( trifluoromethyl)aniline | 696.4 |
| **214** | 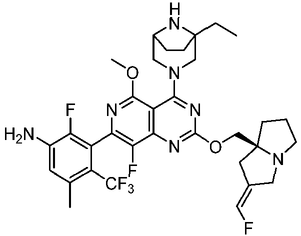 | 3-(4-(1-ethyl-3,8-diazabicycl o[3.2.1]octan-3-yl)-8-fluoro-2-(((S,Z)-2-(fluoromethylene )tetrahydro-1H-pyrrolizin-7a (5H)-yl)methoxy)-5-methoxy pyrido[4,3-d]pyrimidin-7-yl) -2-fluoro-5-methyl-4-(trifluo romethyl)aniline | 678.4 |
| **215** | 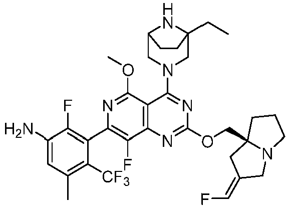 | 3-(4-(1-ethyl-3,8-diazabicycl o[3.2.1]octan-3-yl)-8-fluoro-2-(((S,E)-2-(fluoromethylene )tetrahydro-1H-pyrrolizin-7a (5H)-yl)methoxy)-5-methoxy pyrido[4,3-d]pyrimidin-7-yl) -2-fluoro-5-methyl-4-(trifluo romethyl)aniline | 678.4 |
| **216** | 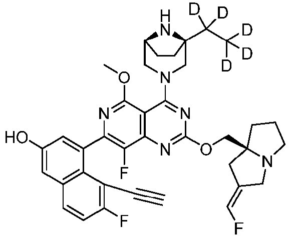 | 4-(4-((1R,5S)-1-(ethyl-*d*₅)-3 ,8-diazabicyclo[3.2.1]octan-3-yl)-8-fluoro-2-(((S,Z)-2-(f luoromethylene)tetrahydro-1H-pyrrolizin-7a(5H)-yl)me thoxy)-5-methoxypyrido[4, 3-d]pyrimidin-7-yl)-5-ethyn yl-6-fluoronaphthalen-2-ol | 676.4 |
| **217** | 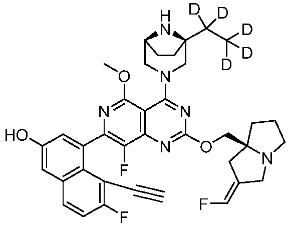 | 4-(4-((1R,5S)-1-(ethyl-*d*₅)-3 ,8-diazabicyclo[3.2.1]octan-3-yl)-8-fluoro-2-(((S,E)-2-(f luoromethylene)tetrahydro-1H-pyrrolizin-7a(5H)-yl)me thoxy)-5-methoxypyrido[4, 3-d]pyrimidin-7-yl)-5-ethyn yl-6-fluoronaphthalen-2-ol | 676.4 |
| **218** | 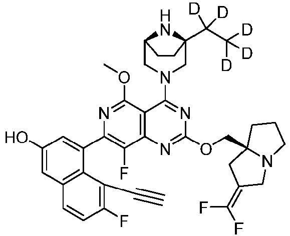 | 4-(2-(((S)-2-(difluoromethyl ene)tetrahydro-1H-pyrrolizi n-7a(5H)-yl)methoxy)-4-((1 R,5S)-1-(ethyl-*d*₅)-3,8-diaza bicyclo[3.2.1]octan-3-yl)-8-fluoro-5-methoxypyrido[4,3 -d]pyrimidin-7-yl)-5-ethyny 1-6-fluoronaphthalen-2-ol | 694.4 |
| **219** | 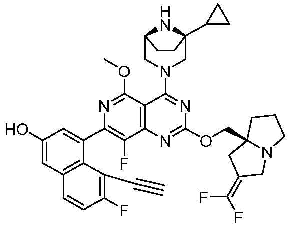 | 4-(4-((1S,5S)-1-cyclopropyl -3,8-diazabicyclo[3.2.1]octa n-3-yl)-2-(((S)-2-(difluorom ethylene)tetrahydro-1H-pyrr olizin-7a(5H)-yl)methoxy)-8-fluoro-5-methoxypyrido[4 ,3-d]pyrimidin-7-yl)-5-ethy nyl-6-fluoronaphthalen-2-ol | 701.4 |
| **358** | 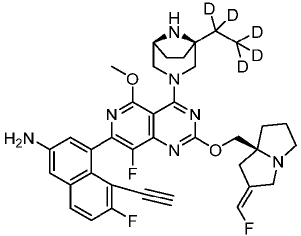 | 4-(4-((1R,5S)-1-(ethyl-*d₅*)-3 ,8-diazabicyclo[3.2.1]octan-3-yl)-8-fluoro-2-(((S,Z)-2-(f luoromethylene)tetrahydro-1H-pyrrolizin-7a(5H)-yl)me thoxy)-5-methoxypyrido[4, 3-d]pyrimidin-7-yl)-5-ethyn yl-6-fluoronaphthalen-2-am ine | 675.4 |
| **359** | 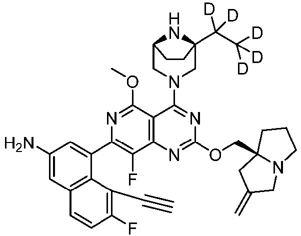 | 4-(4-((1R,5S)-1-(ethyl-*d*₅)-3 ,8-diazabicyclo[3.2.1]octan-3-yl)-8-fluoro-5-methoxy-2-(((S)-2-methylenetetrahydro -1H-pyrrolizin-7a(5H)-yl)m ethoxy)pyrido[4,3-d]pyrimi din-7-yl)-5-ethynyl-6-fluoro naphthalen-2-amine | 657.4 |

### Example 220: Preparation of 4-(6-chloro-2-((2,6-dimethylenetetrahydro-1H-pyrrolizin-7a(5H)-yl)methoxy)-8-flu oro-4-(1-((methoxy-d₃)methyl)-3,8-diazabicyclo[3.2.1]octan-3-yl)quinazolin-7-yl)-5-ethynyl-6-fluoronaphthalen-2-ol

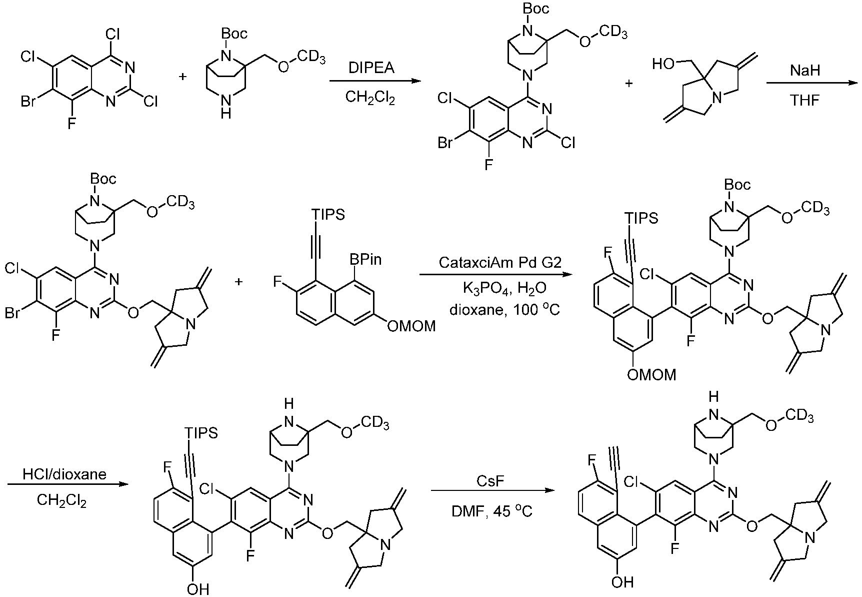

Refer to the preparation conditions of Example 1 and select the corresponding raw materials for preparation. 4-(6-chloro-2-((2,6-dimethylenetetrahydro-1H-pyrrolizi n-7a(5H)-yl)methoxy)-8-fluoro-4-(1-((methoxy-***d***₃)methyl)-3,8-diazabicyclo[3.2.1]octa n-3-yl)quinazolin-7-yl)-5-ethynyl-6-fluoronaphthalen-2-ol: ESI-MS: 687.4 [M+1]⁺.

¹H NMR (400 MHz, MeOH-*d*₄) δ 7.87 - 7.77 (m, 2H), 7.35 - 7.27 (m, 2H), 7.04 (d, *J* = 2.0 Hz, 1H), 5.02 (d, *J* = 7.7 Hz, 4H), 4.59 - 4.42 (m, 2H), 4.29 (d, *J* = 11.0 Hz, 2H), 3.78 (d, *J* = 14.8 Hz, 2H), 3.69 - 3.60 (m, 2H), 3.57 - 3.45 (m, 4H), 3.37 (s, 1H), 2.80 (d, *J* = 17.2 Hz, 2H), 2.59 (d, *J* = 16.6 Hz, 2H), 1.97 - 1.77 (m, 4H), 1.64 (d, *J* = 14.0 Hz, 1H).

**Examples 221 to 354 and 360 to 363 can be prepared by selecting the corresponding raw materials with reference to the route of Example 220.**

| **Example No.** | **Structural Formula** | **Chemical Name** | **ESI-MS: [M+1]⁺** |
|---|---|---|---|
| **221** | 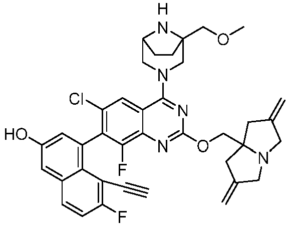 | 4-(6-chloro-2-((2,6-dimethylen etetrahydro-1H-pyrrolizin-7a(5 H)-yl)methoxy)-8-fluoro-4-(1-( methoxymethyl)-3,8-diazabicy clo[3.2.1]octan-3-yl)quinazolin -7-yl)-5-ethynyl-6-fluoronapht halen-2-ol | 684.4 |
| **222** | 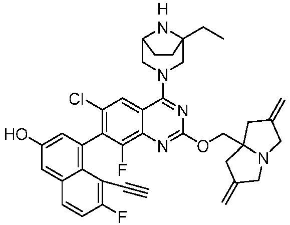 | 4-(6-chloro-2-((2,6-dimethylen etetrahydro-1H-pyrrolizin-7a(5 H)-yl)methoxy)-4-(1-ethyl-3,8-diazabicyclo[3.2.1]octan-3-yl)-8-fluoroquinazolin-7-yl)-5-ethy nyl-6-fluoronaphthalen-2-ol | 668.4 |
| **223** | 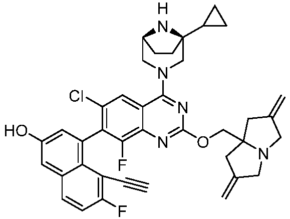 | 4-(6-chloro-4-((1S,5S)-1-cyclo propyl-3,8-diazabicyclo[3.2.1] octan-3-yl)-2-((2,6-dimethylen etetrahydro-1H-pyrrolizin-7a(5 H)-yl)methoxy)-8-fluoroquinaz olin-7-yl)-5-ethynyl-6-fluorona phthalen-2-ol | 680.4 |
| **224** | 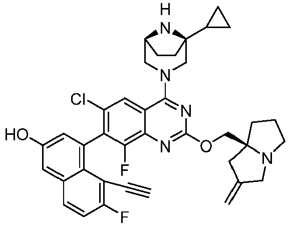 | 4-(6-chloro-4-((1S,5S)-1-cyclo propyl-3,8-diazabicyclo[3.2.1] octan-3-yl)-8-fluoro-2-(((S)-2-methylenetetrahydro-1H-pyrrol izin-7a(5H)-yl)methoxy)quinaz olin-7-yl)-5-ethynyl-6-fluorona phthalen-2-ol | 668.4 |
| **225** | 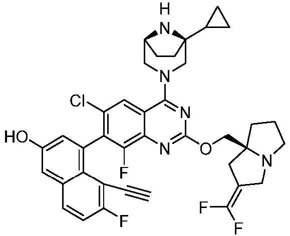 | 4-(6-chloro-2-(((S)-2-(difluoro methylene)tetrahydro-1H-pyrro lizin-7a(5H)-yl)methoxy)-4-((1 S,5S)-1-cyclopropyl-3,8-diazab icyclo[3.2.1]octan-3-yl)-8-fluor oquinazolin-7-yl)-5-ethynyl-6-f luoronaphthalen-2-ol | 704.4 |
| **226** | 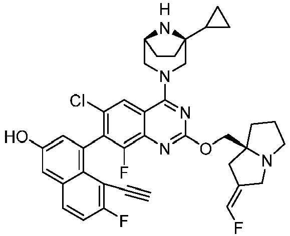 | 4-(6-chloro-4-((1S,5S)-1-cyclo propyl-3,8-diazabicyclo[3.2.1] octan-3-yl)-8-fluoro-2-(((S,Z)-2-(fluoromethylene)tetrahydro-1H-pyrrolizin-7a(5H)-yl)metho xy)quinazolin-7-yl)-5-ethynyl-6-fluoronaphthalen-2-ol | 686.4 |
| **227** | 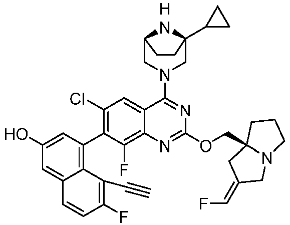 | 4-(6-chloro-4-((1S,5S)-1-cyclo propyl-3,8-diazabicyclo[3.2.1] octan-3-yl)-8-fluoro-2-(((S,E)-2-(fluoromethylene)tetrahydro-1H-pyrrolizin-7a(5H)-yl)metho xy)quinazolin-7-yl)-5-ethynyl-6-fluoronaphthalen-2-ol | 686.4 |
| **228** | 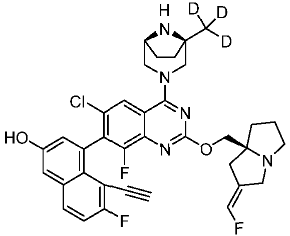 | 4-(6-chloro-8-fluoro-2-(((S,Z)-2-(fluoromethylene)tetrahydro-1H-pyrrolizin-7a(5H)-yl)metho xy)-4-((1R,5S)-1-(methyl-*d*₃)-3 ,9-diazabicyclo[3.2.1]octan-3-y l)quinazolin-7-yl)-5-ethynyl-6-fluoronaphthalen-2-ol | 663.4 |
| **229** | 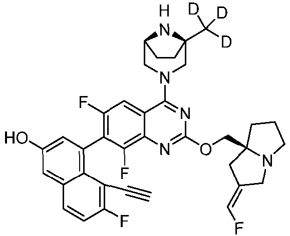 | 4-(6,8-difluoro-2-(((S,Z)-2-(flu oromethylene)tetrahydro-1H-p yrrolizin-7a(5H)-yl)methoxy)-4-((1R,5S)-1-(methyl-*d*₃)-3,8-d iazabicyclo[3.2.1]octan-3-yl)qu inazolin-7-yl)-5-ethynyl-6-fluo ronaphthalen-2-ol | 647.4 |
| **230** | 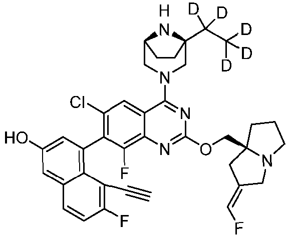 | 4-(6-chloro-4-((1R,5S)-1-(ethyl -*d*₅)-3,8-diazabicyclo[3.2.1]octa n-3-yl)-8-fluoro-2-(((S,Z)-2-(fl uoromethylene)tetrahydro-1H-p yrrolizin-7a(5H)-yl)methoxy)q uinazolin-7-yl)-5-ethynyl-6-flu oronaphthalen-2-ol | 679.4 |
| **231** | 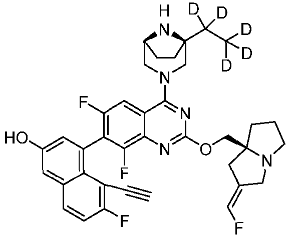 | 4-(4-((1R,5S)-1-(ethyl-ds)-3,8-diazabicyclo[3.2.1]octan-3-yl)-6,8-difluoro-2-(((S,Z)-2-(fluoro methylene)tetrahydro-1H-pyrro lizin-7a(5H)-yl)methoxy)quina zolin-7-yl)-5-ethynyl-6-fluoron aphthalen-2-ol | 663.4 |
| **232** | 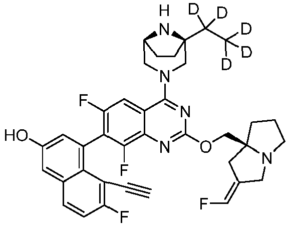 | 4-(4-((1R,5S)-1-(ethyl-*d*₅)-3,8-diazabicyclo[3.2.1]octan-3-yl)-6,8-difluoro-2-(((S,E)-2-(fluoro methylene)tetrahydro-1H-pyrro lizin-7a(5H)-yl)methoxy)quina zolin-7-yl)-5-ethynyl-6-fluoron aphthalen-2-ol | 663.4 |
| **233** | 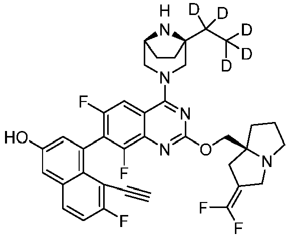 | 4-(2-(((S)-2-(difluoromethylen e)tetrahydro-1H-pyrrolizin-7a( 5H)-yl)methoxy)-4-((1R,5S)-1-(ethyl-*d*₅)-3,8-diazabicyclo[3.2. 1]octan-3-yl)-6,8-difluoroquina zolin-7-yl)-5-ethynyl-6-fluoron aphthalen-2-ol | 681.4 |
| **234** | 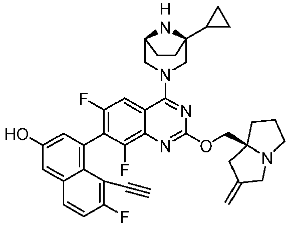 | 4-(4-((1S,5S)-1-cyclopropyl-3, 8-diazabicyclo[3.2.1]octan-3-yl )-6,8-difluoro-2-(((S)-2-methyl enetetrahydro-1H-pyrrolizin-7a (5H)-yl)methoxy)quinazolin-7-yl)-5-ethynyl-6-fluoronaphthal en-2-ol | 652.4 |
| **235** | 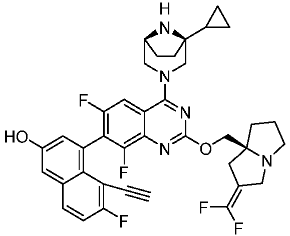 | 4-(4-((1S,5S)-1-cyclopropyl-3, 8-diazabicyclo[3.2.1]octan-3-yl )-2-(((S)-2-(difluoromethylene) tetrahydro-1H-pyrrolizin-7a(5 H)-yl)methoxy)-6,8-difluoroqu inazolin-7-yl)-5-ethynyl-6-fluo ronaphthalen-2-ol | 688.4 |
| **236** | 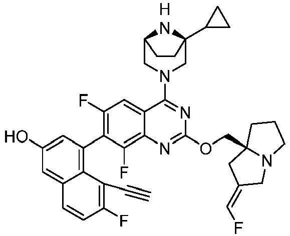 | 4-(4-((1S,5S)-1-cyclopropyl-3, 8-diazabicyclo[3.2.1]octan-3-yl )-6,8-difluoro-2-(((S,Z)-2-(fluo romethylene)tetrahydro-1H-pyr rolizin-7a(5H)-yl)methoxy)qui nazolin-7-yl)-5-ethynyl-6-fluor onaphthalen-2-ol | 670.4 |
| **237** | 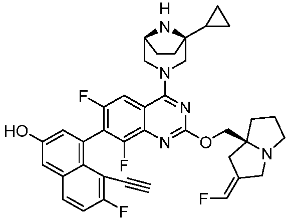 | 4-(4-((1S,5S)-1-cyclopropyl-3, 8-diazabicyclo[3.2.1]octan-3-yl )-6,8-difluoro-2-(((S,E)-2-(fluo romethylene)tetrahydro-1H-pyr rolizin-7a(5H)-yl)methoxy)qui nazolin-7-yl)-5-ethynyl-6-fluor onaphthalen-2-ol | 670.4 |
| **238** | 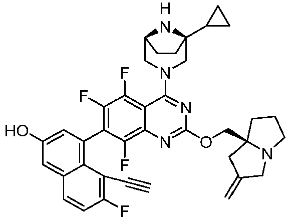 | 4-(4-((1S,5S)-1-cyclopropyl-3, 8-diazabicyclo[3.2.1]octan-3-yl )-5,6,8-trifluoro-2-(((S)-2-meth ylenetetrahydro-1H-pyrrolizin-7a(5H)-yl)methoxy)quinazolin-7-yl)-5-ethynyl-6-fluoronaphth alen-2-ol | 670.4 |
| **239** | 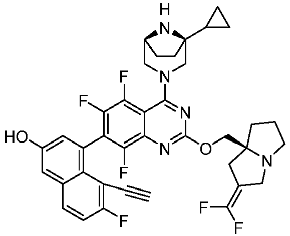 | 4-(4-((1S,5S)-1-cyclopropyl-3, 8-diazabicyclo[3.2.1]octan-3-yl )-2-(((S)-2-(difluoromethylene) tetrahydro-1H-pyrrolizin-7a(5 H)-yl)methoxy)-5,6,8-trifluoro quinazolin-7-yl)-5-ethynyl-6-fl uoronaphthalen-2-ol | 706.4 |
| **240** | 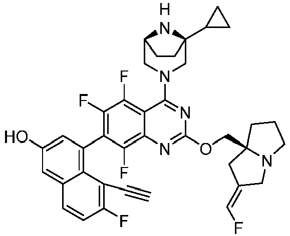 | 4-(4-((1S,5S)-1-cyclopropyl-3, 8-diazabicyclo[3.2.1]octan-3-yl )-5,6,8-trifluoro-2-(((S,Z)-2-(fl uoromethylene)tetrahydro-1H-pyrrolizin-7a(5H)-yl)methoxy) quinazolin-7-yl)-5-ethynyl-6-fl uoronaphthalen-2-ol | 688.4 |
| **241** | 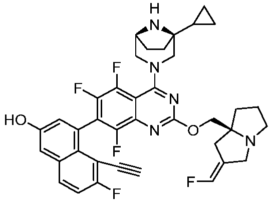 | 4-(4-((1S,5S)-1-cyclopropyl-3, 8-diazabicyclo[3.2.1]octan-3-yl )-5,6,8-trifluoro-2-(((S,E)-2-(fl uoromethylene)tetrahydro-1H-pyrrolizin-7a(5H)-yl)methoxy) quinazolin-7-yl)-5-ethynyl-6-fi uoronaphthalen-2-ol | 688.4 |
| **242** | 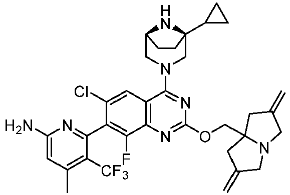 | 6-(6-chloro-2-((2,6-dimethylen etetrahydro-1H-pyrrolizin-7a(5 H)-yl)methoxy)-4-((1S,5S)-1-c yclopropyl-3,8-diazabicyclo[3. 2.1]octan-3-yl)-8-fluoroquinaz olin-7-yl)-4-methyl-5-(trifluoro methyl)pyridin-2-amine | 670.4 |
| **243** | 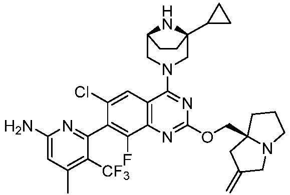 | 6-(6-chloro-4-((1S,5S)-1-cyclo propyl-3,8-diazabicyclo[3.2.1] octan-3-yl)-8-fluoro-2-(((S)-2-methylenetetrahydro-1H-pyrrol izin-7a(5H)-yl)methoxy)quinaz olin-7-yl)-4-methyl-5-(trifluoro methyl)pyridin-2-amine | 658.4 |
| **244** | 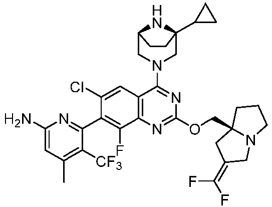 | 6-(6-chloro-2-(((S)-2-(difluoro methylene)tetrahydro-1H-pyrro lizin-7a(5H)-yl)methoxy)-4-((1 S,5S)-1-cyclopropyl-3,8-diazab icyclo[3.2.1]octan-3-yl)-8-fluor oquinazolin-7-yl)-4-methyl-5-(t rifluoromethyl)pyridin-2-amine | 694.4 |
| **245** | 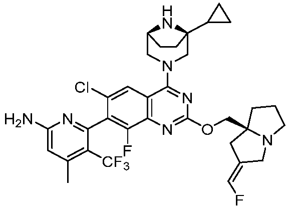 | 6-(6-chloro-4-((1S,5S)-1-cyclo propyl-3,8-diazabicyclo[3.2.1] octan-3-yl)-8-fluoro-2-(((S,Z)-2-(fluoromethylene)tetrahydro-1H-pyrrolizin-7a(5H)-yl)metho xy)quinazolin-7-yl)-4-methyl-5 -(trifluoromethyl)pyridin-2-ami ne | 676.4 |
| **246** | 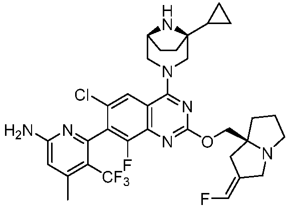 | 6-(6-chloro-4-((1S,5S)-1-cyclo propyl-3,8-diazabicyclo[3.2.1] octan-3-yl)-8-fluoro-2-(((S,E)-2-(fluoromethylene)tetrahydro-1H-pyrrolizin-7a(5H)-yl)metho xy)quinazolin-7-yl)-4-methyl-5 -(trifluoromethyl)pyridin-2-ami ne | 676.4 |
| **247** | 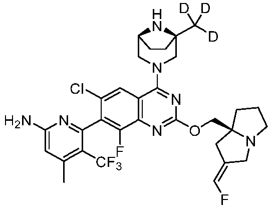 | 6-(6-chloro-8-fluoro-2-(((S,Z)-2-(fluoromethylene)tetrahydro-1H-pyrrolizin-7a(5H)-yl)metho xy)-4-((1R,5S)-1-(methyl-*d*₃)-3 ,8-diazabicyclo[3.2.1]octan-3-y l)quinazolin-7-yl)-4-methyl-5-( trifluoromethyl)pyridin-2-amin e | 653.4 |
| **248** | 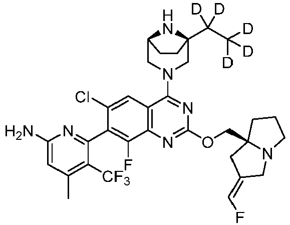 | 6-(6-chloro-4-((1R,5S)-1-(ethyl -*d*₅)-3,8-diazabicyclo[3.2.1]octa n-3-yl)-8-fluoro-2-(((S,Z)-2-(fl uoromethylene)tetrahydro-1H-p yrrolizin-7a(5H)-yl)methoxy)q uinazolin-7-yl)-4-methyl-5-(trif luoromethyl)pyridin-2-amine | 669.4 |
| **249** | 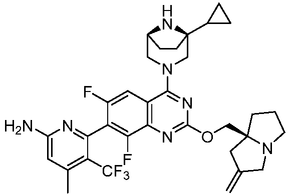 | 6-(4-((1S,5S)-1-cyclopropyl-3, 8-diazabicyclo[3.2.1]octan-3-yl )-6,8-difluoro-2-(((S)-2-methyl enetetrahydro-1H-pyrrolizin-7a (5H)-yl)methoxy)quinazolin-7-yl)-4-methyl-5-(trifluoromethyl )pyridin-2-amine | 642.4 |
| **250** | 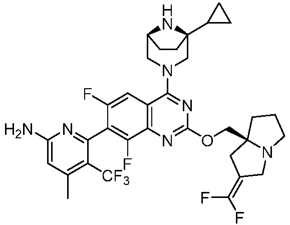 | 6-(4-((1S,5S)-1-cyclopropyl-3, 8-diazabicyclo[3.2.1]octan-3-yl )-2-(((S)-2-(difluoromethylene) tetrahydro-1H-pyrrolizin-7a(5 H)-yl)methoxy)-6,8-difluoroqu inazolin-7-yl)-4-methyl-5-(trifl uoromethyl)pyridin-2-amine | 678.4 |
| **251** | 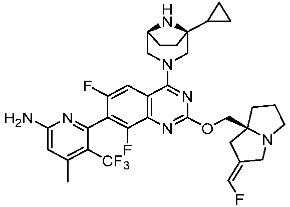 | 6-(4-((1S,5S)-1-cyclopropyl-3, 8-diazabicyclo[3.2.1]octan-3-yl )-6,8-difluoro-2-(((S,Z)-2-(fluo romethylene)tetrahydro-1H-pyr rolizin-7a(5H)-yl)methoxy)qui nazolin-7-yl)-4-methyl-5-(triflu oromethyl)pyridin-2-amine | 660.4 |
| **252** | 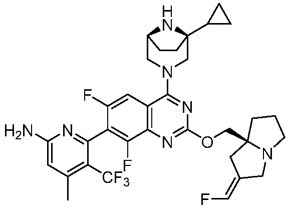 | 6-(4-((1S,5S)-1-cyclopropyl-3, 8-diazabicyclo[3.2.1]octan-3-yl )-6,8-difluoro-2-(((S,E)-2-(fluo romethylene)tetrahydro-1H-pyr rolizin-7a(5H)-yl)methoxy)qui nazolin-7-yl)-4-methyl-5-(triflu oromethyl)pyridin-2-amine | 660.4 |
| **253** | 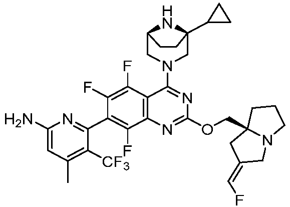 | 6-(4-((1S,5S)-1-cyclopropyl-3, 8-diazabicyclo[3.2.1]octan-3-yl )-5,6,8-trifluoro-2-(((S,Z)-2-(fl uoromethylene)tetrahydro-1H-pyrrolizin-7a(5H)-yl)methoxy) quinazolin-7-yl)-4-methyl-5-(tr ifluoromethyl)pyridin-2-amine | 678.4 |
| **254** | 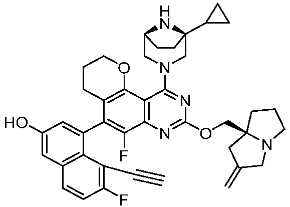 | 4-(10-((1S,5S)-1-cyclopropyl-3 ,8-diazabicyclo[3.2.1]octan-3-y l)-6-fluoro-8-(((S)-2-methylene tetrahydro-1H-pyrrolizin-7a(5 H)-yl)methoxy)-3,4-dihydro-2 H-pyrano[2,3-f]quinazolin-5-yl )-5-ethynyl-6-fluoronaphthalen -2-ol | 690.4 |
| **255** | 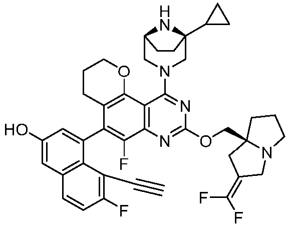 | 4-(8-(((S)-2-(difluoromethylen e)tetrahydro-1H-pyrrolizin-7a( 5H)-yl)methoxy)-10-((1S,5S)-1-cyclopropyl-3,8-diazabicyclo [3.2.1]octan-3-yl)-6-fluoro-3,4-dihydro-2H-pyrano[2,3-f]quina zolin-5-yl)-5-ethynyl-6-fluoron aphthalen-2-ol | 726.4 |
| **256** | 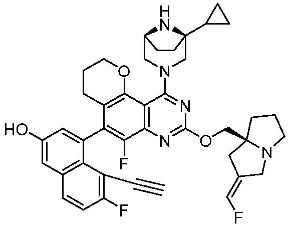 | 4-(10-((1S,5S)-1-cyclopropyl-3 ,8-diazabicyclo[3.2.1]octan-3-y 1)-6-fluoro-8-(((S,Z)-2-(fluoro methylene)tetrahydro-1H-pyrro lizin-7a(5H)-yl)methoxy)-3,4-d ihydro-2H-pyrano[2,3-f]quinaz olin-5-yl)-5-ethynyl-6-fluorona phthalen-2-ol | 708.4 |
| **257** | 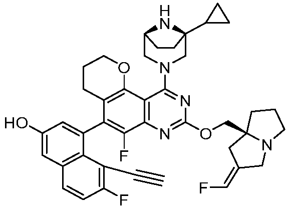 | 4-(10-((1S,5S)-1-cyclopropyl-3 ,8-diazabicyclo[3.2.1]octan-3-y l)-6-fluoro-8-(((S,E)-2-(fluoro methylene)tetrahydro-1H-pyrro lizin-7a(5H)-yl)methoxy)-3,4-d ihydro-2H-pyrano[2,3-f]quinaz olin-5-yl)-5-ethynyl-6-fluorona phthalen-2-ol | 708.4 |
| **258** | 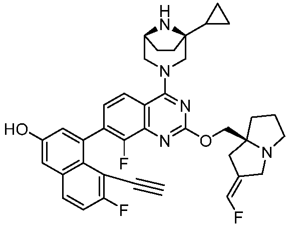 | 4-(4-((1S,5S)-1-cyclopropyl-3, 8-diazabicyclo[3.2.1]octan-3-yl )-8-fluoro-2-(((S,Z)-2-(fluorom ethylene)tetrahydro-1H-pyrroli zin-7a(5H)-yl)methoxy)quinaz olin-7-yl)-5-ethynyl-6-fluorona phthalen-2-ol | 652.4 |
| **259** | 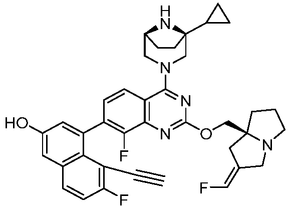 | 4-(4-((1S,5S)-1-cyclopropyl-3, 8-diazabicyclo[3.2.1]octan-3-yl )-8-fluoro-2-(((S,E)-2-(fluorom ethylene)tetrahydro-1H-pyrroli zin-7a(5H)-yl)methoxy)quinaz olin-7-yl)-5-ethynyl-6-fluorona phthalen-2-ol | 652.4 |
| **260** | 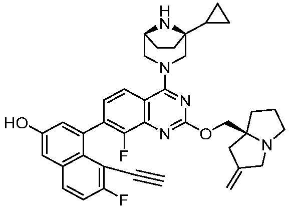 | 4-(4-((1S,5S)-1-cyclopropyl-3, 8-diazabicyclo[3.2.1]octan-3-yl )-8-fluoro-2-(((S)-2-methylenet etrahydro-1H-pyrrolizin-7a(5H )-yl)methoxy)quinazolin-7-yl)-5-ethynyl-6-fluoronaphthalen-2 -ol | 634.4 |
| **261** | 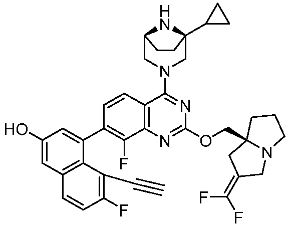 | 4-(4-((1S,5S)-1-cyclopropyl-3, 8-diazabicyclo[3.2.1]octan-3-yl )-2-(((S)-2-(difluoromethylene) tetrahydro-1H-pyrrolizin-7a(5 H)-yl)methoxy)-8-fluoroquinaz olin-7-yl)-5-ethynyl-6-fluorona phthalen-2-ol | 670.4 |
| **262** | 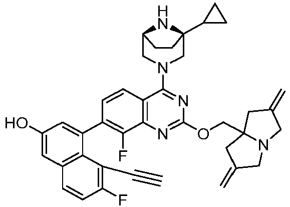 | 4-(4-((1S,5S)-1-cyclopropyl-3, 8-diazabicyclo[3.2.1]octan-3-yl )-2-((2,6-dimethylenetetrahydr o-1H-pyrrolizin-7a(5H)-yl)met hoxy)-8-fluoroquinazolin-7-yl) -5-ethynyl-6-fluoronaphthalen-2-ol | 646.4 |
| **263** | 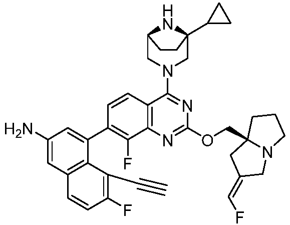 | 4-(4-((1S,5S)-1-cyclopropyl-3, 8-diazabicyclo[3.2.1]octan-3-yl )-8-fluoro-2-(((S,Z)-2-(fluorom ethylene)tetrahydro-1H-pyrroli zin-7a(5H)-yl)methoxy)quinaz olin-7-yl)-5-ethynyl-6-fluorona phthalen-2-amine | 651.4 |
| **264** | 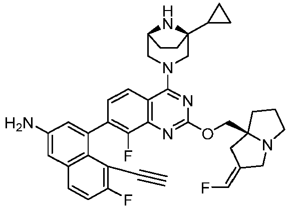 | 4-(4-((1S,5S)-1-cyclopropyl-3, 8-diazabicyclo[3.2.1]octan-3-yl )-8-fluoro-2-(((S,E)-2-(fluorom ethylene)tetrahydro-1H-pyrroli zin-7a(5H)-yl)methoxy)quinaz olin-7-yl)-5-ethynyl-6-fluorona phthalen-2-amine | 651.4 |
| **265** | 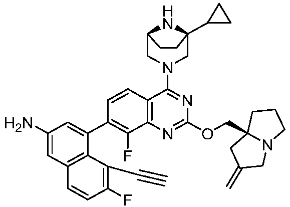 | 4-(4-((1S,5S)-1-cyclopropyl-3, 8-diazabicyclo[3.2.1]octan-3-yl )-8-fluoro-2-(((S)-2-methylenet etrahydro-1H-pyrrolizin-7a(5H )-yl)methoxy)quinazolin-7-yl)-5-ethynyl-6-fluoronaphthalen-2 -amine | 633.4 |
| **266** | 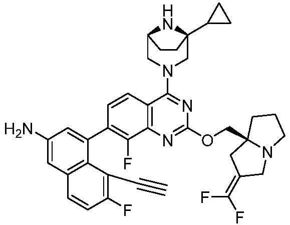 | 4-(4-((1S,5S)-1-cyclopropyl-3, 8-diazabicyclo[3.2.1]octan-3-yl )-2-(((S)-2-(difluoromethylene) tetrahydro-1H-pyrrolizin-7a(5H )-yl)methoxy)-8-fluoroquinazol in-7-yl)-5-ethynyl-6-fluoronaph thalen-2-amine | 669.4 |
| **267** | 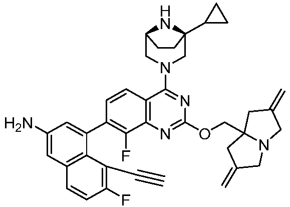 | 4-(4-((1S,5S)-1-cyclopropyl-3, 8-diazabicyclo[3.2.1]octan-3-yl )-2-((2,6-dimethylenetetrahydr o-1H-pyrrolizin-7a(5H)-yl)met hoxy)-8-fluoroquinazolin-7-yl) -5-ethynyl-6-fluoronaphthalen-2-amine | 645.4 |
| **268** | 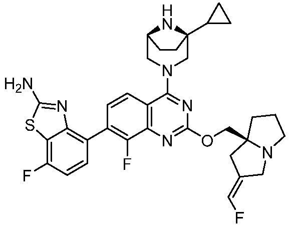 | 4-(4-((1S,5S)-1-cyclopropyl-3, 8-diazabicyclo[3.2.1]octan-3-yl )-8-fluoro-2-(((S,Z)-2-(fluorom ethylene)tetrahydro-1H-pyrroli zin-7a(5H)-yl)methoxy)quinaz olin-7-yl)-7-fluorobenzo[d]thia zol-2-amine | 634.4 |
| **269** | 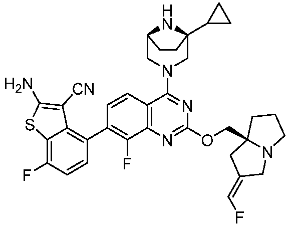 | 2-amino-4-(4-((1S,5S)-1-cyclo propyl-3,8-diazabicyclo[3.2.1] octan-3-yl)-8-fluoro-2-(((S,Z)-2-(fluoromethylene)tetrahydro-1H-pyrrolizin-7a(5H)-yl)metho xy)quinazolin-7-yl)-7-fluorobe nzo[b]thiophene-3-carbonitrile | 658.4 |
| **270** | 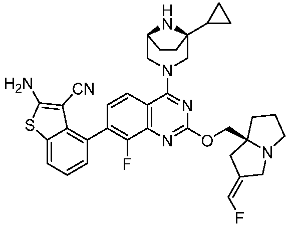 | 2-amino-4-(4-((1S,5S)-1-cyclo propyl-3,8-diazabicyclo[3.2.1] octan-3-yl)-8-fluoro-2-(((S,Z)-2-(fluoromethylene)tetrahydro-1H-pyrrolizin-7a(5H)-yl)metho xy)quinazolin-7-yl)benzo[b]thi ophene-3-carbonitrile | 640.4 |
| **271** | 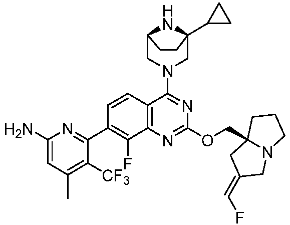 | 6-(4-((1S,5S)-1-cyclopropyl-3, 8-diazabicyclo[3.2.1]octan-3-yl )-8-fluoro-2-(((S,Z)-2-(fluorom ethylene)tetrahydro-1H-pyrroli zin-7a(5H)-yl)methoxy)quinaz olin-7-yl)-4-methyl-5-(trifluoro methyl)pyridin-2-amine | 642.4 |
| **272** | 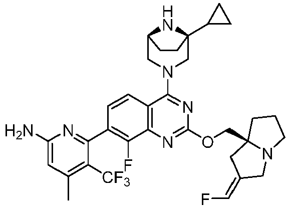 | 6-(4-((1S,5S)-1-cyclopropyl-3, 8-diazabicyclo[3.2.1]octan-3-yl )-8-fluoro-2-(((S,E)-2-(fluorom ethylene)tetrahydro-1H-pyrroli zin-7a(5H)-yl)methoxy)quinaz olin-7-yl)-4-methyl-5-(trifluoro methyl)pyridin-2-amine | 642.4 |
| **273** | 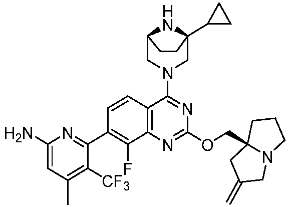 | 6-(4-((1S,5S)-1-cyclopropyl-3, 8-diazabicyclo[3.2.1]octan-3-yl )-8-fluoro-2-(((S)-2-methylenet etrahydro-1H-pyrrolizin-7a(5H )-yl)methoxy)quinazolin-7-yl)-4-methyl-5-(trifluoromethyl)py ridin-2-amine | 624.4 |
| **274** | 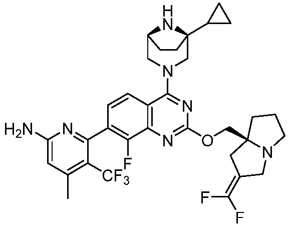 | 6-(4-((1S,5S)-1-cyclopropyl-3, 8-diazabicyclo[3.2.1]octan-3-yl )-2-(((S)-2-(difluoromethylene) tetrahydro-1H-pyrrolizin-7a(5 H)-yl)methoxy)-8-fluoroquinaz olin-7-yl)-4-methyl-5-(trifluoro methyl)pyridin-2-amine | 660.4 |
| **275** | 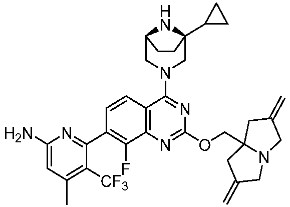 | 6-(4-((1S,5S)-1-cyclopropyl-3, 8-diazabicyclo[3.2.1]octan-3-yl )-2-((2,6-dimethylenetetrahydr o-1H-pyrrolizin-7a(5H)-yl)met hoxy)-8-fluoroquinazolin-7-yl) -4-methyl-5-(trifluoromethyl)p yridin-2-amine | 636.4 |
| **276** | 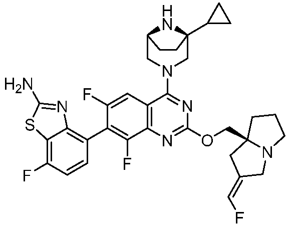 | 4-(4-((1S,5S)-1-cyclopropyl-3, 8-diazabicyclo[3.2.1]octan-3-yl )-6,8-difluoro-2-(((S,Z)-2-(fluo romethylene)tetrahydro-1H-pyr rolizin-7a(5H)-yl)methoxy)qui nazolin-7-yl)-7-fluorobenzo[d]t hiazol-2-amine | 652.4 |
| **277** | 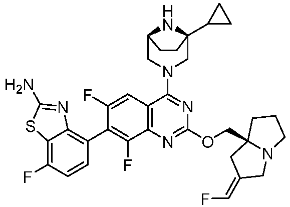 | 4-(4-((1S,5S)-1-cyclopropyl-3, 8-diazabicyclo[3.2.1]octan-3-yl )-6,8-difluoro-2-(((S,E)-2-(fluo romethylene)tetrahydro-1H-pyr rolizin-7a(5H)-yl)methoxy)qui nazolin-7-yl)-7-fluorobenzo[d]t hiazol-2-amine | 652.4 |
| **278** | 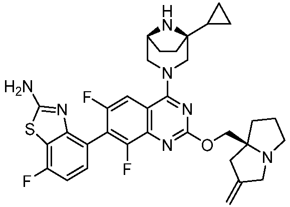 | 4-(4-((1S,5S)-1-cyclopropyl-3, 8-diazabicyclo[3.2.1]octan-3-yl )-6,8-difluoro-2-(((S)-2-methyl enetetrahydro-1H-pyrrolizin-7a (5H)-yl)methoxy)quinazolin-7-yl)-7-fluorobenzo[d]thiazol-2-a mine | 634.4 |
| **279** | 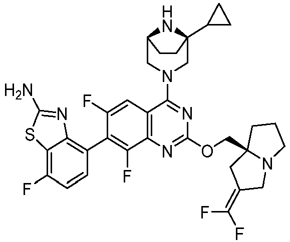 | 4-(4-((1S,5S)-1-cyclopropyl-3, 8-diazabicyclo[3.2.1]octan-3-yl )-2-(((S)-2-(difluoromethylene) tetrahydro-1H-pyrrolizin-7a(5 H)-yl)methoxy)-6,8-difluoroqu inazolin-7-yl)-7-fluorobenzo[d] thiazol-2-amine | 670.4 |
| **280** | 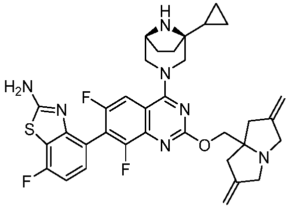 | 4-(4-((1S,5S)-1-cyclopropyl-3, 8-diazabicyclo[3.2.1]octan-3-yl )-2-((2,6-dimethylenetetrahydr o-1H-pyrrolizin-7a(5H)-yl)met hoxy)-6,8-difluoroquinazolin-7 -yl)-7-fluorobenzo[d]thiazol-2-amine | 646.4 |
| **281** | 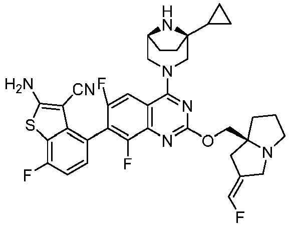 | 2-amino-4-(4-((1S,5S)-1-cyclo propyl-3,8-diazabicyclo[3.2.1] octan-3-yl)-6,8-difluoro-2-(((S, Z)-2-(fluoromethylene)tetrahyd ro-1H-pyrrolizin-7a(5H)-yl)me thoxy)quinazolin-7-yl)-7-fluor obenzo[b]thiophene-3-carbonit rile | 676.4 |
| **282** | 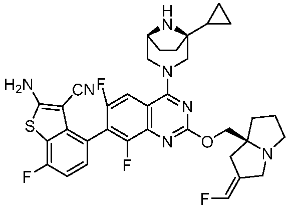 | 2-amino-4-(4-((1S,5S)-1-cyclo propyl-3,8-diazabicyclo[3.2.1] octan-3-yl)-6,8-difluoro-2-(((S, E)-2-(fluoromethylene)tetrahyd ro-1H-pyrrolizin-7a(5H)-yl)me thoxy)quinazolin-7-yl)-7-fluor obenzo[b]thiophene-3-carbonit rile | 676.4 |
| **283** | 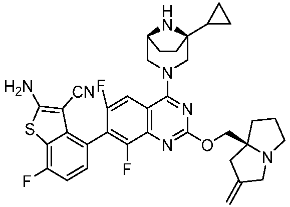 | 2-amino-4-(4-((1S,5S)-1-cyclo propyl-3,8-diazabicyclo[3.2.1] octan-3-yl)-6,8-difluoro-2-(((S) -2-methylenetetrahydro-1H-pyr rolizin-7a(5H)-yl)methoxy)qui nazolin-7-yl)-7-fluorobenzo[b]t hiophene-3-carbonitrile | 658.4 |
| **284** | 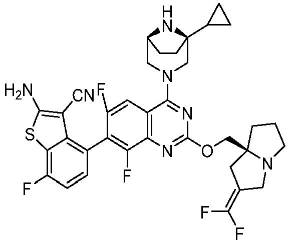 | 2-amino-4-(4-((1S,5S)-1-cyclo propyl-3,8-diazabicyclo[3.2.1] octan-3-yl)-2-(((S)-2-(difluoro methylene)tetrahydro-1H-pyrro lizin-7a(5H)-yl)methoxy)-6,8-d ifluoroquinazolin-7-yl)-7-fluor obenzo[b]thiophene-3-carbonit rile | 694.4 |
| **285** | 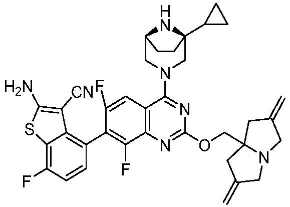 | 2-amino-4-(4-((1S,5S)-1-cyclo propyl-3,8-diazabicyclo[3.2.1] octan-3-yl)-2-((2,6-dimethylen etetrahydro-1H-pyrrolizin-7a(5 H)-yl)methoxy)-6,8-difluoroqu inazolin-7-yl)-7-fluorobenzo[b] thiophene-3-carbonitrile | 670.4 |
| **286** | 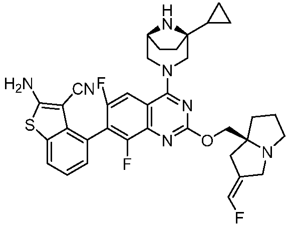 | 2-amino-4-(4-((1S,5S)-1-cyclo propyl-3,8-diazabicyclo[3.2.1] octan-3-yl)-6,8-difluoro-2-(((S, Z)-2-(fluoromethylene)tetrahyd ro-1H-pyrrolizin-7a(5H)-yl)me thoxy)quinazolin-7-yl)benzo[b] thiophene-3-carbonitrile | 658.4 |
| **287** | 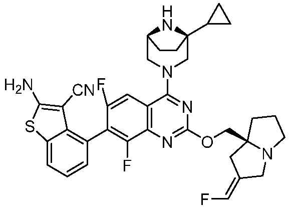 | 2-amino-4-(4-((1S,5S)-1-cyclo propyl-3,8-diazabicyclo[3.2.1] octan-3-yl)-6,8-difluoro-2-(((S, E)-2-(fluoromethylene)tetrahyd ro-1H-pyrrolizin-7a(5H)-yl)me thoxy)quinazolin-7-yl)benzo[b] thiophene-3-carbonitrile | 658.4 |
| **288** | 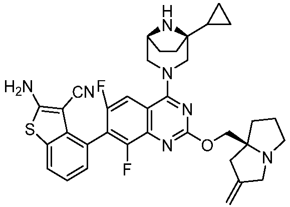 | 2-amino-4-(4-((1S,5S)-1-cyclo propyl-3,8-diazabicyclo[3.2.1] octan-3-yl)-6,8-difluoro-2-(((S) -2-methylenetetrahydro-1H-pyr rolizin-7a(5H)-yl)methoxy)qui nazolin-7-yl)benzo[b]thiophen e-3-carbonitrile | 640.4 |
| **289** | 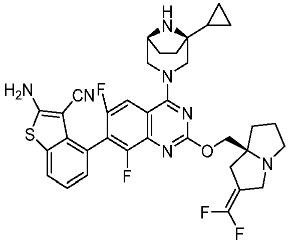 | 2-amino-4-(4-((1S,5S)-1-cyclo propyl-3,8-diazabicyclo[3.2.1] octan-3-yl)-2-(((S)-2-(difluoro methylene)tetrahydro-1H-pyrro lizin-7a(5H)-yl)methoxy)-6,8-d ifluoroquinazolin-7-yl)benzo[b ]thiophene-3-carbonitrile | 676.4 |
| **290** | 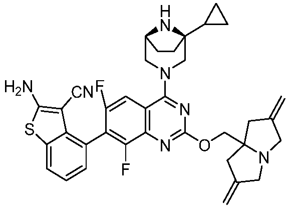 | 2-amino-4-(4-((1S,5S)-1-cyclo propyl-3,8-diazabicyclo[3.2.1] octan-3-yl)-2-((2,6-dimethylen etetrahydro-1H-pyrrolizin-7a(5 H)-yl)methoxy)-6,8-difluoroqu inazolin-7-yl)benzo[b]thiophen e-3-carbonitrile | 652.4 |
| **291** | 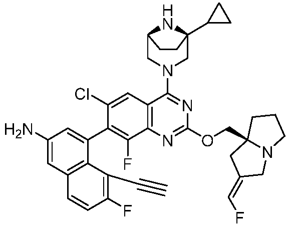 | 4-(6-chloro-4-((1S,5S)-1-cyclo propyl-3,8-diazabicyclo[3.2.1] octan-3-yl)-8-fluoro-2-(((S,Z)-2-(fluoromethylene)tetrahydro-1H-pyrrolizin-7a(5H)-yl)metho xy)quinazolin-7-yl)-5-ethynyl-6-fluoronaphthalen-2-amine | 685.4 |
| **292** | 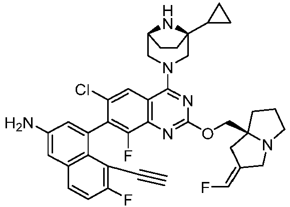 | 4-(6-chloro-4-((1S,5S)-1-cyclo propyl-3,8-diazabicyclo[3.2.1] octan-3-yl)-8-fluoro-2-(((S,E)-2-(fluoromethylene)tetrahydro-1H-pyrrolizin-7a(5H)-yl)metho xy)quinazolin-7-yl)-5-ethynyl-6-fluoronaphthalen-2-amine | 685.4 |
| **293** | 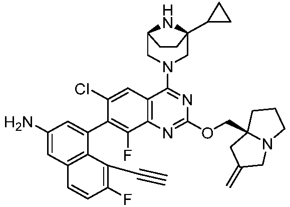 | 4-(6-chloro-4-((1S,5S)-1-cyclo propyl-3,8-diazabicyclo[3.2.1] octan-3-yl)-8-fluoro-2-(((S)-2-methylenetetrahydro-1H-pyrrol izin-7a(5H)-yl)methoxy)quinaz olin-7-yl)-5-ethynyl-6-fluorona phthalen-2-amine | 667.4 |
| **294** | 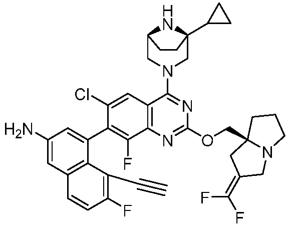 | 4-(6-chloro-4-((1S,5S)-1-cyclo propyl-3,8-diazabicyclo[3.2.1] octan-3-yl)-2-(((S)-2-(difluoro methylene)tetrahydro-1H-pyrro lizin-7a(5H)-yl)methoxy)-8-flu oroquinazolin-7-yl)-5-ethynyl-6-fluoronaphthalen-2-amine | 703.4 |
| **295** | 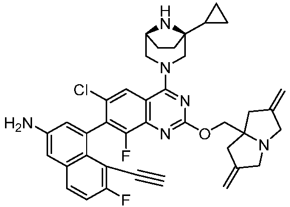 | 4-(6-chloro-4-((1S,5S)-1-cyclo propyl-3,8-diazabicyclo[3.2.1] octan-3-yl)-2-((2,6-dimethylen etetrahydro-1H-pyrrolizin-7a(5 H)-yl)methoxy)-8-fluoroquinaz olin-7-yl)-5-ethynyl-6-fluorona phthalen-2-amine | 679.4 |
| **296** | 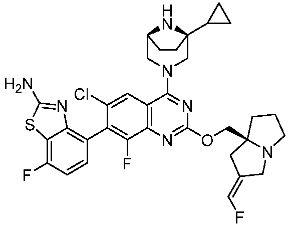 | 4-(6-chloro-4-((1S,5S)-1-cyclo propyl-3,8-diazabicyclo[3.2.1] octan-3-yl)-8-fluoro-2-(((S,Z)-2-(fluoromethylene)tetrahydro-1H-pyrrolizin-7a(5H)-yl)metho xy)quinazolin-7-yl)-7-fluorobe nzo[d]thiazol-2-amine | 668.4 |
| **297** | 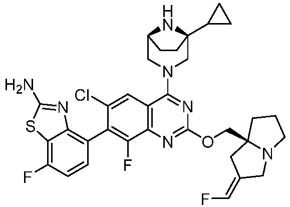 | 4-(6-chloro-4-((1S,5S)-1-cyclo propyl-3,8-diazabicyclo[3.2.1] octan-3-yl)-8-fluoro-2-(((S,E)-2-(fluoromethylene)tetrahydro-1H-pyrrolizin-7a(5H)-yl)metho xy)quinazolin-7-yl)-7-fluorobe nzo[d]thiazol-2-amine | 668.4 |
| **298** | 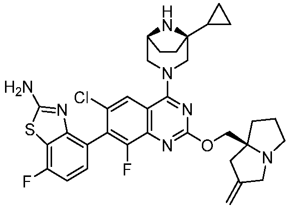 | 4-(6-chloro-4-((1S,5S)-1-cyclo propyl-3,8-diazabicyclo[3.2.1] octan-3-yl)-8-fluoro-2-(((S)-2-methylenetetrahydro-1H-pyrrol izin-7a(5H)-yl)methoxy)quinaz olin-7-yl)-7-fluorobenzo[d]thia zol-2-amine | 650.4 |
| **299** | 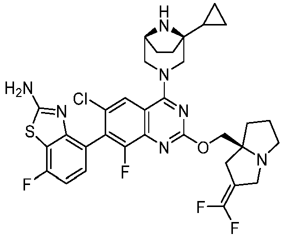 | 4-(6-chloro-4-((1S,5S)-1-cyclo propyl-3,8-diazabicyclo[3.2.1] octan-3-yl)-2-(((S)-2-(difluoro methylene)tetrahydro-1H-pyrro lizin-7a(5H)-yl)methoxy)-8-flu oroquinazolin-7-yl)-7-fluorobe nzo[d]thiazol-2-amine | 686.4 |
| **300** | 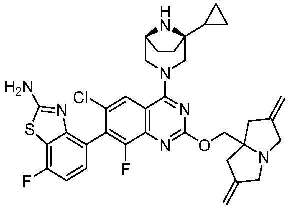 | 4-(6-chloro-4-((1S,5S)-1-cyclo propyl-3,8-diazabicyclo[3.2.1] octan-3-yl)-2-((2,6-dimethylen etetrahydro-1H-pyrrolizin-7a(5 H)-yl)methoxy)-8-fluoroquinaz olin-7-yl)-7-fluorobenzo[d]thia zol-2-amine | 662.4 |
| **301** | 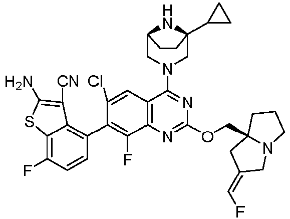 | 2-amino-4-(6-chloro-4-((1S,5S)-1-cyclopropyl-3,8-diazabicyclo[ 3.2.1]octan-3-yl)-8-fluoro-2-(((S ,Z)-2-(fluoromethylene)tetrahyd ro-1H-pyrrolizin-7a(5H)-yl)met hoxy)quinazolin-7-yl)-7-fluorob enzo[b]thiophene-3-carbonitrile | 692.4 |
| **302** | 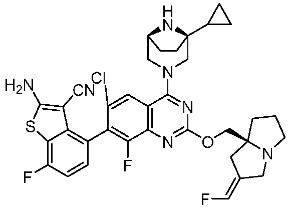 | 2-amino-4-(6-chloro-4-((1S,5S)-1-cyclopropyl-3,8-diazabicyclo[ 3.2.1]octan-3-yl)-8-fluoro-2-(((S ,E)-2-(fluoromethylene)tetrahyd ro-1H-pyrrolizin-7a(5H)-yl)met hoxy)quinazolin-7-yl)-7-fluorob enzo[b]thiophene-3-carbonitrile | 692.4 |
| **303** | 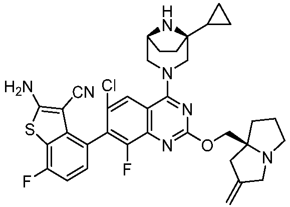 | 2-amino-4-(6-chloro-4-((1S,5S)-1-cyclopropyl-3,8-diazabicyclo[ 3.2.1]octan-3-yl)-8-fluoro-2-(((S )-2-methylenetetrahydro-1H-pyr rolizin-7a(5H)-yl)methoxy)quin azolin-7-yl)-7-fluorobenzo[b]thi ophene-3-carbonitrile | 674.4 |
| **304** | 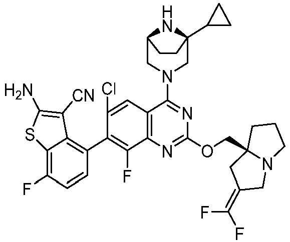 | 2-amino-4-(6-chloro-4-((1S,5S)-1-cyclopropyl-3,8-diazabicyclo[ 3.2.1]octan-3-yl)-2-(((S)-2-(difl uoromethylene)tetrahydro-1H-p yrrolizin-7a(5H)-yl)methoxy)-8-fluoroquinazolin-7-yl)-7-fluorob enzo[b]thiophene-3-carbonitrile | 710.4 |
| **305** | 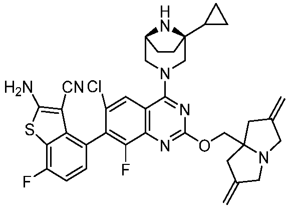 | 2-amino-4-(6-chloro-4-((1S,5S)-1-cyclopropyl-3,8-diazabicyclo[ 3.2.1]octan-3-yl)-2-((2,6-dimeth ylenetetrahydro-1H-pyrrolizin-7 a(5H)-yl)methoxy)-8-fluoroquin azolin-7-yl)-7-fluorobenzo[b]thi ophene-3-carbonitrile | 686.4 |
| **306** | 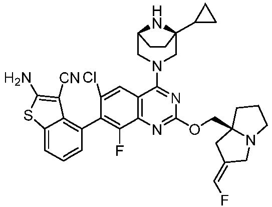 | 2-amino-4-(6-chloro-4-((1S,5S)-1-cyclopropyl-3,8-diazabicyclo[ 3.2.1]octan-3-yl)-8-fluoro-2-(((S ,Z)-2-(fluoromethylene)tetrahyd ro-1H-pyrrolizin-7a(5H)-yl)met hoxy)quinazolin-7-yl)benzo[b]th iophene-3-carbonitrile | 674.4 |
| **307** | 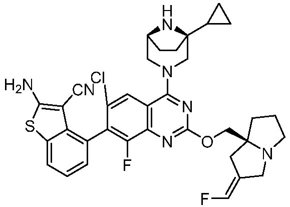 | 2-amino-4-(6-chloro-4-((1S,5S)-1-cyclopropyl-3,8-diazabicyclo[ 3.2.1]octan-3-yl)-8-fluoro-2-(((S ,E)-2-(fluoromethylene)tetrahyd ro-1H-pyrrolizin-7a(5H)-yl)met hoxy)quinazolin-7-yl)benzo[b]th iophene-3-carbonitrile | 674.4 |
| **308** | 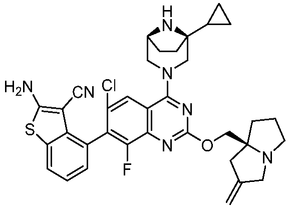 | 2-amino-4-(6-chloro-4-((1S,5S)-1-cyclopropyl-3,8-diazabicyclo[ 3.2.1]octan-3-yl)-8-fluoro-2-(((S )-2-methylenetetrahydro-1H-pyr rolizin-7a(5H)-yl)methoxy)quin azolin-7-yl)benzo[b]thiophene-3 -carbonitrile | 656.4 |
| **309** | 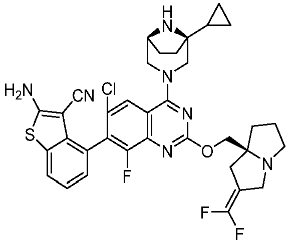 | 2-amino-4-(6-chloro-4-((1S,5S)-1-cyclopropyl-3,8-diazabicyclo[ 3.2.1]octan-3-yl)-2-(((S)-2-(difl uoromethylene)tetrahydro-1H-p yrrolizin-7a(5H)-yl)methoxy)-8-fluoroquinazolin-7-yl)benzo[b]t hiophene-3-carbonitrile | 692.4 |
| **310** | 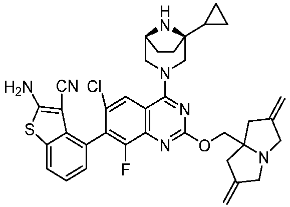 | 2-amino-4-(6-chloro-4-((1S,5S)-1-cyclopropyl-3,8-diazabicyclo[ 3.2.1]octan-3-yl)-2-((2,6-dimeth ylenetetrahydro-1H-pyrrolizin-7 a(5H)-yl)methoxy)-8-fluoroquin azolin-7-yl)benzo[b]thiophene-3 -carbonitrile | 668.4 |
| **311** | 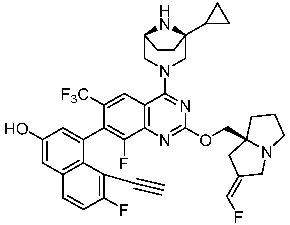 | 4-(4-((1S,5S)-1-cyclopropyl-3, 8-diazabicyclo[3.2.1]octan-3-yl )-8-fluoro-2-(((S,Z)-2-(fluorom ethylene)tetrahydro-1H-pyrroli zin-7a(5H)-yl)methoxy)-6-(trifl uoromethyl)quinazolin-7-yl)-5-ethynyl-6-fluoronaphthalen-2-o l | 720.4 |
| **312** | 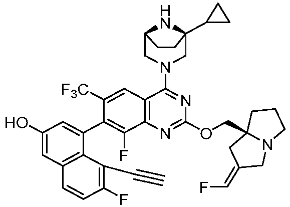 | 4-(4-((1S,5S)-1-cyclopropyl-3, 8-diazabicyclo[3.2.1]octan-3-yl )-8-fluoro-2-(((S,E)-2-(fluorom ethylene)tetrahydro-1H-pyrroli zin-7a(5H)-yl)methoxy)-6-(trifl uoromethyl)quinazolin-7-yl)-5-ethynyl-6-fluoronaphthalen-2-o l | 720.4 |
| **313** | 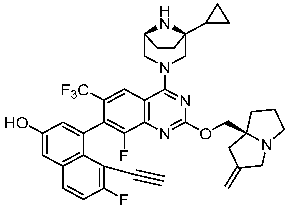 | 4-(4-((1S,5S)-1-cyclopropyl-3, 8-diazabicyclo[3.2.1]octan-3-yl )-8-fluoro-2-(((S)-2-methylenet etrahydro-1H-pyrrolizin-7a(5H )-yl)methoxy)-6-(trifluorometh yl)quinazolin-7-yl)-5-ethynyl-6 -fluoronaphthalen-2-ol | 702.4 |
| **314** | 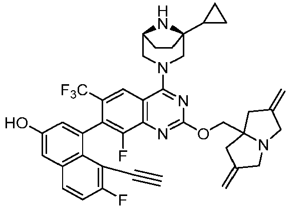 | 4-(4-((1S,5S)-1-cyclopropyl-3, 8-diazabicyclo[3.2.1]octan-3-yl )-2-((2,6-dimethylenetetrahydr o-1H-pyrrolizin-7a(5H)-yl)met hoxy)-8-fluoro-6-(trifluoromet hyl)quinazolin-7-yl)-5-ethynyl-6-fluoronaphthalen-2-ol | 714.4 |
| **315** | 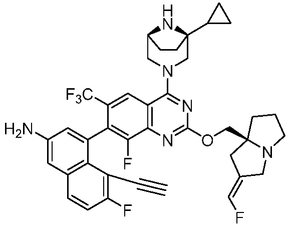 | 4-(4-((1S,5S)-1-cyclopropyl-3, 8-diazabicyclo[3.2.1]octan-3-yl )-8-fluoro-2-(((S,Z)-2-(fluorom ethylene)tetrahydro-1H-pyrroli zin-7a(5H)-yl)methoxy)-6-(trifl uoromethyl)quinazolin-7-yl)-5-ethynyl-6-fluoronaphthalen-2-a mine | 719.4 |
| **316** | 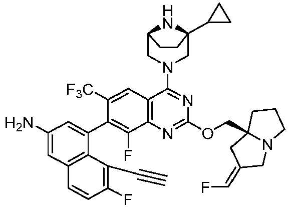 | 4-(4-((1S,5S)-1-cyclopropyl-3, 8-diazabicyclo[3.2.1]octan-3-yl )-8-fluoro-2-(((S,E)-2-(fluorom ethylene)tetrahydro-1H-pyrroli zin-7a(5H)-yl)methoxy)-6-(trifl uoromethyl)quinazolin-7-yl)-5-ethynyl-6-fluoronaphthalen-2-a mine | 719.4 |
| **317** | 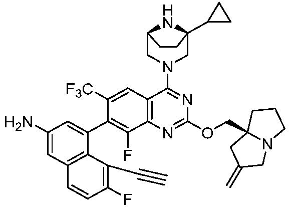 | 4-(4-((1S,5S)-1-cyclopropyl-3, 8-diazabicyclo[3.2.1]octan-3-yl )-8-fluoro-2-(((S)-2-methylenet etrahydro-1H-pyrrolizin-7a(5H )-yl)methoxy)-6-(trifluorometh yl)quinazolin-7-yl)-5-ethynyl-6 -fluoronaphthalen-2-amine | 701.4 |
| **318** | 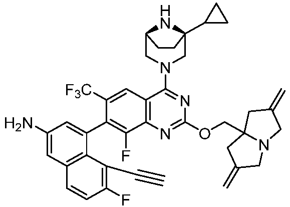 | 4-(4-((1S,5S)-1-cyclopropyl-3, 8-diazabicyclo[3.2.1]octan-3-yl )-2-((2,6-dimethylenetetrahydr o-1H-pyrrolizin-7a(5H)-yl)met hoxy)-8-fluoro-6-(trifluoromet hyl)quinazolin-7-yl)-5-ethynyl-6-fluoronaphthalen-2-amine | 713.4 |
| **319** | 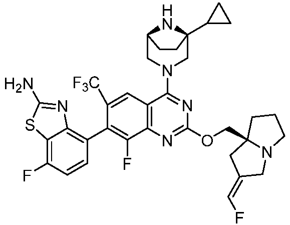 | 4-(4-((1S,5S)-1-cyclopropyl-3, 8-diazabicyclo[3.2.1]octan-3-yl )-8-fluoro-2-(((S,Z)-2-(fluorom ethylene)tetrahydro-1H-pyrroli zin-7a(5H)-yl)methoxy)-6-(trifl uoromethyl)quinazolin-7-yl)-7-fluorobenzo[d]thiazol-2-amine | 702.4 |
| **320** | 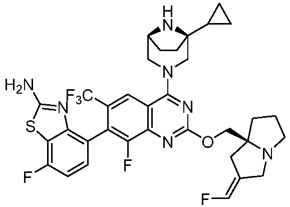 | 4-(4-((1S,5S)-1-cyclopropyl-3, 8-diazabicyclo[3.2.1]octan-3-yl )-8-fluoro-2-(((S,E)-2-(fluorom ethylene)tetrahydro-1H-pyrroli zin-7a(5H)-yl)methoxy)-6-(trifl uoromethyl)quinazolin-7-yl)-7-fluorobenzo[d]thiazol-2-amine | 702.4 |
| **321** | 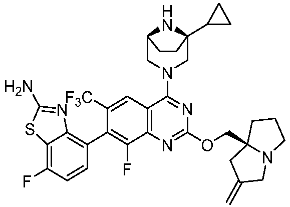 | 4-(4-((1S,5S)-1-cyclopropyl-3, 8-diazabicyclo[3.2.1]octan-3-yl )-8-fluoro-2-(((S)-2-methylenet etrahydro-1H-pyrrolizin-7a(5H )-yl)methoxy)-6-(trifluorometh yl)quinazolin-7-yl)-7-fluoroben zo[d]thiazol-2-amine | 684.4 |
| **322** | 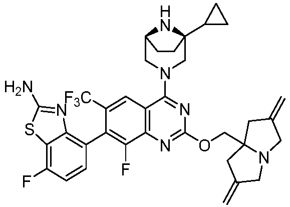 | 4-(4-((1S,5S)-1-cyclopropyl-3, 8-diazabicyclo[3.2.1]octan-3-yl )-2-((2,6-dimethylenetetrahydr o-1H-pyrrolizin-7a(5H)-yl)met hoxy)-8-fluoro-6-(trifluoromet hyl)quinazolin-7-yl)-7-fluorobe nzo[d]thiazol-2-amine | 696.4 |
| **323** | 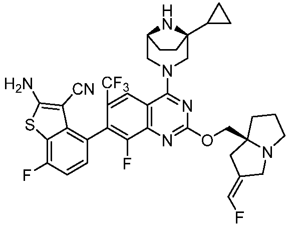 | 2-amino-4-(4-((1S,5S)-1-cyclo propyl-3,8-diazabicyclo[3.2.1] octan-3-yl)-8-fluoro-2-(((S,Z)-2-(fluoromethylene)tetrahydro-1H-pyrrolizin-7a(5H)-yl)metho xy)-6-(trifluoromethyl)quinazo lin-7-yl)-7-fluorobenzo[b]thiop hene-3-carbonitrile | 726.4 |
| **324** | 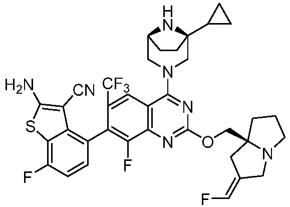 | 2-amino-4-(4-((1S,5S)-1-cyclo propyl-3,8-diazabicyclo[3.2.1] octan-3-yl)-8-fluoro-2-(((S,E)-2-(fluoromethylene)tetrahydro-1H-pyrrolizin-7a(5H)-yl)metho xy)-6-(trifluoromethyl)quinazo lin-7-yl)-7-fluorobenzo[b]thiop hene-3-carbonitrile | 726.4 |
| **325** | 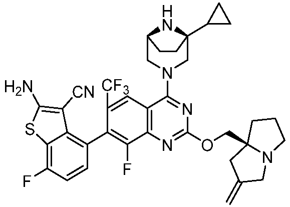 | 2-amino-4-(4-((1S,5S)-1-cyclo propyl-3,8-diazabicyclo[3.2.1] octan-3-yl)-8-fluoro-2-(((S)-2-methylenetetrahydro-1H-pyrrol izin-7a(5H)-yl)methoxy)-6-(trif luoromethyl)quinazolin-7-yl)-7 -fluorobenzo[b]thiophene-3-car bonitrile | 708.4 |
| **326** | 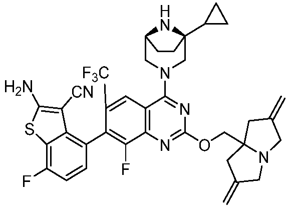 | 2-amino-4-(4-((1S,5S)-1-cyclo propyl-3,8-diazabicyclo[3.2.1] octan-3-yl)-2-((2,6-dimethylen etetrahydro-1H-pyrrolizin-7a(5 H)-yl)methoxy)-8-fluoro-6-(trif luoromethyl)quinazolin-7-yl)-7 -fluorobenzo[b]thiophene-3-car bonitrile | 720.4 |
| **327** | 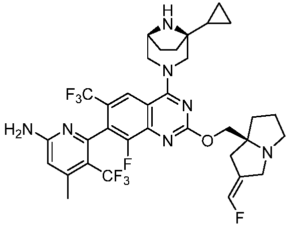 | 6-(4-((1S,5S)-1-cyclopropyl-3, 8-diazabicyclo[3.2.1]octan-3-yl )-8-fluoro-2-(((S,Z)-2-(fluorom ethylene)tetrahydro-1H-pyrroli zin-7a(5H)-yl)methoxy)-6-(trifl uoromethyl)quinazolin-7-yl)-4-methyl-5-(trifluoromethyl)pyri din-2-amine | 710.4 |
| **328** | 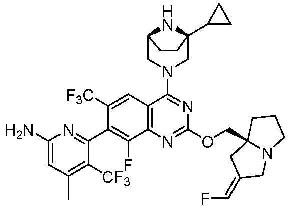 | 6-(4-((1S,5S)-1-cyclopropyl-3, 8-diazabicyclo[3.2.1]octan-3-yl )-8-fluoro-2-(((S,E)-2-(fluorom ethylene)tetrahydro-1H-pyrroli zin-7a(5H)-yl)methoxy)-6-(trifl uoromethyl)quinazolin-7-yl)-4-methyl-5-(trifluoromethyl)pyri din-2-amine | 710.4 |
| **329** | 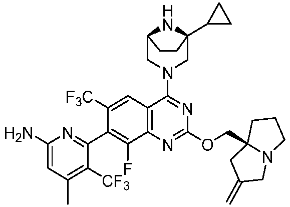 | 6-(4-((1S,5S)-1-cyclopropyl-3, 8-diazabicyclo[3.2.1]octan-3-yl )-8-fluoro-2-(((S)-2-methylenet etrahydro-1H-pyrrolizin-7a(5H )-yl)methoxy)-6-(trifluorometh yl)quinazolin-7-yl)-4-methyl-5 -(trifluoromethyl)pyridin-2-ami ne | 692.4 |
| **330** | 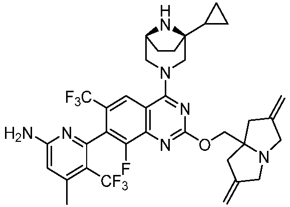 | 6-(4-((1S,5S)-1-cyclopropyl-3, 8-diazabicyclo[3.2.1]octan-3-yl )-2-((2,6-dimethylenetetrahydr o-1H-pyrrolizin-7a(5H)-yl)met hoxy)-8-fluoro-6-(trifluoromet hyl)quinazolin-7-yl)-4-methyl-5-(trifluoromethyl)pyridin-2-a mine | 704.4 |
| **331** | 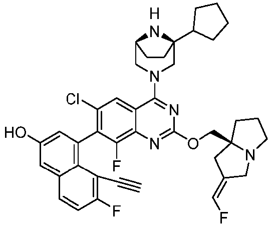 | 4-(6-chloro-4-((1S,5S)-1-cyclo pentyl-3,8-diazabicyclo[3.2.1]o ctan-3-yl)-8-fluoro-2-(((S,Z)-2-(fluoromethylene)tetrahydro-1 H-pyrrolizin-7a(5H)-yl)methox y)quinazolin-7-yl)-5-ethynyl-6-fluoronaphthalen-2-ol | 714.4 |
| **332** | 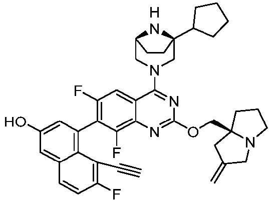 | 4-(4-((1S,5S)-1-cyclopentyl-3, 8-diazabicyclo[3.2.1]octan-3-yl )-6,8-difluoro-2-(((S)-2-methyl enetetrahydro-1H-pyrrolizin-7a (5H)-yl)methoxy)quinazolin-7-yl)-5-ethynyl-6-fluoronaphthal en-2-ol | 680.4 |
| **333** | 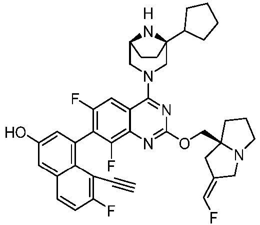 | 4-(4-((1S,5S)-1-cyclopentyl-3, 8-diazabicyclo[3.2.1]octan-3-yl )-6,8-difluoro-2-(((S,Z)-2-(fluo romethylene)tetrahydro-1H-pyr rolizin-7a(5H)-yl)methoxy)qui nazolin-7-yl)-5-ethynyl-6-fluor onaphthalen-2-ol | 698.4 |
| **334** | 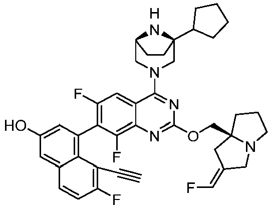 | 4-(4-((1S,5S)-1-cyclopentyl-3, 8-diazabicyclo[3.2.1]octan-3-yl )-6,8-difluoro-2-(((S,Z)-2-(fluo romethylene)tetrahydro-1H-pyr rolizin-7a(5H)-yl)methoxy)qui nazolin-7-yl)-5-ethynyl-6-fluor onaphthalen-2-ol | 698.4 |
| **335** | 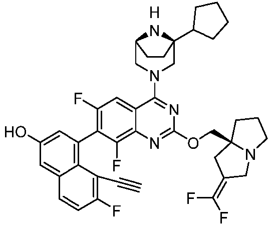 | 4-(4-((1S,5S)-1-cyclopentyl-3, 8-diazabicyclo[3.2.1]octan-3-yl )-2-(((S)-2-(difluoromethylene) tetrahydro-1H-pyrrolizin-7a(5 H)-yl)methoxy)-6,8-difluoroqu inazolin-7-yl)-5-ethynyl-6-fluo ronaphthalen-2-ol | 716.4 |
| **336** | 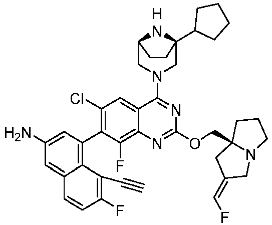 | 4-(6-chloro-4-((1S,5S)-1-cyclo pentyl-3,8-diazabicyclo[3.2.1]o ctan-3-yl)-8-fluoro-2-(((S,Z)-2-(fluoromethylene)tetrahydro-1 H-pyrrolizin-7a(5H)-yl)methox y)quinazolin-7-yl)-5-ethynyl-6-fluoronaphthalen-2-amine | 713.4 |
| **337** | 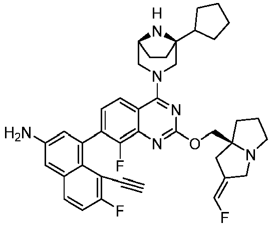 | 4-(4-((1S,5S)-1-cyclopentyl-3,8 -diazabicyclo[3.2.1]octan-3-yl)-8-fluoro-2-(((S,Z)-2-(fluoromet hylene)tetrahydro-1H-pyrrolizi n-7a(5H)-yl)methoxy)quinazoli n-7-yl)-5-ethynyl-6-fluoronapht halen-2-amine | 679.4 |
| **338** | 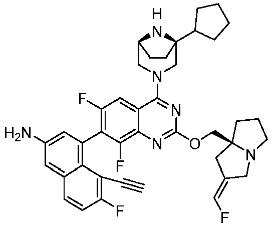 | 4-(4-((1S,5S)-1-cyclopentyl-3, 8-diazabicyclo[3.2.1]octan-3-yl )-6,8-difluoro-2-(((S,Z)-2-(fluo romethylene)tetrahydro-1H-pyr rolizin-7a(5H)-yl)methoxy)qui nazolin-7-yl)-5-ethynyl-6-fluor onaphthalen-2-amine | 697.4 |
| **339** | 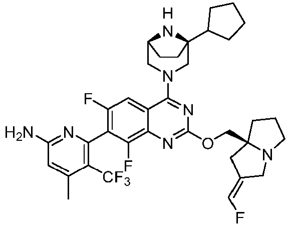 | 6-(4-((1S,5S)-1-cyclopentyl-3, 8-diazabicyclo[3.2.1]octan-3-yl )-6,8-difluoro-2-(((S,Z)-2-(fluo romethylene)tetrahydro-1H-pyr rolizin-7a(5H)-yl)methoxy)qui nazolin-7-yl)-4-methyl-5-(triflu oromethyl)pyridin-2-amine | 688.4 |
| **340** | 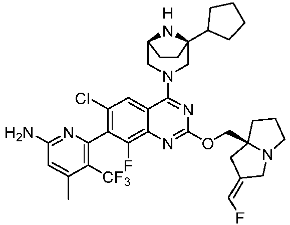 | 6-(6-chloro-4-((1S,5S)-1-cyclo pentyl-3,8-diazabicyclo[3.2.1]o ctan-3-yl)-8-fluoro-2-(((S,Z)-2-(fluoromethylene)tetrahydro-1 H-pyrrolizin-7a(5H)-yl)methox y)quinazolin-7-yl)-4-methyl-5-(trifluoromethyl)pyridin-2-ami ne | 704.4 |
| **341** | 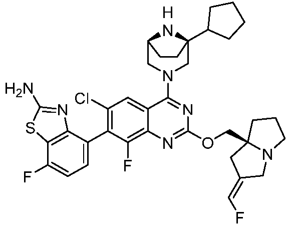 | 4-(6-chloro-4-((1S,5S)-1-cyclo pentyl-3,8-diazabicyclo[3.2.1]o ctan-3-yl)-8-fluoro-2-(((S,Z)-2-(fluoromethylene)tetrahydro-1 H-pyrrolizin-7a(5H)-yl)methox y)quinazolin-7-yl)-7-fluoroben zo[d]thiazol-2-amine | 696.4 |
| **342** | 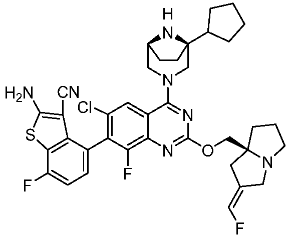 | 2-amino-4-(6-chloro-4-((1S,5S) -1-cyclopentyl-3,8-diazabicycl o[3.2.1]octan-3-yl)-8-fluoro-2-( ((S,Z)-2-(fluoromethylene)tetra hydro-1H-pyrrolizin-7a(5H)-yl )methoxy)quinazolin-7-yl)-7-fl uorobenzo[b]thiophene-3-carbo nitrile | 720.4 |
| **343** | 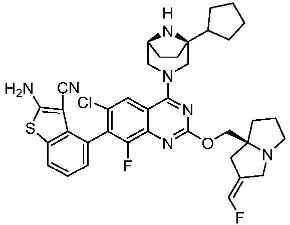 | 2-amino-4-(6-chloro-4-((1S,5S) -1-cyclopentyl-3,8-diazabicycl o[3.2.1]octan-3-yl)-8-fluoro-2-( ((S,Z)-2-(fluoromethylene)tetra hydro-1H-pyrrolizin-7a(5H)-yl )methoxy)quinazolin-7-yl)benz o[b]thiophene-3-carbonitrile | 702.4 |
| **344** | 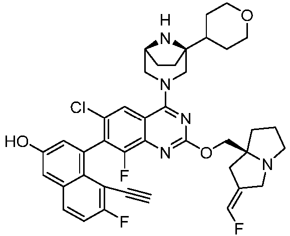 | 4-(6-chloro-8-fluoro-2-(((S,Z)-2-(fluoromethylene)tetrahydro-1H-pyrrolizin-7a(5H)-yl)metho xy)-4-((1S,5S)-1-(tetrahydro-2 H-pyran-4-yl)-3,8-diazabicyclo [3.2.1]octan-3-yl)quinazolin-7-yl)-5-ethynyl-6-fluoronaphthal en-2-ol | 730.4 |
| **345** | 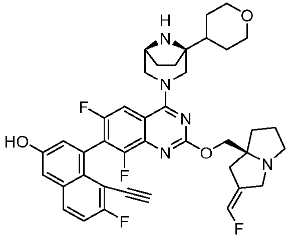 | 4-(6,8-difluoro-2-(((S,Z)-2-(flu oromethylene)tetrahydro-1H-p yrrolizin-7a(5H)-yl)methoxy)-4-((1S,5S)-1-(tetrahydro-2H-p yran-4-yl)-3,8-diazabicyclo[3.2 .1]octan-3-yl)quinazolin-7-yl)-5-ethynyl-6-fluoronaphthalen-2 -ol | 714.4 |
| **346** | 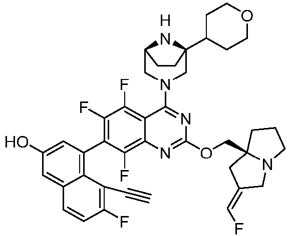 | 5-ethynyl-6-fluoro-4-(5,6,8-trif luoro-2-(((S,Z)-2-(fluoromethy lene)tetrahydro-1H-pyrrolizin-7a(5H)-yl)methoxy)-4-((1S,5S) -1-(tetrahydro-2H-pyran-4-yl)-3,8-diazabicyclo[3.2.1]octan-3-yl)quinazolin-7-yl)naphthalen-2-ol | 732.4 |
| **347** | 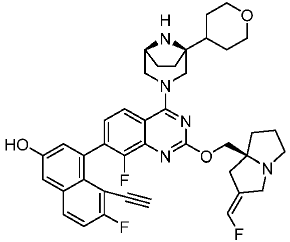 | 5-ethynyl-6-fluoro-4-(8-fluoro-2-(((S,Z)-2-(fluoromethylene)t etrahydro-1H-pyrrolizin-7a(5H )-yl)methoxy)-4-((1S,5S)-1-(tet rahydro-2H-pyran-4-yl)-3,8-di azabicyclo[3.2.1]octan-3-yl)qui nazolin-7-yl)naphthalen-2-ol | 696.4 |
| **348** | 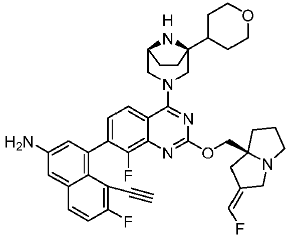 | 5-ethynyl-6-fluoro-4-(8-fluoro-2-(((S,Z)-2-(fluoromethylene)t etrahydro-1H-pyrrolizin-7a(5H )-yl)methoxy)-4-((1S,5S)-1-(tet rahydro-2H-pyran-4-yl)-3,8-di azabicyclo[3.2.1]octan-3-yl)qui nazolin-7-yl)naphthalen-2-ami ne | 695.4 |
| **349** | 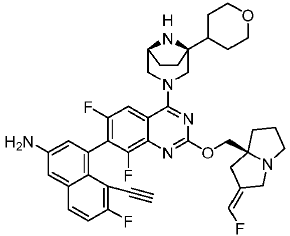 | 4-(6,8-difluoro-2-(((S,Z)-2-(flu oromethylene)tetrahydro-1H-p yrrolizin-7a(5H)-yl)methoxy)-4-((1S,5S)-1-(tetrahydro-2H-p yran-4-yl)-3,8-diazabicyclo[3.2 .1]octan-3-yl)quinazolin-7-yl)-5-ethynyl-6-fluoronaphthalen-2 -amine | 713.4 |
| **350** | 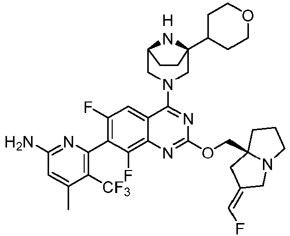 | 6-(6,8-difluoro-2-(((S,2)-2-(flu oromethylene)tetrahydro-1H-p yrrolizin-7a(5H)-yl)methoxy)-4-((1S,5S)-1-(tetrahydro-2H-p yran-4-yl)-3,8-diazabicyclo[3.2 .1]octan-3-yl)quinazolin-7-yl)-4-methyl-5-(trifluoromethyl)py ridin-2-amine | 704.4 |
| **351** | 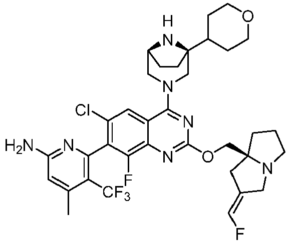 | 6-(6-chloro-8-fluoro-2-(((S,Z)-2-(fluoromethylene)tetrahydro-1H-pyrrolizin-7a(5H)-yl)metho xy)-4-((1S,5S)-1-(tetrahydro-2 H-pyran-4-yl)-3,8-diazabicyclo [3.2.1]octan-3-yl)quinazolin-7-yl)-4-methyl-5-(trifluoromethyl )pyridin-2-amine | 720.4 |
| **352** | 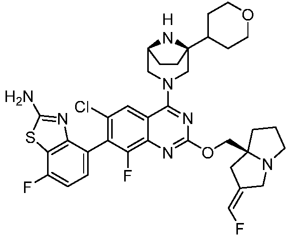 | 4-(6-chloro-8-fluoro-2-(((S,Z)-2-(fluoromethylene)tetrahydro-1H-pyrrolizin-7a(5H)-yl)metho xy)-4-((1S,5S)-1-(tetrahydro-2 H-pyran-4-yl)-3,8-diazabicyclo [3.2.1]octan-3-yl)quinazolin-7-yl)-7-fluorobenzo[d]thiazol-2-a mine | 712.4 |
| **353** | 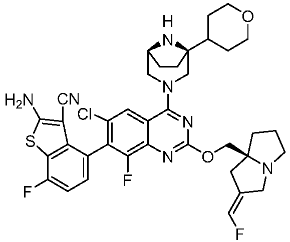 | 2-amino-4-(6-chloro-8-fluoro-2 -(((S,Z)-2-(fluoromethylene)tet rahydro-1H-pyrrolizin-7a(SH)-yl)methoxy)-4-((1S,5S)-1-(tetr ahydro-2H-pyran-4-yl)-3,8-dia zabicyclo[3.2.1]octan-3-yl)qui nazolin-7-yl)-7-fluorobenzo[b]t hiophene-3-carbonitrile | 736.4 |
| **354** | 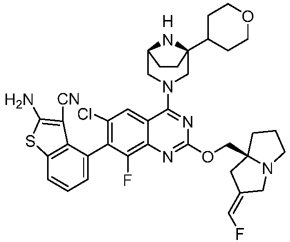 | 2-amino-4-(6-chloro-8-fluoro-2 -(((S,Z)-2-(fluoromethylene)tet rahydro-1H-pyrrolizin-7a(SH)-yl)methoxy)-4-((1S,5S)-1-(tetr ahydro-2H-pyran-4-yl)-3,8-dia zabicyclo[3.2.1]octan-3-yl)qui nazolin-7-yl)benzo[b]thiophen e-3-carbonitrile | 718.4 |
| **360** | 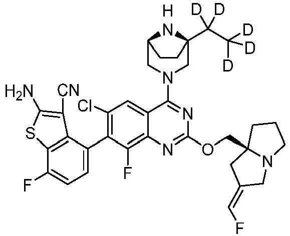 | 2-amino-4-(6-chloro-4-((1R,5S )-1-(ethyl-*d₅*)-3,8-diazabicyclo[ 3.2.1]octan-3-yl)-8-fluoro-2-((( S,Z)-2-(fluoromethylene)tetrah ydro-1H-pyrrolizin-7a(5H)-yl) methoxy)quinazolin-7-yl)-7-flu orobenzo[b]thiophene-3-carbo nitrile | 685.4 |
| **361 (SFC, Peak 2)** | 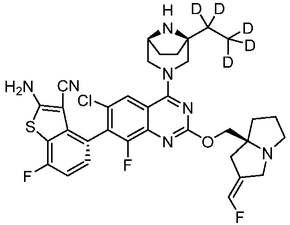 | (Ra)-2-amino-4-(6-chloro-4-((1 R,5S)-1-(ethyl-*d₅*)-3,8-diazabic yclo[3.2.1]octan-3-yl)-8-fluoro -2-(((S,Z)-2-(fluoromethylene)t etrahydro-1H-pyrrolizin-7a(5H )-yl)methoxy)quinazolin-7-yl)-7-fluorobenzo[b]thiophene-3-c arbonitrile | 685.4 |
| **362** | 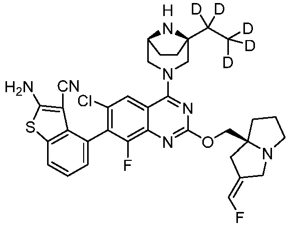 | 2-amino-4-(6-chloro-4-((1R,5S )-1-(ethyl-*d₅*)-3,8-diazabicyclo[ 3.2.1]octan-3-yl)-8-fluoro-2-((( S,Z)-2-(fluoromethylene)tetrah ydro-1H-pyrrolizin-7a(5H)-yl) methoxy)quinazolin-7-yl)benz o[b]thiophene-3-carbonitrile | 667.4 |
| **363** | 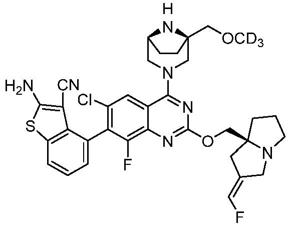 | 2-amino-4-(6-chloro-8-fluoro-2 -(((S,Z)-2-(fluoromethylene)tet rahydro-1H-pyrrolizin-7a(5H)-yl)methoxy)-4-((1R,5S)-1-((me thoxy-*d₃*)methyl)-3,8-diazabicy clo[3.2.1]octan-3-yl)quinazolin -7-yl)benzo[b]thiophene-3-carb onitrile | 681.4 |

| | | | |
|---|---|---|---|
| **Notes: Embodiment 361 can be obtained by separation by SFC ((chiral column: DAICEL CHIRALPAK IG (250 mm*30 mm, 10 µm) mobile phase: CO₂-i-PrOH (0.1% NH₃•H₂O).** | | | |

**HNMR data for the compounds prepared in the above examples are as follows:**

| **Example No.** | **¹H NMR** |
|---|---|
| **5** | ¹H NMR (400 MHz, MeOH-*d*₄) δ 9.01 (s, 1H), 7.86 (dd, *J* = 9.1, 5.7 Hz, 1H), 7.37 - 7.28 (m, 2H), 7.21 (dd, *J* = 4.4, 2.6 Hz, 1H), 5.03 (d, *J* = 8.1 Hz, 4H), 4.77 - 4.68 (m, 1H), 4.54 (dd, *J* = 28.8, 11.5 Hz, 1H), 4.35 (d, *J* = 2.6 Hz, 2H), 3.82 - 3.66 (m, 4H), 3.42 - 3.34 (m, 4H), 2.79 (d, *J* = 16.4 Hz, 2H), 2.60 (d, *J* = 15.4 Hz, 2H), 2.04 - 1.89 (m, 1H), 1.89 - 1.64 (m, 4H), 1.61 - 1.51 (m, 1H), 1.06 (td, *J* = 7.5, 2.6 Hz, 3H). |
| **6** | ¹H NMR (400 MHz, MeOH-d₄) δ 9.02 (d, *J* = 2.4 Hz, 1H), 7.86 (dd, *J* = 9.1, 5.8 Hz, 1H), 7.38 - 7.28 (m, 2H), 7.21 (t, *J* = 2.2 Hz, 1H), 5.04 (d, *J* = 6.8 Hz, 4H), 4.60 (dd, *J* = 12.2, 6.9 Hz, 2H), 4.36 (s, 2H), 3.86 - 3.70 (m, 4H), 3.51 - 3.36 (m, 4H), 2.81 (d, *J* = 15.8 Hz, 2H), 2.61 (d, *J* = 16.9 Hz, 2H), 2.09 - 1.88 (m, 2H), 1.86 - 1.76 (m, 1H), 1.58 (t, *J* = 12.4 Hz, 1H). |
| **8** | ¹H NMR (400 MHz, MeOH-*d*₄) δ 9.00 (d, *J* = 4.1 Hz, 1H), 7.86 (dd, *J* = 9.1, 5.7 Hz, 1H), 7.37 - 7.28 (m, 2H), 7.21 (s, 1H), 5.02 (d, *J* = 7.6 Hz, 4H), 4.70 - 4.55 (m, 2H), 4.33 (d, *J* = 3.3 Hz, 2H), 3.81 - 3.64 (m, 4H), 3.59 (dd, *J* = 12.5, 5.5 Hz, 1H), 3.52 (d, *J* = 2.6 Hz, 2H), 3.41 (d, *J* = 3.3 Hz, 3H), 3.37 - 3.35 (m, 3H), 2.79 (d, *J* = 16.5 Hz, 2H), 2.59 (d, *J* = 16.4 Hz, 2H), 1.94 (dq, *J* = 13.6, 7.4 Hz, 1H), 1.77 (td, *J* = 12.7, 4.8 Hz, 2H), 1.68 - 1.57 (m, 1H). |
| **12** | ¹H NMR (400 MHz, MeOH-*d*₄) δ 9.02 (s, 1H), 7.86 (dd, *J* = 9.2, 5.7 Hz, 1H), 7.40 - 7.27 (m, 2H), 7.21 (t, *J* = 3.3 Hz, 1H), 5.02 (d, *J* = 7.9 Hz, 4H), 4.75 - 4.47 (m, 3H), 4.37 - 4.30 (m, 2H), 3.85 - 3.61 (m, 4H), 3.53 (d, *J* = 12.5 Hz, 1H), 3.40 - 3.34 (m, 2H), 2.79 (d, *J* = 16.5 Hz, 2H), 2.59 (d, *J* = 16.5 Hz, 2H), 1.95 - 1.81 (m, 1H), 1.77 - 1.55 (m, 2H), 1.37 - 1.27 (m, 1H), 1.18 - 1.08 (m, 1H), 0.64 - 0.30 (m, 4H). |
| **20** | ¹H NMR (400 MHz, DMSO-*d*₆) δ 10.16 (s, 1H), 9.04 (s, 1H), 7.98 (dd, *J* = 9.2, 5.9 Hz, 1H), 7.47 (t, *J* = 9.0 Hz, 1H), 7.39 (d, *J* = 2.6 Hz, 1H), 7.18 (t, *J* = 2.9 Hz, 1H), 4.90 (s, 2H), 4.55 - 4.26 (m, 2H), 4.09 - 3.90 (m, 3H), 3.66 - 3.52 (m, 3H), 3.32 - 3.27 (m, 1H), 3.23 - 3.16 (m, 1H), 3.03 - 2.96 (m, 1H), 2.62 - 2.56 (m, 2H), 2.39 - 2.33 (m, 1H), 2.01 - 1.93 (m, 1H), 1.88 - 1.55 (m, 8H), 1.41 - 1.30 (m, 1H), 0.95 (q, *J* = 7.2 Hz, 3H). |
| **22** | ¹H NMR (400 MHz, DMSO-d₆) δ 10.18 (s, 1H), 9.03 (s, 1H), 7.98 (dd, *J* = 9.2, 5.9 Hz, 1H), 7.47 (t, *J* = 9.0 Hz, 1H), 7.39 (d, *J* = 2.6 Hz, 1H), 7.21 - 7.15 (m, 1H), 4.91 (s, 2H), 4.57 - 4.30 (m, 2H), 4.11 - 3.97 (m, 2H), 3.97 - 3.87 (m, 1H), 3.65 - 3.52 (m, 3H), 3.50 - 3.40 (m, 3H), 3.33 - 3.32 (m, 2H), 3.24 - 3.16 (m, 1H), 3.03 - 2.98 (m, 1H), 2.64 - 2.53 (m, 3H), 2.41 - 2.34 (m, 1H), 2.00 - 1.66 (m, 7H), 1.52 - 1.41 (m, 1H). |
| **29** | ¹H NMR (400 MHz, MeOH-*d*₄) δ 9.02 (s, 1H), 7.86 (dd, *J* = 9.2, 5.7 Hz, 1H), 7.42 - 7.25 (m, 2H), 7.21 (t, *J* = 3.0 Hz, 1H), 5.00 (s, 2H), 4.69 - 4.54 (m, 2H), 4.35 - 4.26 (m, 2H), 3.81 - 3.60 (m, 3H), 3.54 (d, *J* = 12.5 Hz, 1H), 3.36 (q, *J* = 5.1 Hz, 2H), 3.23 - 3.14 (m, 1H), 2.87 -2.68 (m, 2H), 2.50 (d, *J* = 15.8 Hz, 1H), 2.19 - 2.12 (m, 1H), 2.06 - 1.80 (m, 4H), 1.77 - 1.52 (m, 2H), 1.41 - 1.20 (m, 1H), 1.19 - 1.08 (m, 1H), 0.70 - 0.28 (m, 4H). |
| **45** | ¹H NMR (400 MHz, MeOH-*d*₄) δ 9.02 (s, 1H), 7.86 (dd, *J* = 9.1, 5.7 Hz, 1H), 7.40 - 7.27 (m, 2H), 7.21 (t, *J* = 3.3 Hz, 1H), 4.71 - 4.52 (m, 2H), 4.40 - 4.26 (m, 2H), 3.86 - 3.61 (m, 3H), 3.58 - 3.51 (m, 1H), 3.43 (d, *J* = 14.5 Hz, 1H), 3.35 (d, *J* = 8.5 Hz, 1H), 3.19 - 3.09 (m, 1H), 2.79 (d, *J* = 15.8 Hz, 1H), 2.70 (q, *J* = 8.4 Hz, 1H), 2.51 (d, *J* = 16.0 Hz, 1H), 2.11 (d, *J* = 9.7 Hz, 1H), 2.05 - 1.81 (m, 4H), 1.77 - 1.57 (m, 2H), 1.38 - 1.26 (m, 1H), 1.17 - 1.09 (m, 1H), 0.62 - 0.33 (m, 4H). |
| **47** | ¹H NMR (400 MHz, DMSO-*d*₆) δ 10.16 (s, 1H), 9.05 (s, 1H), 7.98 (dd, *J* = 8.0, 4.0 Hz, 1H), 7.46 (t, *J* = 8.0 Hz, 1H), 7.39 (d, *J* = 4.0 Hz, 1H), 7.18 (dd, *J* = 8.0, 4.0 Hz, 1H), 4.56 - 4.24 (m, 2H), 4.18 - 4.09 (m, 2H), 3.91 (d, *J* = 12.0 Hz, 1H), 3.70 - 3.53 (m, 3H), 3.02 - 2.98 (m, 1H), 2.68 - 2.55 (m, 3H), 2.43 - 2.39 (m,2H), 1.89 - 1.58 (m, 8H). |
| **53** | ¹H NMR (400 MHz, DMSO-*d*₆) δ 10.16 (s, 1H), 9.05 (s, 1H), 7.98 (dd, *J* = 9.2, 5.9 Hz, 1H), 7.47 (t, *J* = 9.0 Hz, 1H), 7.39 (d, *J* = 2.6 Hz, 1H), 7.18 (s, 1H), 6.75 (d, *J* = 85.7 Hz, 1H), 4.42 (t, *J* = 10.3 Hz, 1H), 4.29 (dd, *J* = 19.9, 11.9 Hz, 1H), 4.13 - 3.99 (m, 2H), 3.97 - 3.91 (m, 1H), 3.70 (d, *J* = 14.8 Hz, 1H), 3.63 - 3.49 (m, 2H), 3.33 - 3.22 (m, 2H), 3.00 (m, 1H), 2.60 - 2.52 (m, 2H), 2.37 - 2.26 (m, 1H), 2.00 - 1.57 (m, 8H). |
| **55** | ¹H NMR (400 MHz, DMSO-*d*₆) δ 10.17 (s, 1H) 9.05 (s, 1H), 7.98 (dd, *J* = 8.0 Hz, 1H), 7.47 (t, *J* = 8.0 Hz, 1H), 7.40 (d, *J* = 4.0 Hz, 1H), 7.19 (t, *J* = 4.0 Hz, 1H), 6.71 (dd, *J* = 84.0 Hz,1H) 4.56 - 4.27 (m, 2H), 4.11 - 4.03 (m, 2H), 3.96 - 3.90 (m, 1H), 3.72 - 3.59 (m, 3H), 3.27 (s, 1H), 3.03 - 3.28 (m, 1H), 2.60 - 2.56 (m, 1H), 2.34 - 2.31 (m, 1H), 2.01 - 1.53 (m, 10H), 0.98 - 0.92 (m, 3H). |
| **56** | ¹H NMR (400 MHz, DMSO-*d*₆) δ 10.15 (s, 1H) 9.03 (s, 1H), 7.97 (dd, *J* = 8.0 Hz, 1H), 7.46 (t, *J* = 8.0 Hz, 1H), 7.39 (d, *J* = 4.0 Hz, 1H), 7.19 (t, *J* = 4.0 Hz, 1H), 6.70 (dd, *J* = 84.0 Hz,1H) 4.55 - 4.27 (m, 2H), 4.11 - 4.00 (m, 2H), 3.96 - 3.90 (m, 1H), 3.72 - 3.58 (m, 3H), 3.27 (s, 1H), 3.03 - 3.28 (m, 1H), 2.59 - 2.56 (m, 1H), 2.33 - 2.31 (m, 1H), 2.01 - 1.52 (m, 10H), 0.97 - 0.90 (m, 3H). |
| **58** | ¹H NMR (400 MHz, DMSO-*d*₆) δ 9.03 (s, 1H), 8.00 - 7.96 (m, 1H), 7.47 (t, *J* = 8.0 Hz, 1H), 7.40 (d, *J* = 4.0 Hz, 1H), 7.19 (s, 1H), 6.71 (dd, *J* = 84.0 Hz,1H), 4.57 - 4.30 (m, 2H), 4.14 - 4.01 (m, 2H), 3.98 - 3.86 (m, 1H), 3.70 (d, *J* = 16.0 Hz, 1H), 3.65 - 3.55 (m, 2H), 3.50 - 3.46 (m, 2H), 3.33 - 3.27 (m, 5H), 3.03 - 3.98 (m, 1H), 2.60 - 2.54 (m, 2H), 2.33 (d, *J* = 12.0 Hz, 1H), 2.02 - 1.58 (m, 8H). |
| **61** | ¹H NMR (400 MHz, DMSO-*d*₆) δ 10.23 (s, 1H), 9.03 (s, 1H), 7.98 (dd, *J* = 8.0, 4.0 Hz, 1H), 7.47 (t, *J* = 8.0 Hz, 1H), 7.39 (d, *J* = 4.0 Hz, 1H), 7.19 (s, 1H), 6.71 (dd, *J* = 84.0 Hz,1H), 4.57 - 4.30 (m, 2H), 4.12 - 4.00 (m, 2H), 3.96 - 3.88 (m, 1H), 3.70 (d, *J* = 12.0 Hz, 1H), 3.64 - 3.53 (m, 2H), 3.48 - 3.38 (m, 4H), 3.03 - 2.98 (m, 1H), 2.59 - 2.53 (m, 2H), 2.34 - 2.30 (m, 1H), 2.02 - 1.58 (m, 8H). |
| **62** | ¹H NMR (400 MHz, DMSO-*d*₆) δ 10.19 (s, 1H), 9.02 (s, 1H), 7.96 (dd, *J* = 8.0, 4.0 Hz, 1H), 7.45 (t, *J* = 8.0 Hz, 1H), 7.38 (d, *J* = 4.0 Hz, 1H), 7.17 (s, 1H), 6.71 (dd, *J* = 84.0 Hz,1H), 4.57 - 4.29 (m, 2H), 4.12 - 4.00 (m, 2H), 3.92 - 3.88 (m, 1H), 3.70 (d, *J* = 12.0 Hz, 1H), 3.60 - 3.52 (m, 2H), 3.48 - 3.38 (m, 4H), 3.03 - 2.98 (m, 1H), 2.58 - 2.53 (m, 2H), 2.32 - 2.30 (m, 1H), 2.01 - 1.58 (m, 8H). |
| **68** | ¹H NMR (400 MHz, MeOH-*d*₄) δ 9.02 (s, 1H), 7.86 (dd, *J* = 9.2, 5.7 Hz, 1H), 7.40 - 7.27 (m, 2H), 7.21 (t, *J* = 2.8 Hz, 1H), 6.65 (d, *J* = 84.8 Hz, 1H), 4.69 - 4.52 (m, 2H), 4.35 - 4.23 (m, 2H), 3.84 (d, *J* = 15.0 Hz, 1H), 3.74 - 3.61 (m, 2H), 3.57 - 3.50 (m, 1H), 3.49 - 3.42 (m, 1H), 3.35 (d, *J* = 7.9 Hz, 1H), 3.18 - 3.12 (m, 1H), 2.75 - 2.67 (m, 2H), 2.44 (d, *J* = 15.5 Hz, 1H), 2.17 - 2.09 (m, 1H), 2.04 - 1.79 (m, 4H), 1.77 - 1.56 (m, 2H), 1.36 - 1.28 (m, 1H), 1.16 - 1.10 (m, 1H), 0.63 - 0.35 (m, 4H). |
| **71** | ¹H NMR (400 MHz, DMSO-*d*₆) δ 10.16 (s, 1H), 9.05 (s, 1H), 7.98 (dd, *J* = 9.2, 5.9 Hz, 1H), 7.46 (t, *J* = 9.0 Hz, 1H), 7.39 (d, *J* = 2.6 Hz, 1H), 7.18 (t, *J* = 3.0 Hz, 1H), 6.75 (d, *J* = 85.8 Hz, 1H), 4.54 - 4.26 (m, 2H), 4.18 - 3.98 (m, 2H), 3.92 (d, *J* = 20.1 Hz, 1H), 3.70 (d, *J* = 14.8 Hz, 1H), 3.67 - 3.48 (m, 2H), 3.31 - 3.26 (m, 2H), 3.03 - 2.97 (m, 1H), 2.59 - 2.53 (m, 2H), 2.36 - 2.28 (m, 1H), 2.01 - 1.56 (m, 7H), 1.42 - 1.27 (m, 1H). |
| **75** | ¹H NMR (400 MHz, MeOH-*d*₄) δ 9.02 (s, 1H), 7.86 (dd, *J* = 9.2, 5.7 Hz, 1H), 7.37 - 7.29 (m, 2H), 7.21 (dd, *J* = 5.6, 2.5 Hz, 1H), 6.81 - 6.54 (m, 1H), 4.83 - 4.71 (m, 1H), 4.63 - 4.47 (m, 1H), 4.41 - 4.27 (m, 2H), 3.97 - 3.88 (m, 1H), 3.85 - 3.70 (m, 2H), 3.60 - 3.52 (m, 1H), 3.47 - 3.41 (m, 1H), 3.38 - 3.36 (m, 1H), 3.27 - 3.17 (m, 1H), 2.82 - 2.67 (m, 3H), 2.55 - 2.46 (m, 1H), 2.27 - 2.11 (m, 2H), 2.05 - 1.62 (m, 14H). |
| **80** | ¹H NMR (400 MHz, MeOH-*d*₄) δ 9.02 (s, 1H), 7.86 (dd, *J* = 9.1, 5.7 Hz, 1H), 7.40 - 7.28 (m, 2H), 7.22 (dd, *J* = 4.6, 2.6 Hz, 1H), 6.72 (dt, *J* = 84.6, 2.1 Hz, 1H), 4.72 (dd, *J* = 16.4, 12.5 Hz, 1H), 4.55 (dd, *J* = 28.6, 12.4 Hz, 1H), 4.42 - 4.20 (m, 2H), 3.79 - 3.63 (m, 3H), 3.44 - 3.32 (m, 3H), 3.12 (ddd, *J* = 10.7, 6.3, 4.4 Hz, 1H), 2.84 (d, *J* = 16.4 Hz, 1H), 2.68 (dt, *J* = 9.5, 6.9 Hz, 1H), 2.55 (d, *J* = 16.4 Hz, 1H), 2.16 - 2.11 (m, 1H), 1.99 - 1.50 (m, 9H), 1.06 (td, *J* = 7.7, 3.0 Hz, 3H). |
| **82** | ¹H NMR (400 MHz, DMSO-*d*₆) δ 10.16 (s, 1H), 9.03 (s, 1H), 7.98 (dd, *J* = 9.1, 5.9 Hz, 1H), 7.47 (t, *J* = 9.0 Hz, 1H), 7.39 (d, *J* = 2.6 Hz, 1H), 7.18 (t, *J* = 2.5 Hz, 1H), 6.82 (d, *J* = 85.8 Hz, 1H), 4.51 (dd, *J* = 39.2, 12.2 Hz, 1H), 4.35 (dd, *J* = 38.2, 12.1 Hz, 1H), 4.13 (d, *J* = 10.8 Hz, 1H), 4.02 (d, *J* = 10.6 Hz, 1H), 3.91 (d, *J* = 31.7 Hz, 1H), 3.66 - 3.55 (m, 2H), 3.54 - 3.47 (m, 1H), 3.46 (s, 1H), 3.44 - 3.37 (m, 2H), 3.36 - 3.35 (m, 2 H), 3.20 (d, *J* = 13.8 Hz, 1H), 3.01 - 2.94 (m, 1H), 2.71 - 2.64 (m, 1H), 2.58 - 2.52 (m, 1H), 2.43 - 2.30 (m, 2H), 2.02 - 1.93 (m, 1H), 1.88 - 1.61 (m, 6H), 1.50 - 1.39 (m, 1H). |
| **84** | ¹H NMR (400 MHz, MeOH-*d*₄) δ 9.01 (d, *J* = 4.3 Hz, 1H), 7.86 (dd, *J* = 9.2, 5.7 Hz, 1H), 7.39 - 7.27 (m, 2H), 7.22 (t, *J* = 2.2 Hz, 1H), 6.72 (d, *J* = 84.4 Hz, 1H), 4.64 (dt, *J* = 26.4, 14.3 Hz, 3H), 4.37 (dd, *J* = 10.7, 6.5 Hz, 1H), 4.28 (dd, *J* = 10.7, 3.9 Hz, 1H), 3.85 - 3.45 (m, 7H), 3.20 - 3.08 (m, 1H), 2.85 (d, *J* = 16.4 Hz, 1H), 2.71 (q, *J* = 8.2 Hz, 1H), 2.57 (d, *J* = 16.4 Hz, 1H), 2.23 - 2.10 (m, 1H), 2.01 - 1.61 (m, 7H). |
| **90** | ¹H NMR (400 MHz, MeOH-*d*₄) δ 9.03 (d, *J* = 1.3 Hz, 1H), 7.86 (dd, *J* = 9.2, 5.7 Hz, 1H), 7.43 - 7.26 (m, 2H), 7.26 - 7.15 (m, 1H), 6.72 (dt, *J* = 84.7, 2.0 Hz, 1H), 4.72 - 4.52 (m, 2H), 4.38 - 4.25 (m, 2H), 3.77 - 3.62 (m, 3H), 3.59 - 3.51 (m, 1H), 3.35 - 3.33 (m, 1H), 3.16 - 3.06 (m, 1H), 3.01 (s, 1H), 2.84 (d, *J* = 16.5 Hz, 1H), 2.73 - 2.63 (m, 1H), 2.55 (d, *J* = 16.4 Hz, 1H), 2.20 - 2.09 (m, 1H), 1.99 - 1.83 (m, 4H), 1.80 - 1.67 (m, 1H), 1.68 - 1.57 (m, 1H), 1.37 - 1.29 (m, 1H), 1.18 - 1.07 (m, 1H), 0.61 - 0.36 (m, 4H). |
| **137** | ¹H NMR (400 MHz, MeOH-*d*₄) δ 9.00 (s, 1H), 7.74 (dd, *J* = 9.1, 5.7 Hz, 1H), 7.27 - 7.12 (m, 3H), 6.79 - 6.51 (m, 1H), 4.77 - 4.49 (m, 2H), 4.37 - 4.25 (m, 2H), 3.91 - 3.83 (m, 1H), 3.81 - 3.67 (m, 2H), 3.53 - 3.33 (m, 3H), 3.21 - 3.14 (m, 1H), 2.76 - 2.70 (m, 2H), 2.50 - 2.41 (m, 1H), 2.17 - 2.10 (m, 1H), 2.02 - 1.75 (m, 6H), 1.61 - 1.51 (m, 1H). |
| **138** | ¹H NMR (400 MHz, MeOH-*d*₄) δ 9.01 (s, 1H), 7.74 (dd, *J* = 9.1, 5.7 Hz, 1H), 7.24 (t, *J* = 9.0 Hz, 1H), 7.19 (d, *J* = 2.5 Hz, 1H), 7.14 (t, *J* = 2.6 Hz, 1H), 6.65 (d, *J* = 84.6 Hz, 1H), 4.72 - 4.51 (m, 3H), 4.37 - 4.20 (m, 2H), 3.84 (d, *J* = 15.0 Hz, 1H), 3.74 - 3.62 (m, 2H), 3.57 - 3.43 (m, 2H), 3.21 - 3.11 (m, 1H), 2.76 - 2.68 (m, 2H), 2.45 (d, *J* = 15.6 Hz, 1H), 2.20 - 1.52 (m, 8H), 1.32 (td, *J* = 12.6, 4.2 Hz, 1H), 1.17 - 1.07 (m, 1H), 0.55 (q, *J* = 5.2 Hz, 3H), 0.46 - 0.33 (m, 1H). |
| **201** | ¹H NMR (400 MHz, MeOH-*d*₄) δ 7.84 (dd, *J* = 9.2, 5.7 Hz, 1H), 7.34 - 7.27 (m, 2H), 7.26 - 7.18 (m, 1H), 5.03 (d, *J* = 8.4 Hz, 4H), 4.58 - 3.93 (m, 4H), 3.78 (d, *J* = 14.7 Hz, 2H), 3.69 - 3.50 (m, 2H), 3.39 - 3.33 (m, 3H), 3.28 - 3.15 (m, 0H), 2.79 (d, *J* = 15.9 Hz, 2H), 2.59 (d, *J* = 16.4 Hz, 2H), 1.95 - 1.49 (m, 6H), 1.04 (q, *J* = 7.4 Hz, 3H). |
| **204** | ¹H NMR (400 MHz, MeOH-*d*₄) δ 7.83 (dd, *J* = 9.1, 5.7 Hz, 1H), 7.38 - 7.18 (m, 3H), 6.81 - 6.50 (m, 1H), 4.69 - 3.92 (m, 4H), 3.89 - 3.80 (m, 1H), 3.67 - 3.43 (m, 3H), 3.29 - 3.11 (m, 3H), 2.78 - 2.65 (m, 2H), 2.44 (d, *J* = 15.6 Hz, 1H), 2.18 - 2.08 (m, 1H), 2.03 - 1.46 (m, 9H), 1.04 (q, *J* = 7.5 Hz, 3H). |
| **229** | ¹H NMR (400 MHz, MeOH-d₄) δ 7.85 (dd, *J* = 9.2, 5.7 Hz, 1H), 7.50 (dd, *J* = 10.0, 1.8 Hz, 1H), 7.41 - 7.24 (m, 2H), 7.13 (d, *J* = 2.6 Hz, 1H), 6.64 (d, *J* = 84.6, 1H), 4.66 - 4.18 (m, 5H), 3.85 (d, *J* = 14.9 Hz, 1H), 3.70 - 3.53 (m, 2H), 3.47 (d, *J* = 14.8 Hz, 1H), 3.27 - 3.10 (m, 2H), 2.78 - 2.64 (m, 2H), 2.44 (d, *J* = 15.6 Hz, 1H), 2.20 - 2.07 (m, 1H), 2.05 - 1.68 (m, 6H), 1.62 - 1.44 (m, 1H). |
| **303** | ¹H NMR (400 MHz, MeOH-*d*₄) δ 7.96 (s, 1H), 7.26 - 7.19 (m, 1H), 7.06 (t, *J* = 8.9 Hz, 1H), 5.27 (d, *J* = 12.2 Hz, 2H), 4.81 - 4.64 (m, 5H), 4.36 (t, *J* = 15.2 Hz, 1H), 4.22 (d, *J* = 6.6 Hz, 1H), 3.99 - 3.76 (m, 4H), 3.14 - 3.03 (m, 1H), 2.81 (d, *J* = 15.6 Hz, 1H), 2.44 - 2.32 (m, 1H), 2.30 - 2.21 (m, 1H), 2.19 - 2.00 (m, 4H), 1.95 - 1.80(m, 1H), 1.60 - 1.50 (m, 1H), 1.34 - 1.27 (m, 1H), 0.86 - 0.70 (m, 3H), 0.58 - 0.48 (m, 1H). |
| **355** | ¹H NMR (400 MHz, MeOH-d₄) δ 9.00 (d, *J* = 2.6 Hz, 1H), 7.74 (dd, *J* = 9.1, 5.7 Hz, 1H), 7.24 (t, *J* = 9.0 Hz, 1H), 7.19 (d, *J* = 2.5 Hz, 1H), 7.14 (t, *J* = 2.6 Hz, 1H), 6.65 (d, *J* = 84.6 Hz, 1H), 4.61 - 4.48 (m, 2H), 4.39 - 4.19 (m, 2H), 3.84 (d, *J* = 14.8 Hz, 1H), 3.74 - 3.59 (m, 2H), 3.44 (t, *J* = 14.3 Hz, 2H), 3.15 (ddd, *J* = 10.4, 6.4, 4.5 Hz, 1H), 2.76 - 2.64 (m, 2H), 2.44 (d, *J* = 15.4 Hz, 1H), 2.18 - 2.07 (m, 1H), 2.04 - 1.29 (m, 8H). |
| **357** | ¹H NMR (400 MHz, MeOH-*d*₄) δ 9.00 (s, 1H), 7.74 (dd, *J* = 9.1, 5.7 Hz, 1H), 7.24 (t, *J* = 9.0 Hz, 1H), 7.19 (d, *J* = 2.5 Hz, 1H), 7.14 (t, *J* = 2.6 Hz, 1H), 5.02 - 4.98 (m, 2H), 4.77 - 4.52 (m, 3H), 4.36 - 4.22 (m, 2H), 3.82 - 3.63 (m, 3H), 3.47 - 3.35 (m, 2H), 3.22 - 3.11 (m, 1H), 2.81 - 2.70 (m, 2H), 2.49 (d, *J* = 15.9 Hz, 1H), 2.21 - 2.08 (m, 1H), 2.04 - 1.73 (m, 6H), 1.61 - 1.49 (m, 1H). |
| **360** | ¹H NMR (400 MHz, MeOH-*d*₄) δ 7.86 (s, 1H), 7.21 (ddd, *J* = 8.2, 5.1, 1.2 Hz, 1H), 7.07 (t, *J* = 8.0Hz, 1H), 6.73 - 6.53 (m, 1H), 4.65 - 4.44 (m, 3H), 4.36 - 4.22 (m, 2H), 3.85 (d, *J* = 14.4 Hz, 1H), 3.69 - 3.59 (m, 2H), 3.48 - 3.43 (m, 1H), 3.18 - 3.12 (m, 1H), 2.73 (d, *J* = 14.4 Hz, 2H), 2.43 (d, *J* = 14.3 Hz, 1H), 2.17 - 2.09 (m, 1H), 2.05 - 1.68 (m, 6H), 1.57 - 1.45 (m, 1H). |
| **361** | ¹H NMR (400 MHz, DMSO-*d*₆) δ 8.11 (s, 2H), 7.85(s, 1H), 7.27 (dd, *J* = 5.3, 8.4 Hz, 1H), 7.15 (t, *J* = 8.9Hz, 1H), 6.88 - 6.60 (m, 1H), 4.35 (br d, *J* = 12.1 Hz,1H), 4.24 (br d, *J* = 11.0 Hz, 1H), 4.13 - 3.98(m, 2H),3.70 (br d, *J* = 15.0 Hz, 1H), 3.59 - 3.47 (m, 2H), 3.30(br s, 1H), 3.28 - 3.19 (m, 2H),3.00 (ddd, *J* = 4.2, 6.2,9.9 Hz, 1H), 2.60 - 2.54 (m, 3H), 2.36 - 2.27 (m, 1H),2.02 - 1.92 (m, 1H), 1.91 - 1.75 (m, 2H), 1.75 - 1.64(m, 2H), 1.61 - 1.50 (m, 2H), 1.37 - 1.19 (m, 1H). |
| **362** | ¹H NMR (400 MHz, MeOH-*d*₄) δ 7.86 (s, 1H), 7.67 (d, *J* = 7.7 Hz, 1H), 7.25 (t, *J* = 7.7 Hz, 1H), 7.17 (d, *J* = 7.1 Hz, 1H), 6.74 - 6.53 (m, 1H), 4.65 - 4.44 (m, 3H), 4.38 - 4.19 (m, 2H), 3.85 (d, *J* = 15.2 Hz, 1H), 3.69 - 3.60 (m, 2H), 3.46 (d, *J* = 14.0 Hz, 1H), 3.15 (dd, *J* = 10.6, 5.9 Hz, 1H), 2.71 (t, *J* = 14.1 Hz, 2H), 2.44 (d, *J* = 15.2 Hz, 1H), 2.22 - 2.09 (m, 1H), 2.07 - 1.57 (m, 6H), 1.50 (d, *J* = 18.1 Hz, 1H). |
| **363** | ¹H NMR (400 MHz, MeOH-*d*₄) δ 7.86 (s, 1H), 7.67 (d, *J* = 7.9 Hz, 1H), 7.25 (t, *J* = 7.7 Hz, 1H), 7.17 (d, *J* = 7.1 Hz, 1H), 6.74 - 6.53 (m, 1H), 4.59 - 4.41 (m, 2H), 4.31 - 4.22 (m, 2H), 3.85 (d, *J* = 15.1 Hz, 1H), 3.67 - 3.58 (m, 2H), 3.55 - 3.42 (m, 4H), 3.19 - 3.11 (m, 1H), 2.76 - 2.68 (m, 2H), 2.43 (d, *J* = 15.7 Hz, 1H), 2.13 (dd, *J* = 10.6, 6.3 Hz, 1H), 2.04 - 1.72 (m, 6H), 1.59 (t, *J* = 12.4 Hz, 1H). |

### Biological Test Evaluation

### I. KRAS^{G12D}/cRAF Binding Test

### 1. Compound Preparation

The compound was dissolved in 100% DMSO at a starting concentration of 5 mM and diluted in DMSO with a 3-fold serial dilution method for a total of 8 concentration points. 1 µL of the compound or DMSO at each concentration point was added into 99 µL of the experimental buffer to obtain 10X stock solution.

### 2. Test Steps

1) 4 µL of the working mixture solution of His-tagged KRAS^{G12D} and GMPPNP was added to the 384-well plate.
2) The 10X compound stock solution or 1% DMSO was added to the 384-well plate.
3) The plate was centrifuged at 1,000 rpm for 1 minute.
4) 4 µL of GST-tagged cRAF solution was added to the 384-well plate.
5) The plate was centrifuged at 1,000 rpm for 1 minute.
6) The plate was incubated at room temperature for 15 minutes.
7) 10 µL of detection working solution was added to the 384-well plate.
8) The plate was centrifuged at 1,000 rpm for 1 minute.
9) The plate was incubated at room temperature for 60 minutes.
10) The 384-well plate was placed into Envision and assay data was obtained.

### 3. Data Processing

The ratio for each compound concentration was calculated from the signal of the DMSO control group contained in each assay plate and the Alphalisa signal in each compound well. The compound concentration required for inhibitory concentration at 50% (IC₅₀) was determined using a four-parameter logarithmic dose-response equation by testing the concentration of the compound and the value of the ratio. The endpoint value (IC₅₀) of the reference compound was assessed in each experiment as a quality control measure. If the endpoint value is within three times the expected value, the experiment may be considered acceptable.

**Table 1: Biology Test Results**

| **Example No.** | **KRAS G12D/ cRAF Alphalisa IC₅₀ (nM)** | **Example No.** | **KRAS G12D/ cRAF Alphalisa IC₅₀ (nM)** |
|---|---|---|---|
| **Example 1** | 37.4 | **Example 68** | 40.1 |
| **Example 2** | 48.2 | **Example 71** | 5.6 |
| **Example 5** | 16.5 | **Example 75** | 34.5 |
| **Example 6** | 3.2 | **Example 80** | 6.2 |
| **Example 8** | NT | **Example 82** | 8.1 |
| **Example 12** | 101.5 | **Example 84** | 3.9 |
| **Example 20** | 3.0 | **Example 90** | 40.7 |
| **Example 22** | 2.9 | **Example 137** | 9.5 |
| **Example 29** | 25.0 | **Example 173** | NT |
| **Example 45** | 52.5 | **Example 201** | NT |
| **Example 47** | 14.6 | **Example 204** | 3.9 |
| **Example 53** | 16.6 | **Example 229** | 20.6 |
| **Example 55** | 3.0 | **Example 355** | 11.0 |
| **Example 56** | NT | **Example 360** | 20.2 |
| **Example 58** | 6.0 | **Example 361** | 7.1 |
| **Example 61** | 2.5 | **Example 362** | 27.7 |
| **Example 62** | 5.1 | **Example 363** | 18.6 |
| **Positive Compound 1** | 7.7 | | |
| **Notes** | "NT" indicates not tested. | | |

The "positive compound 1" used in the biological test evaluation of this application is Example 252 in WO2021041671A1 (the same positive compound 1 used in the following anti-proliferative 2D CTG assay and mouse pharmacokinetic experiment) with the chemical structure as follows:

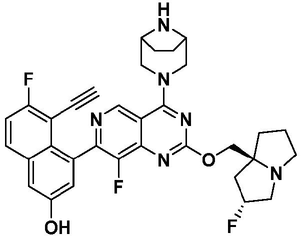

From the KRAS^{G12D}/cRAF binding inhibitory data of the compounds in specific examples, it can be seen that the series of compounds of the present invention had a strong inhibitory effect on KRAS^{G12D}/cRAF binding. Under the same test conditions, the cell inhibitory activity of the compounds in some examples was even equivalent to that of the positive compounds, or improved to a certain extent.

### II. Anti-Proliferative 2D CTG Assay

### 1. Experiment Steps:

### 1.1 Day 0 Cell Culture and Plating

Cells in a logarithmic growth phase were harvested and the cells were counted by using a platelet counter. Cell viability was tested by using a trypan blue exclusion method to ensure that cell viability was above 90%. The cell concentration was adjusted to the desired final density, and 90 µL of cell suspension was added to a 96-well plate, then the cells were incubated overnight at 37°C, 5% CO₂ and 95% humidity. Cell density and cell culture are shown in the figure below.

| **No.** | **Cell Line** | **Cell Medium** | **Cell Density** |
|---|---|---|---|
| **1** | **HPAC** | **DMEM/F12 (1:1) + 10% FBS** | **1,000 cells/well** |
| **2** | **LS513** | **RPMI-1640+10% FBS** | **2,000 cells/well** |
| **3** | **AsPC-1** | **RPMI-1640+10% FBS** | **1,000 cells/well** |
| **4** | **AGS** | **RPMI-1640+10% FBS** | **1,000 cells/well** |

### 1.2 Day 1 Baseline Readings

10 µL PBS was added to each well of the T0 plate containing cells. The CTG reagent was thawed and the plate was equilibrated to room temperature for 30 minutes. The CTG solution of 50 µL/well was added to each well. The cells were lysed by shaking on an orbital shaker for 5 minutes. The plate was left at room temperature for 20 minutes to stabilize the luminescence signal. The T0 fluorescence signal value was read.

### 1.3 Day 1 Compound Treatment

From the 10 mM stock solution, with a ratio of 1:5, 9-point serial dilutions were performed. The 10X medium containing the compound was transferred into corresponding wells of the 96 wells. The final maximum compound concentration was 10 µM and the final DMSO concentration was 0.1%. The 96-well plate was placed in a 37°C incubator and incubated for 4 days.

### 1.4 Day 5 Signal Reading

50 µL/well of detection reagent (CTG) was added to the cells, and the signal was read in an Envision machine.

### 2. Data Processing

The percent inhibition (%) at each compound concentration was calculated from the signal in the HPE and ZPE control wells and the fluorescence signal in the individual compound wells included in each assay plate. The ZPE control wells containing enzyme and substrate had an inhibition rate of 0%, and the HPE control wells containing only substrate had an inhibition rate of 100%. The compound concentration required for inhibitory concentration at 50% (IC₅₀) was determined using a four-parameter logarithmic dose-response equation by testing the concentration of the compound and the inhibition percentage value. The endpoint value (IC₅₀) of the reference compound was assessed in each experiment as a quality control measure. If the endpoint value is within three times the expected value, the experiment may be considered acceptable.

**Table 2: Biology Test Results**

| **Example No.** | **AGS cell IC₅₀ (nM)** | **LS513 cell IC₅₀ (nM)** | **HPAC cell IC₅₀ (nM)** | **AsPC-1 cell IC₅₀ (nM)** |
|---|---|---|---|---|
| **Example 1** | 22.9 | NT | NT | NT |
| **Example 2** | 251.2 | NT | NT | NT |
| **Example 5** | 14.1 | NT | NT | 45.8 |
| **Example 6** | 14.3 | NT | NT | 48.9 |
| **Example 8** | 36.8 | NT | NT | NT |
| **Example 12** | 103.7 | NT | NT | 219.0 |
| **Example 20** | 3.1 | NT | NT | 17.2 |
| **Example 22** | 21.7 | NT | NT | 14.3 |
| **Example 29** | 21.0 | NT | NT | 102.2 |
| **Example 45** | NT | 163.3 | 133.2 | NT |
| **Example 47** | 4.8 | 23.9 | 23.1 | 46.3 |
| **Example 53** | 1.0 | NT | NT | NT |
| **Example 55** | 1.3 | NT | NT | 16.3 |
| **Example 56** | 0.6 | 2.6 | 2.5 | 9.1 |
| **Example 58** | 1.7 | NT | NT | 21.8 |
| **Example 61** | 3.4 | NT | NT | 37.9 |
| **Example 62** | 1.5 | 4.9 | 9.7 | 19.8 |
| **Example 68** | 33.4 | 59.8 | 83.2 | 190.3 |
| **Example 71** | 1.4 | 4.1 | 2.3 | 16.7 |
| **Example 75** | 14.2 | 56.1 | 93.6 | NT |
| **Example 80** | 5.8 | NT | NT | 39.6 |
| **Example 84** | 11.4 | NT | NT | 59.3 |
| **Example 90** | 28.1 | NT | NT | 103.4 |
| **Example 137** | 9.4 | 14.4 | 11.7 | 37.6 |
| **Example 173** | 26.4 | NT | NT | NT |
| **Example 201** | 21.6 | NT | NT | NT |
| **Example 204** | 5.1 | NT | NT | NT |
| **Example 229** | NT | 33.3 | 87.5 | NT |
| **Example 355** | 17.7 | NT | NT | 66.1 |
| **Example 357** | 23.0 | 57.4 | 46.9 | NT |
| **Example 360** | NT | NT | NT | NT |
| **Example 361** | NT | 40.0 | 38.0 | NT |
| **Example 362** | NT | 65.7 | 61.8 | NT |
| **Example 363** | NT | 82.3 | 66.3 | NT |
| **Positive Compound 1** | 4.2 | 18.1 | 10.3 | 34.9 |
| **Positive Compound 2** | 9.2 | 59.4 | 33.0 | 83.3 |
| **Notes** | "NT" indicates not tested. | | | |

The "positive compound 2" used in the biological test evaluation of the anti-proliferation 2D CTG assay of this application is Example 54 in WO2023098425A1 with the chemical structure as follows:

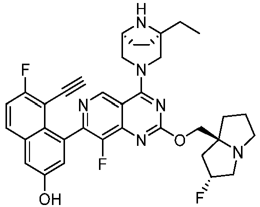

From the cell activity data of the compounds in specific examples, it can be seen that the series of compounds of the present invention had a strong inhibitory effect on KRAS cell activity. Under the same test conditions, compared with the positive compounds, the cell inhibitory activity of the compounds in some examples increased several times or even more than ten times.

### III. Pharmacokinetic Experiment in Mice

### (I) Study Purpose

The purpose of this trial is to study the pharmacokinetic behavior of some compounds of the present invention, and the administration method is: single oral administration (PO) or intravenous administration (IV) to ICR mice.

### (II) Trial Protocol

### 1. Test Drug

The compounds used in this trial are from the compounds in the specific examples of the present invention and the listed positive compounds.

### 2. Test Animals

ICR mice male N=9 original source: Shanghai Sippr-Bk Lab Animal Co., Ltd.

### 3. Drug Preparation and Administration

The compounds were weighed and dissolved in the corresponding solution (0.5% CMC + 1% tween water solution or 10% PG + 10% Solutol^{®} + 80% pH3 citrate buffer). The mixture was then shaken well and sonicated to prepare a 1 mg/mL stock solution. Nine mice were orally administered at a dose of 10 mg/kg or intravenously administered at a dose of 2 mg/kg after an overnight fast.

### 4. Sample Collection

About 90 µL of blood was collected via the submandibular vein per time point, heparin sodium anticoagulant was added, the sample was placed on ice after collection, and plasma was centrifuged within 1 hour (centrifugation conditions: 8,000 rpm, 6 minutes, 2-8°C). The blood collection timepoints were 0, 0.25, 0.5, 1, 2, 4, 6, 8 and 24 h. The samples were stored in a -20°C refrigerator.

The plasma sample was 40 µL, 160 µL of ice-cold acetonitrile containing internal standard was added, vortexed for 3 minutes, and centrifuged at 11,000 rpm for 5 minutes. 100 µL of the supernatant was taken and added to 100 µL of water, 5 µL of the sample was taken and injected into LC/MS/MS for analysis.

### 5. Test Results

| **Example No.** | **Route of Administration /Dose** | **Cmax (ng/mL)** | **T_{1/2} (h)** | **AUC (ng/mL*hr)** | **Oral Bioavailability (%)** |
|---|---|---|---|---|---|
| **Example 62** | PO/10 mpk | 223.5 | 5.7 | 619.1 | 14.6% |
| | IV/2 mpk | 763.7 | 4.9 | 849.2 | / |
| **Example 68** | PO/10 mpk | 300.2 | 5.6 | 1,074.3 | 26.4% |
| | IV/2 mpk | 886.7 | 6.3 | 812.6 | / |
| **Example 71** | PO/10 mpk | 461.2 | 4.9 | 1,021.4 | 41.1% |
| | IV/2 mpk | 637.5 | 6.6 | 496.6 | / |
| **Example 137** | PO/10 mpk | 298.4 | 2.9 | 1,881.9 | 48.6% |
| | IV/2 mpk | 466.8 | 5.9 | 774.6 | / |
| **Example 355** | PO/10 mpk | 119.9 | 9.1 | 465.5 | 41.7% |
| | IV/2 mpk | 68.9 | 7.2 | 223.2 | / |
| **Example 361** | PO/10 mpk | 163.6 | 9.6 | 369.6 | 33.5% |
| | IV/2 mpk | 178.9 | 13.7 | 220.8 | / |
| **Positive Compound 1** | PO/10 mpk | 20.6 | 1.6 | 40.2 | 2.2% |
| | IV/2 mpk | 1131 | 3.1 | 366.6 | / |
| **Reference Compound 1** | PO/10 mpk | 33.2 | 2.6 | 82.6 | 2.4% |
| | IV/2 mpk | 1,691.1 | 10.5 | 688.2 | / |
| **Reference Compound 2** | PO/10 mpk | 27.7 | 2.3 | 43.2 | 1.2% |
| | IV/2 mpk | 1,348 | 14.1 | 718 | / |
| **Reference Compound 3** | PO/10 mpk | 10.7 | 2.6 | 27.1 | NT |
| | / | / | / | / | / |
| **Reference Compound 4** | PO/10 mpk | 14.8 | 4.6 | 52.8 | 6.5% |
| | IV/2 mpk | 104.1 | 8.4 | 163.3 | / |
| **Notes** | 1. "NT" indicates not tested. | | | | |
| | 2. Oral bioavailability = (AUC_{PO}/dose (PO)) ÷ (AUC_{IV}/dose (IV)) * 100% | | | | |

From the pharmacokinetic data of the compounds in the above specific examples, with the substituents introduced on the bridge rings, this series of compounds of the present invention were absorbed into the mice via a single oral dose (10 mg/kg) and showed very good exposure and oral bioavailability. With head-to-head comparison of multiple groups of positive compounds, the exposure or oral bioavailability of the series of compounds of the present invention increased by about 5-10 times. The experiment results demonstrate that this series of compounds of the present invention have good oral bioavailability, and are likely to solve the problem that pharmacokinetic limitations that make the positive compounds unsuitable for oral administration.

All documents mentioned in the present invention are incorporated by reference in this application, just as each document is cited separately as a reference. In addition, it should be understood that various modifications or changes may be made by those skilled in the art after reading the above disclosed content of the present invention, and these equivalent forms also fall within the scope defined by the claims appended hereto.

## Claims

1. A compound of formula (I), a stereoisomer or pharmaceutically acceptable salt thereof: 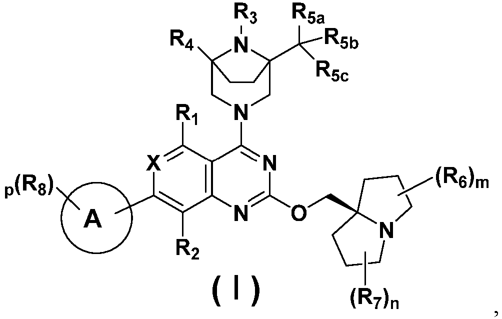
wherein, X is CR₉ or N;
ring A is C₆-₁₀ aryl or 5-10 membered heteroaryl, preferably, the C₆₋₁₀ aryl or 5-10 membered heteroaryl is selected from the group consisting of phenyl, naphthyl, pyridyl, pyrimidinyl, pyridazinyl, quinolinyl, isoquinolinyl, indolyl, benzothiophenyl, benzimidazolyl, benzopyrazolyl, benzoxazolyl and benzothiazolyl;
R₁ is selected from the group consisting of hydrogen, deuterium, halogen, cyano,
nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₂ is selected from the group consisting of hydrogen, deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₃ is selected from the group consisting of hydrogen, C₁₋₁₀ alkyl, C₂₋₁₀ alkenyl, C₃₋₁₂ cycloalkyl, 3-12 membered heterocyclyl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-C(O)R₁₅, -C₀₋₈ alkyl-O-C(O)R₁₅, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅, and the above groups are each independently optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₄ is selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, and the above groups are each independently optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₅ₐ is selected from the group consisting of hydrogen, deuterium, halogen, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-NH-S(O)₂R₁₃, -C₀₋₈ alkyl-NH-S(O)₂NR₁₆R₁₇, -C₀₋₈ alkyl-S(O)ᵣR₁₃, -C₀₋₈ alkyl-S(O)ᵣNR₁₆R₁₇, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, and the above groups are each independently optionally further substituted by one or more substituents selected from the group consisting of deuterium, halogen, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R_{5b} and R_{5c} are each independently selected from the group consisting of hydrogen, deuterium, halogen, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-NH-S(O)₂R₁₃, -C₀₋₈ alkyl-NH-S(O)₂NR₁₆R₁₇, -C₀₋₈ alkyl-S(O)ᵣR₁₃, -C₀₋₈ alkyl-S(O)ᵣNR₁₆R₁₇, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, and the above groups are each independently optionally further substituted by one or more substituents selected from the group consisting of deuterium, halogen, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, provided that R₅ₐ, R_{5b} and R_{5c} are not hydrogen at the same time;
or, R_{5b} and R_{5c}, together with the carbon atom to which they are directly attached, form a C(O), C₃₋₁₀ cycloalkyl or 3-10 membered heterocyclyl, and the above groups are each independently optionally further substituted by one or more substituents selected from the group consisting of deuterium, halogen, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆-C(O)R₁₅ wherein R₅ₐ is defined as above;
or, R_{5b} and R_{5c}, together with the carbon atom to which they are directly attached, form 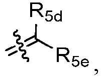 wherein R₅ₐ is defined as above;
or, R₅ₐ, R_{5b} and R_{5c}, together with the carbon atom to which they are directly attached, form 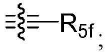
R_{5d}, R₅ₑ and R_{5f} are each independently selected from the group consisting of halogen, cyano, C₁₋₄ alkyl, C₃₋₆ cycloalkyl, 3-6 membered heterocyclyl and -C₀₋₈ alkyl-NR₁₆R₁₇, and the above groups are each independently optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl and 5-10 membered heteroaryl;
each R₆ and each R₇ are independently selected from the group consisting of hydrogen, deuterium, halogen, cyano, nitro, azido, C₁₋₁₀ alkyl, C₁₋₁₀ haloalkyl, C₁₋₁₀ deuterioalkyl, C₁₋₁₀ alkylene, C₁₋₁₀ haloalkylene, C₁₋₁₀ deuterioalkylene, C₂₋₁₀ alkenyl, C₂₋₁₀ alkynyl, C₃₋₁₂ cycloalkyl, C₃₋₁₂ cycloalkylene, C₃₋₁₂ halocycloalkylene, C₃₋₁₂ deuteriocycloalkylene, 3-12 membered heterocyclyl, C₃₋₁₂ heterocycloalkylene, C₃₋₁₂ haloheterocycloalkylene, C₃₋₁₂ deuterioheterocycloalkylene, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, provided that at least one of R₆ or R₇ is C₁₋₁₀ alkylene, C₁₋₁₀ haloalkylene, C₁₋₁₀ deuterioalkylene, C₁₋₁₀ cycloalkylene, C₁₋₁₀ halocycloalkylene, C₁₋₁₀ deuteriocycloalkylene, C₁₋₁₀ heterocycloalkylene, C₁₋₁₀ haloheterocycloalkylene or C₁₋₁₀ deuterioheterocycloalkylene;
each R₈ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, or, when p≥2, two adjacent R₈, together with the moiety to which they are directly attached, form a C₅₋₁₂ cycloalkyl or 5-12 membered heterocyclyl, and the above groups are each independently optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₉ is selected from the group consisting of hydrogen, deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
or, R₉ and R₁, together with the moiety to which they are directly attached, form a C₃₋₁₀ cycloalkyl or 3-10 membered heterocyclyl, and the C₃₋₁₀ cycloalkyl or 3-10 membered heterocyclyl is independently optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
each R₁₀ is independently selected from the group consisting of hydrogen, deuterium, C₀₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-C(O)OR₁₄, -C₀₋₈ alkyl-C(O)R₁₅ and -C₀₋₈ alkyl-C(O)NR₁₆R₁₇, and the above groups are each independently optionally further substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)R₁₅, -C₀₋₈ alkyl-O-C(O)R₁₅, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
each R₁₁ and each R₁₂ are independently selected from the group consisting of hydrogen, deuterium, hydroxy, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl and 5-10 membered heteroaryl, or, R₁₁ and R₁₂, together with the sulfur atom to which they are directly attached, form a 3-10 membered heterocyclyl, and the above groups are each independently optionally further substituted by one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)R₁₅, -C₀₋₈ alkyl-O-C(O)R₁₅, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
each R₁₃ is independently selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl and -NR₁₆R₁₇, and the above groups are each independently optionally further substituted by one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₄ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl and 5-10 membered heteroaryl, and the above groups are each independently optionally further substituted by one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₅ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇, and the above groups are each independently optionally further substituted by one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₆ and each R₁₇ are independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and C₁₋₁₀ alkanoyl, and the above groups are each independently optionally further substituted by one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, monoC₁₋₁₀ alkylamino, diC₁₋₁₀ alkylamino and C₁₋₁₀ alkanoyl;
or, R₁₆ and R₁₇, together with the nitrogen atom to which they are directly attached, form a 4-10 membered heterocyclyl or 5-10 membered heteroaryl, and the 4-10 membered heterocyclyl or 5-10 membered heteroaryl is optionally further substituted by one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, monoC₁₋₁₀ alkylamino, diC₁₋₁₀ alkylamino and C₁₋₁₀ alkanoyl;
m is 0, 1 or 2;
n is 0, 1 or 2;
p is 0, 1, 2, 3, 4, 5 or 6; and
each r is independently 0, 1 or 2.

2. The compound of formula (I), the stereoisomer or pharmaceutically acceptable salt thereof according to claim 1, wherein X is CR₉ or N;
R₁ is selected from the group consisting of hydrogen, deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₂ is selected from the group consisting of hydrogen, deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₃ is selected from the group consisting of hydrogen, C₁₋₄ alkyl, C₂₋₄ alkenyl, C₃₋₆ cycloalkyl, 3-6 membered heterocyclyl, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-C(O)R₁₅, -C₀₋₄ alkyl-O-C(O)R₁₅, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₄ is selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₅ₐ is selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-NH-S(O)₂R₁₃, -C₀₋₄ alkyl-NH-S(O)₂NR₁₆R₁₇, -C₀₋₄ alkyl-S(O)ᵣR₁₃, -C₀₋₄ alkyl-S(O)ᵣNR₁₆R₁₇, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R_{5b} and R_{5c} are each independently selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-NH-S(O)₂R₁₃, -C₀₋₄ alkyl-NH-S(O)₂NR₁₆R₁₇, -C₀₋₄ alkyl-S(O)ᵣR₁₃, -C₀₋₄ alkyl-S(O)ᵣNR₁₆R₁₇, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, provided that R₅ₐ, R_{5b} and R_{5c} are not hydrogen at the same time;
or, R_{5b} and R_{5c}, together with the carbon atom to which they are directly attached, form a C(O), C₃₋₆ cycloalkyl or 3-6 membered heterocyclyl, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, wherein R₅ₐ is defined as above;
or, R_{5b} and R_{5c}, together with the carbon atom to which they are directly attached, form 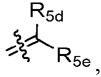 wherein R₅ₐ is defined as above;
or, R₅ₐ, R_{5b} and R_{5c}, together with the carbon atom to which they are directly attached, form 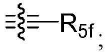
R_{5d}, R₅ₑ and R_{5f} are each independently selected from the group consisting of halogen, cyano, C₁₋₄ alkyl, C₃₋₆ cycloalkyl, 3-6 membered heterocyclyl and -C₀₋₄ alkyl-NR₁₆R₁₇, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl and 5-8 membered heteroaryl;
each R₆ and each R₇ are independently selected from the group consisting of hydrogen, deuterium, halogen, cyano, nitro, azido, C₁₋₄ alkyl, C₁₋₄ haloalkyl, C₁₋₄ deuterioalkyl, C₁₋₄ alkylene, C₁₋₄ haloalkylene, C₁₋₄ deuterioalkylene, C₂₋₄ alkenyl, C₂₋₄ alkynyl, C₃₋₆ cycloalkyl, C₃₋₆ cycloalkylene, C₃₋₆ halocycloalkylene, C₃₋₆ deuteriocycloalkylene, 3-6 membered heterocyclyl, C₃₋₆ heterocycloalkylene, C₃₋₆ haloheterocycloalkylene, C₃₋₆ deuterioheterocycloalkylene, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, provided that at least one of R₆ or R₇ is C₁₋₄ alkylene, C₁₋₄ haloalkylene, C₁₋₄ deuterioalkylene, C₃₋₆ cycloalkylene, C₃₋₆ halocycloalkylene, C₃₋₆ deuteriocycloalkylene, C₃₋₆ heterocycloalkylene, C₃₋₆ haloheterocycloalkylene or C₃₋₆ deuterioheterocycloalkylene;
each R₈ is independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, or, when p≥2, two adjacent R₈, together with the moiety to which they are directly attached, form a C₅₋₁₀ cycloalkyl or 5-10 membered heterocyclyl, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₉ is selected from the group consisting of hydrogen, deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
wherein, R₁₀, R₁₁, R₁₂, R₁₃, R₁₄, R₁₅, R₁₆, R₁₇ and r are defined as in claim 1.

3. The compound of formula (I), the stereoisomer or pharmaceutically acceptable salt thereof according to claim 1, wherein each R₁₀ is independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-C(O)OR₁₄, -C₀₋₄ alkyl-C(O)R₁₅ and -C₀₋₄ alkyl-C(O)NR₁₆R₁₇, and the above groups are each independently optionally further substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
each R₁₁ and each R₁₂ are independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, or, R₁₁ and R₁₂, together with the sulfur atom to which they are directly attached, form a 3-6 membered heterocyclyl, and the above groups are each independently optionally further substituted by one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
each R₁₃ is independently selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, 5-8 membered heteroaryl and -NR₁₆R₁₇, and the above groups are each independently optionally further substituted by one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₄ is independently selected from the group consisting of hydrogen, deuterium, C₁₋₄ alkyl, C₂₋₄ alkenyl, C₃₋₆ cycloalkyl, 3-6 membered heterocyclyl, C₆₋₈ aryl and 5-8 membered heteroaryl, and the above groups are each independently optionally further substituted by one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₅ is independently selected from the group consisting of hydrogen, deuterium, hydroxy, C₁₋₄ alkyl, C₁₋₄ alkoxy, C₂₋₄ alkenyl, C₂₋₄ alkynyl, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇, and the above groups are each independently optionally further substituted by one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₆ and each R₁₇ are independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and C₁₋₄ alkanoyl, and the above groups are independently optionally further substituted by one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, monoC₁₋₄ alkylamino, diC₁₋₄ alkylamino and C₁₋₄ alkanoyl;
or, R₁₆ and R₁₇, together with the nitrogen atom to which they are directly attached, form a 4-8 membered heterocyclyl or 5-8 membered heteroaryl, and the 4-8 membered heterocyclyl or 5-8 membered heteroaryl is optionally further substituted by one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, monoC₁₋₄ alkylamino, diC₁₋₄ alkylamino and C₁₋₄ alkanoyl,
each r is independently 0, 1 or 2.

4. The compound of formula (I), the stereoisomer or pharmaceutically acceptable salt thereof according to claim 1, wherein R₂ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R₃ is selected from the group consisting of hydrogen, C₁₋₄ alkyl, C₂₋₄ alkenyl, C₃₋₆ cycloalkyl, 3-6 membered heterocyclyl, -S(O)ᵣR₁₃, -O-R₁₄, -C(O)OR₁₄, -C(O)SR₁₄, -C(O)R₁₅, -O-C(O)R₁₅, -NR₁₆R₁₇, -C(O)NR₁₆R₁₇ and -N(R₁₆)-C(O)R₁₅, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R₄ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇ and r are defined as in claim 1;
**preferably,** R₂ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
R₃ is selected from the group consisting of hydrogen, C₁₋₄ alkyl, C₂₋₄ alkenyl, C₃₋₆ cycloalkyl, 3-6 membered heterocyclyl, -S(O)ᵣR₁₃, -O-R₁₄, -C(O)OR₁₄, -C(O)SR₁₄, -C(O)R₁₅, -O-C(O)R₁₅, -NR₁₆R₁₇, -C(O)NR₁₆R₁₇ and -N(R₁₆)-C(O)R₁₅, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
R₄ is selected from the group consisting of hydrogen, deuterium, halogen, cyano, C₁₋₄ alkyl, C₂₋₄ alkenyl, C₂₋₄ alkynyl, C₃₋₆ cycloalkyl and 3-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O and =S;
wherein, R₁₀, R₁₁, R₁₂, R₁₃, R₁₄, R₁₅, R₁₆, R₁₇ and r are defined as in claim 1.

5. The compound of formula (I), the stereoisomer or pharmaceutically acceptable salt thereof according to claim 1, wherein the compound of formula (I) is a compound of formula (IIa) or a compound of formula (IIb) as shown below: 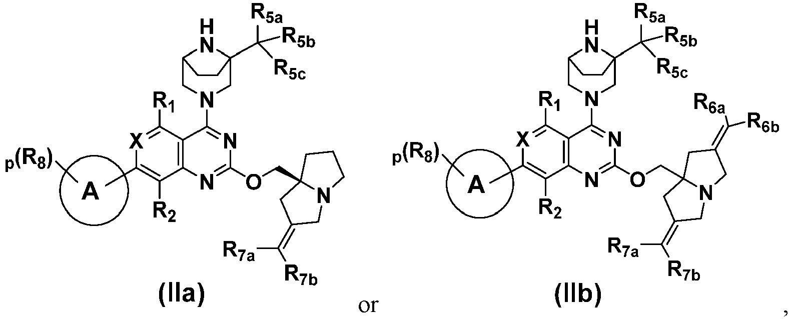
wherein, each X is independently CR₉ or N;
each ring A is independently phenyl, naphthyl, pyridyl, pyrimidinyl, pyridazinyl, quinolinyl, isoquinolinyl, benzothiophenyl or benzothiazolyl;
each R₁ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
each R₂ is independently selected from the group consisting of hydrogen, deuterium, fluorine, chlorine, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
each R₅ₐ is independently selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
each R_{5b} and each R_{5c} are independently selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, provided that R₅ₐ, R_{5b} and R_{5c} are not hydrogen at the same time;
or, R_{5b} and R_{5c}, together with the carbon atom to which they are directly attached, form a C(O), C₃₋₆ cycloalkyl or 3-6 membered heterocyclyl, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, wherein R₅ₐ is defined as above;
or, R_{5b} and R_{5c}, together with the carbon atom to which they are directly attached, form 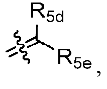 wherein R₅ₐ is defined as above;
or, R₅ₐ, R_{5b} and R_{5c}, together with the carbon atom to which they are directly attached, form 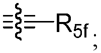
each R_{5d} and each R₅ₑ are independently selected from the group consisting of hydrogen, halogen, cyano, C₁₋₄ alkyl, C₃₋₆ cycloalkyl, 3-6 membered heterocyclyl and -C₀₋₄ alkyl-NR₁₆R₁₇, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl and 5-8 membered heteroaryl, provided that R_{5d} and R₅ₑ are not hydrogen at the same time;
each R_{5f} is independently selected from the group consisting of halogen, cyano, C₁₋₄ alkyl, C₃₋₆ cycloalkyl, 3-6 membered heterocyclyl and -C₀₋₄ alkyl-NR₁₆R₁₇, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl and 5-8 membered heteroaryl;
in the compound of formula (IIb), R₆ₐ and R_{6b} are each independently selected from the group consisting of hydrogen, deuterium, halogen, C₁₋₃ alkyl, C₁₋₃ haloalkyl and C₁₋₃ deuterioalkyl, or, R₆ₐ and R_{6b}, together with the carbon atom to which they are directly attached, form a C₃₋₆ cycloalkyl or 3-6 membered heterocyclyl, and the C₃₋₆ cycloalkyl or 3-6 membered heterocyclyl is independently optionally further substituted by one or more substituents selected from the group consisting of deuterium or halogen;
each R₇ₐ and each R_{7b} are independently selected from the group consisting of hydrogen, deuterium, halogen, C₁₋₃ alkyl, C₁₋₃ haloalkyl and C₁₋₃ deuterioalkyl;
or, R₇ₐ and R_{7b}, together with the carbon atom to which they are directly attached, form a C₃₋₆ cycloalkyl or 3-6 membered heterocyclyl, and the C₃₋₆ cycloalkyl or 3-6 membered heterocyclyl is independently optionally further substituted by one or more substituents which are deuterium or halogen;
each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, or, when p≥2, two adjacent R₈, together with the moiety to which they are directly attached, form a C₅₋₆ cycloalkyl or 5-6 membered heterocyclyl, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
each R₉ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇, p and r are defined as in claim 1.

6. The compound of formula (I), the stereoisomer or pharmaceutically acceptable salt thereof according to claim 5, wherein R₇ₐ and R_{7b} are each independently selected from the group consisting of hydrogen, deuterium, fluorine, chlorine, bromine, methyl, ethyl, propyl, isopropyl, monofluoromethyl, difluoromethyl, trifluoromethyl, monodeuteriomethyl, dideuteriomethyl and trideuteriomethyl;
or, R₇ₐ and R_{7b}, together with the carbon atom to which they are directly attached, form a C₃₋₆ cycloalkyl or 3-6 membered heterocyclyl, and the C₃₋₆ cycloalkyl or 3-6 membered heterocyclyl is independently optionally further substituted by one or more substituents selected from deuterium, fluorine, chlorine and bromine.

7. The compound of formula (I), the stereoisomer or pharmaceutically acceptable salt thereof according to claim 5, wherein in the compound of formula (IIb), R₆ₐ and R_{6b} are each independently selected from the group consisting of hydrogen, deuterium, fluorine, chlorine, bromine, methyl, ethyl, propyl, isopropyl, monofluoromethyl, difluoromethyl, trifluoromethyl, monodeuteriomethyl, dideuteriomethyl and trideuteriomethyl, or, R₆ₐ and R_{6b}, together with the carbon atom to which they are directly attached, form a C₃₋₆ cycloalkyl or 3-6 membered heterocyclyl, and the C₃₋₆ cycloalkyl or 3-6 membered heterocyclyl is independently optionally further substituted by one or more substituents selected from the group consisting of deuterium, fluorine, chlorine and bromine.

8. The compound of formula (I), the stereoisomer or pharmaceutically acceptable salt thereof according to claim 5, wherein X is CR₉ or N;
R₁ is selected from the group consisting of hydrogen, deuterium, halogen, cyano, C₁₋₄ alkyl, C₁₋₄ haloalkyl, C₁₋₄ deuterioalkyl, C₃₋₆ cycloalkyl, 3-6 membered heterocyclyl, -O-R₁₄ and -NR₁₆R₁₇;
R₉ is selected from the group consisting of hydrogen, deuterium, halogen, cyano, C₁₋₄ alkyl, C₁₋₄ haloalkyl, C₁₋₄ deuterioalkyl, C₃₋₆ cycloalkyl, 3-6 membered heterocyclyl, -O-R₁₄ and -NR₁₆R₁₇;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, C₃₋₆ cycloalkyl, 3-6 membered heterocyclyl, =O, =S, -O-R₁₄ and -NR₁₆R₁₇;
wherein, R₁₄, R₁₆ and R₁₇ are defined as in claim 5;
**preferably,** X is CR₉ or N;
R₁ is selected from the group consisting of hydrogen, deuterium, fluorine, chlorine, bromine, cyano, methyl, ethyl, propyl, isopropyl, butyl, isobutyl, monofluoromethyl, difluoromethyl, trifluoromethyl, monodeuteriomethyl, dideuteriomethyl, trideuteriomethyl, cyclopropyl, cyclobutyl, cyclopentyl, cyclohexyl, oxacyclopropyl, azacyclopropyl, oxacyclobutyl, azacyclobutyl, methoxy, ethoxy, propoxy, trideuteriomethoxy, isopropoxy, butoxy, cyclopropyloxy, cyclobutyloxy, monomethylamino, dimethylamino and amino;
R₉ is selected from the group consisting of hydrogen, deuterium, fluorine, chlorine, bromine, cyano, methyl, ethyl, propyl, isopropyl, butyl, isobutyl, monofluoromethyl, difluoromethyl, trifluoromethyl, monodeuteriomethyl, dideuteriomethyl, trideuteriomethyl, cyclopropyl, cyclobutyl, cyclopentyl, cyclohexyl, oxacyclopropyl, azacyclopropyl, oxacyclobutyl, azacyclobutyl, methoxy, ethoxy, propoxy, isopropoxy, butoxy, cyclopropyloxy, cyclobutyloxy, monomethylamino, dimethylamino and amino;
or, R₉ and R₁, together with the moiety to which they are directly attached, form an oxacyclohexyl, and the oxacyclohexyl is optionally further substituted by one or more substituents selected from the group consisting of deuterium, fluorine, chlorine, bromine, cyano, methyl, ethyl, propyl, isopropyl, butyl, isobutyl, monofluoromethyl, difluoromethyl, trifluoromethyl, monodeuteriomethyl, dideuteriomethyl, trideuteriomethyl, cyclopropyl, cyclobutyl, cyclopentyl, cyclohexyl, oxacyclopropyl, azacyclopropyl, oxacyclobutyl, azacyclobutyl, =O, =S, methoxy, ethoxy, propoxy, isopropoxy, butoxy, cyclopropyloxy, cyclobutyloxy, monomethylamino, dimethylamino and amino.

9. The compound of formula (I), the stereoisomer or pharmaceutically acceptable salt thereof according to claim 5, wherein each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇ and r are defined as in claim 5;
**preferably,** each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -O-R₁₄ and -NR₁₆R₁₇;
wherein, R₁₄, R₁₆ and R₁₇ are defined as in claim 5;
**more preferably,** each R₈ is independently selected from the group consisting of hydrogen, deuterium, fluorine, chlorine, bromine, cyano, hydroxy, methyl, ethyl, propyl, isopropyl, butyl, isobutyl, vinyl, ethynyl, cyclopropyl, cyclobutyl, cyclopentyl, cyclohexyl, oxacyclopropyl, azacyclopropyl, oxacyclobutyl, azacyclobutyl, methoxy, ethoxy, propoxy, isopropoxy, butoxy, cyclopropyloxy, cyclobutyloxy, monomethylamino, dimethylamino and amino, and the above groups are each independently optionally further substituted by one or more substituents selected from the group consisting of deuterium, fluorine, chlorine, bromine, cyano, hydroxy, methyl, ethyl, propyl, isopropyl, butyl, isobutyl, vinyl, ethynyl, monofluoromethyl, difluoromethyl, trifluoromethyl, monodeuteriomethyl, dideuteriomethyl, trideuteriomethyl, cyclopropyl, cyclobutyl, cyclopentyl, cyclohexyl, oxacyclopropyl, azacyclopropyl, oxacyclobutyl, azacyclobutyl, =O, =S, methoxy, ethoxy, propoxy, isopropoxy, butoxy, cyclopropyloxy, cyclobutyloxy, monomethylamino, dimethylamino and amino.

10. The compound of formula (I), the stereoisomer or pharmaceutically acceptable salt thereof according to claim 5, wherein R₅ₐ is selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R_{5b} and R_{5c} are each independently selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇ and r are defined as in claim 5;
**preferably,** R₅ₐ is selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, -O-R₁₄ and -C(O)NR₁₆R₁₇, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O and =S;
R_{5b} and R_{5c} are each independently hydrogen, deuterium, halogen or C₁₋₄ alkyl, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S and -O-R₁₄;
wherein, R₁₄, R₁₆ and R₁₇ are defined as in claim 5.

11. The compound of formula (I), the stereoisomer or pharmaceutically acceptable salt thereof according to claim 5, wherein R₅ₐ is selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R_{5b} and R_{5c}, together with the carbon atom to which they are directly attached, form a C(O), C₃₋₆ cycloalkyl or 3-6 membered heterocyclyl, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇ and r are defined as in claim 5;
**preferably,** R₅ₐ is selected from the group consisting of hydrogen, deuterium, halogen, C₁₋₄ alkyl and -O-R₁₄, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, and -O-R₁₄;
R_{5b} and R_{5c}, together with the carbon atom to which they are directly attached, form a C(O), C₃₋₆ cycloalkyl or 3-6 membered heterocyclyl, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -O-R₁₄ and -NR₁₆R₁₇,
wherein, R₁₄, R₁₆ and R₁₇ are defined as in claim 5.

12. The compound of formula (I), the stereoisomer or pharmaceutically acceptable salt thereof according to claim 5, wherein R₅ₐ is selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R_{5b} and R_{5c}, together with the carbon atom to which they are directly attached, form 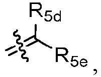
R_{5d} and R₅ₑ are each independently selected from the group consisting of hydrogen, halogen, cyano, C₁₋₄ alkyl, C₃₋₆ cycloalkyl, 3-6 membered heterocyclyl and -C₀₋₄ alkyl-NR₁₆R₁₇, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl and 5-8 membered heteroaryl, provided that R_{5d} and R₅ₑ are not hydrogen at the same time;
wherein, R₁₀, R₁₁, R₁₂, R₁₃, R₁₄, R₁₅, R₁₆, R₁₇ and r are defined as in claim 5;
**preferably,** R₅ₐ is selected from the group consisting of hydrogen, deuterium, halogen and C₁₋₄ alkyl, and the above groups are each independently optionally further substituted by one or more substituents selected from the group consisting of deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, and -O-R₁₄;
R_{5b} and R_{5c}, together with the carbon atom to which they are directly attached, form 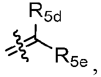
R_{5d} and R₅ₑ are each independently selected from the group consisting of hydrogen, halogen, cyano, C₁₋₄ alkyl, C₃₋₆ cycloalkyl, 3-6 membered heterocyclyl and -C₀₋₄ alkyl-NR₁₆R₁₇, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl and 5-8 membered heteroaryl, provided that R_{5d} and R₅ₑ are not hydrogen at the same time;
wherein, R₁₄, R₁₆ and R₁₇ are defined as in claim 5.

13. The compound of formula (I), the stereoisomer or pharmaceutically acceptable salt thereof according to claim 5, wherein R₅ₐ, R_{5b} and R_{5c}, together with the carbon atom to which they are directly attached, form 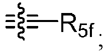
R_{5f} is selected from the group consisting of halogen, cyano, C₁₋₄ alkyl, C₃₋₆ cycloalkyl, 3-6 membered heterocyclyl and -C₀₋₄ alkyl-NR₁₆R₁₇, and the above groups are each independently optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl and 5-8 membered heteroaryl;
wherein, R₁₆ and R₁₇ are defined as in claim 5.

14. The compound of formula (I), the stereoisomer or pharmaceutically acceptable salt thereof according to claim 1, wherein the compound of formula (I) is a compound of formula (IIIa1) or a compound of formula (IIIb1) as shown below: 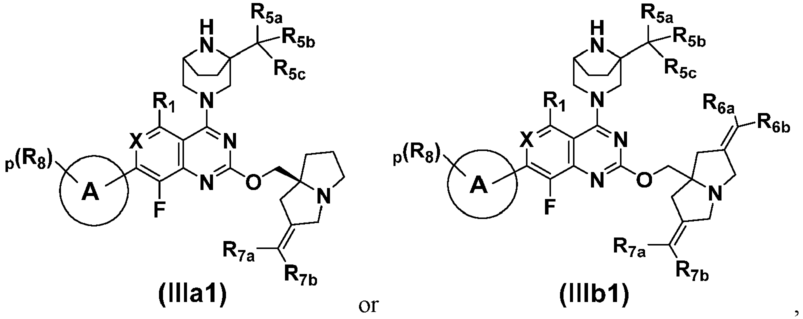
wherein, each X is independently CR₉ or N;
each ring A is independently phenyl, naphthyl, pyridyl, pyrimidinyl, pyridazinyl, quinolinyl, isoquinolinyl, benzothiophenyl or benzothiazolyl;
each R₁ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
each 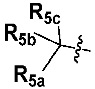 is independently selected from the group consisting of deuteriomethyl, ethyl, isopropyl, methoxymethyl, cyclopropyloxymethyl and 1-hydroxy-1-methyl-ethyl, and the ethyl, isopropyl, methoxymethyl, cyclopropyloxymethyl or 1-hydroxy-1-methyl-ethyl is each independently optionally further substituted by one or more deuterium(s);
in the compound of formula (IIIb 1), R₆ₐ and R_{6b} are each independently selected from the group consisting of hydrogen, deuterium, fluorine, C₁₋₃ alkyl, C₁₋₃ haloalkyl and C₁₋₃ deuterioalkyl;
each R₇ₐ and each R_{7b} are independently selected from the group consisting of hydrogen, deuterium, fluorine, C₁₋₃ alkyl, C₁₋₃ haloalkyl and C₁₋₃ deuterioalkyl;
each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
each R₉ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
wherein, R₁₄, R₁₆, R₁₇ and p are defined as in claim 1.

15. The compound of formula (I), the stereoisomer or pharmaceutically acceptable salt thereof according to claim 14, wherein each 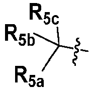 is independently selected from the group consisting of deuteriomethyl, ethyl, isopropyl, cyclopropyloxymethyl and 1-hydroxy-1-methyl-ethyl, and the ethyl, isopropyl, cyclopropyloxymethyl or 1-hydroxy-1-methyl-ethyl is each independently further substituted by 1, 2, 3, 4, 5, 6 or 7 deuterium(s).

16. The compound of formula (I), the stereoisomer or pharmaceutically acceptable salt thereof according to claim 14, wherein each 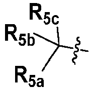 is independently selected from the group consisting of -CH₂D, -CHD₂, -CD₃, 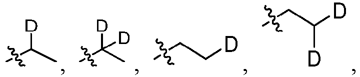 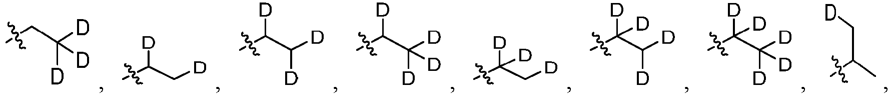 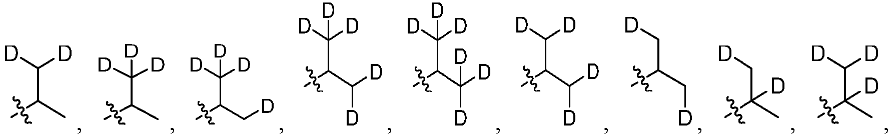 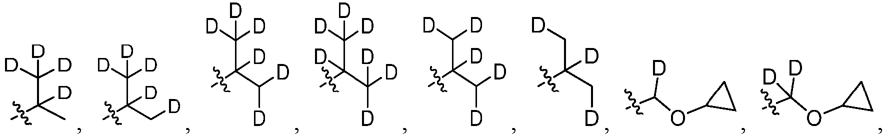 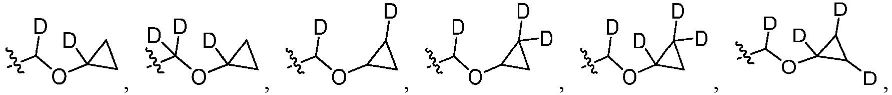 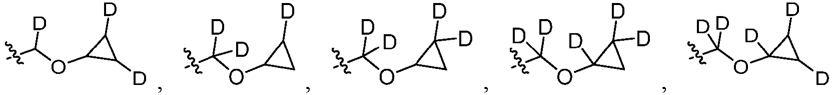 and

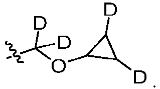

17. The compound of formula (I), the stereoisomer or pharmaceutically acceptable salt thereof according to claim 14, wherein each 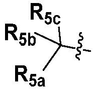 is independently selected from the group consisting of -CH₂D, -CHD₂, -CD₃, 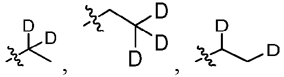 and

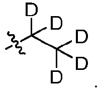

18. The compound of formula (I), the stereoisomer or pharmaceutically acceptable salt thereof according to claim 14, wherein each 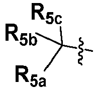 is independently ethyl or isopropyl.

19. The compound of formula (I), the stereoisomer or pharmaceutically acceptable salt thereof according to claim 14, wherein each 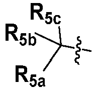 is independently methoxymethyl, and the methoxymethyl is optionally further substituted by one or more deuterium(s); provided that the compound of formula (I), the stereoisomer or pharmaceutically acceptable salt thereof is not the following compound:

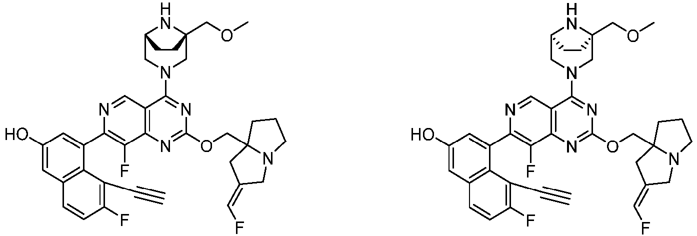

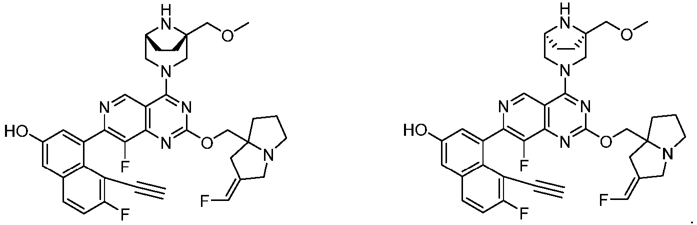

20. The compound of formula (I), the stereoisomer or pharmaceutically acceptable salt thereof according to claim 19, wherein each 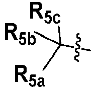 is independently methoxymethyl, and the methoxymethyl is optionally further substituted by 1, 2, 3, 4 or 5 deuterium(s).

21. The compound of formula (I), the stereoisomer or pharmaceutically acceptable salt thereof according to claim 19, wherein each 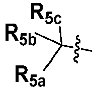 is independently selected from the group consisting of 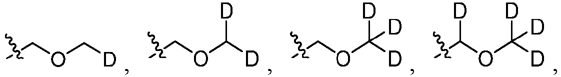 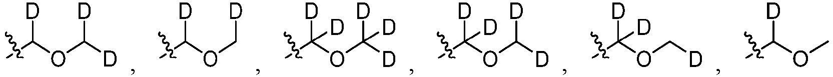 and

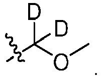

22. The compound of formula (I), the stereoisomer or pharmaceutically acceptable salt thereof according to claim 19, wherein each 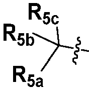 is independently

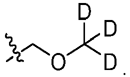

23. The compound of formula (I), the stereoisomer or pharmaceutically acceptable salt thereof according to claim 14, wherein each X is independently CR₉ or N;
each R₁ is independently selected from the group consisting of methoxy, ethoxy, isopropoxy, cyclopropyloxy, trideuteriomethoxy, monomethylamino, dimethylamino and amino;
each R₉ is independently selected from the group consisting of hydrogen, fluorine, chlorine, methyl, monofluoromethyl, difluoromethyl, trifluoromethyl, monodeuteriomethyl, dideuteriomethyl and trideuteriomethyl.

24. The compound of formula (I), the stereoisomer or pharmaceutically acceptable salt thereof according to claim 14, wherein each X is independently CR₉, and R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇,
wherein, R₁₄, R₁₆ and R₁₇ are defined as in claim 1.

25. The compound of formula (I), the stereoisomer or pharmaceutically acceptable salt thereof according to claim 1, wherein the compound of formula (I) is a compound of formula (IIIa2) or a compound of formula (IIIb2) as shown below: 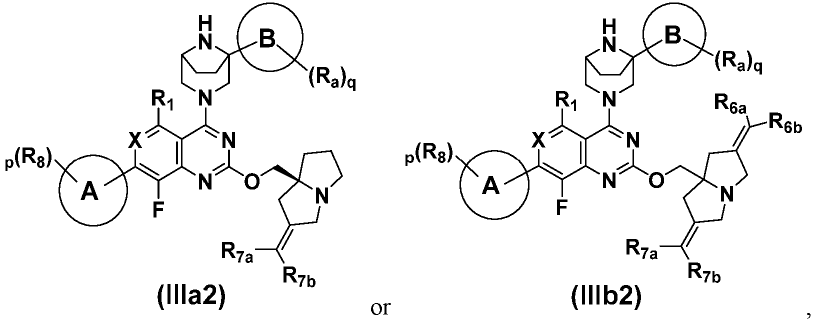
wherein, each X is independently CR₉ or N;
each ring A is independently phenyl, naphthyl, pyridyl, pyrimidinyl, pyridazinyl, quinolinyl, isoquinolinyl, benzothiophenyl or benzothiazolyl;
each ring B is independently selected from the group consisting of oxacyclobutyl, oxacyclopentyl and oxacyclohexyl;
each Rₐ is independently selected from the group consisting of hydrogen, deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -O-R₁₄ and -NR₁₆R₁₇;
each R₁ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
in the compound of formula (IIIb2), R₆ₐ and R_{6b} are each independently selected from the group consisting of hydrogen, deuterium, fluorine, C₁₋₃ alkyl, C₁₋₃ haloalkyl and C₁₋₃ deuterioalkyl;
each R₇ₐ and each R_{7b} are independently selected from the group consisting of hydrogen, deuterium, fluorine, C₁₋₃ alkyl, C₁₋₃ haloalkyl and C₁₋₃ deuterioalkyl;
each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
each R₉ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
q is 0, 1, 2 or 3;
wherein, R₁₄, R₁₆, R₁₇ and p are defined as in claim 1.

26. The compound of formula (I), the stereoisomer or pharmaceutically acceptable salt thereof according to claim 25, wherein each ring B is independently oxacyclobutyl or oxacyclohexyl;
each Rₐ is independently selected from the group consisting of hydrogen, deuterium, fluorine, chlorine, methyl, ethyl, propyl, isopropyl, monofluoromethyl, difluoromethyl, trifluoromethyl, monodeuteriomethyl, dideuteriomethyl and trideuteriomethyl.

27. The compound of formula (I), the stereoisomer or pharmaceutically acceptable salt thereof according to claim 1, wherein the compound of formula (I) is a compound of formula (IIIa3) or a compound of formula (IIIb3) as shown below: 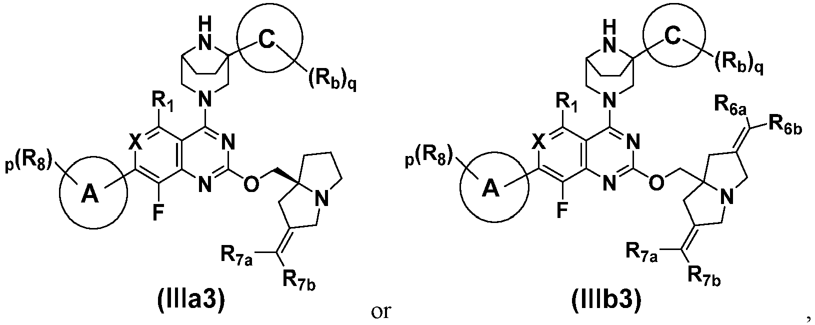
wherein, each X is independently CR₉ or N;
each ring A is independently phenyl, naphthyl, pyridyl, pyrimidinyl, pyridazinyl, quinolinyl, isoquinolinyl, benzothiophenyl or benzothiazolyl;
each ring C is independently selected from the group consisting of cyclopropyl, cyclobutyl, cyclopentyl and cyclohexyl;
each R_{b} is independently selected from the group consisting of hydrogen, deuterium, halogen, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -O-R₁₄ and -NR₁₆R₁₇;
each R₁ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
in the compound of formula (IIIb3), R₆ₐ and R_{6b} are each independently selected from the group consisting of hydrogen, deuterium, fluorine, C₁₋₃ alkyl, C₁₋₃ haloalkyl and C₁₋₃ deuterioalkyl;
each R₇ₐ and each R_{7b} are independently selected from the group consisting of hydrogen, deuterium, fluorine, C₁₋₃ alkyl, C₁₋₃ haloalkyl and C₁₋₃ deuterioalkyl;
each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
each R₉ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
q is 0, 1, 2 or 3;
wherein, R₁₄, R₁₆, R₁₇ and p are defined as in claim 1.

28. The compound of formula (I), the stereoisomer or pharmaceutically acceptable salt thereof according to claim 27, wherein each ring C is independently cyclopropyl;
each R_{b} is independently selected from the group consisting of hydrogen, deuterium, fluorine, chlorine, methyl, ethyl, propyl, isopropyl, monofluoromethyl, difluoromethyl, trifluoromethyl, monodeuteriomethyl, dideuteriomethyl and trideuteriomethyl.

29. The compound of formula (I), the stereoisomer or pharmaceutically acceptable salt thereof according to claim 1, wherein the compound of formula (I) is a compound of formula (IIIa4) or a compound of formula (IIIb4) as shown below: 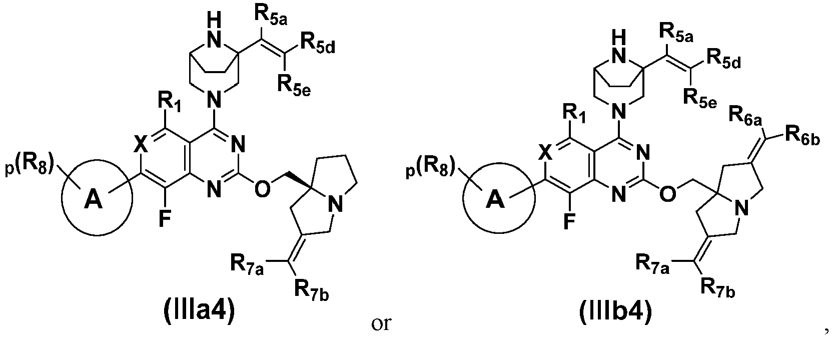
wherein, each X is independently CR₉ or N;
each ring A is independently phenyl, naphthyl, pyridyl, pyrimidinyl, pyridazinyl, quinolinyl, isoquinolinyl, benzothiophenyl or benzothiazolyl;
each R₁ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
R₅ₐ is selected from the group consisting of hydrogen, fluorine and methyl;
R_{5d} is selected from the group consisting of hydrogen, fluorine and methyl;
R₅ₑ is selected from the group consisting of hydrogen, fluorine and methyl;
in the compound of formula (IIIb4), R₆ₐ and R_{6b} are each independently selected from the group consisting of hydrogen, deuterium, fluorine, C₁₋₃ alkyl, C₁₋₃ haloalkyl and C₁₋₃ deuterioalkyl;
each R₇ₐ and each R_{7b} are independently selected from the group consisting of hydrogen, deuterium, fluorine, C₁₋₃ alkyl, C₁₋₃ haloalkyl and C₁₋₃ deuterioalkyl;
each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
each R₉ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
wherein, R₁₄, R₁₆, R₁₇ and p are defined as in claim 1.

30. The compound of formula (I), the stereoisomer or pharmaceutically acceptable salt thereof according to claim 29, wherein R₅ₐ is hydrogen; R_{5d} is fluorine; R₅ₑ is hydrogen or fluorine.

31. The compound of formula (I), the stereoisomer or pharmaceutically acceptable salt thereof according to claim 1, wherein the compound of formula (I) is a compound of formula (IIIa5) or a compound of formula (IIIb5) as shown below: 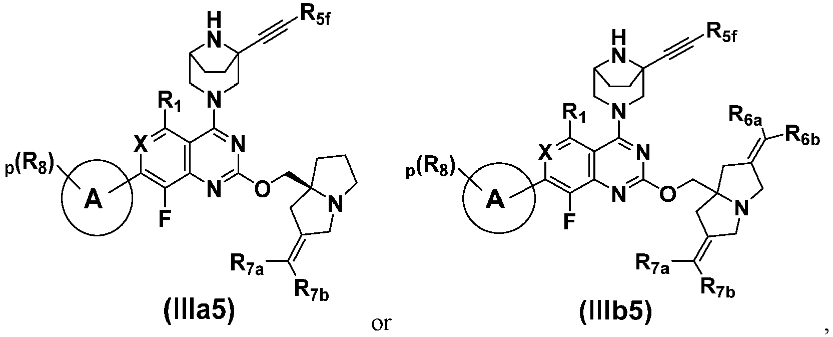
wherein, each X is independently CR₉ or N;
each ring A is independently phenyl, naphthyl, pyridyl, pyrimidinyl, pyridazinyl, quinolinyl, isoquinolinyl, benzothiophenyl or benzothiazolyl;
each R₁ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
R_{5f} is selected from the group consisting of hydrogen, fluorine and methyl;
in the compound of formula (IIIb5), R₆ₐ and R_{6b} are each independently selected from the group consisting of hydrogen, deuterium, fluorine, C₁₋₃ alkyl, C₁₋₃ haloalkyl and C₁₋₃ deuterioalkyl;
each R₇ₐ and each R_{7b} are independently selected from the group consisting of hydrogen, deuterium, fluorine, C₁₋₃ alkyl, C₁₋₃ haloalkyl and C₁₋₃ deuterioalkyl;
each R₈ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -O-R₁₄ and -NR₁₆R₁₇, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
each R₉ is independently selected from the group consisting of hydrogen, deuterium, halogen, cyano, C₁₋₄ alkyl, C₁₋₄ haloalkyl, C₁₋₄ deuterioalkyl, -O-R₁₄ and -NR₁₆R₁₇;
or, R₉ and R₁, together with the moiety to which they are directly attached, form a C₃₋₈ cycloalkyl or 3-8 membered heterocyclyl, and the C₃₋₈ cycloalkyl or 3-8 membered heterocyclyl is independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NR₁₆R₁₇;
wherein, R₁₄, R₁₆, R₁₇ and p are defined as in claim 1.

32. The compound of formula (I), the stereoisomer or pharmaceutically acceptable salt thereof according to claim 31, wherein R_{5f} is methyl.

33. The compound of formula (I), the stereoisomer or pharmaceutically acceptable salt thereof according to claim 14, 19, 25, 27, 29 or 31, wherein each X is independently CR₉ or N;
each R₁ is independently selected from the group consisting of hydrogen, halogen, -O-R₁₄ and -NR₁₆R₁₇;
each R₉ is independently selected from the group consisting of hydrogen, halogen, C₁₋₄ alkyl, C₁₋₄ haloalkyl and C₁₋₄ deuterioalkyl;
or, R₉ and R₁, together with the moiety to which they are directly attached, form a N-containing or O-containing 3-8 membered heterocyclyl;
wherein, R₁₄, R₁₆ and R₁₇ are defined as in claim 1;
**preferably,** each X is independently CR₉ or N;
each R₁ is independently selected from the group consisting of hydrogen, fluorine, chlorine, hydroxy, methoxy, ethoxy, isopropoxy, cyclopropyloxy, trideuteriomethoxy, monomethylamino, dimethylamino and amino;
each R₉ is independently selected from the group consisting of hydrogen, fluorine, chlorine, methyl, monofluoromethyl, difluoromethyl, trifluoromethyl, monodeuteriomethyl, dideuteriomethyl and trideuteriomethyl;
or, R₉ and R₁, together with the moiety to which they are directly attached, form an oxacyclohexyl.

34. The compound of formula (I), the stereoisomer or pharmaceutically acceptable salt thereof according to claim 14, 19, 25, 27, 29 or 31, wherein each ring A is independently selected from the group consisting of phenyl, naphthyl, pyridyl, pyrimidinyl, benzothiophenyl and benzothiazolyl;
each R₈ is independently selected from the group consisting of hydrogen, deuterium, halogen, cyano, C₁₋₄ alkyl, C₂₋₄ alkenyl, C₂₋₄ alkynyl, -O-R₁₄ and -NH₂, and the above groups are each independently optionally further substituted by one or more substituents selected from the group consisting of deuterium, halogen, cyano, C₁₋₄ alkyl, C₁₋₄ haloalkyl, C₁₋₄ deuterioalkyl, =O, -O-R₁₄ and -NH₂;
wherein, R₁₄ is defined as in claim 1.

35. The compound of formula (I), the stereoisomer or pharmaceutically acceptable salt thereof according to claim 14, 19, 25, 27, 29 or 31, wherein each 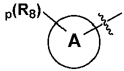 is independently selected from the group consisting of the following structures: 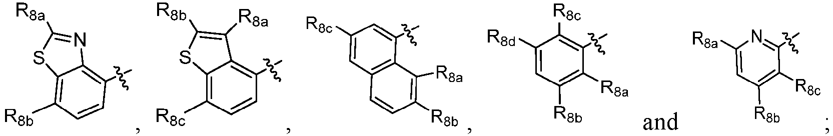 each R₈ₐ, each R_{8b}, each R_{8c} and each R_{8d} are independently selected from the group consisting of hydrogen, deuterium, fluorine, chlorine, bromine, cyano, hydroxy, methyl, ethyl, propyl, isopropyl, butyl, isobutyl, vinyl, ethynyl, cyclopropyl, cyclobutyl, cyclopentyl, cyclohexyl, oxacyclopropyl, azacyclopropyl, oxacyclobutyl, azacyclobutyl, methoxy, ethoxy, propoxy, isopropoxy, butoxy, cyclopropyloxy, cyclobutyloxy, monomethylamino, dimethylamino and amino, and the above groups are each independently optionally further substituted by one or more substituents selected from the group consisting of deuterium, fluorine, chlorine, bromine, cyano, hydroxy, methyl, ethyl, propyl, isopropyl, butyl, isobutyl, vinyl, ethynyl, monofluoromethyl, difluoromethyl, trifluoromethyl, monodeuteriomethyl, dideuteriomethyl, trideuteriomethyl, cyclopropyl, cyclobutyl, cyclopentyl, cyclohexyl, oxacyclopropyl, azacyclopropyl, oxacyclobutyl, azacyclobutyl, =O, methoxy, ethoxy, propoxy, isopropoxy, butoxy, cyclopropyloxy, cyclobutyloxy, monomethylamino, dimethylamino and amino.

36. The compound of formula (I), the stereoisomer or pharmaceutically acceptable salt thereof according to claim 14, 19, 25, 27, 29 or 31, wherein R₆ₐ and R_{6b} are each independently selected from the group consisting of hydrogen, deuterium, fluorine, methyl, ethyl, monofluoromethyl, difluoromethyl, trifluoromethyl, monodeuteriomethyl, dideuteriomethyl and trideuteriomethyl;
**preferably,** R₆ₐ and R_{6b} are each independently hydrogen, deuterium or fluorine.

37. The compound of formula (I), the stereoisomer or pharmaceutically acceptable salt thereof according to claim 14, 19, 25, 27, 29 or 31, wherein each R₇ₐ and each R_{7b} are independently selected from the group consisting of hydrogen, deuterium, fluorine, methyl, ethyl, monofluoromethyl, difluoromethyl, trifluoromethyl, monodeuteriomethyl, dideuteriomethyl and trideuteriomethyl;
**preferably,** each R₇ₐ and each R_{7b} are independently hydrogen, deuterium or fluorine.

38. The compound of formula (I), the stereoisomer or pharmaceutically acceptable salt thereof according to any one of claims 1 to 37, wherein the compound is selected from the following compounds:

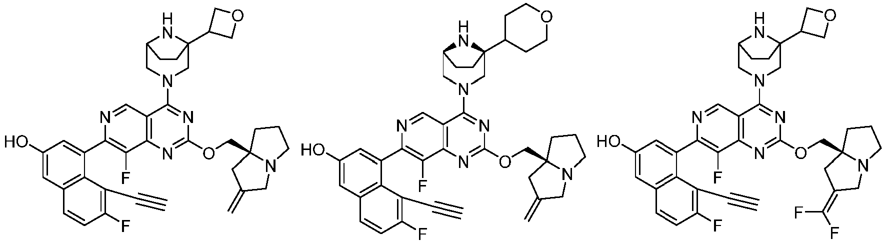

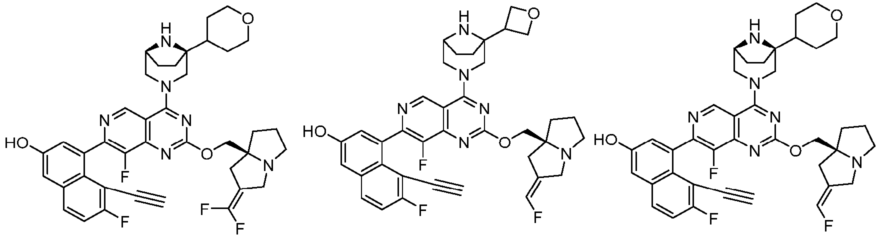

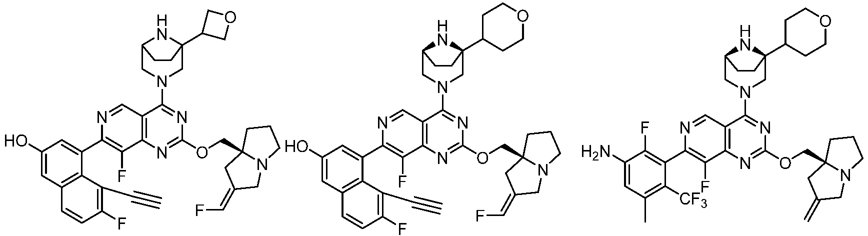

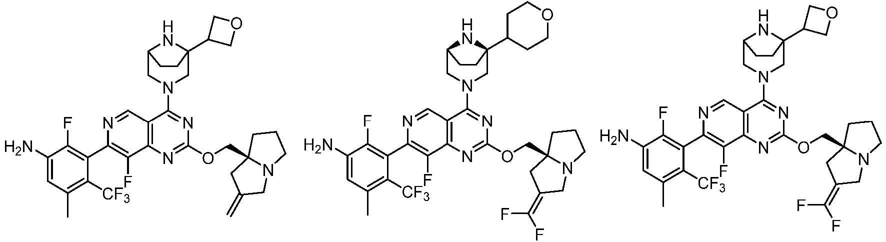

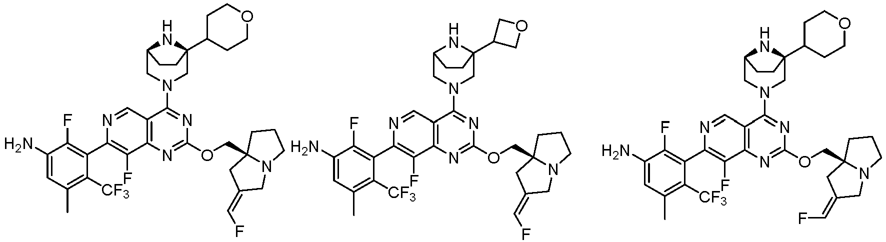

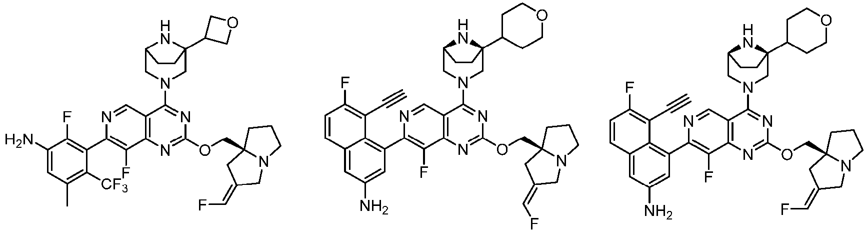

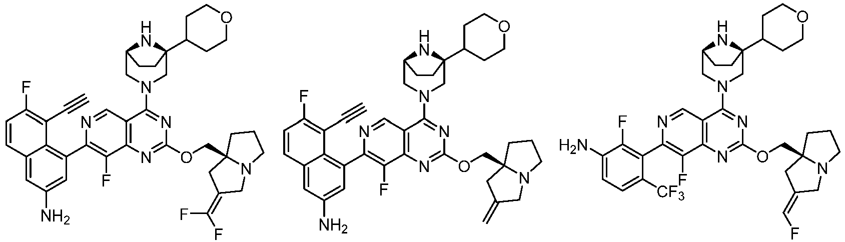

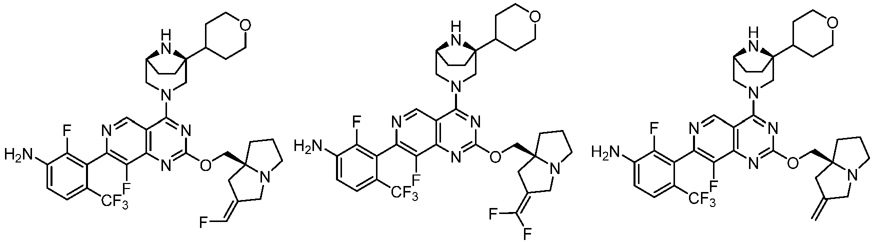

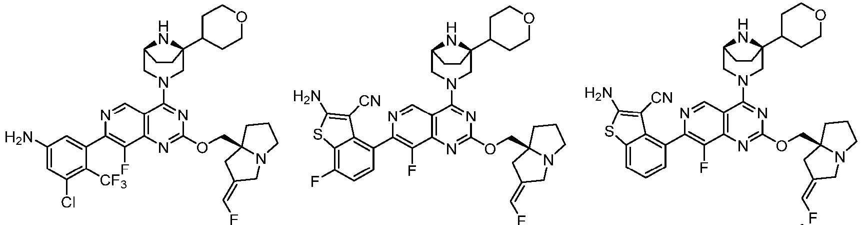

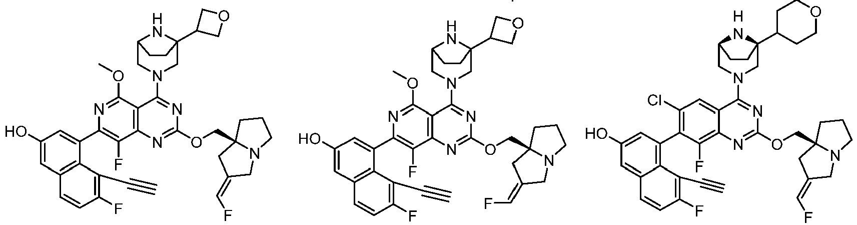

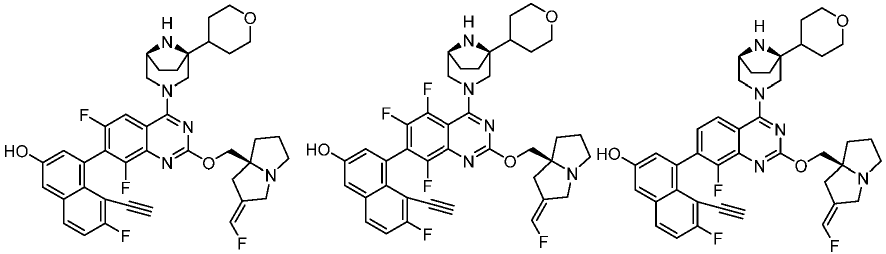

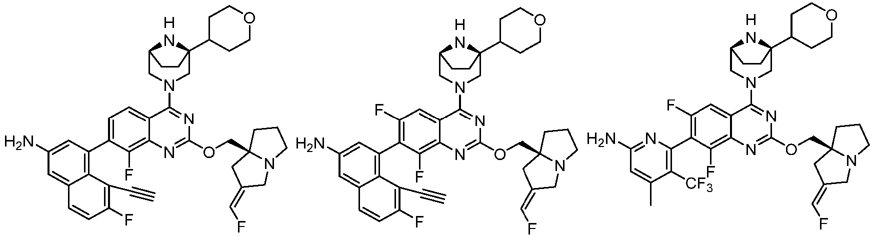

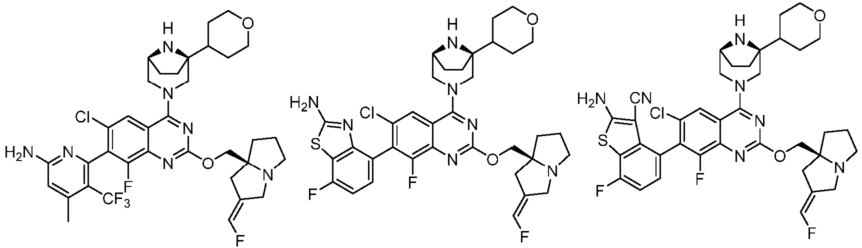

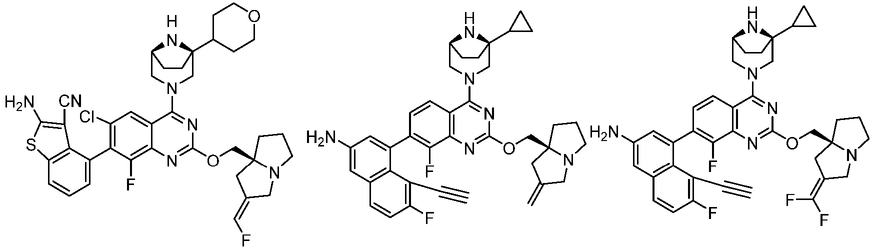

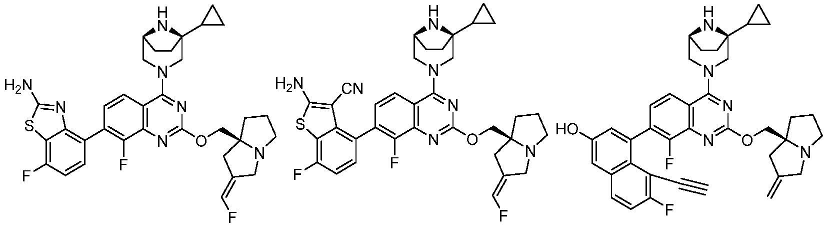

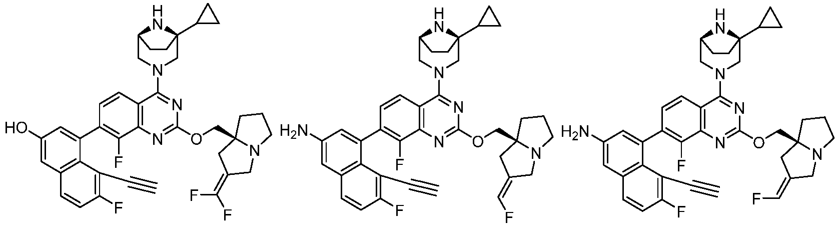

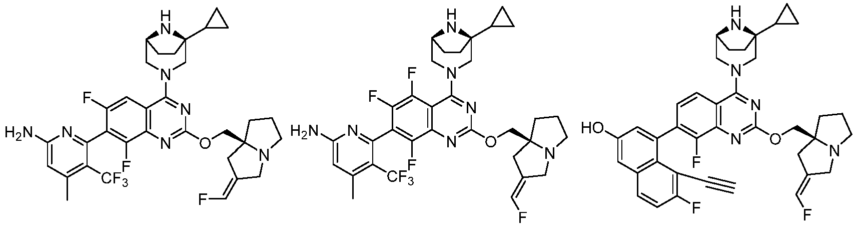

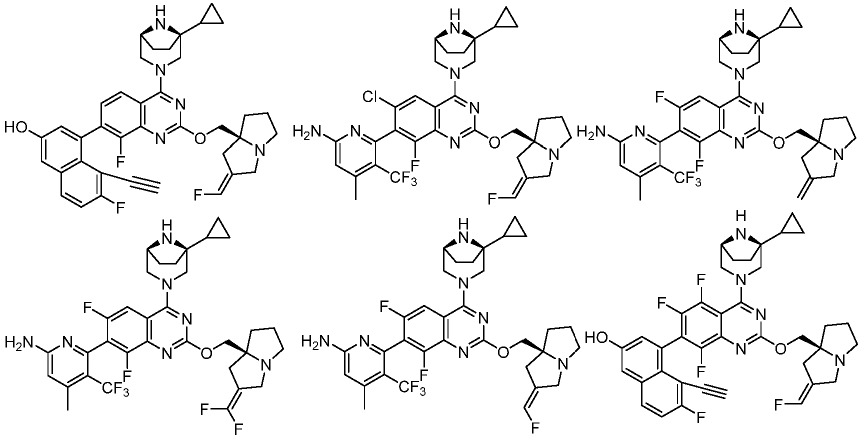

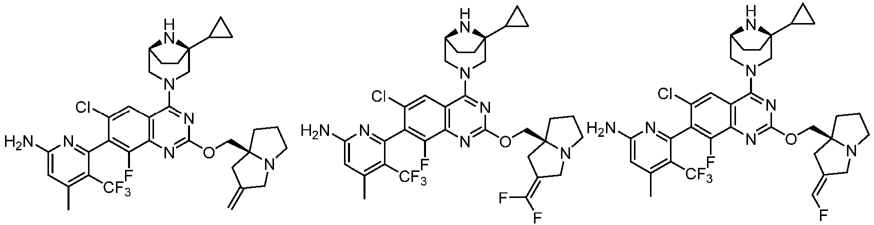

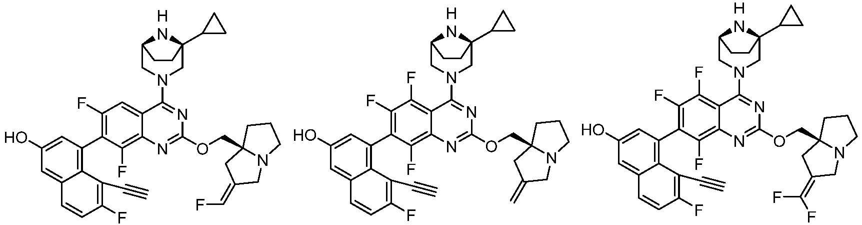

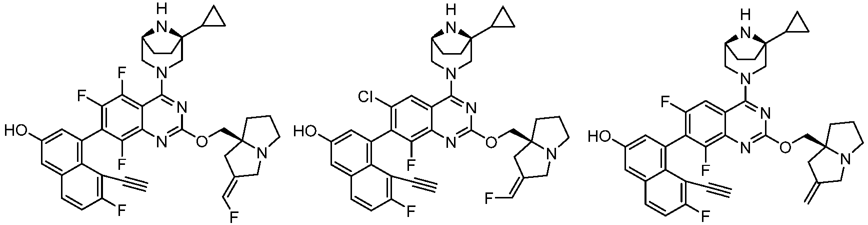

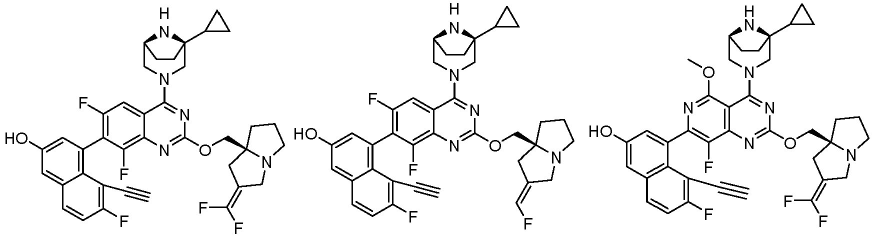

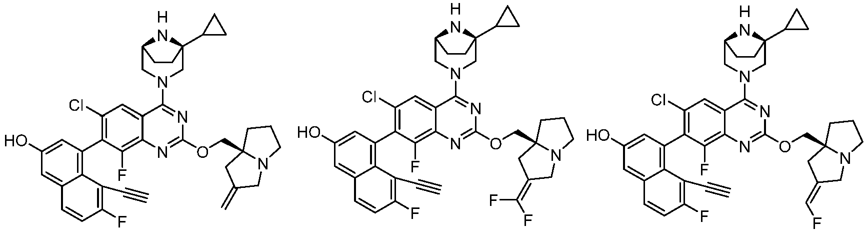

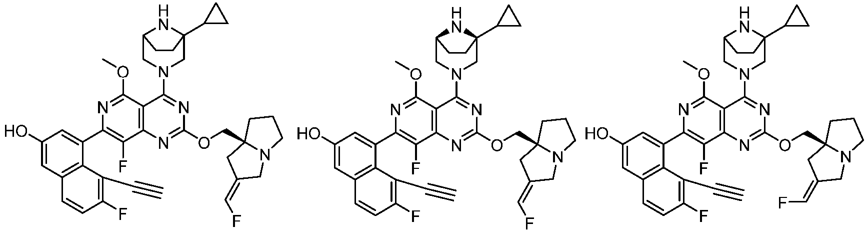

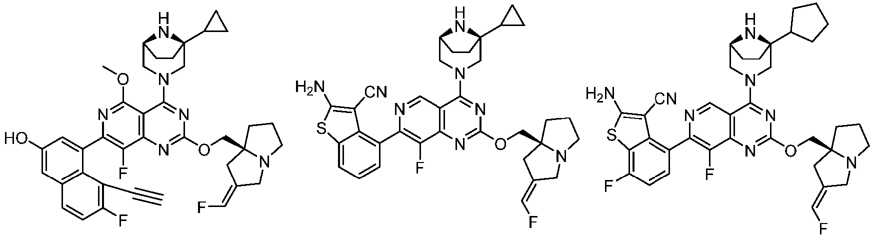

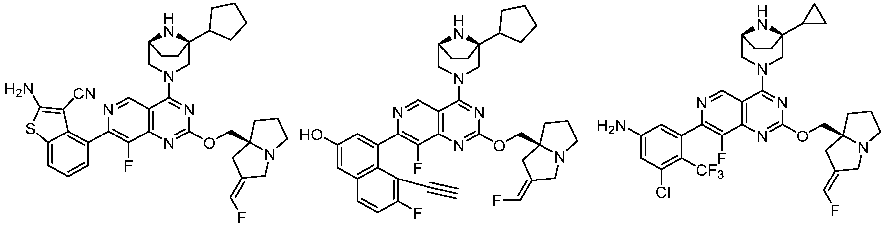

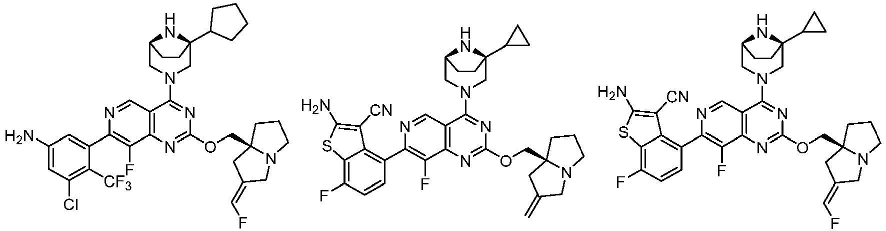

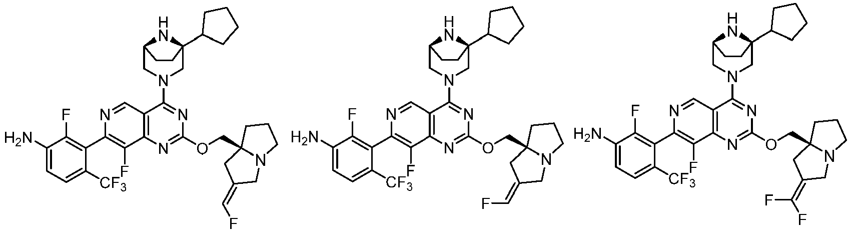

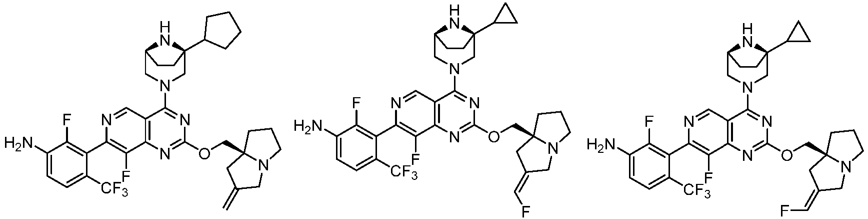

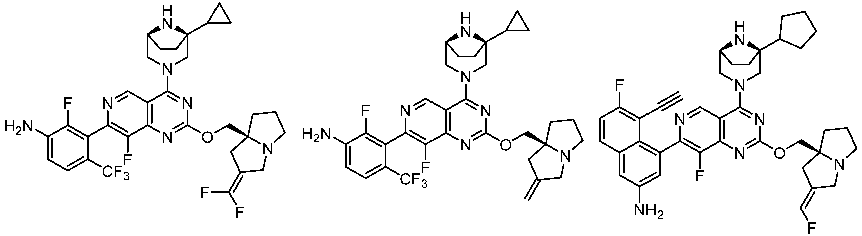

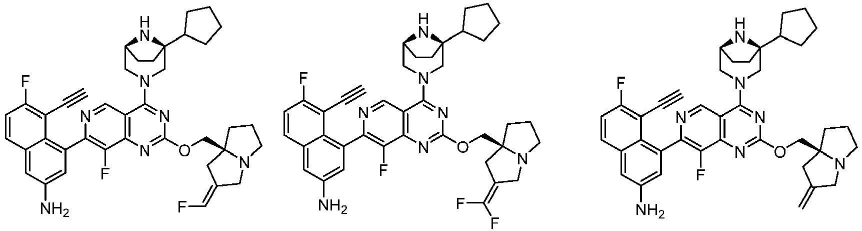

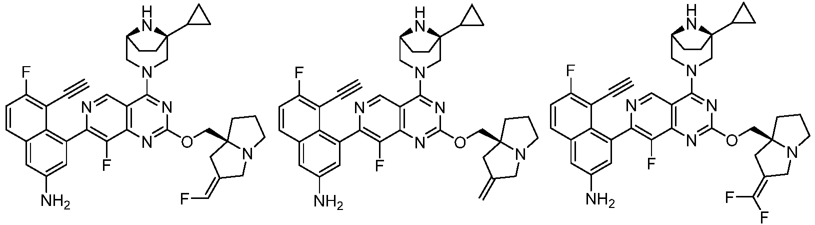

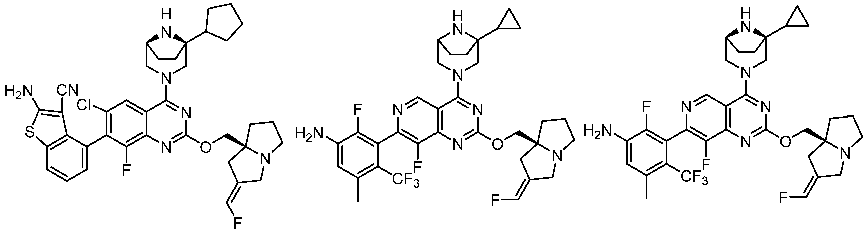

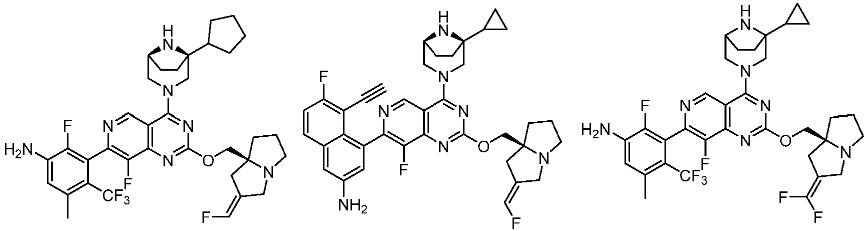

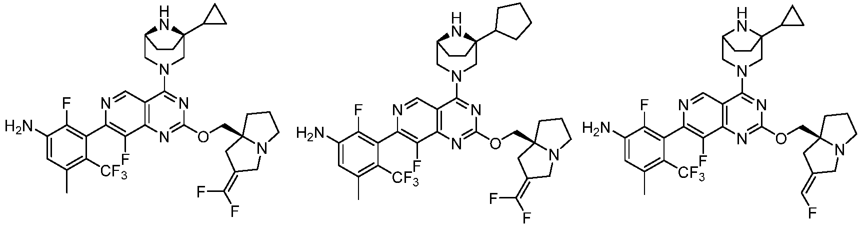

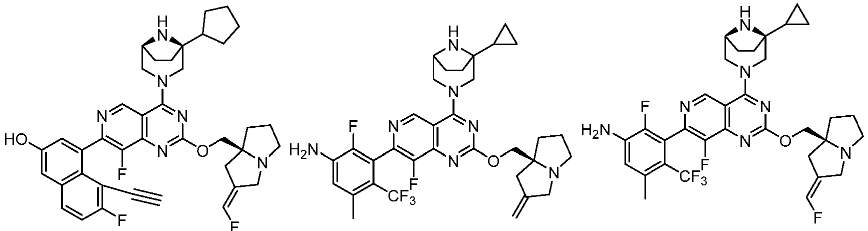

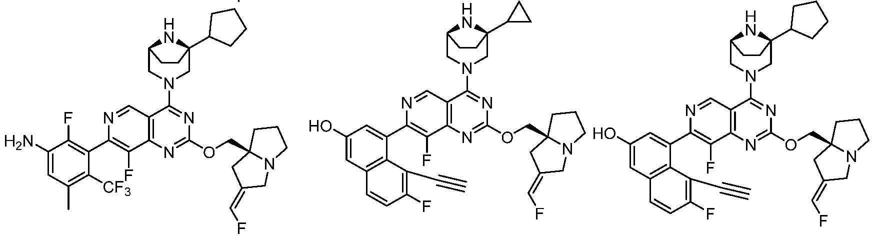

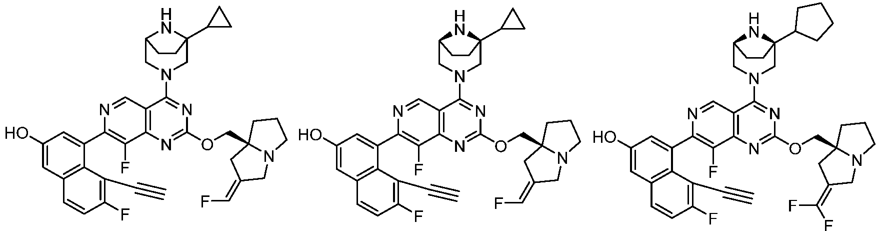

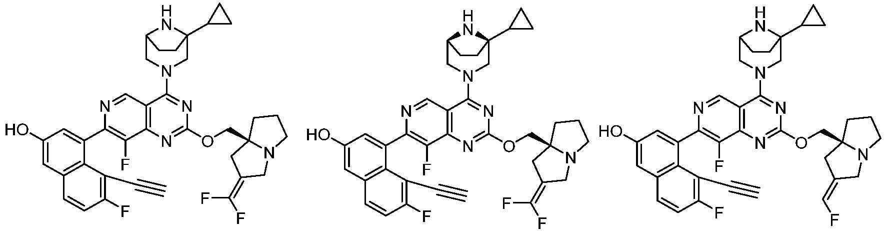

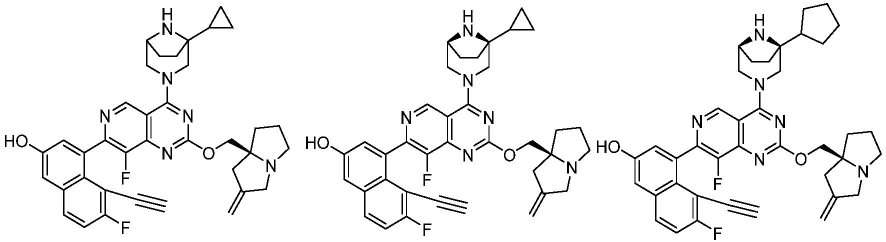

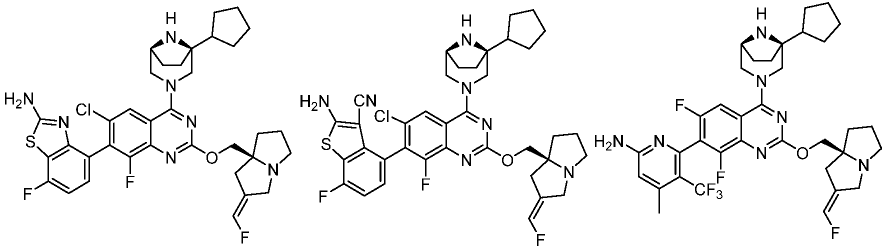

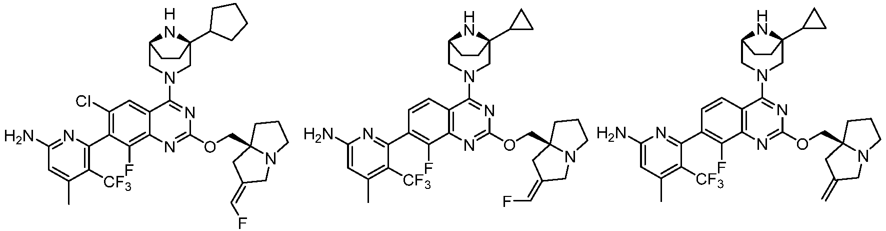

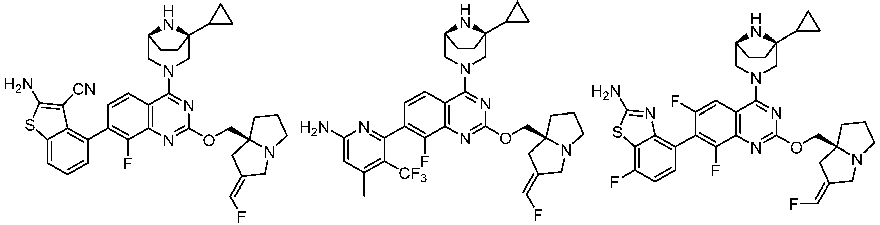

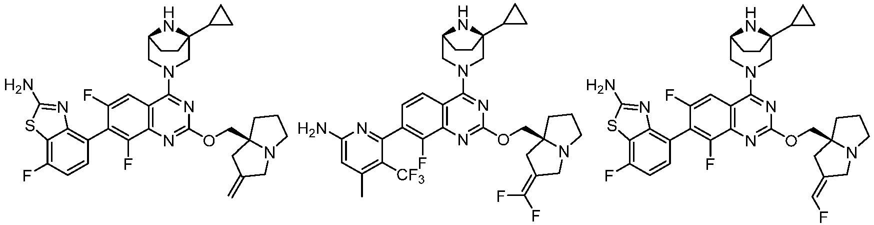

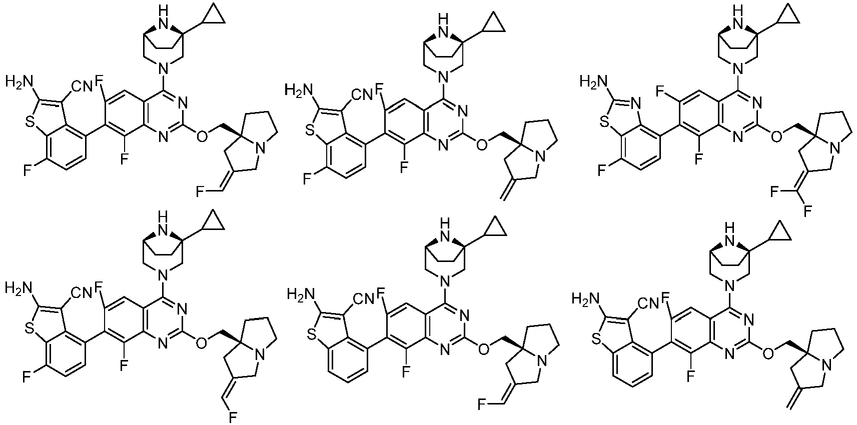

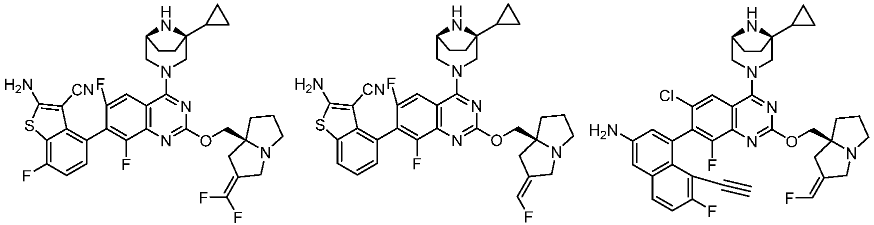

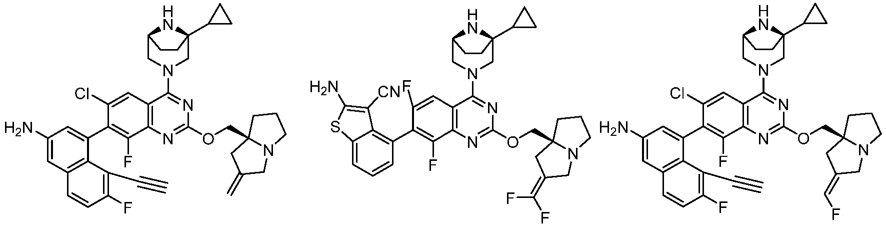

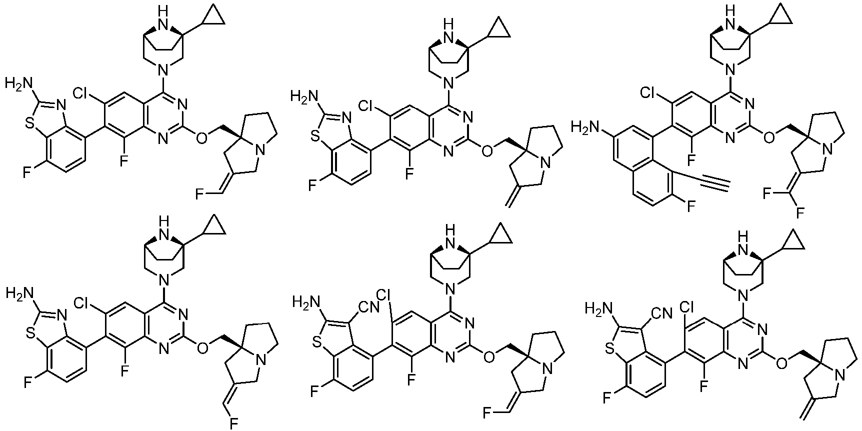

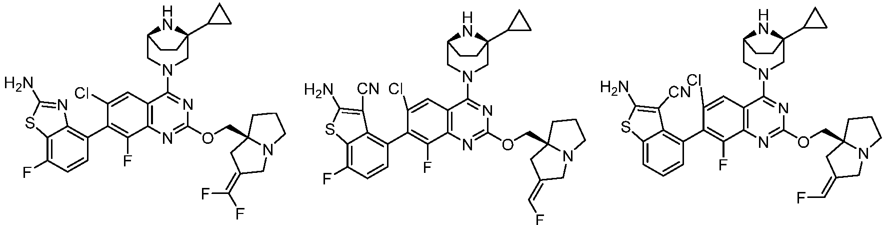

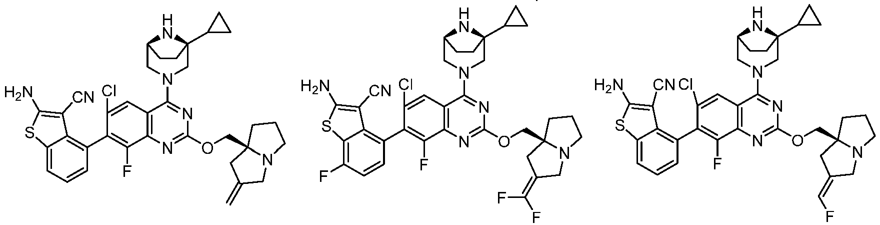

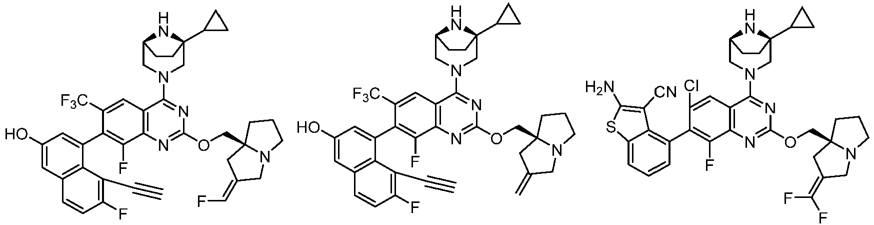

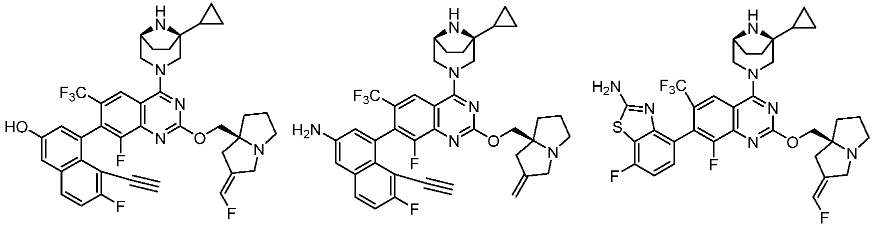

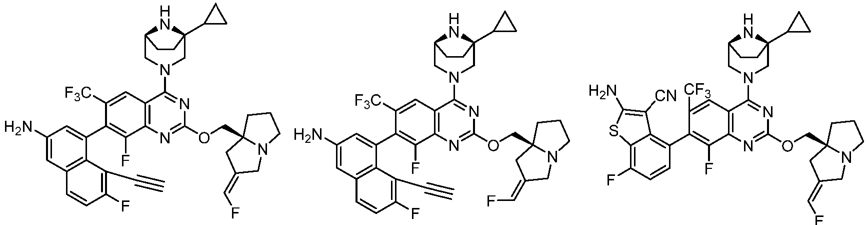

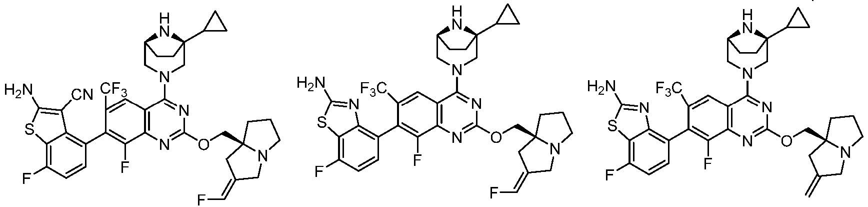

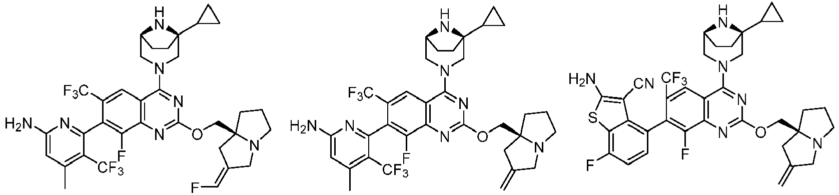

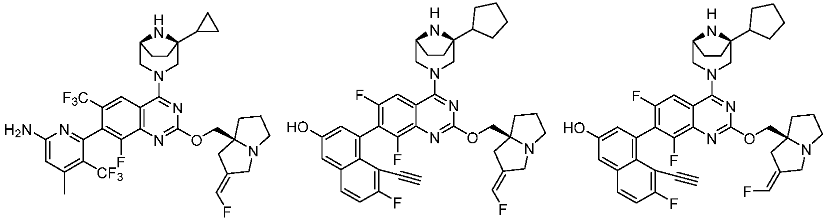

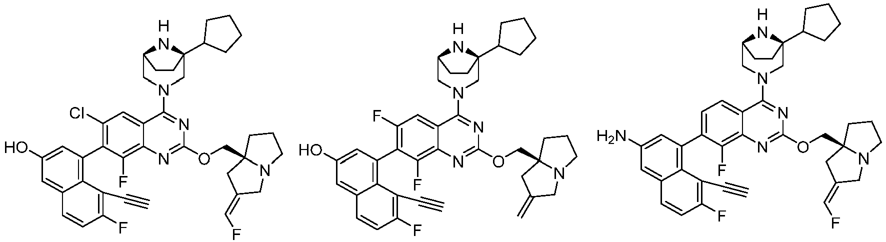

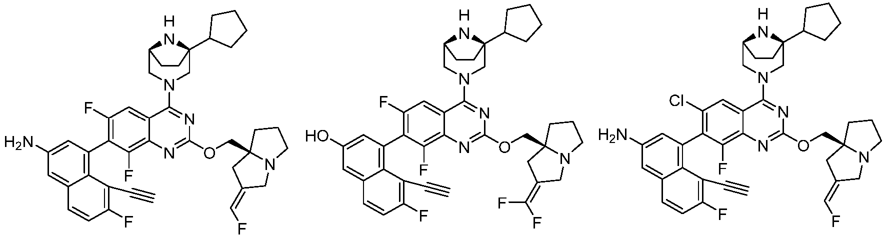

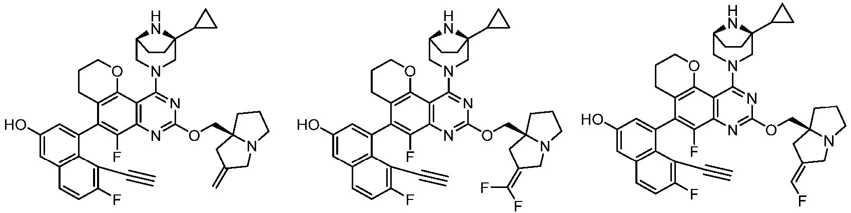

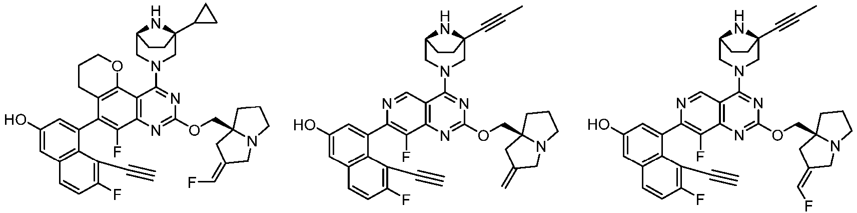

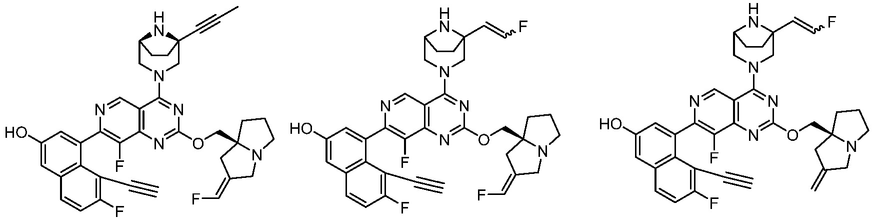

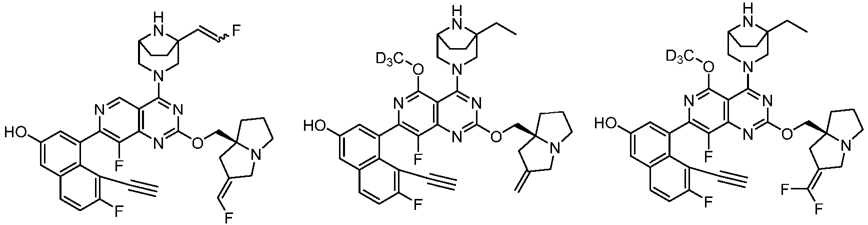

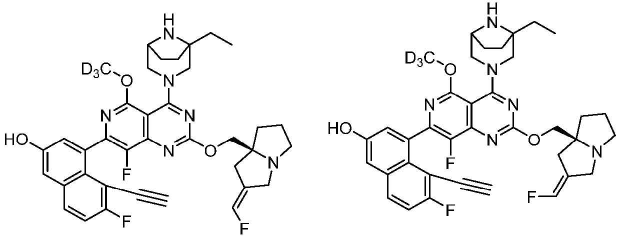

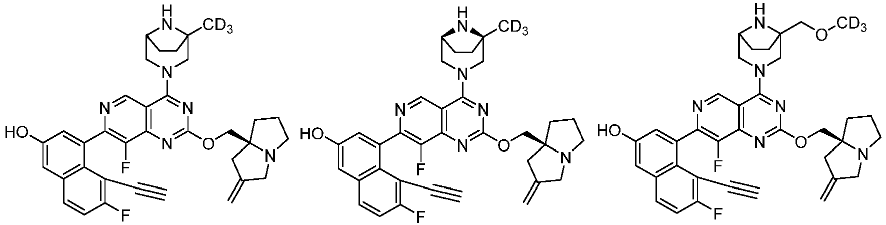

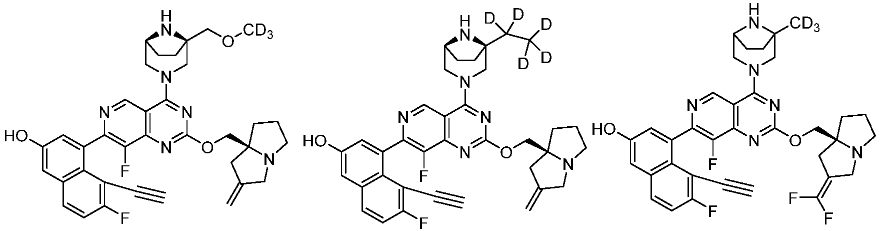

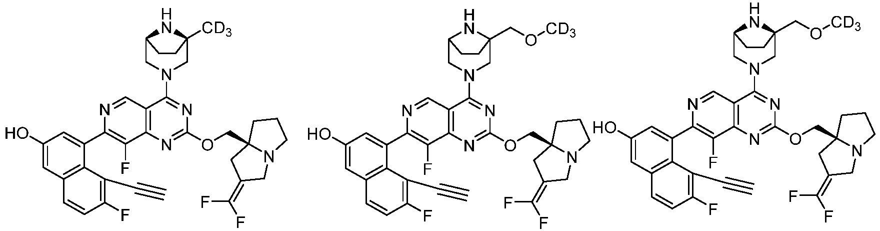

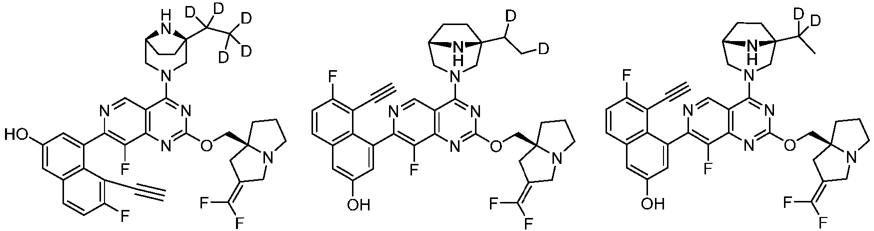

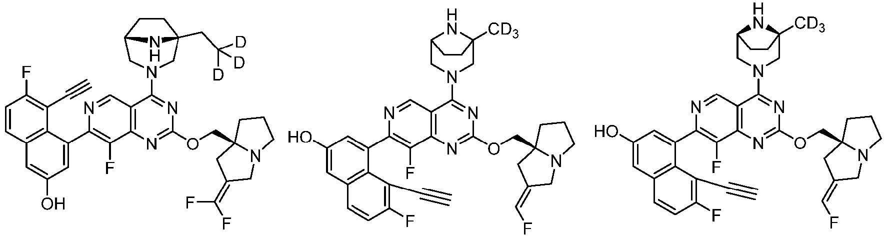

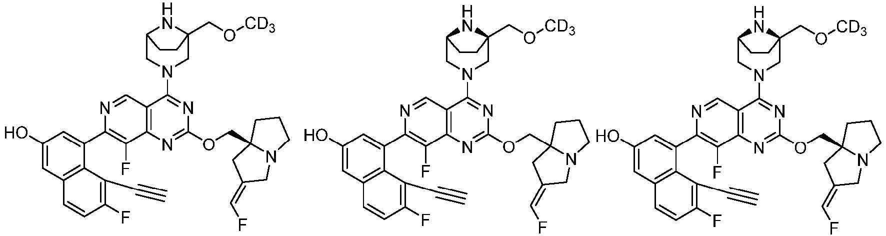

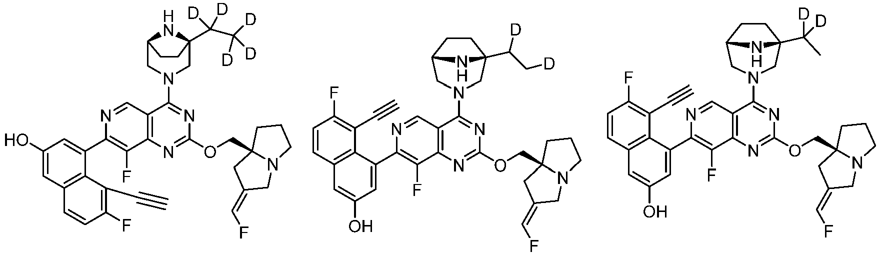

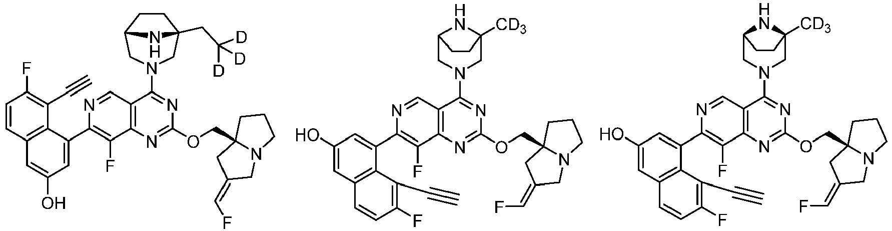

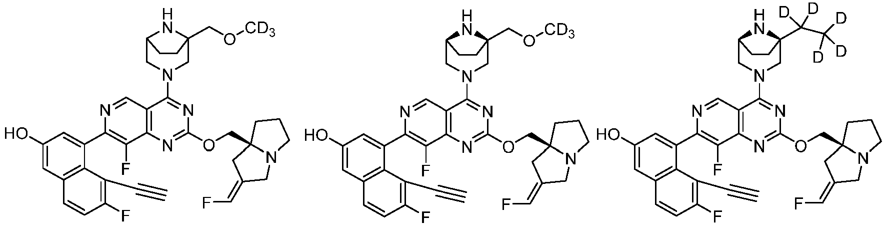

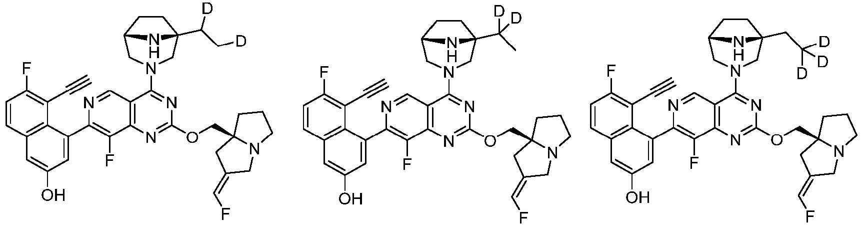

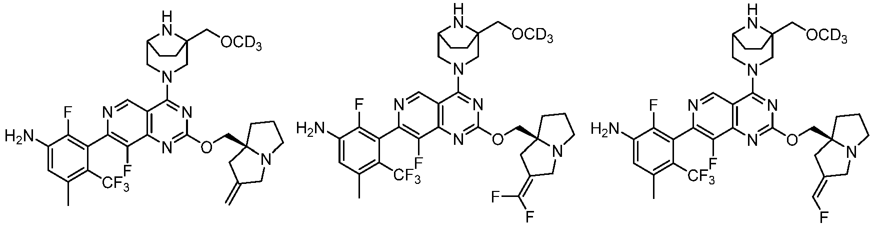

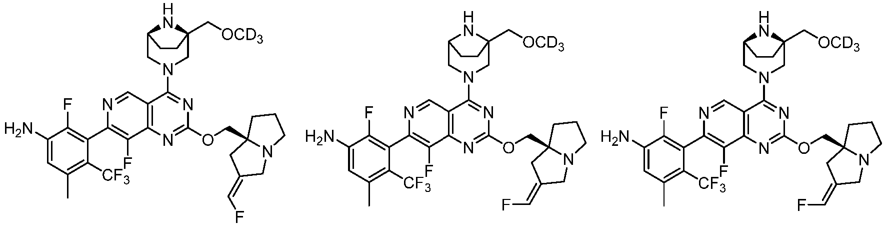

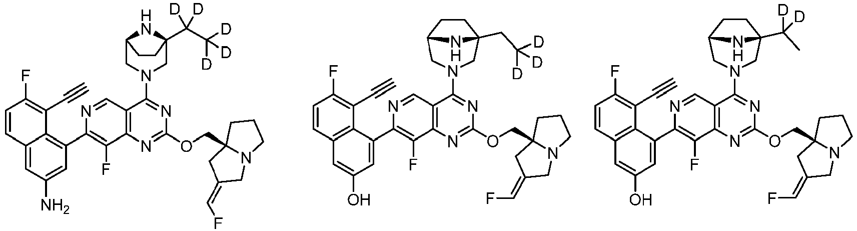

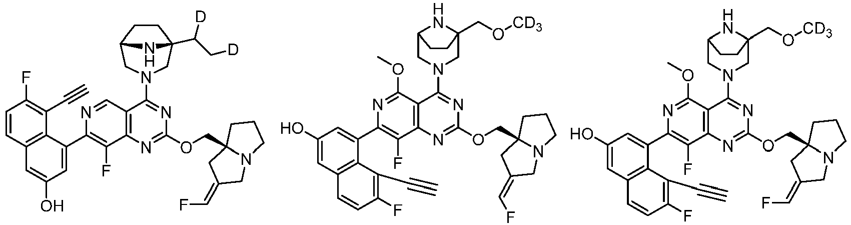

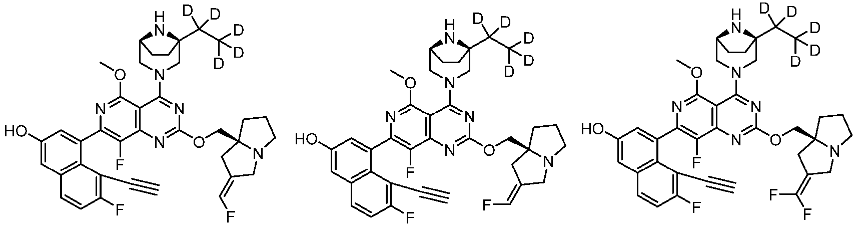

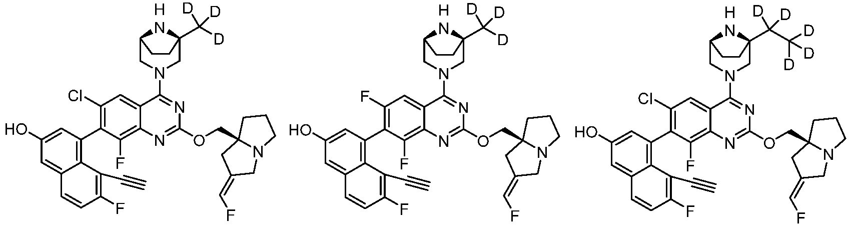

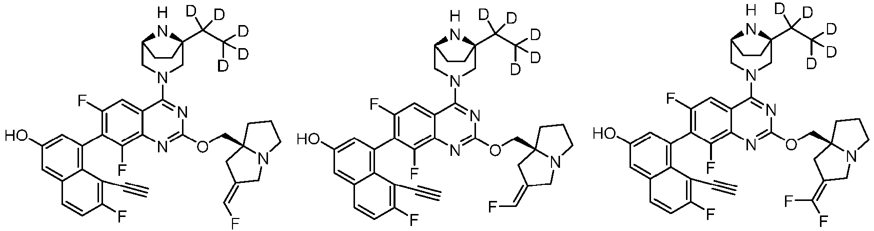

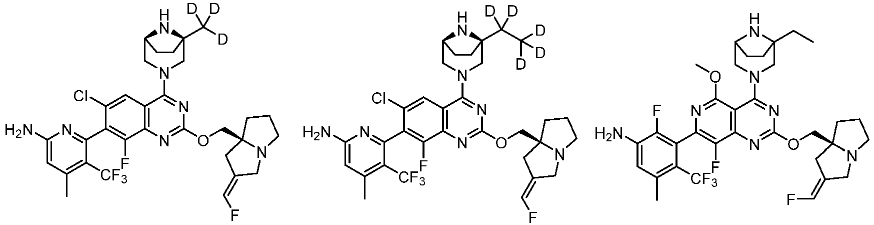

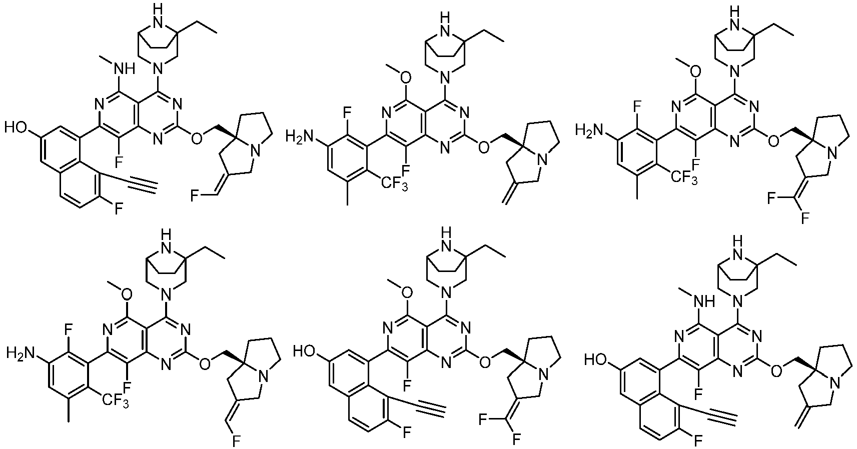

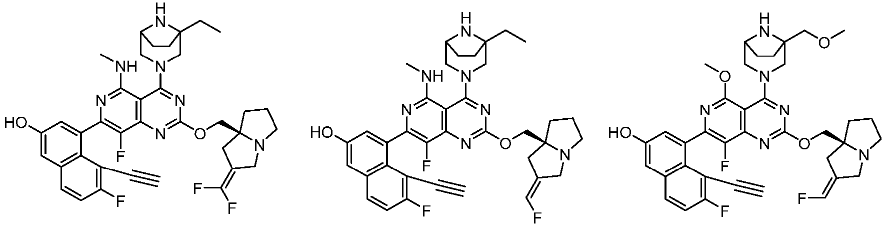

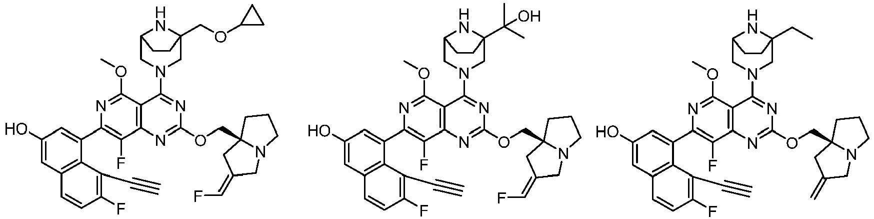

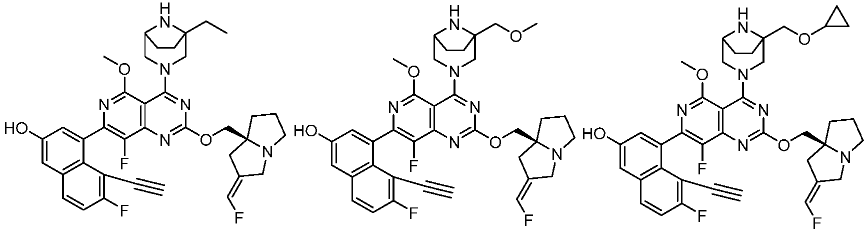

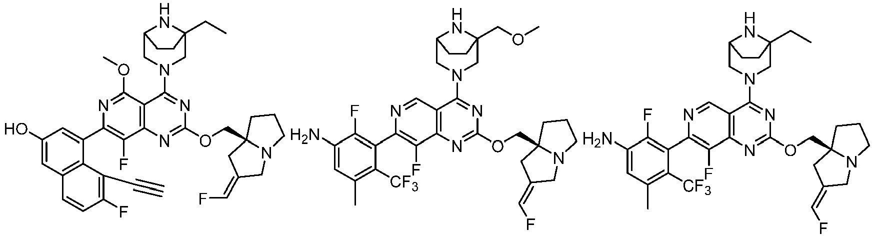

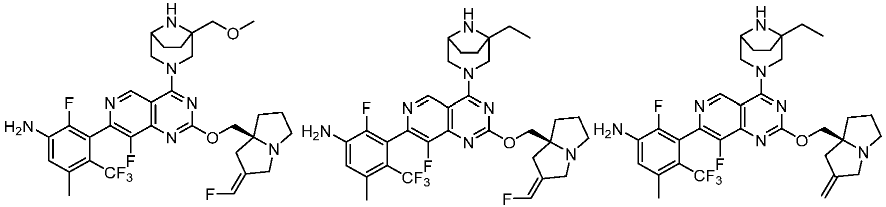

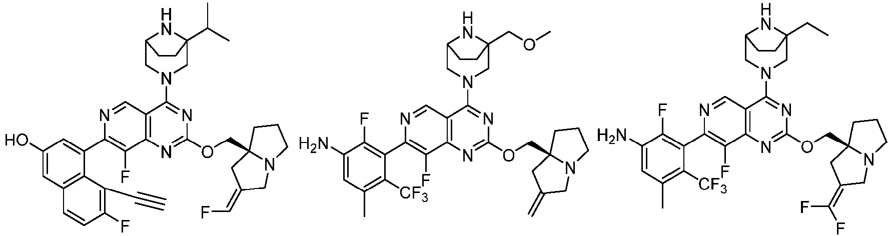

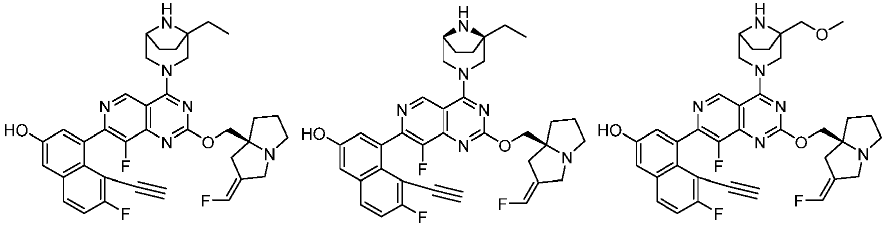

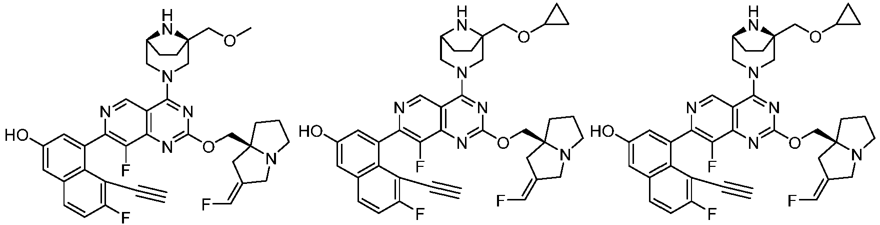

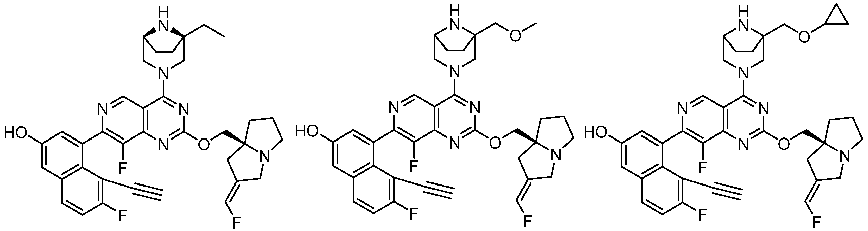

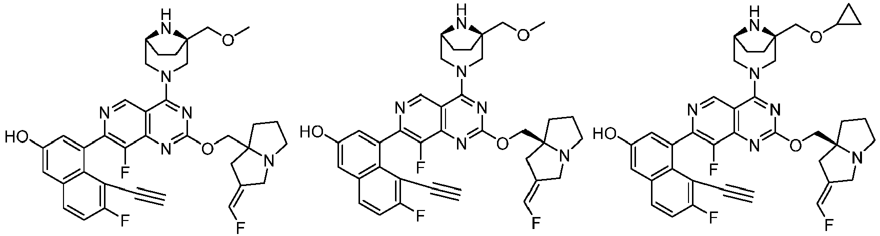

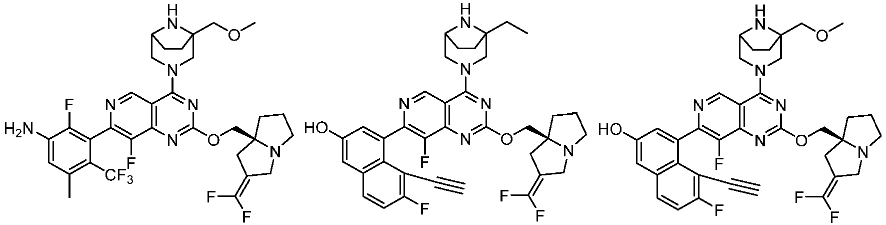

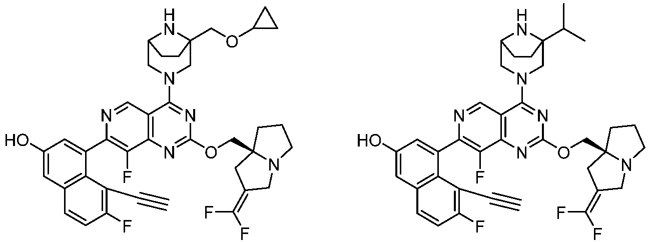

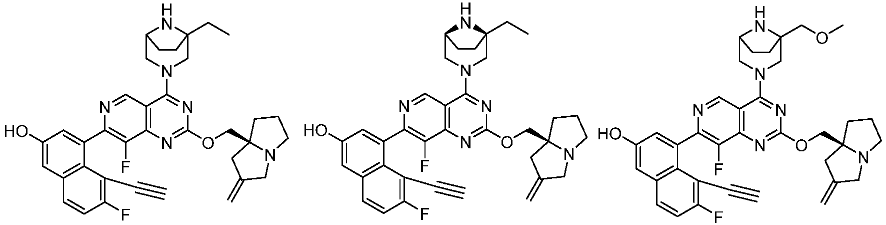

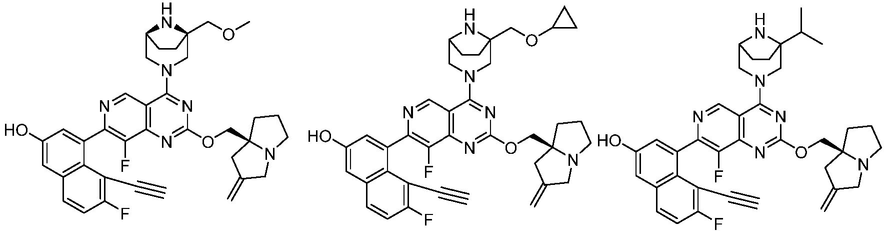

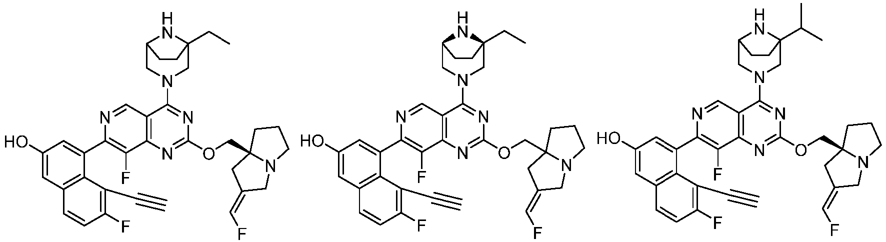

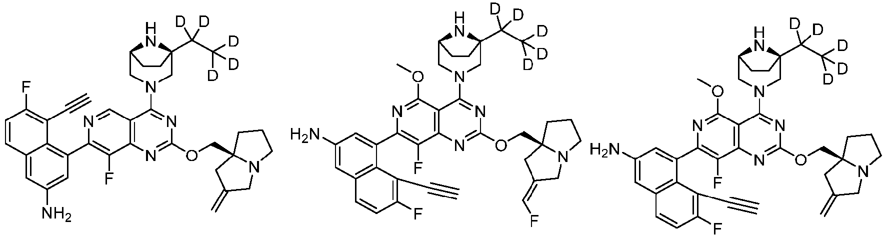

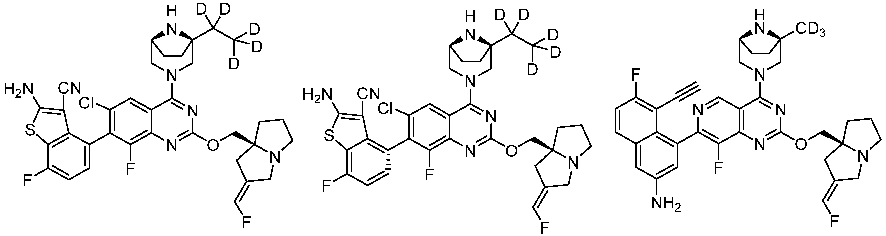

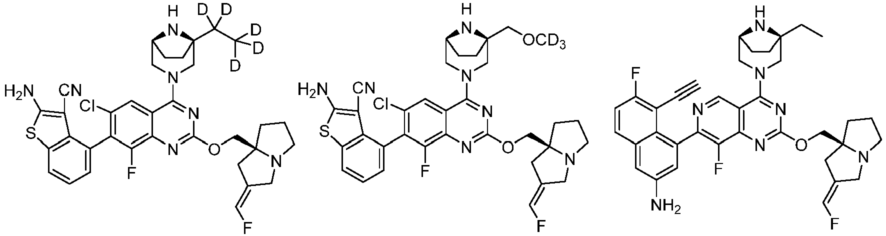

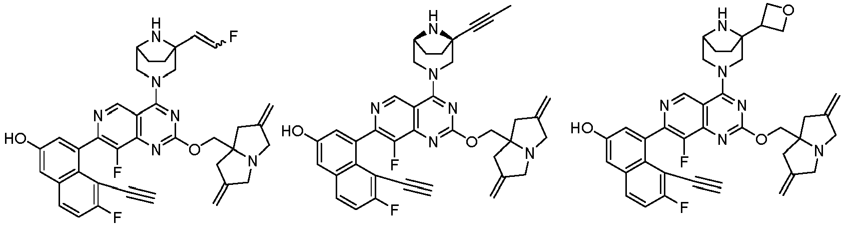

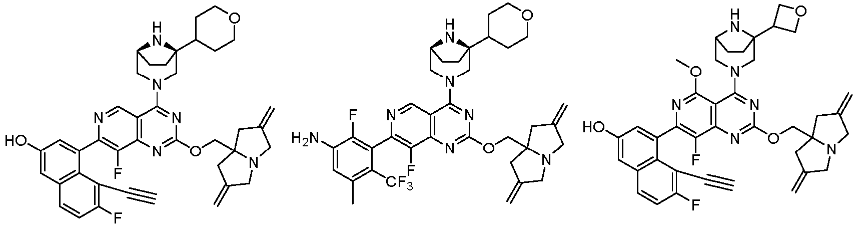

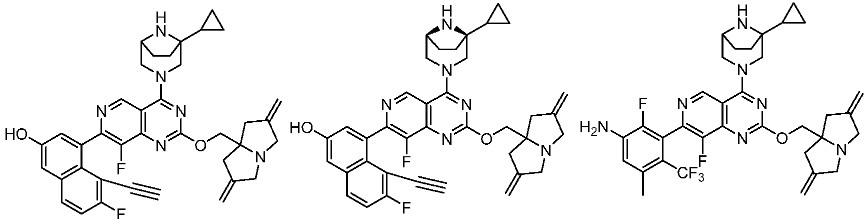

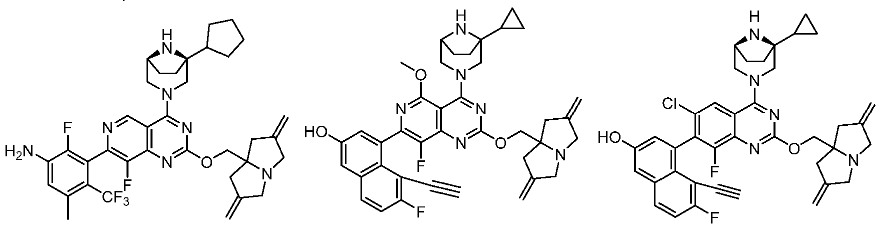

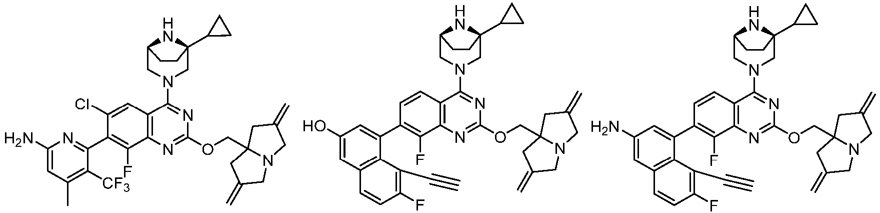

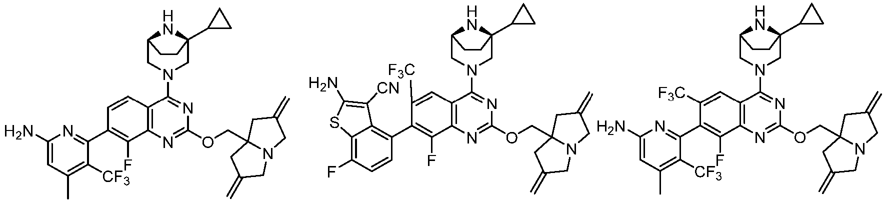

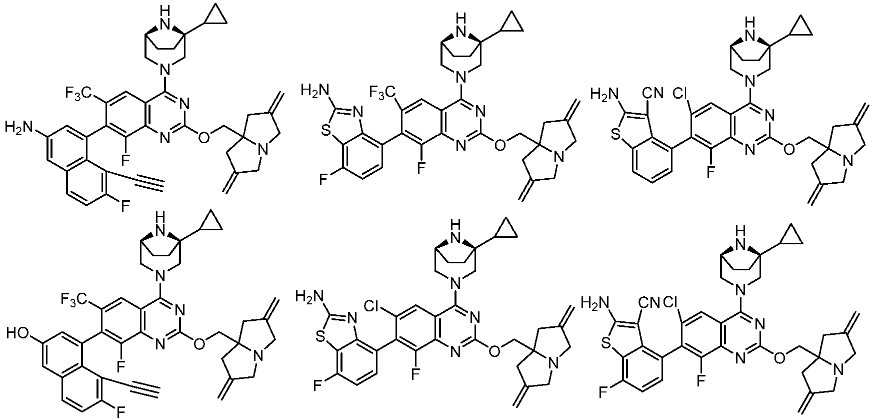

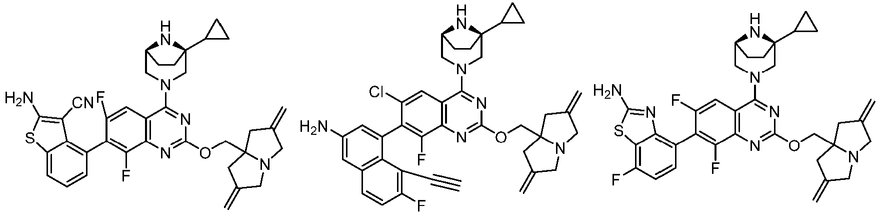

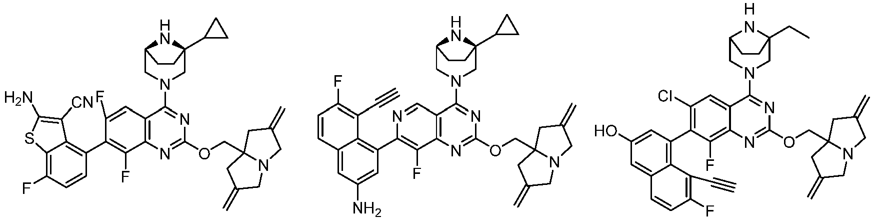

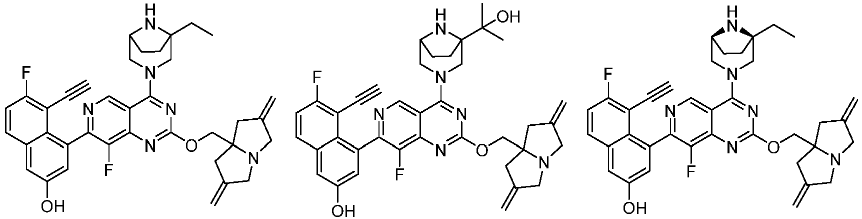

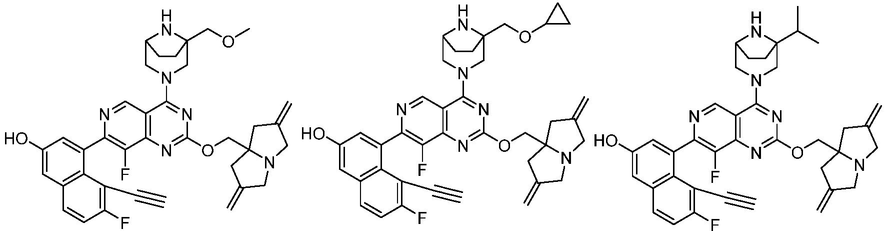

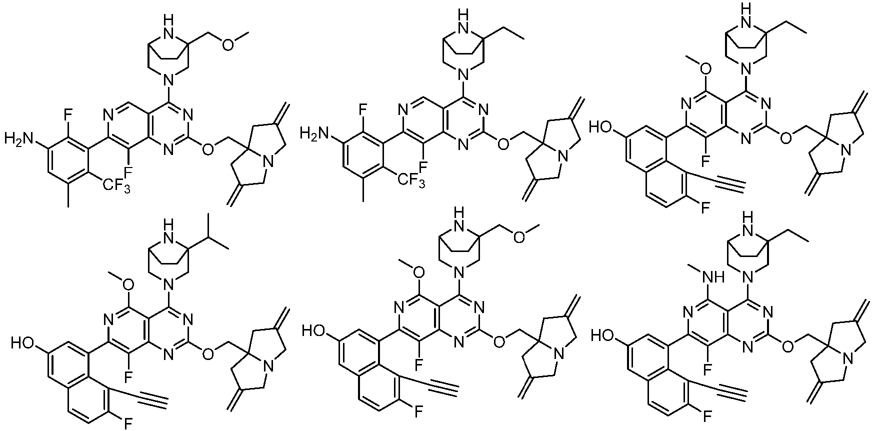

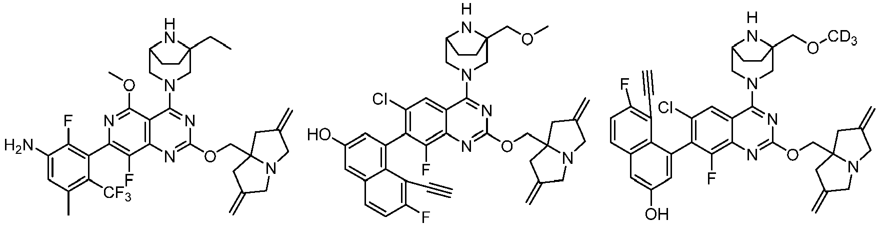

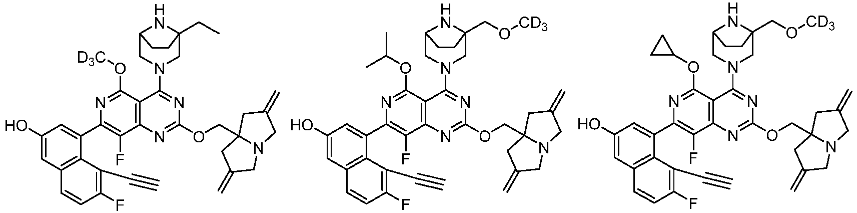

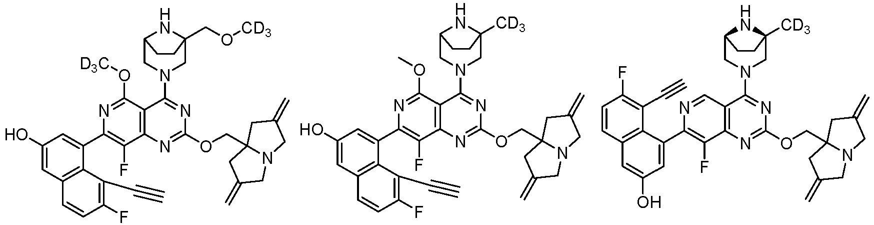

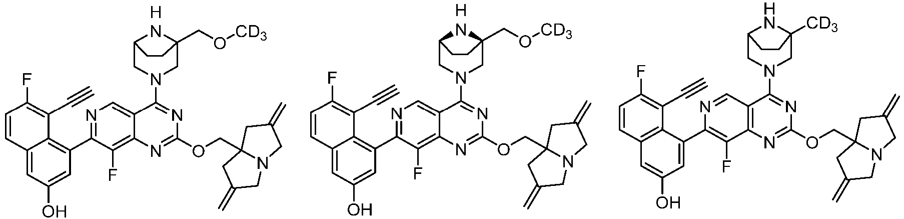

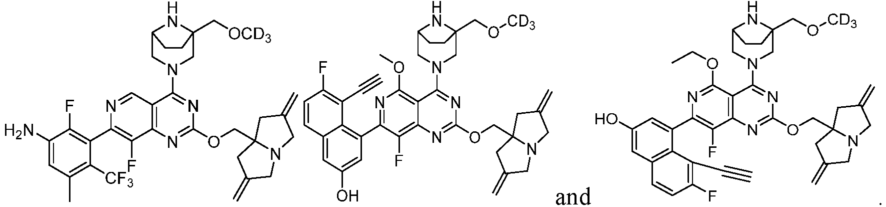

39. A pharmaceutical composition comprising the compound of formula (I), the stereoisomer or pharmaceutically acceptable salt thereof according to any one of claims 1 to 38, and a pharmaceutically acceptable carrier.

40. Use of the compound of formula (I), the stereoisomer or pharmaceutically acceptable salt thereof of any one of claims 1 to 38 in the preparation of a medicament for treating and/or preventing cancers or tumors related to KRAS G12D.

41. The use of the compound of formula (I), the stereoisomer or pharmaceutically acceptable salt thereof according to any one of claims 1 to 38 in the preparation of a medicament for treating and/or preventing KRAS G12D-related sarcoma (angiosarcoma, fibrosarcoma, rhabdomyosarcoma, liposarcoma), myxoma, rhabdomyoma, fibroma, lipoma, teratoma; bronchial carcinoma (squamous cell carcinoma, undifferentiated small cell carcinoma, undifferentiated large cell carcinoma, adenocarcinoma), alveolar (bronchiolar carcinoma) carcinoma, bronchial adenoma, lymphoma, chondromatoid hamartoma, mesothelioma; esophageal carcinoma (squamous cell carcinoma, adenocarcinoma, leiomyosarcoma, lymphoma), gastric carcinoma (lymphoma, leiomyosarcoma), pancreatic carcinoma (ductal adenocarcinoma, insulinoma, glucagonoma, gastrinoma, carcinoid tumor, vasoactive intestinal peptide tumor), small intestine cancer (adenocarcinoma, lymphoma, carcinoid tumor, Kaposi's sarcoma, leiomyoma, hemangioma, lipoma, neurofibroma, fibroma), colorectal carcinoma (adenocarcinoma, tubular adenoma, villous adenoma, hamartoma, leiomyoma); kidney cancer (adenocarcinoma, Wilms' tumor (nephroblastoma), lymphoma, leukemia), bladder cancer and urethral cancer (squamous cell carcinoma, transitional cell carcinoma, adenocarcinoma), prostate cancer (adenocarcinoma, sarcoma), testicular cancer (seminoma, teratoma, embryonal carcinoma, terato-epithelial cancer, choriocarcinoma, sarcoma, mesenchymal cell carcinoma, fibroma, fibroadenoma, adenomatoid tumor, lipoma); liver cancer (hepatocellular carcinoma), cholangiocarcinoma, hepatoblastoma, angiosarcoma, hepatocellular adenoma, hemangioma; gallbladder cancer, ampullary carcinoma, cholangiocarcinoma; osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochondroma (osteochondral exostosis), benign chondroma, chondroblastoma, chondromyxoid fibroma, osteoid osteoma and giant cell tumor; skull cancer (osteoma, hemangioma, granuloma, xanthoma, osteitis deformans), meningeal carcinomatosis (meningioma, meningeal sarcoma, gliomatosis), brain cancer (astrocytoma, medulloblastoma, glioma, ependymoma, blastoma (pineal tumor), glioblastoma multiforme, oligodendroglioma, neurilemmoma, retinoblastoma, congenital tumor, spinal neurofibroma, meningioma, glioma, sarcoma); uterine cancer (endometrial cancer (serous cystadenocarcinoma, mucinous cystadenocarcinoma, unclassified cancer), granulosa-theca cell tumor, Sertoli-Leydig cell tumor, dysgerminoma, malignant teratoma), vulvar cancer (squamous cell carcinoma, intraepithelial carcinoma, adenocarcinoma, fibrosarcoma, melanoma), vaginal cancer (clear cell carcinoma, squamous cell carcinoma, botryoid sarcoma (embryonal rhabdomyosarcoma)), fallopian tube (carcinoma); hematologic cancer (myelogenous leukemia (acute and chronic), acute lymphoblastic leukemia, chronic lymphocytic leukemia, myeloproliferative disease, multiple myeloma, myelodysplastic syndrome), Hodgkin's disease, non-Hodgkin's lymphoma (malignant lymphoma); malignant melanoma, basal cell carcinoma, squamous cell carcinoma, Kaposi's sarcoma, dysplastic nevus, lipoma, hemangioma, dermatofibroma, keloid, psoriasis, adrenal tumor or neuroblastoma.

42. The compound of formula (I), the stereoisomer or pharmaceutically acceptable salt thereof according to any one of claims 1 to 38, for use in treating and/or preventing cancers or tumors related to KRAS G12D.
